# EUROPEAN PATENT APPLICATION

(11) **EP 4 692 338 A1**
(43) Date of publication of application: **11.02.2026**
(21) Application number: 24867518.3
(22) Date of filing: 19.09.2024
(51) Int. Cl.: C12N 9/22, C12N 15/55

(54) **CAS12 PROTEIN AND USE THEREOF**

(30) Priority: 19.09.2023 CN 202311214330; 01.04.2024 CN 202410388592
(71) Applicant: Guangzhou Reforgene Medicine Co., Ltd., Guangzhou, Guangdong 510535 (CN); Zheijiang Synsorbio Technology Co., Ltd., Shaoxing City, Zhejiang 312300 (CN)
(72) Inventor: LIANG, Junbin, Guangzhou, Guangdong 510535 (CN); HUANG, Liancheng, Guangzhou, Guangdong 510535 (CN); CHEN, Chongjian, Shaoxing, Zhejiang 312300 (CN); SUN, Yang, Shaoxing, Zhejiang 312300 (CN); PAN, Weiye, Shaoxing, Zhejiang 312300 (CN); XU, Hui, Guangzhou, Guangdong 510535 (CN); SI, Kaiwei, Guangzhou, Guangdong 510535 (CN); CAI, Jinxiu, Guangzhou, Guangdong 510535 (CN); LIAO, Qing, Guangzhou, Guangdong 510535 (CN); HUANGFU, Desheng, Guangzhou, Guangdong 510535 (CN)
(74) Representative: Dai, Simin
(86) International application number: PCT/CN2024/119862
(87) International publication number: WO 2025/061113

(57) **Abstract**

Cas12 protein, guide polynucleotide, inactivated Cas12 mutant, fusion protein or conjugate including the Cas12 protein, isolated nucleic acid, CRISPR-Cas12 system, vector system, delivery system, cell, pharmaceutical composition, and kit, and the use thereof are provided.

## Description

This application claims priority to Chinese Patent Application No. 202311214330.6, filed on September 19, 2023, and Chinese Patent Application No. 202410388592.2, filled on April 1, 2024, the entire contents of each of which are incorporated herein by reference.

### TECHNICAL FIELD

The present disclosure relates to the field of CRISPR gene editing, and, more particularly, to Cas12 proteins and uses thereof.

### BACKGROUND

A CRISPR-Cas system is an adaptive immune defense developed by bacteria and archaea over a long period of time, which is used to fight against invading viruses and exogenous DNA. The clustered regularly interspaced short palindromic repeat (CRISPR) and the CRISPR-associated protein system (CRISPR-Cas system) can be used to make changes to gene sequences directly in cells, which is a fast and effective manner.

Many researchers in this field are working on finding new Cas12 proteins and CRISPR-Cas12 gene editing systems.

### SUMMARY

The present disclosure provides Cas12 proteins and uses thereof.

Embodiments of the present disclosure provide a Cas12 protein. In some embodiments, the Cas12 protein is selected from the group consisting of a CLUSTER1 protein, a CLUSTER2 protein, a CLUSTER3 protein, a CLUSTER4 protein, a CLUSTERS protein, a CLUSTER6 protein, a CLUSTER7 protein, a CLUSTER8 protein, a CLUSTER9 protein, a CLUSTER10 protein, a CLUSTER11 protein, a CLUSTER12 protein, and a CLUSTER13 protein.

In some embodiments, the Cas12 protein comprises an amino acid sequence having at least 50% sequence identity to any one of the amino acid sequences shown in SEQ ID NO: 1-53, SEQ ID NO: 696, or SEQ ID NO: 728.

In some embodiments, the Cas12 protein comprises an amino acid sequence having at least 50%, at least 55%, at least 60%, at least 65%, at least 70%,at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to any one of the amino acid sequences shown in SEQ ID NO: 1-53, SEQ ID NO: 696, or SEQ ID NO: 728.

In some embodiments, the Cas12 protein comprises an amino acid sequence having at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% sequence identity to any one of the amino acid sequences shown in SEQ ID NO: 1-53, SEQ ID NO: 696, or SEQ ID NO: 728.

In some embodiments, the Cas12 protein comprises an amino acid sequence having at least 80% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 85% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 90% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 95% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 97% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 98% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 99% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 99.5% sequence identity to any one of SEQ ID NO: 1-53, SEQ ID NO: 696, or SEQ ID NO: 728. In some embodiments, the Cas12 protein comprises an amino acid sequence having at least 99.7% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 99.8% sequence identity to any one of the amino acid sequences shown in SEQ ID NO: 1-53, SEQ ID NO: 696, or SEQ ID NO: 728. In some embodiments, the Cas12 protein comprises an amino acid sequence having at least 100% sequence identity to any one of the amino acid sequences shown in SEQ ID NO: 1-53, SEQ ID NO: 696, or SEQ ID NO: 728.

In some embodiments, the Cas12 protein retains a function of a protein having an amino acid sequence as shown in any one of SEQ ID NO: 1-53, SEQ ID NO: 696, or SEQ ID NO: 728.

In some embodiments, the Cas12 protein forms a complex with a guide polynucleotide. In some embodiments, the Cas12 protein and the guide polynucleotide specifically bind to a target nucleic acid.

In some embodiments, the Cas12 protein forms a complex with a guide polynucleotide, and the complex specifically binds to a target nucleic acid. In some embodiments, the Cas12 protein forms a complex with a guide polynucleotide, and the complex specifically binds to a target DNA.

In some embodiments, the Cas12 protein and a guide polynucleotide specifically binds to and cleaves a target nucleic acid. In some embodiments, the Cas12 protein and a guide polynucleotide specifically binds to and cleaves a target DNA. In some embodiments, the Cas12 protein forms a complex with a guide polynucleotide, and the complex specifically binds and cleaves a target nucleic acid. In some embodiments, the Cas12 protein forms a complex with the guide polynucleotide, and the complex specifically binds and cleaves the target DNA.

As used herein, the phrase "retaining a function of a protein having an amino acid sequence as shown in any one of SEQ ID NO: 1-53, SEQ ID NO: 696, or SEQ ID NO: 728" refers to retaining the ability to form a complex with a guide polynucleotide, retaining the ability to bind a target nucleic acid complementary to the guide sequence, retaining the ability to specifically cleave the target nucleic acid with the guide polynucleotide, and/or retaining the ability to process an RNA transcript containing the guide sequence into guide polynucleotide molecules.

In some embodiments, the retaining a function of a protein having an amino acid sequence as shown in any one of SEQ ID NO: 1-53, SEQ ID NO: 696, or SEQ ID NO: 728 refers to retaining the ability to form a complex with a guide polynucleotide.

In some embodiments, the retaining a function of a protein having an amino acid sequence as shown in any one of SEQ ID NO: 1-53, SEQ ID NO: 696, or SEQ ID NO: 728 refers to retaining the ability to bind a target nucleic acid complementary to the guide sequence of the guide polynucleotide.

In some embodiments, the retaining a function of a protein having an amino acid sequence as shown in any one of SEQ ID NO: 1-53, SEQ ID NO: 696, or SEQ ID NO: 728 refers to retaining the ability to specifically cleave a target nucleic acid with a guide polynucleotide.

In some embodiments, the retaining a function of a protein having an amino acid sequence as shown in any one of SEQ ID NO: 1-53, SEQ ID NO: 696, or SEQ ID NO: 728 refers to retaining the ability to process the RNA transcript containing the guide sequence into guide polynucleotide molecules.

In some embodiments, the Cas12 protein comprises an amino acid sequence as shown in any one of SEQ ID NO: 1-53, SEQ ID NO: 696, or SEQ ID NO: 728.

In some embodiments, a protospacer adjacent motif (PAM) sequence (5'→3') recognized by the Cas12 protein is selected from any one or more of the following:
A, C, T, G,
TA, TC, GN, AA, AG, TG, AN, GG, CG, TN, NT, NG, GT, NA, CC, AC, GC, AT, CT, GA, TT, CN, NC, CA,
NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG,
NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, NNGC.

N is A, T, C, or G.

In some embodiments, a PAM sequence recognized by the Cas12 protein is 5'-T-3'.

In some embodiments, a PAM sequence recognized by the Cas12 protein is 5'-G-3'.

In some embodiments, a PAM sequence recognized by the Cas12 protein is 5'-A-3'.

In some embodiments, a PAM sequence recognized by the Cas12 protein is 5'-C-3'.

In some embodiments, a PAM sequence recognized by the Cas12 protein is 5'-TA-3'.

In some embodiments, a PAM sequence recognized by the Cas12 protein is 5'-TC-3'.

In some embodiments, a PAM sequence recognized by the Cas12 protein is 5'-TG-3'.

In some embodiments, a PAM sequence recognized by the Cas12 protein is 5'-TT-3'.

In some embodiments, a PAM sequence recognized by the Cas12 protein is 5'-TN-3'.

In some embodiments, a PAM sequence recognized by the Cas12 protein is 5'-TTN-3'.

In some embodiments, a PAM sequence recognized by the Cas12 protein is 5'-TTT-3'.

In some embodiments, a PAM sequence recognized by the Cas12 protein is 5'-TTG-3'.

In some embodiments, a PAM sequence recognized by the Cas12 protein is 5'-TTC-3'.

In some embodiments, a PAM sequence recognized by the Cas12 protein is 5'-TTA-3'.

In some embodiments, a PAM sequence recognized by the Cas12 protein is 5'-WTN-3'.

In some embodiments, a PAM sequence recognized by the Cas12 protein is 5'-ATN-3'.

N is A, T, C, or G, and W is A or T.

In some embodiments of the present disclosure, the Cas12 protein is an inactivated Cas12 mutant. In some embodiments of the present disclosure, the Cas12 protein is a nuclease-inactivated mutant. In some embodiments of the present disclosure, the Cas12 protein is a dead Cas12 mutant or a nickase Cas12 mutant. In some embodiments, the Cas12 protein has an inactivated Ruvc domain.

In some embodiments of the present disclosure, the Cas12 protein is selected from an active fragment constituting the Cas12 protein described in the present disclosure.

In some embodiments of the present disclosure, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to any one of the amino acid sequences shown in SEQ ID NO: 46, SEQ ID NO: 696, SEQ ID NO: 52, or SEQ ID NO: 728.

In some embodiments, the Cas12 protein forms a complex with a guide polynucleotide. Further, the complex specifically binds to a target nucleic acid. Further, the complex cleaves the target nucleic acid, modifies the target nucleic acid, and/or modulates the expression of the target nucleic acid.

In some embodiments, the Cas12 protein forms a complex with a guide polynucleotide, and the guide polynucleotide comprises a guide sequence that is reverse complementary to a target nucleic acid. Further, the guide polynucleotide comprises a scaffold sequence that interacts with the Cas12 protein. Further, the scaffold sequence comprises a direct repeat (DR) sequence. Further, the DR sequence comprises a nucleotide sequence having at least 50%, at least 55%, at least 60%, at least 65%, at least 70%,at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NO: 704, SEQ ID NO: 529, or SEQ ID NO: 534.

In some embodiments, the scaffold sequence does not comprise a tracrRNA sequence.

In some embodiments, a PAM sequence recognized by the Cas12 protein is 5-TTN-3' and/or 5'-TTNC-3'. N may be A, T, C, or G.

Some embodiments of the present disclosure provide a Cas12 protein. The Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 46.

In some embodiments, the Cas12 protein forms a complex with a guide polynucleotide. Further, the complex specifically binds to a target nucleic acid. Further, the complex cleaves the target nucleic acid, modifies the target nucleic acid, and/or modulates an expression of the target nucleic acid.

In some embodiments, the Cas12 protein forms a complex with a guide polynucleotide, and the guide polynucleotide comprises a guide sequence that is reverse complementary to the target nucleic acid. Further, the guide polynucleotide comprises a scaffold sequence that interacts with the Cas12 protein. Further, the scaffold sequence comprises a DR sequence. Further, the scaffold sequence comprises a sequence having at least 80%, at least 85%, at least 90%, at least 95%, at least 96%, or at least 97% sequence identity to the sequence shown in SEQ ID NO: 704.

Some embodiments of the present disclosure provide a Cas12 protein. In some embodiments, the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 696.

In some embodiments, the Cas12 protein forms a complex with a guide polynucleotide. Further, the complex specifically binds to a target nucleic acid. Further, the complex cleaves the target nucleic acid, modifies the target nucleic acid, and/or modulates the expression of the target nucleic acid.

In some embodiments, the Cas12 protein forms a complex with a guide polynucleotide, and the guide polynucleotide comprises a guide sequence that is reverse complementary to a target nucleic acid. Further, the guide polynucleotide comprises a scaffold sequence that interacts with the Cas12 protein. Further, the scaffold sequence comprises a DR sequence. Further, the scaffold sequence comprises a sequence having at least 80%, at least 85%, at least 90%, at least 95%, at least 96%, or at least 97% sequence identity to the sequence shown in SEQ ID NO: 704.

Some embodiments of the present disclosure provide a Cas12 protein. The Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 52.

In some embodiments, the Cas12 protein forms a complex with a guide polynucleotide. Further, the complex specifically binds to a target nucleic acid. Further, the complex cleaves the target nucleic acid, modifies the target nucleic acid, and/or modulates the expression of the target nucleic acid.

In some embodiments, the Cas12 protein forms a complex with a guide polynucleotide, and the guide polynucleotide comprises a guide sequence that is reverse complementary to a target nucleic acid. Further, the guide polynucleotide comprises a scaffold sequence that interacts with the Cas12 protein. Further, the scaffold sequence comprises a DR sequence. Further, the scaffold sequence comprises a sequence having at least 80%, at least 85%, at least 90%, at least 95%, at least 96%, or at least 97% sequence identity to the sequence shown in SEQ ID NO: 534.

Some embodiments of the present disclosure provide a Cas12 protein. The Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 728.

In some embodiments, the Cas12 protein forms a complex with a guide polynucleotide. Further, the complex specifically binds to a target nucleic acid. Further, the complex cleaves the target nucleic acid, modifies the target nucleic acid, and/or modulates the expression of the target nucleic acid.

In some embodiments, the Cas12 protein forms a complex with a guide polynucleotide, and the guide polynucleotide comprises a guide sequence that is reverse complementary to a target nucleic acid. Further, the guide polynucleotide comprises a scaffold sequence that interacts with the Cas12 protein. Further, the scaffold sequence comprises a DR sequence. Further, the scaffold sequence comprises a sequence having at least 80%, at least 85%, at least 90%, at least 95%, at least 96%, or at least 97% sequence identity to the sequence shown in SEQ ID NO: 534.

In some embodiments of the present disclosure, the Cas12 protein comprises an amino acid sequence having at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 46.

The Cas12 protein forms a complex with a guide polynucleotide; and the guide polynucleotide comprises a guide sequence that is reverse complementary to a target nucleic acid and a DR sequence.

In some embodiments, the DR sequence comprises a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 704.

In some embodiments, the complex binds to the target nucleic acid under the guidance of the guide sequence.

In some embodiments, a PAM sequence recognized by the Cas12 protein is 5'-TTN-3'.

N may be A, T, C or G.

In some embodiments of the present disclosure, the Cas12 protein comprises an amino acid sequence having at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 696.

The Cas12 protein may form a complex with a guide polynucleotide; and the guide polynucleotide comprises a guide sequence that is reverse complementary to a target nucleic acid and a DR sequence.

In some embodiments, the DR sequence comprises a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in the SEQ ID NO: 704.

In some embodiments, the complex binds to the target nucleic acid under the guidance of the guide sequence.

In some embodiments, a PAM sequence recognized by the Cas12 protein is 5'-TTN-3' In some embodiments, a PAM sequence recognized by the Cas12 protein is 5'-ATN-3'. In some embodiments, a PAM sequence recognized by the Cas12 protein is 5'-WTN-3'.

In some embodiments of the present disclosure, the Cas12 protein comprises an amino acid sequence having at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 52.

The Cas12 protein may form a complex with a guide polynucleotide; and the guide polynucleotide comprises a guide sequence that is reverse complementary to a target nucleic acid and a DR sequence.

In some embodiments, the DR sequence comprises a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 534.

In some embodiments, the complex binds to the target nucleic acid under the guidance of the guide sequence.

In some embodiments, a PAM sequence recognized by the Cas12 protein is 5'-TTN-3'.

In some embodiments of the present disclosure, the Cas12 protein comprises an amino acid sequence having at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the amino acid sequence shown in SEQ ID NO: 728.

The Cas12 protein may form a complex with a guide polynucleotide; and the guide polynucleotide comprises a guide sequence that is reverse complementary to a target nucleic acid and a DR sequence.

In some embodiments, the DR sequence comprises a nucleotide sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in SEQ ID NO: 534.

In some embodiments, the complex binds to the target nucleic acid under the guidance of the guide sequence.

In some embodiments, a PAM sequence recognized by the Cas12 protein is 5'-TTN-3'.

N may be A, T, C, or G.

In some embodiments, the reverse complementation is partially complementary or fully complementary. In some embodiments, the guide sequence hybridizes to the target nucleic acid.

In some embodiments, the Cas12 protein is a mutant of a Cas protein having an amino acid sequence shown in any one of SEQ ID NO: 1-53, 696, or 728.

In some embodiments, the Cas12 protein is an inactivated mutant of a Cas protein having an amino acid sequence shown in any one of SEQ ID NO: 1-53, 696, or 728.

In some embodiments, the Cas12 protein provided herein comprises one, two, or more mutations as compared to the Cas protein with the sequence shown in any one of SEQ ID NO: 1-53, SEQ ID NO: 696, and SEQ ID NO: 728, such as a single amino acid insertion, a single amino acid deletion, a single amino acid substitution, or combinations thereof. In some examples, compared to the Cas protein with the sequence shown in any one of SEQ ID NO: 1-53, SEQ ID NO: 696, and SEQ ID NO: 728, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions) while retaining the ability to bind the target nucleic acid molecule complementary to the guide sequence of the guide polynucleotide, and/or retaining the ability to process an RNA transcript containing the guide sequence into the guide polynucleotide molecules. In some embodiments, compared to the Cas protein with the sequence shown in any one of SEQ ID NO: 1-53, SEQ ID NO: 696, and SEQ ID NO: 728, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions) while retaining the ability to bind the target nucleic acid molecule complementary to the guide sequence of the guide polynucleotide.

In some embodiments of the present disclosure, the Cas12 protein comprises a mutation at any amino acid residue corresponding to the sequence shown in SEQ ID NO: 696. In some embodiments, the mutation is a mutation to any other natural amino acid residue. In some embodiments, the mutation is a mutation to residue R, H, K, or A. In some embodiments, the mutation is a mutation to residue R. In some embodiments, the mutation is a mutation to residue A. In some embodiments, the mutation is mutated to residue H. In some embodiments, the mutation is a mutation to residue K.

In some embodiments of the present disclosure, the Cas12 protein has mutations at the amino acid residues corresponding to positions 1-41, 42-195, 196-290, 291-358, 359-479, 480-636, 637-689, 690-846, 847-884, 885-959, 960-1080 or 1081-1139 of the sequence shown in SEQ ID NO: 696.

In some embodiments of the present disclosure, the Cas12 protein has the mutation in the RuvC domain corresponding to the sequence shown in SEQ ID NO: 696. In some embodiments of the present disclosure, the Cas12 protein has mutations at the amino acid residues corresponding to positions 637-689, 885-959, or 1081-1139 of the sequence shown in SEQ ID NO: 696.

In some embodiments of the present disclosure, the Cas12 protein has the mutation in a helical domain corresponding to the sequence shown in SEQ ID NO: 696. In some embodiments of the present disclosure, the Cas12 protein has the mutations at the amino acid residues corresponding to positions 42-195, 291-479, or 690-846 of the sequence as shown in SEQ ID NO: 696.

In some embodiments of the present disclosure, the Cas12 protein has the mutation at the amino acid residue corresponding to positions 1-41 of the sequence as shown in SEQ ID NO: 696. In some embodiments of the present disclosure, the Cas12 protein has the mutation at the amino acid residue corresponding to positions 42-195 of the sequence as shown in SEQ ID NO: 696. In some embodiments of the present disclosure, the Cas12 protein has the mutation at the amino acid residue corresponding to positions 196-290 of the sequence as shown in SEQ ID NO: 696. In some embodiments of the present disclosure, the Cas12 protein has the mutation at the amino acid residue corresponding to positions 291-358 of the sequence as shown in SEQ ID NO: 696. In some embodiments of the present disclosure, the Cas12 protein has the mutation at the amino acid residue corresponding to positions 359-479 of the sequence as shown in SEQ ID NO: 696. In some embodiments of the present disclosure, the Cas12 protein has the mutation at the amino acid residue corresponding to positions 480-636 of the sequence as shown in SEQ ID NO: 696. In some embodiments of the present disclosure, the Cas12 protein has the mutation at the amino acid residue corresponding to positions 637-689 of the sequence as shown in SEQ ID NO: 696. In some embodiments of the present disclosure, the Cas12 protein has the mutation at the amino acid residue corresponding to positions 690-846 of the sequence as shown in SEQ ID NO: 696. In some embodiments of the present disclosure, the Cas12 protein has the mutation at the amino acid residue corresponding to positions 847-884 of the sequence as shown in SEQ ID NO: 696. In some embodiments of the present disclosure, the Cas12 protein has the mutation at the amino acid residue corresponding to positions 885-959 of the sequence as shown in SEQ ID NO: 696. In some embodiments of the present disclosure, the Cas12 protein has the mutation at the amino acid residue corresponding to positions 960-1080 of the sequence as shown in SEQ ID NO: 696. In some embodiments of the present disclosure, the Cas12 protein has the mutation at the amino acid residue corresponding to positions 1081-1139 of the sequence as shown in SEQ ID NO: 696.

In some embodiments of the present disclosure, at the amino acid residues corresponding to positions 1-41 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 42-195 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2% , at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 196-290 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 291-358 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 359-479 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 480-636 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 637-689 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 690-846 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 847-884 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 885-959 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 960-1080 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696. In some embodiments of the present disclosure, at the amino acid residues corresponding to positions 1081-1139 of the sequence as shown in SEQ ID NO: 696, the Cas12 protein has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in SEQ ID NO: 696.

In some embodiments of the present disclosure, the Cas12 protein has at least one mutation in at least 1, at least 2, at least 3, at least 4, at least 5, at least 6, at least 7, at least 8, at least 9, at least 10, at least 11, or at least 12 of the amino acid residues corresponding to positions 1, 2, 3, 4, 5, 6, 7, 8, 9, 10, 12, 13, 14, 15, 16, 19, 21, 22, 23, 24, 25, 26, 27, 28, 29, 30, 32, 33, 34, 35, 36, 37, 38, 39, 40, 41, 42, 43, 44, 46, 47, 48, 49, 50, 51, 53, 54, 55, 56, 57, 58, 59, 60, 61, 62, 64, 65, 66, 67, 68, 69, 70, 71, 72, 73, 74, 75, 76, 77, 78, 79, 80, 81, 82, 83, 84, 85, 86, 88, 89, 90, 91, 92, 93, 94, 95, 96, 97, 98, 99, 101, 102, 103, 104, 105, 106, 108, 109, 110, 111, 112, 114, 115, 116, 117, 118, 119, 120, 121, 123, 124, 125, 126, 127, 128, 129, 130, 131, 132, 133, 134, 135, 136, 137, 138, 139, 140, 141, 142, 143, 144, 145, 146, 147, 148, 149, 151, 152, 153, 154, 155, 156, 157, 158, 159, 160, 161, 162, 163, 164, 165, 166, 167, 169, 170, 171, 172, 174, 175, 176, 177, 178, 179, 180, 182, 183, 184, 185, 186, 187, 188, 189, 190, 191, 192, 194, 195, 196, 197, 198, 199, 200, 202, 203, 204, 205, 206, 207, 208, 209, 210, 211, 212, 213, 214, 215, 216, 217, 218, 219, 220, 221, 222, 223, 224, 225, 227, 228, 229, 230, 231, 232, 233, 234, 235, 236, 237, 238, 239, 240, 242, 243, 244, 245, 247, 248, 249, 250, 251, 252, 253, 255, 256, 257, 258, 259, 260, 261, 262, 263, 265, 266, 267, 268, 269, 270, 271, 272, 273, 274, 275, 276, 278, 279, 281, 282, 283, 284, 285, 286, 287, 288, 289, 290, 291, 292, 293, 294, 295, 296, 297, 298, 299, 300, 301, 302, 303, 305, 306, 308, 309, 310, 313, 315, 316, 317, 318, 319, 320, 321, 322, 323, 324, 325, 327, 328, 329, 330, 331, 332, 333, 334, 335, 336, 337, 339, 340, 341, 342, 343, 344, 345, 346, 347, 348, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 431, 432, 433, 435, 436, 437, 439, 440, 441, 442, 443, 444, 446, 447, 448, 449, 450, 451, 452, 453, 454, 455, 456, 457, 458, 459, 460, 461, 462, 463, 464, 467, 469, 470, 471, 472, 473, 474, 475, 476, 477, 478, 479, 480, 481, 482, 483, 484, 485, 486, 487, 488, 489, 490, 491, 492, 493, 494, 496, 497, 499, 500, 501, 502, 503, 504, 506, 507, 508, 509, 510, 511, 512, 513, 514, 515, 516, 517, 518, 519, 520, 521, 522, 523, 524, 525, 526, 527, 528, 529, 531, 532, 533, 534, 535, 536, 537, 538, 539, 540, 541, 542, 543, 544, 545, 546, 547, 548, 549, 550, 552, 553, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 589, 590, 592, 593, 594, 595, 596, 597, 598, 599, 601, 602, 603, 604, 605, 606, 607, 608, 609, 610, 611, 612, 613, 614, 615, 616, 618, 619, 620, 621, 622, 623, 624, 625, 626, 627, 628, 630, 631, 632, 633, 634, 635, 636, 637, 638, 639, 640, 641, 642, 643, 644, 645, 646, 647, 648, 649, 650, 651, 652, 653, 654, 655, 656, 657, 658, 659, 660, 661, 662, 663, 664, 665, 666, 667, 668, 669, 670, 671, 672, 673, 674, 675, 676, 678, 679, 680, 681, 683, 684, 685, 686, 688, 689, 691, 692, 693, 694, 695, 696, 697, 698, 699, 700, 701, 702, 703, 704, 705, 706, 707, 708, 709, 710, 711, 712, 713, 715, 716, 717, 719, 720, 721, 722, 723, 724, 725, 727, 728, 729, 730, 731, 732, 733, 734, 736, 737, 738, 739, 740, 741, 742, 743, 744, 745, 746, 747, 748, 749, 751, 752, 753, 754, 755, 756, 758, 759, 760, 761, 762, 764, 765, 766, 767, 768, 769, 771, 772, 773, 774, 775, 776, 779, 780, 781, 782, 783, 784, 785, 786, 787, 789, 790, 791, 792, 794, 795, 797, 798, 800, 801, 802, 804, 805, 806, 807, 808, 809, 810, 811, 812, 813, 814, 815, 817, 818, 819, 821, 822, 823, 824, 825, 826, 827, 828, 829, 830, 831, 832, 833, 834, 835, 836, 837, 838, 839, 840, 841, 842, 844, 845, 846, 847, 848, 849, 850, 851, 852, 853, 854, 855, 856, 857, 858, 859, 860, 862, 863, 864, 865, 866, 867, 868, 870, 872, 873, 874, 875, 876, 877, 879, 880, 881, 882, 883, 884, 885, 886, 887, 888, 890, 891, 892, 893, 894, 895, 896, 897, 898, 899, 900, 901, 902, 903, 904, 905, 906, 909, 910, 911, 912, 913, 914, 916, 917, 918, 919, 920, 921, 922, 923, 924, 925, 926, 927, 928, 930, 931, 932, 933, 934, 935, 936, 937, 938, 939, 940, 941, 942, 943, 944, 945, 946, 947, 948, 949, 950, 951, 952, 953, 954, 955, 956, 957, 958, 959, 960, 961, 963, 964, 965, 966, 967, 968, 969, 970, 971, 972, 973, 974, 975, 976, 977, 979, 980, 981, 982, 983, 985, 986, 987, 988, 989, 990, 991, 992, 993, 994, 995, 996, 997, 998, 999, 1000, 1001, 1002, 1003, 1004, 1005, 1006, 1007, 1008, 1009, 1010, 1011, 1012, 1013, 1014, 1015, 1016, 1017, 1018, 1021, 1023, 1024, 1025, 1026, 1027, 1028, 1029, 1030, 1031, 1032, 1033, 1035, 1036, 1037, 1038, 1039, 1040, 1041, 1042, 1043, 1044, 1045, 1046, 1047, 1048, 1049, 1050, 1052, 1053, 1055, 1056, 1057, 1058, 1059, 1060, 1061, 1062, 1063, 1064, 1065, 1066, 1067, 1068, 1069, 1070, 1071, 1072, 1073, 1074, 1075, 1076, 1077, 1078, 1079, 1080, 1081, 1082, 1083, 1084, 1085, 1086, 1087, 1088, 1089, 1090, 1091, 1092, 1093, 1094, 1095, 1096, 1097, 1098, 1100, 1101, 1102, 1103, 1104, 1105, 1107, 1108, 1109, 1110, 1111, 1112, 1113, 1115, 1116, 1117, 1120, 1122, 1123, 1124, 1125, 1128, 1129, 1130, 1131, 1132, 1133, 1134, and 1135 of the amino acid sequence shown in SEQ ID NO: 696. In some embodiments, the mutation is a mutation to any other natural amino acid residue. In some embodiments, the mutation is a mutation to residue R, H, K, or A. In some embodiments, the mutation is a mutation to residue R. In some embodiments, the mutation is a mutation to residue A. In some embodiments, the mutation is a mutation to residue H. In some embodiments, the mutation is a mutation to residue K.

In some embodiments of the present disclosure, the Cas12 protein has at least one mutation in at least 1, at least 2, at least 3, at least 4, at least 5, at least 6, at least 7, at least 8, at least 9, at least 10, at least 11, or at least 12 of the amino acid residues corresponding to positions 1, 2, 3, 4, 5, 7, 10, 24, 30, 48, 51, 55, 58, 59, 66, 108, 118, 138, 141, 175, 178, 185, 186, 257, 333, 352, 356, 375, 376, 378, 379, 383, 397, 400, 416, 426, 443, 449, 456, 459, 462, 469, 484, 485, 509, 561, 597 607, 609, 623, 638, 639, 640, 697, 722, 731, 733, 755, 758, 771, 773, 779, 781, 784, 785, 786, 789, 792, 794, 798, 822, 823, 825, 826, 829, 830, 833, 834, 836, 842, 845, 846, 847, 850, 851, 853, 855, 856, 858, 859, 860, 866, 884, 892, 893, 900, 904, 926, 956, 985, 988, 989, 992, 993, 996, 1016, 1033, 1045, 1050, 1073, 1074, 1095, 1100, 1124, 1129, and 1132 of the amino acid sequence shown in SEQ ID NO: 696. In some embodiments, the mutation is a mutation to residue R, H, or K. In some embodiments, the mutation is a mutation to residue R.

In some embodiments of the present disclosure, the Cas12 protein has at least one mutation in at least 1, at least 2, at least 3, at least 4, at least 5, at least 6, at least 7, at least 8, at least 9,at least 10, at least 11, or at least 12 of the amino acid residues corresponding to positions 12, 29, 35, 36, 40, 53, 57, 60, 64, 71, 72, 73, 75, 94, 95, 96, 97, 99, 137, 148, 149, 153, 164, 167, 171, 172, 174, 177, 190, 192, 194, 199, 204, 207, 208, 211, 215, 228, 232, 236, 238, 244, 248, 253, 256, 258, 261, 262, 275, 282, 286, 292, 298, 300, 302, 320, 324, 328, 332, 336, 339, 366, 373, 374, 384, 389, 393, 395, 415, 432, 436, 440, 453, 458, 460, 471, 472, 474, 506, 508, 510, 519, 523, 526, 528, 531, 534, 544, 550, 570, 571, 573, 592, 594, 596, 613, 615, 616, 619, 622, 624, 626, 648, 650, 651, 652, 658, 661, 663, 684, 686, 689, 693, 704, 744, 783, 787, 800, 849, 854, 876, 888, 890, 891, 894, 912, 940, 941, 942, 945, 946, 948, 961, 971, 979, 987, 998, 1000, 1002, 1006, 1013, 1015, 1035, 1042, 1048, 1049, 1052, 1053, 1057, 1058, 1063, 1079, 1081, 1082, 1083, 1084, 1085, 1086, 1087, 1088, 1089, 1090, and 1091 of the amino acid sequence shown in SEQ ID NO: 696. In some embodiments, the mutation is a mutation to residue A.

In some embodiments of the present disclosure, the Cas12 protein has the mutations at the amino acid residues corresponding to any 1, any 2, any 3, any 4, any 5, any 6, any 7, any 8, any 9, any 10, any 11, any 12, any 13, any 14, any 15, any 16, or more positions in the amino acid sequence shown in SEQ ID NO: 696, and the positions are selected from 133, G184, S185, Q186, G194, N195, G196, G197, N245, G256, L260, Y278, S285, Y316, H350, D352, A355, A356, C385, P386, H387, G390, K391, N392, D429, Q461, Q462, Q469, E485, S491, K521, P525, L611, K629, K631, N633, D841, N898, K987, A988, G989, Q990, T991, D1010, E1013, A1136, K1138, and T1139.

In some embodiments of the present disclosure, the Cas 12 protein has any 1, any 2, any 3, any 4, any 5, any 6, any 7, any 8, any 9, any 10, any 11, any 12, any 13, any 14, any 15, any 16, or more amino acid mutations of the amino acid sequence shown in SEQ ID NO: 696 at positions corresponding to the amino acid sequence shown in SEQ ID NO: 696, and the amino acid mutations are selected from I33R, G184R, S185R, Q186R, G194R, N195R, G196R, G197R, N245R, G256R, L260R, Y278R, S285R, Y316R, H350R, D352R, A355R, A356R, C385R, P386R, H387R, G390R, K391R, N392R, D429R, Q461R, Q462R, Q469R, E485R, S491R, K521R, P525R, L611R, K629R, K631R, N633R, D841R, N898R, K987R, A988R, G989R, Q990R, T991R, D1010R, E1013R, A1136R, K1138R, and T1139R.

In some embodiments of the present disclosure, the Cas12 protein has any 1, any 2, any 3, any 4, any 5, or more amino acid mutation combinations of the amino acid sequence shown in SEQ ID NO: 696 at the position corresponding to the amino acid sequence shown in SEQ ID NO: 696, and the amino acid mutation combinations are selected from 186+352+1+426+846+858+860, 186+352+1+426+846+860, 186+352+1+5+426+858+860, 186+352+1+3+426+858+860, 186+352+3+426+860, 186+352+1+333+426+858+860, 186+352+1+426+485+858+860, 186+352+1+5+426+860, 186+352+3+426+858+860, 186+352+5+426+858+860, 186+352+333+426+858+860, 186+352+333+426+860, 186+352+426+846+860, 186+352+5+426+860, 186+352+1+3+426+860, 5+426+860, 186+352+426+846+85+860, 186+352+1+426+485+860, 426+858+860, 7+426+858, 186+352+426+860, 186+352+426+485+858+860, 186+352+426+485+860, 426+846+858+860, 7+426+846, 186+352+860, 184+186+352+376+1132, 5+333+426, 5+426+858, 186+352+1+333+426+860, 184+186+352+3+107+426, 186+352+5+426, 333+376+426, 186+352+5, 2+5+846+858, 186+352+3+426, 5+846 +858, 186+352+426+846, 186+352+7, 186+352+3+376+426, 186+352+376+426+860+865, 186+352+376+426, 3+846+860, 846+858+988, 3+426+858, 3+846+858+860, 3+858+860, 186+352+858, 184+186+352+860, 333+426, 184+186+352+3+376, 3+426+860, 846+860+988, 3+860, 184+186+352+3+639, 186+352+333+426, 846 +585+860, 186+352+426, 333+426+846, 333+426+485, 5+846+860, 3+846+988, 184+186+352+376, 3+333+426, 186+352+426+485, 333+426+858, 333+426+ 1132, 428+485, 186+352+333+352+376+426, 858+860, 186+352+376+426+485+860, 184+186+352+426, 186+352+639, 5+858+988, 3+858+988, 5+858, 3+858, 184+186+352+846, 184+186+352+639, 858+988, 184+186+352+426+1132, 186+352+1132, 184+186+352+5, 184+186+352+858, 858+860+1132, 3+5, 426+649, 186+352+426+485+860, 186+352+333, 184+186+352, 186+376, 846+860, 858+988+1132, 846+858, 333+376, 376+426, 184+186+352+3, 3+846+1132, 5+846+ 1132, 186+352+426+1132, 376+426+485+660, 426, 5+846, 846+860+1132, 333+376+485, 184+186+352+639+1132, 352+426, 333+485, 184+186+352+333, 846+ 858+1132, 333+426+860, 186+352+988, 5+860, 846+988, 186+352, 3+846, 846+1132, 184+186+352+1132, 186+485, 988+1132, 184+186+352+485, 376+485, 5+ 1132, 3+7, 186+352+485, 184+186+352+7, 184+186+352+333+336, 3+1132, 426+858+988, 186+352+376, 186+352+3, and 186+352+333+336+352+376+426 (mutation combinations are separated by commas). In some embodiments, the mutation is a mutation to residue R or A.

In some embodiments of the present disclosure, the Cas12 protein has any one amino acid mutation combination of the amino acid sequence shown in SEQ ID NO: 696 at a position corresponding to the amino acid sequence shown in SEQ ID NO: 696, and the amino acid mutation combination is selected from 186+352+1+426+846+858+860, 186+352+1+426+846+860, 186+352+1+5+426+858+860, 186+352+1+3+426+858+860, 186+352+3+426+860, 186+352+1+333+426+858+860, 186+352+1+426+485+858+860, 186+352+1+5+426+860, 186+352+3+426+858+860, 186+352+5+426+858+860, 186+352+333+426+858+860, 186+352+333+426+860, 186+352+426+846+860, 186+352+5+426+860, 186+352+1+3+426+860, 5+426+860, 186+352+426+846+858+860, 186+352+1+426+485+860, 426+858+860, 7+426+858, 186+352+426+860, 186+352+426+485+858+860, 186+352+426+485+860, 426+846+858+860, 7+426+846, and 186+352+860 (mutation combinations are separated by commas). In some embodiments, the mutation is a mutation to residue R or A.

In some embodiments of the present disclosure, the Cas12 protein has any 1, any 2, any 3, any 4, any 5, or more amino acid mutations of the amino acid sequence shown in SEQ ID NO: 696 at the position corresponding to the amino acid sequence shown in SEQ ID NO: 696, and the amino acid mutations are selected from D352R+Q186R, D352R+L260R, D352R+A355R, A355R+L260R, P386R+C385R, E485R+Q462R, D352R+Q186R, A355R+L260R, G184R+R186Q, D352R+Q186R+I33R, D352R+Q186R+G184R, D352R+Q186R+S185R, D352R+Q186R+G256R, D352R+Q186R+Y278R, D352R+Q186R+S285R, D352R+Q186R+Y316R, D352R+Q186R+H350R, D352R+Q186R+A356R, D352R+Q186R+Q469R, D352R+Q186R+S491R, D352R+Q186R+K521R, D352R+Q186R+P525R, D352R+Q186R+K629R, D352R+Q186R+N633R, D352R+Q186R+D841R, D352R+Q186R+N898R, D352R+Q186R+K987R, D352R+Q186R+T991R, D352R+Q186R+D1010R, and D352R+Q186R+E1013R.

In some embodiments of the present disclosure, the Cas12 protein comprises a mutation at an amino acid residue corresponding to position 1 of the sequence as shown in SEQ ID NO: 696. In some embodiments of the present disclosure, the Cas12 protein comprises a mutation at an amino acid residue corresponding to position 2 of the sequence as shown in SEQ ID NO: 696. In some embodiments of the present disclosure, the Cas12 protein comprises a mutation at an amino acid residue corresponding to position 3 of the sequence as shown in SEQ ID NO: 696. In some embodiments of the present disclosure, the Cas12 protein comprises a mutation at an amino acid residue corresponding to position 4 of the sequence as shown in SEQ ID NO: 696. In some embodiments of the present disclosure, the Cas12 protein comprises a mutation at an amino acid residue corresponding to position 5 of the sequence as shown in SEQ ID NO: 696. In some embodiments of the present disclosure, the Cas12 protein comprises a mutation at an amino acid residue corresponding to position 7 of the sequence as shown in SEQ ID NO: 696. In some embodiments of the present disclosure, the Cas12 protein comprises a mutation at an amino acid residue corresponding to position 10 of the sequence as shown in SEQ ID NO: 696. In some embodiments of the present disclosure, the Cas12 protein comprises a mutation at an amino acid residue corresponding to position 24 of the sequence as shown in SEQ ID NO: 696. In some embodiments of the present disclosure, the Cas12 protein comprises a mutation at amino acid residue corresponding to position 30 of the as shown in SEQ ID NO: 696. In some embodiments of the present disclosure, the Cas12 protein comprises a mutation at an amino acid residue corresponding to position 48 of the sequence as shown in SEQ ID NO: 696. In some embodiments of the present disclosure, the Cas12 protein comprises a mutation at an amino acid residue corresponding to position 51 of the sequence as shown in SEQ ID NO: 696. In some embodiments of the present disclosure, the Cas12 protein comprises a mutation at an amino acid residue corresponding to position 55 of the sequence as shown in SEQ ID NO: 696. In some embodiments of the present disclosure, the Cas12 protein comprises a mutation at an amino acid residue corresponding to position 58 of the sequence as shown in SEQ ID NO: 696. In some embodiments of the present disclosure, the Cas12 protein comprises a mutation at an amino acid residue corresponding to position 59 of the sequence as shown in SEQ ID NO: 696. In some embodiments of the present disclosure, the Cas12 protein comprises a mutation at an amino acid residue corresponding to position 66 of the sequence as shown in SEQ ID NO: 696. In some embodiments of the present disclosure, the Cas12 protein comprises a mutation at amino acid residue corresponding to position 108 of the sequence as shown in SEQ ID NO: 696. In some embodiments of the present disclosure, the Cas12 protein comprises a mutation at an amino acid residue corresponding to position 118 of the sequence as shown in SEQ ID NO: 696. In some embodiments of the present disclosure, the Cas12 protein comprises a mutation at an amino acid residue corresponding to position 138 of the sequence as shown in SEQ ID NO: 696. In some embodiments of the present disclosure, the Cas12 protein comprises a mutation at an amino acid residue corresponding to position 141 of the sequence as shown in SEQ ID NO: 696. In some embodiments of the present disclosure, the Cas12 protein comprises a mutation at an amino acid residue corresponding to position 175 of the sequence as shown in SEQ ID NO: 696. In some embodiments of the present disclosure, the Cas12 protein comprises a mutation at an amino acid residue corresponding to position 178 of the sequence as shown in SEQ ID NO: 696. In some embodiments of the present disclosure, the Cas12 protein comprises a mutation at an amino acid residue corresponding to position 185 of the sequence as shown in SEQ ID NO: 696. In some embodiments of the present disclosure, the Cas12 protein comprises a mutation at an amino acid residue corresponding to position 186 of the sequence as shown in SEQ ID NO: 696. In some embodiments of the present disclosure, the Cas12 protein comprises a mutation at an amino acid residue corresponding to position 257 of the sequence as shown in SEQ ID NO: 696. In some embodiments of the present disclosure, the Cas12 protein comprises a mutation at an amino acid residue corresponding to position 333 of the sequence as shown in SEQ ID NO: 696. In some embodiments of the present disclosure, the Cas12 protein comprises a mutation at an amino acid residue corresponding to position 352 of the sequence as shown in SEQ ID NO: 696. In some embodiments of the present disclosure, the Cas12 protein comprises a mutation at an amino acid residue corresponding to position 356 of the sequence as shown in SEQ ID NO: 696. In some embodiments of the present disclosure, the Cas12 protein comprises a mutation at an amino acid residue corresponding to position 375 of the sequence as shown in SEQ ID NO: 696. In some embodiments of the present disclosure, the Cas12 protein comprises a mutation at an amino acid residue corresponding to position 376 of the sequence as shown in SEQ ID NO: 696. In some embodiments of the present disclosure, the Cas12 protein comprises a mutation at an amino acid residue corresponding to position 378 of the sequence as shown in SEQ ID NO: 696. In some embodiments of the present disclosure, the Cas12 protein comprises a mutation at an amino acid residue corresponding to position 379 of the sequence as shown in SEQ ID NO: 696. In some embodiments of the present disclosure, the Cas12 protein comprises a mutation at an amino acid residue corresponding to position 383 of the sequence as shown in SEQ ID NO: 696. In some embodiments of the present disclosure, the Cas12 protein comprises a mutation at an amino acid residue corresponding to position 397 of the sequence as shown in SEQ ID NO: 696. In some embodiments of the present disclosure, the Cas12 protein comprises a mutation at an amino acid residue corresponding to position 400 of the sequence as shown in SEQ ID NO: 696. In some embodiments of the present disclosure, the Cas12 protein comprises a mutation at an amino acid residue corresponding to position 416 of the sequence as shown in SEQ ID NO: 696. In some embodiments of the present disclosure, the Cas12 protein comprises a mutation at an amino acid residue corresponding to position 426 of the sequence as shown in SEQ ID NO: 696. In some embodiments of the present disclosure, the Cas12 protein comprises a mutation at an amino acid residue corresponding to position 443 of the sequence as shown in SEQ ID NO: 696. In some embodiments of the present disclosure, the Cas12 protein comprises a mutation at an amino acid residue corresponding to position 449 of the sequence as shown in SEQ ID NO: 696. In some embodiments of the present disclosure, the Cas12 protein comprises a mutation at an amino acid residue corresponding to position 456 of the sequence as shown in SEQ ID NO: 696. In some embodiments of the present disclosure, the Cas12 protein comprises a mutation at an amino acid residue corresponding to position 459 of the sequence as shown in SEQ ID NO: 696. In some embodiments of the present disclosure, the Cas12 protein comprises a mutation at an amino acid residue corresponding to position 462 of the sequence as shown in SEQ ID NO: 696. In some embodiments of the present disclosure, the Cas12 protein comprises a mutation at an amino acid residue corresponding to position 469 of the sequence as shown in SEQ ID NO: 696. In some embodiments of the present disclosure, the Cas12 protein comprises a mutation at an amino acid residue corresponding to position 484 of the sequence as shown in SEQ ID NO: 696. In some embodiments of the present disclosure, the Cas12 protein comprises a mutation at an amino acid residue corresponding to position 485 of the sequence as shown in SEQ ID NO: 696. In some embodiments of the present disclosure, the Cas12 protein comprises a mutation at an acid residue corresponding to position 509 of the sequence as shown in SEQ ID NO: 696. In some embodiments of the present disclosure, the Cas12 protein comprises a mutation at an amino acid residue corresponding to position 561 of the sequence as shown in SEQ ID NO: 696. In some embodiments of the present disclosure, the Cas12 protein comprises a mutation at an amino acid residue corresponding to position 597 of the sequence as shown in SEQ ID NO: 696. In some embodiments of the present disclosure, the Cas12 protein comprises a mutation at an amino acid residue corresponding to position 607 of the sequence as shown in SEQ ID NO: 696. In some embodiments of the present disclosure, the Cas12 protein comprises a mutation at an amino acid residue corresponding to position 609 of the sequence as shown in SEQ ID NO: 696. In some embodiments of the present disclosure, the Cas12 protein comprises a mutation at an amino acid residue corresponding to position 623 of the sequence as shown in SEQ ID NO: 696. In some embodiments of the present disclosure, the Cas12 protein comprises a mutation at an amino acid residue corresponding to position 638 of the sequence as shown in SEQ ID NO: 696. In some embodiments of the present disclosure, the Cas12 protein comprises a mutation at an amino acid residue corresponding to position 639 of the sequence as shown in SEQ ID NO: 696. In some embodiments of the present disclosure, the Cas12 protein comprises a mutation at an amino acid residue corresponding to position 640 of the sequence as shown in SEQ ID NO: 696. In some embodiments of the present disclosure, the Cas12 protein comprises a mutation at an amino acid residue corresponding to position 697 of the sequence as shown in SEQ ID NO: 696. In some embodiments of the present disclosure, the Cas12 protein comprises a mutation at an amino acid residue corresponding to position 722 of the sequence as shown in SEQ ID NO: 696. In some embodiments of the present disclosure, the Cas12 protein comprises a mutation at an amino acid residue corresponding to position 731 of the sequence as shown in SEQ ID NO: 696. In some embodiments of the present disclosure, the Cas12 protein comprises a mutation at an amino acid residue corresponding to position 733 of the sequence as shown in SEQ ID NO: 696. In some embodiments of the present disclosure, the Cas12 protein comprises a mutation at an amino acid residue corresponding to position 755 of the sequence as shown in SEQ ID NO: 696. In some embodiments of the present disclosure, the Cas12 protein comprises a mutation at an amino acid residue corresponding to position 758 of the sequence as shown in SEQ ID NO: 696. In some embodiments of the present disclosure, the Cas12 protein comprises a mutation at an amino acid residue corresponding to position 771 of the sequence as shown in SEQ ID NO: 696. In some embodiments of the present disclosure, the Cas12 protein comprises a mutation at an amino acid residue corresponding to position 773 of the sequence as shown in SEQ ID NO: 696. In some embodiments of the present disclosure, the Cas12 protein comprises a mutation at an amino acid residue corresponding to position 779 of the sequence as shown in SEQ ID NO: 696. In some embodiments of the present disclosure, the Cas12 protein comprises a mutation at an amino acid residue corresponding to position 781 of the sequence as shown in SEQ ID NO: 696. In some embodiments of the present disclosure, the Cas12 protein comprises a mutation at an amino acid residue corresponding to position 784 of the sequence as shown in SEQ ID NO: 696. In some embodiments of the present disclosure, the Cas12 protein comprises a mutation at an amino acid residue corresponding to position 785 of the sequence as shown in SEQ ID NO: 696. In some embodiments of the present disclosure, the Cas12 protein comprises a mutation at an amino acid residue corresponding to position 786 of the sequence as shown in SEQ ID NO: 696. In some embodiments of the present disclosure, the Cas12 protein comprises a mutation at an amino acid residue corresponding to position 789 of the sequence as shown in SEQ ID NO: 696. In some embodiments of the present disclosure, the Cas12 protein comprises a mutation at an amino acid residue corresponding to position 792 of the sequence as shown in SEQ ID NO: 696. In some embodiments of the present disclosure, the Cas12 protein comprises a mutation at an amino acid residue corresponding to position 794 of the sequence as shown in SEQ ID NO: 696. In some embodiments of the present disclosure, the Cas12 protein comprises a mutation at an amino acid residue corresponding to position 798 of the sequence as shown in SEQ ID NO: 696. In some embodiments of the present disclosure, the Cas12 protein comprises a mutation at an amino acid residue corresponding to position 822 of the sequence as shown in SEQ ID NO: 696. In some embodiments of the present disclosure, the Cas12 protein comprises a mutation at an amino acid residue corresponding to position 823 of the sequence as shown in SEQ ID NO: 696. In some embodiments of the present disclosure, the Cas12 protein comprises a mutation at an amino acid residue corresponding to position 825 of the sequence as shown in SEQ ID NO: 696. In some embodiments of the present disclosure, the Cas12 protein comprises a mutation at an amino acid residue corresponding to position 826 of the sequence as shown in SEQ ID NO: 696. In some embodiments of the present disclosure, the Cas12 protein comprises a mutation at an amino acid residue corresponding to position 829 of the sequence as shown in SEQ ID NO: 696. In some embodiments of the present disclosure, the Cas12 protein comprises a mutation at an amino acid residue corresponding to position 830 of the sequence as shown in SEQ ID NO: 696. In some embodiments of the present disclosure, the Cas12 protein comprises a mutation at an amino acid residue corresponding to position 833 of the sequence as shown in SEQ ID NO: 696. In some embodiments of the present disclosure, the Cas12 protein comprises a mutation at an amino acid residue corresponding to position 834 of the sequence as shown in SEQ ID NO: 696. In some embodiments of the present disclosure, the Cas12 protein comprises a mutation at an amino acid residue corresponding to position 836 of the sequence as shown in SEQ ID NO: 696. In some embodiments of the present disclosure, the Cas12 protein comprises a mutation at an amino acid residue corresponding to position 842 of the sequence as shown in SEQ ID NO: 696. In some embodiments of the present disclosure, the Cas12 protein comprises a mutation at an amino acid residue corresponding to position 845 of the sequence as shown in SEQ ID NO: 696. In some embodiments of the present disclosure, the Cas12 protein comprises a mutation at an amino acid residue corresponding to position 846 of the sequence as shown in SEQ ID NO: 696. In some embodiments of the present disclosure, the Cas12 protein comprises a mutation at an amino acid residue corresponding to position 847 of the sequence as shown in SEQ ID NO: 696. In some embodiments of the present disclosure, the Cas12 protein comprises a mutation at an amino acid residue corresponding to position 850 of the sequence as shown in SEQ ID NO: 696. In some embodiments of the present disclosure, the Cas12 protein comprises a mutation at an amino acid residue corresponding to position 851 of the sequence as shown in SEQ ID NO: 696. In some embodiments of the present disclosure, the Cas12 protein comprises a mutation at an amino acid residue corresponding to position 853 of the sequence as shown in SEQ ID NO: 696. In some embodiments of the present disclosure, the Cas12 protein comprises a mutation at an amino acid residue corresponding to position 855 of the sequence as shown in SEQ ID NO: 696. In some embodiments of the present disclosure, the Cas12 protein comprises a mutation at an amino acid residue corresponding to position 856 of the sequence as shown in SEQ ID NO: 696. In some embodiments of the present disclosure, the Cas12 protein comprises a mutation at an amino acid residue corresponding to position 858 of the sequence as shown in SEQ ID NO: 696. In some embodiments of the present disclosure, the Cas12 protein comprises a mutation at an amino acid residue corresponding to position 859 of the sequence as shown in SEQ ID NO: 696. In some embodiments of the present disclosure, the Cas12 protein comprises a mutation at an amino acid residue corresponding to position 860 of the sequence as shown in SEQ ID NO: 696. In some embodiments of the present disclosure, the Cas12 protein comprises a mutation at an amino acid residue corresponding to position 866 of the sequence as shown in SEQ ID NO: 696. In some embodiments of the present disclosure, the Cas12 protein comprises a mutation at an amino acid residue corresponding to position 884 of the sequence as shown in SEQ ID NO: 696. In some embodiments of the present disclosure, the Cas12 protein comprises a mutation at an amino acid residue corresponding to position 892 of the sequence as shown in SEQ ID NO: 696. In some embodiments of the present disclosure, the Cas12 protein comprises a mutation at an amino acid residue corresponding to position 893 of the sequence as shown in SEQ ID NO: 696. In some embodiments of the present disclosure, the Cas12 protein comprises a mutation at an amino acid residue corresponding to position 900 of the sequence as shown in SEQ ID NO: 696. In some embodiments of the present disclosure, the Cas12 protein comprises a mutation at an amino acid residue corresponding to position 904 of the sequence as shown in SEQ ID NO: 696. In some embodiments of the present disclosure, the Cas12 protein comprises a mutation at an amino acid residue corresponding to position 926 of the sequence as shown in SEQ ID NO: 696. In some embodiments of the present disclosure, the Cas12 protein comprises a mutation at an amino acid residue corresponding to position 956 of the sequence as shown in SEQ ID NO: 696. In some embodiments of the present disclosure, the Cas12 protein comprises a mutation at an amino acid residue corresponding to position 985 of the sequence as shown in SEQ ID NO: 696. In some embodiments of the present disclosure, the Cas12 protein comprises a mutation at an amino acid residue corresponding to position 988 of the sequence as shown in SEQ ID NO: 696. In some embodiments of the present disclosure, the Cas12 protein comprises a mutation at an amino acid residue corresponding to position 989 of the sequence as shown in SEQ ID NO: 696. In some embodiments of the present disclosure, the Cas12 protein comprises a mutation at an amino acid residue corresponding to position 992 of the sequence as shown in SEQ ID NO: 696. In some embodiments of the present disclosure, the Cas12 protein comprises a mutation at an amino acid residue corresponding to position 993 of the sequence as shown in SEQ ID NO: 696. In some embodiments of the present disclosure, the Cas12 protein comprises a mutation at an amino acid residue corresponding to position 996 of the sequence as shown in SEQ ID NO: 696. In some embodiments of the present disclosure, the Cas12 protein comprises a mutation at an amino acid residue corresponding to position 1016 of the sequence as shown in SEQ ID NO: 696. In some embodiments of the present disclosure, the Cas12 protein comprises a mutation at an amino acid residue corresponding to position 1033 of the sequence as shown in SEQ ID NO: 696. In some embodiments of the present disclosure, the Cas12 protein comprises a mutation at an amino acid residue corresponding to position 1045 of the sequence as shown in SEQ ID NO: 696. In some embodiments of the present disclosure, the Cas12 protein comprises a mutation at an amino acid residue corresponding to position 1050 of the sequence as shown in SEQ ID NO: 696. In some embodiments of the present disclosure, the Cas12 protein comprises a mutation at an amino acid residue corresponding to position 1073 of the sequence as shown in SEQ ID NO: 696. In some embodiments of the present disclosure, the Cas12 protein comprises a mutation at an amino acid residue corresponding to position 1074 of the sequence as shown in SEQ ID NO: 696. In some embodiments of the present disclosure, the Cas12 protein comprises a mutation at an amino acid residue corresponding to position 1095 of the sequence as shown in SEQ ID NO: 696. In some embodiments of the present disclosure, the Cas12 protein comprises a mutation at an amino acid residue corresponding to position 1100 of the sequence as shown in SEQ ID NO: 696. In some embodiments of the present disclosure, the Cas12 protein comprises a mutation at an amino acid residue corresponding to position 1124 of the sequence as shown in SEQ ID NO: 696. In some embodiments of the present disclosure, the Cas12 protein comprises a mutation at an amino acid residue corresponding to position 1129 of the sequence as shown in SEQ ID NO: 696. In some embodiments of the present disclosure, the Cas12 protein comprises a mutation at an amino acid residue corresponding to position 1132 of the sequence as shown in SEQ ID NO: 696. In some embodiments, the mutation is a mutation to residue R.

In some embodiments of the present disclosure, the Cas12 protein comprises a mutation at an amino acid residue corresponding to position 651 of the sequence as shown in SEQ ID NO: 696. In some embodiments, the mutation is a mutation to residue A.

In some embodiments of the present disclosure, the Cas12 protein comprises a mutation at an amino acid residue corresponding to position 891 of the sequence as shown in SEQ ID NO: 696. In some embodiments, the mutation is a mutation to residue A.

In some embodiments of the present disclosure, the Cas12 protein comprises a mutation at an amino acid residue corresponding to position 1082 of the sequence as shown in SEQ ID NO: 696. In some embodiments, the mutation is a mutation to residue A.

In some embodiments of the present disclosure, the Cas12 protein has mutations at amino acid residues corresponding to any 1, any 2, any 3, any 4, any 5, any 6, any 7, any 8, any 9,any 10, any 11, any 12, any 13, any 14, any 15, any 16, or more positions of the amino acid sequence shown in SEQ ID NO: 52, and the positions are selected from V15, Q172, A173, G182, E183, G184, K185, K186, G239, V243, D264, E271, Y295, L297, N317, T329, E331, I335, K339, N347, E363, H366, V426, K429, S430, L433, S452, S455, S465, E493, P497, K587, E768, T825, A911, G914, K915, I916, K918, T919, T920, A922, and E940.

In some embodiments of the present disclosure, the Cas12 protein has any 1, any 2, any 3, any 4, any 5, any 6, any 7, any 8, any 9, any 10, any 11, any 12, any 13, any 14, any 15, any 16, or more amino acid mutations of the amino acid sequence shown in SEQ ID NO: 52 at positions corresponding to the amino acid sequence shown in SEQ ID NO: 52, and the amino acid mutations are selected from V15R, Q172R, A173W, G182R, E183R, G184R, K185R, K186R, G239R, V243R, D264R, E271R, Y295R, L297R, N317R, T329R, E331R, I335R, K339R, N347E, E363R, H366R, V426R, K429R, S430R, L433R, S452R, S455R, S465R, E493R, P497R, K587R, E768R, T825R, A911R, G914R, K915R, I916R, K918R, T919R, T920R, A922R, and E940R.

In some embodiments of the present disclosure, the Cas12 protein has any 1, any 2, any 3, any 4, any 5, or more amino acid mutations of the amino acid sequence shown in SEQ ID NO: 52 at positions corresponding to the amino acid sequence shown in SEQ ID NO: 52, and the amino acid mutations are selected from N347E+K339R, Q172R+S452R, Q172R+T920R, Q172R+V426R, S452R+T920R, V426R+S452R, V426R+T920R.

In some embodiments of the present disclosure, the Cas12 protein comprises a mutation at an amino acid residue corresponding to position 15 of the sequence as shown in SEQ ID NO: 52. In some embodiments of the present disclosure, the Cas12 protein comprises a mutation at an amino acid residue corresponding to position 172 of the sequence as shown in SEQ ID NO: 52. In some embodiments of the present disclosure, the Cas12 protein comprises a mutation at an amino acid residue corresponding to position 173 of the sequence as shown in SEQ ID NO: 52. In some embodiments of the present disclosure, the Cas12 protein comprises a mutation at an amino acid residue corresponding to position 182 of the sequence as shown in SEQ ID NO: 52. In some embodiments of the present disclosure, the Cas12 protein comprises a mutation at an amino acid residue corresponding to position 183 of the sequence as shown in SEQ ID NO: 52. In some embodiments of the present disclosure, the Cas12 protein comprises a mutation at an amino acid residue corresponding to position 184 of the sequence as shown in SEQ ID NO: 52. In some embodiments of the present disclosure, the Cas12 protein comprises a mutation at an amino acid residue corresponding to position 185 of the sequence as shown in SEQ ID NO: 52. In some embodiments of the present disclosure, the Cas12 protein comprises a mutation at an amino acid residue corresponding to position 186 of the sequence as shown in SEQ ID NO: 52. In some embodiments of the present disclosure, the Cas12 protein comprises a mutation at an amino acid residue corresponding to position 239 of the sequence as shown in SEQ ID NO: 52. In some embodiments of the present disclosure, the Cas12 protein comprises a mutation at an amino acid residue corresponding to position 243 of the sequence as shown in SEQ ID NO: 52. In some embodiments of the present disclosure, the Cas12 protein comprises a mutation at an amino acid residue corresponding to position 264 of the sequence as shown in SEQ ID NO: 52. In some embodiments of the present disclosure, the Cas12 protein comprises a mutation at an amino acid residue corresponding to position 271 of the sequence as shown in SEQ ID NO: 52. In some embodiments of the present disclosure, the Cas12 protein comprises a mutation at an amino acid residue corresponding to position 295 of the sequence as shown in SEQ ID NO: 52. In some embodiments of the present disclosure, the Cas12 protein comprises a mutation at an amino acid residue corresponding to position 297 of the sequence as shown in SEQ ID NO: 52. In some embodiments of the present disclosure, the Cas12 protein comprises a mutation at an amino acid residue corresponding to position 317 of the sequence as shown in SEQ ID NO: 52. In some embodiments of the present disclosure, the Cas12 protein comprises a mutation at an amino acid residue corresponding to position 329 of the sequence as shown in SEQ ID NO: 52. In some embodiments of the present disclosure, the Cas12 protein comprises a mutation at an amino acid residue corresponding to position 331 of the sequence as shown in SEQ ID NO: 52. In some embodiments of the present disclosure, the Cas12 protein comprises a mutation at an amino acid residue corresponding to position 335 of the sequence as shown in SEQ ID NO: 52. In some embodiments of the present disclosure, the Cas12 protein comprises a mutation at an amino acid residue corresponding to position 339 of the sequence as shown in SEQ ID NO: 52. In some embodiments of the present disclosure, the Cas12 protein comprises a mutation at an amino acid residue corresponding to position 347 of the sequence as shown in SEQ ID NO: 52. In some embodiments of the present disclosure, the Cas12 protein comprises a mutation at an amino acid residue corresponding to position 363 of the sequence as shown in SEQ ID NO: 52. In some embodiments of the present disclosure, the Cas12 protein comprises a mutation at an amino acid residue corresponding to position 366 of the sequence as shown in SEQ ID NO: 52. In some embodiments of the present disclosure, the Cas12 protein comprises a mutation at an amino acid residue corresponding to position 426 of the sequence as shown in SEQ ID NO: 52. In some embodiments of the present disclosure, the Cas12 protein comprises a mutation at an amino acid residue corresponding to position 429 of the sequence as shown in SEQ ID NO: 52. In some embodiments of the present disclosure, the Cas12 protein comprises a mutation at an amino acid residue corresponding to position 430 of the sequence as shown in SEQ ID NO: 52. In some embodiments of the present disclosure, the Cas12 protein comprises a mutation at an amino acid residue corresponding to position 433 of the sequence as shown in SEQ ID NO: 52. In some embodiments of the present disclosure, the Cas12 protein comprises a mutation at an amino acid residue corresponding to position 452 of the sequence as shown in SEQ ID NO: 52. In some embodiments of the present disclosure, the Cas12 protein comprises a mutation at an amino acid residue corresponding to position 455 of the sequence as shown in SEQ ID NO: 52. In some embodiments of the present disclosure, the Cas12 protein comprises a mutation at an amino acid residue corresponding to position 465 of the sequence as shown in SEQ ID NO: 52. In some embodiments of the present disclosure, the Cas12 protein comprises a mutation at an amino acid residue corresponding to position 493 of the sequence as shown in SEQ ID NO: 52. In some embodiments of the present disclosure, the Cas12 protein comprises a mutation at an amino acid residue corresponding to position 497 of the sequence as shown in SEQ ID NO: 52. In some embodiments of the present disclosure, the Cas12 protein comprises a mutation at an amino acid residue corresponding to position 587 of the sequence as shown in SEQ ID NO: 52. In some embodiments of the present disclosure, the Cas12 protein comprises a mutation at an amino acid residue corresponding to position 768 of the sequence as shown in SEQ ID NO: 52. In some embodiments of the present disclosure, the Cas12 protein comprises a mutation at an amino acid residue corresponding to position 825 of the sequence as shown in SEQ ID NO: 52. In some embodiments of the present disclosure, the Cas12 protein comprises a mutation at an amino acid residue corresponding to position 911 of the sequence as shown in SEQ ID NO: 52. In some embodiments of the present disclosure, the Cas12 protein comprises a mutation at an amino acid residue corresponding to position 914 of the sequence as shown in SEQ ID NO: 52. In some embodiments of the present disclosure, the Cas12 protein comprises a mutation at an amino acid residue corresponding to position 915 of the sequence as shown in SEQ ID NO: 52. In some embodiments of the present disclosure, the Cas12 protein comprises a mutation at an amino acid residue corresponding to position 916 of the sequence as shown in SEQ ID NO: 52. In some embodiments of the present disclosure, the Cas12 protein comprises a mutation at an amino acid residue corresponding to position 918 of the sequence as shown in SEQ ID NO: 52. In some embodiments of the present disclosure, the Cas12 protein comprises a mutation at an amino acid residue corresponding to position 919 of the sequence as shown in SEQ ID NO: 52. In some embodiments of the present disclosure, the Cas12 protein comprises a mutation at an amino acid residue corresponding to position 920 of the sequence as shown in SEQ ID NO: 52. In some embodiments of the present disclosure, the Cas12 protein comprises a mutation at an amino acid residue corresponding to position 922 of the sequence as shown in SEQ ID NO: 52. In some embodiments of the present disclosure, the Cas12 protein comprises a mutation at an amino acid residue corresponding to position 940 of the sequence as shown in SEQ ID NO: 52. In some embodiments, the mutation is a mutation to residue R.

Embodiments of the present disclosure provides a guide polynucleotide, wherein the guide polynucleotide comprises (i) a scaffold sequence, and (ii) a guide sequence. The guide sequence has at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to a sequence shown in any one of SEQ ID NO: 722, SEQ ID NO: 761-782, and SEQ ID NO: 825-877. The guide polynucleotide is able to form a complex with a nucleic acid-binding polypeptide and guide a sequence-specific binding of the complex to a target nucleic acid.

In some embodiments, the guide sequence may be obtained by adding and/or deleting 1, 2, 3, 4, 5, 6, or 7 nucleotides to/from the sequence shown in any one of SEQ ID NO: 722, SEQ ID NO: 761-782, and SEQ ID NO: 825-877. In some embodiments, the guide sequence is as shown in any one of SEQ ID NO: 722, SEQ ID NO: 761-782, and SEQ ID NO: 825-877.

In some embodiments, the guide sequence hybridizes to the target nucleic acid. In some embodiments, the target nucleic acid is randomly selected from TTR, HBG, BCL11A, BACH2, KLKB1, PCSK9, SOD1, and BACE1 genes.

In some embodiments, the guide sequence is located at the 5' end or 3' end of the scaffold sequence.

In some embodiments, the nucleic acid-binding polypeptide and the guide polynucleotide form a complex. Further, the complex specifically binds to a target nucleic acid. Further, the complex cleaves the target nucleic acid, modifies the target nucleic acid, and/or modulates an expression of the target nucleic acid.

In some embodiments, the nucleic acid-binding polypeptide comprises a DNA-binding polypeptide. In some embodiments, the nucleic acid-binding polypeptide comprises an RNA-binding polypeptide. In some embodiments, the nucleic acid-binding polypeptide comprises a TALEN nuclease, a zinc finger nuclease, a CRISPR-Cas nuclease, or a meganuclease. In some embodiments, the nucleic acid-binding polypeptide is an RNA-guided nuclease. In some embodiments, the DNA-binding polypeptide is a CRISPR-associated nuclease, i.e., a Cas enzyme (also known as a Cas protein, a CRISPR-Cas nuclease). In some embodiments, the nucleic acid-binding polypeptide is selected from Cas1, Cas1B, Cas2, Cas3, Cas4, Cas5, Cas5d, Cas5t, Cas5h, Cas5a, Cas6, Cas7, Cas8, Cas8a, Cas8b, Cas8c, Cas9, Cas10, Cas10d, Cas12a/Cpfl, Cas12b/C2cl, Cas12c/C2c3, Cas12d/CasY, Cas12e/CasX, Cas12f/CasZ, Cas12g, Cas12h, Cas12i, Csy1, Csy2, Csy3, Csy4, Cse1, Cse2, Cse3, Cse4, Cse5e, Csc1, Csc2, Csa5, Csn1, Csn2, Csm1, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx1S, Csx11, Csf1, Csf2, CsO, Csf4, Csd1, Csd2, Cst1, Cst2, Csh1, Csh2, Csa1, Csa2, Csa3, Csa4, Csa5, Cas13a, Cas13b, Cas13c, Cas13d, Cas13e, Cas13f, TnpB, IscB, IsrB, and Fancor, or fragments thereof. Non-limiting examples of the fragments include nucleic acid-binding domain fragments. In some embodiments, the nucleic acid-binding polypeptide is selected from Cas9, Cas12, Cas13, TnpB, IscB, IsrB, Fancor nuclease, or fragments thereof, including but not limited to the nucleic acid-binding domain fragments. In some embodiments, the Cas9 is selected from SpCas9, SaCas9, Nme2Cas9, Nme3Cas9, CjCas9, NmCas9, FnCas9, PpnCas9, FrCas9, SauCas9, SauriCas9, ScaCas9, St1Cas9, BlatCas9, CdiCas9, GeoCas9, fragments thereof, and mutations thereof or fragments of the mutations. In some embodiments, the nucleic acid-binding polypeptide is selected from AsCpf1, enAsCas12a (addgene plasmid #196724), dFnCas12a (addgene plasmid #136379), ErCas12a, LbCas12a D832A, LbCas12a H759A, LbCas12a E795L, FnCas12a3, FnCas12a D917A, AsCas12a R1226A, AsCas12a D908A, AsCas12a E174R/S542R, AsCas12a (S542R/K548V/N552R), PrCas12a, PxCas12a, PcCas12a, PdCas12a, Mb2Cas12a, Mb3Cas12a, MICas12a, CMaCas12a, CMtCas12a, HkCas12a, Lb5Cas12a, ErCas12a, TsCas12a, FnCpf1, LbCas12a, dLbCpf1, ttHsCas12a, AaCas12b, AaCas12b D570A, AaCas12b Q119F/E475R/E758R, BhCas12b, BvCas12b, BrCas12b, AkCas12b, AmCas12b, BsCas12b, OspCas12c, Cas12c2 (addgene plasmid #183072), Cas12c_4 (addgene plasmid #183071), Cas12c1 (addgene plasmid #120872), CasY.1 (from Katanobacteria), CasY.2 (from Vogelbacteria), CasY.3 (from Vogelbacteria), CasY.4 (fromParcubacteria), CasY.5 (from Komeilibacteria), CasY.6 (from Kerfeldbacteria), PlmCasX, DpbCasX, Un1Cas12f, CnCas12f1, enRhCas12f1, AsCas12f1, SpaCas12f1, Cas12g1 (addgene plasmid #120879), Cas12h from the international application WO2021113522A1 (SEQ ID NO: 1 from the application), Cas12i1 (addgene plasmid #171670), Cas12i2 (addgene plasmid #188275), Cas12i1 (addgene plasmid #120882), Cas12i2 (addgene plasmid #120883), Cas12i protein named as Cas12f.4/Cas12f.5/Cas12f.6 in CN111757889B, dSiCas12i(D1049A), SiCas12i, Si2Cas12i, WiCas12i, Wi2Cas12i, Wi3Cas12i, SaCas12i, Sa2Cas12i, Sa3Cas12i, WaCas12i, Wa2Cas12i, xCas12i, hfCas12Max, Cas12i-Max (addgene plasmid #188276), Cas12i1 D647A (addgene plasmid #171671), Cas12i-HiFi (addgene plasmid #188269), Cas12i1 D647A, Cas12j3 (addgene plasmid #188497), Cas12j2 (addgene plasmid #188498), AsCas12j-2 (addgene plasmid #191655), Cas12j-8 (addgene plasmid #194966), ShCas12k, N7Cas12k, AcCas12k, Cas12k-TniQ (addgene plasmid #181787), Cas12k-TnsC (addgene plasmid #181789), Cas12l, MmCas12m, MmCas12m ΔZF (H549A, C552A), dCas12m-ΔZF (D485A, H549A, C552A), AcCas12n, dAcCas12n(D240), TnpB Actinomadura_cellulosilytica_strain_DSM_45823, TnpB Actinomadura_namibiensis_strain_DSM_44197, TnpB Actinomadura_umbrina_strain_DSM_43927_$, TnpB Actinoplanes_lobatus_strain_DSM_43150 (TnpB-1 and TnpB-2), TnpB Alicyclobacillus_macrosporagiidus_strain_DSM_17980, TnpB Haloactinospora_alba_Strain_DSM_45015, TnpB Lipingzhangella_halophila_strain_DSM_102030, TnpB Meiothermus_Silvanus_DSM_9946, TnpB QNFX01000004, ISDra2 TnpB(PDB: 8H1J), KraIscB-1, AwaIscB, OgeuIscB, GtFz1 (from Guillardia theta), SpuFz1 (fromSpizellomyces punctatus), NlovFz2 (from Percolozoa Naegleria lovaniensis), and MmeFz2 (from Mercenaria mercenaria), fragments thereof, and mutations thereof or fragments of the mutationss. In some embodiments, the DNA-binding polypeptide is able to cleave one strand of a double-stranded nucleic acid molecule (e.g., a double-stranded DNA molecule). Alternatively, the DNA-binding polypeptide is an RNA-guided nuclease with nickase activity. In some embodiments, the Cas enzyme cleaves a target strand of a double-stranded nucleic acid molecule, which means that the Cas enzyme cleaves the strand that is base-paired (complementary) with gRNA (e.g., sgRNA) bound to the Cas enzyme. In some embodiments, the nucleic acid-binding polypeptide is an RNA-guided nuclease with inactivated nuclease activity. In some embodiments, the nucleic acid-binding polypeptide is a completely inactivated mutant relative to nuclease activity of the wild-type RNA-guided nuclease, such as a dCas enzyme, which comprises but is not limited to Cas9, Cas12, Cas13, TnpB, IscB, IsrB, and Fancor with completely inactivated nuclease activity (which are referred to as dead Cas9, dead Cas12, dead Cas13, dead TnpB, dead IscB, dead IsrB, and dead Fancor); or fragments or mutations thereof, such as a SpRYs Cas9 mutant. In some embodiments of the present disclosure, the nucleic acid-binding polypeptide is a partially inactivated mutant relative to nuclease activity of a wild-type RNA-guided nuclease, such as an nCas enzyme, e.g., a polypeptide fragment that retains the nuclease activity for cleavage of a single strand of double-stranded DNA, which comprises but is not limited to nickase Cas9 and nickase Cas12.

Some embodiments of the present disclosure provide a guide polynucleotide comprising (i) a DR sequence having at least 50% sequence identity to the sequence shown in any one of SEQ ID NO: 54-583 or 704, and (ii) a guide sequence engineered to hybridize to a target nucleic acid. The DR sequence is linked to the guide sequence, and the guide polynucleotide forms a complex with a Cas12 protein, and guide sequence-specific binding of the complex to the target nucleic acid.

In some embodiments of the present disclosure, the DR sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the sequence shown in any one of SEQ ID NO: 54-583 or 704.

In some embodiments of the present disclosure, the DR sequence has at least 60% sequence identity to the sequence shown in any one of SEQ ID NO: 54-583 or 704.

In some embodiments of the present disclosure, the DR sequence has at least 65% sequence identity to the sequence shown in any one of SEQ ID NO: 54-583 or 704.

In some embodiments of the present disclosure, the DR sequence has at least 70% sequence identity to the sequence shown in any one of SEQ ID NO: 54-583 or 704.

In some embodiments of the present disclosure, the DR sequence has at least 75% sequence identity to the sequence shown in any one of SEQ ID NO: 54-583 or 704.

In some embodiments of the present disclosure, the DR sequence has at least 80% sequence identity to the sequence shown in any one of SEQ ID NO: 54-583 or 704.

In some embodiments of the present disclosure, the DR sequence has at least 85% sequence identity to the sequence shown in any one of SEQ ID NO: 54-583 or 704.

In some embodiments of the present disclosure, the DR sequence has at least 90% sequence identity to the sequence shown in any one of SEQ ID NO: 54-583 or 704.

In some embodiments of the present disclosure, the DR sequence has at least 95% sequence identity to the sequence shown in any one of SEQ ID NO: 54-583 or 704.

In some embodiments of the present disclosure, the DR sequence has at least 96% sequence identity to the sequence shown in any one of SEQ ID NO: 54-583 or 704.

In some embodiments of the present disclosure, the DR sequence has at least 97% sequence identity to the sequence shown in any one of SEQ ID NO: 54-583 or 704.

In some embodiments of the present disclosure, the DR sequence has at least 98% sequence identity to the sequence shown in any one of SEQ ID NO: 54-583 or 704.

In some embodiments of the present disclosure, the DR sequence has at least 100% sequence identity to the sequence shown in any one of SEQ ID NO: 54-583 or 704.

In some embodiments, the Cas12 protein is a Cas12 protein as described herein.

In some embodiments, the guide sequence comprises 15-60 nucleotides. In some embodiments, the guide sequence comprises 15-50 nucleotides. In some embodiments, the guide sequence comprises 15-40 nucleotides. In some embodiments, the guide sequence comprises 15-35 nucleotides. In some embodiments, the guide sequence comprises 15-30 nucleotides. In some embodiments, the guide sequence comprises 15-25 nucleotides. In some embodiments, the guide sequence comprises 18-25 nucleotides. In some embodiments, the guide sequence comprises 20-25 nucleotides. In some embodiments, the guide sequence comprises 18-22 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides.

In some embodiments, the guide sequence hybridizes to the target nucleic acid, and the guide sequence is 90%-100% complementary to the target nucleic acid.

In some embodiments, the guide sequence hybridizes to the target nucleic acid.

In some embodiments, the guide sequence hybridizes to the target nucleic acid, and the guide sequence is mismatched to the target nucleic acid by no more than one nucleotide.

In some embodiments, the DR sequence comprises 15-100 nucleotides. In some embodiments, the DR sequence comprises 15-90 nucleotides. In some embodiments, the DR sequence comprises 15-80 nucleotides. In some embodiments, the DR sequence comprises 15-70 nucleotides. In some embodiments, the DR sequence comprises 15-60 nucleotides. In some embodiments, the guide sequence comprises 15-50 nucleotides. In some embodiments, the guide sequence comprises 15-40 nucleotides. In some embodiments, the guide sequence comprises 20-40 nucleotides. In some embodiments, the guide sequence comprises 20-30 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides.

In some embodiments, the guide sequence is located at the 3' end of the DR sequence.

In some embodiments, the guide sequence is located at the 5' end of the DR sequence.

In some embodiments, the guide polynucleotide further comprises tracrRNA.

In some embodiments of the present disclosure, the tracrRNA sequence has at least 50% sequence identity to any one of the sequences shown in SEQ ID NO: 584-695. In some embodiments of the present disclosure, the tracrRNA sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to any one of the sequences shown in SEQ ID NO: 584-695.

In some embodiments of the present disclosure, the tracrRNA sequence is selected from the sequences shown in SEQ ID NO: 584-695.

In some embodiments, the tracrRNA is complementarily paired with the DR sequence. In general, the complementary pairing is a complementary pairing for partial bases. In some embodiments, the tracrRNA interacts with the DR sequence.

In some embodiments, the tracrRNA sequence is linked to the DR sequence. In some embodiments, the tracrRNA sequence is linked to the DR sequence by a nucleotide sequence. In some embodiments, the tracrRNA sequence is linked to the DR sequence by a nucleotide sequence including 1-10 nucleotides. In some embodiments, the tracrRNA sequence is linked to the DR sequence by a nucleotide sequence including 1, 2, 3, 4, 5, 6, 7, 8, 9, or 10 nucleotides. In some embodiments, the tracrRNA sequence is linked to the DR sequence by a nucleotide sequence including 4 nucleotides. In some embodiments, the tracrRNA sequence is linked to the DR sequence by a 5'-GAAA-3' linker.

In some embodiments, the tracrRNA sequence is located at the 3' end of the DR sequence.

In some embodiments, the tracrRNA sequence is located at the 5' end of the DR sequence.

In some embodiments, the tracrRNA comprises 10-200 nucleotides. In some embodiments, the tracrRNA comprises 10-190, 10-180, 10-170, 10-160, 10-150, 10-140, 10-130, 10-120, 10-110, 10-100, 10-90, 10-80, 10-70, 10-60, 10-50, 10-40, 10-30, 10-20, 20-100, 30-100, 40-100, 20-90, 20-80, 20-70, 20-60, 20-50, or 30-50 nucleotides. In some embodiments, the tracrRNA comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 nucleotides.

SEQ ID NO: 1-53 shows the amino acid sequence of the Cas protein.

SEQ ID NO: 54-583 shows the DR sequence corresponding to the Cas protein. When more than one DR sequences corresponding to a particular Cas protein are listed, any one DR sequence may be used.

SEQ ID NO: 584-695 shows the tracrRNA sequence corresponding to the Cas protein. When SEQ ID NO: 584-695 does not list the tracrRNA sequence corresponding to a specific Cas protein, then gRNA may comprise only the guide sequence and the DR sequence, and do not comprise the tracrRNA sequence. When SEQ ID NO: 584-695 lists the tracrRNA sequence corresponding to the particular Cas protein, then the gRNA may comprise the guide sequence and the DR sequence and optionally comprise the tracrRNA sequence. When more than one tracrRNA sequences corresponding to the particular Cas protein are listed, any one of the tracrRNA sequences may be selected for use.

Some embodiments of the present disclosure provide an inactivated Cas12 mutant. The inactivated Cas12 mutant is a nuclease-inactivated mutant of the Cas12 protein as described in the present disclosure.

In the present disclosure, depending on the context, a reference scope of the Cas12 protein may encompass the inactivated Cas12 mutant. However, given the importance of the inactivated Cas12 mutant (non-limiting examples including that the inactivated Cas12 mutant is fused with a deaminase for single-base editing, fused with a transcriptional activation domain or a transcriptional repression domain for transcription regulation, etc.), the inactivated Cas12 mutant may be described separately and in detail herein; which does not imply that the reference scope of the Cas12 protein necessarily excludes the inactivated Cas12 mutant.

In some embodiments, the inactivated Cas12 mutant is a mutant in which the nuclease activity is completely inactivated, i.e., a dead Cas12 mutant (dCas12). The dCas12 only binds the target nucleic acid under the mediation of the guide polynucleotide and has no or negligible cleavage activity against the target nucleic acid. For example, a target nucleic acid cleavage efficiency of the dCas12 is no more than 20%, 15%, 10%, 5%, 4%, 3%, 2%, or 1% of the target nucleic acid cleavage efficiency of the Cas12 protein before inactivating mutation.

In some embodiments, the inactivated Cas12 mutant is a mutant in which the nuclease activity is partially inactivated. Further, the mutant with partially inactivated nuclease activity is a nickase Cas12 (nCas12), which binds the target nucleic acid under mediation of the guide polynucleotide, and then cleaves one single strand of the double-stranded target nucleic acid without cleaving the other single strand.

In some embodiments, the inactivated Cas12 mutant is a Cas12 protein with an inactivated Ruvc domain.

In some embodiments, the inactivated Cas12 mutant is a Cas12 protein with an inactivated Ruvc-I, Ruvc-II, or Ruvc-III domain.

In some embodiments, the inactivated Cas12 mutant is obtained by introducing the inactivating mutation into the Ruvc-I, Ruvc-II, or Ruvc-III domain of the Cas12 protein.

In some embodiments, the inactivating mutation is selected from one or more of D651A, E891A, and D1082A corresponding to the amino acid sequence as shown in SEQ ID NO: 696.

In some embodiments, the inactivating mutation is D651A, E891A, and D1082A corresponding to the amino acid sequence shown in SEQ ID NO: 696.

In some embodiments, a PAM sequence recognized by the inactivated Cas12 mutant is the same as the PAM sequence recognized by the Cas12 protein.

In some embodiments, the PAM sequence (5'→3') recognized by the inactivated Cas12 mutant is selected from any one or more of the following: A, C, T, G, TA, TC, GN, AA, AG, TG, AN, GG, CG, TN, NT, NG, GT, NA, CC, AC, GC, AT, CT, GA, TT, CN, NC, CA, NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG, NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, and NNGC.

N is A, T, C, or G.

Some embodiments of the present disclosure provide a fusion protein or conjugate. The fusion protein or conjugate comprises: (1) the Cas12 protein as described herein, or the inactivated Cas12 mutant as described herein; and (2) a homologous or heterologous functional domain.

In the present disclosure, depending on the context, the reference scope of the Cas12 protein may encompass the inactivated Cas12 mutant. However, given the importance of the inactivated Cas12 mutant (non-limiting examples including the fusion of the inactivated Cas12 mutant with the deaminase for single base editing, fusion with a transcriptional activation domain or a transcriptional repression domain for transcriptional regulation, etc.), the inactivated Cas12 mutant is described separately and in detail herein, which does not imply that the reference scope of the Cas12 protein necessarily excludes the inactivated Cas12 mutant.

In some embodiments of the present disclosure, a fusion protein is provided. The fusion protein comprises (1) the Cas12 protein as described herein, or the inactivated Cas12 mutant as described herein; and (2) the homologous or heterologous functional domain.

In some embodiments of the present disclosure, a fusion protein is provided. The fusion protein comprises (1) the Cas12 protein as described herein; and (2) the homologous or heterologous functional domain.

In some embodiments, a conjugate is provided. The conjugate comprises (1) the Cas12 protein as described herein, or the inactivated Cas12 mutant as described herein; and (2) the homologous or heterologous functional domain.

In some embodiments, a conjugate is provided. The conjugate comprises (1) the Cas12 protein as described herein; and (2) the homologous or heterologous functional domain.

In some embodiments, the functional domain has an enzyme activity for modifying the target nucleic acid sequence; the enzyme activity comprising a nuclease activity, a methyltransferase activity, a demethylase activity, a DNA repair activity, a DNA damage activity, a deamination activity, a dismutase activity, an alkylation activity, a depurination activity, an oxidation activity, a pyrimidine dimer formation activity, an integrase activity, a transposase activity, a recombinase activity, a polymerase activity, a ligase activity, a helicase activity, a photolyase activity, a glycosylase activity, a deglycosylation activity, an acetyltransferase activity, a deacetylase activity, a kinase activity, a phosphatase activity, a ubiquitin ligase activity, a deubiquitination activity, an adenylylation activity, a deadenylation activity, a SUMOylating activity, a deSUMOylating activity, a myristoylation activity, and/or a demyristoylation activity.

In some embodiments, the inactivating mutation is selected from one or more of D651A, E891A, and D1082A corresponding to the amino acid sequence shown in SEQ ID NO: 696.

In some embodiments, the inactivating mutation is D651A, E891A, and D1082A corresponding to the amino acid sequence as shown in SEQ ID NO: 696.

In some embodiments, the functional domain is selected from one or more of the following: a nuclease (e.g., FokI), a DNA methyltransferase, a DNA demethylase, a histone methyltransferase, a histone demethylase, a histone acetylase domain, a histone deacetylase domain, a DNA repair enzyme, a DNA damage enzyme, a deaminase, a dismutase, an alkylase, a depurinase, an oxidase, a pyrimidine dimer-forming enzyme, an integrase, a transposase, a recombinase, a polymerase, a ligase, a helicase, a photolyase, a glycosylase, a deglycosylase, an acetyltransferase, a deacetylase, a kinase, a phosphatase, a ubiquitin ligase, a deubiquitinating enzyme, an adenylylase, a deadenylase, a SUMOylating enzyme, a deSUMOylating enzyme, a myristoylase, and/or a demyristoylase.

In some embodiments, the homologous or heterologous functional domain is selected from any one, two, three, four, or more of the following: a subcellular positioning signal, a DNA binding domain, a protease domain, a transcriptional activation domain, a transcriptional repression domain, a nuclease domain, a deaminase domain, a uracil DNA glycosylase domain (UDG), a uracil DNA glycosylase inhibitory domain (UGI), a DNA methyltransferase, a DNA demethylase, a histone methyltransferase, a histone demethylase, a transcription release factor, a histone acetylase domain, a histone deacetylase domain, a DNA ligase, an affinity tag, a reporter tag, an affinity domain, and a reporter domain.

In some embodiments, the homologous or heterologous functional domain comprises a transcriptional repressor. In some embodiments, the homologous or heterologous functional domain comprises a DNA methyltransferase. In some embodiments, the homologous or heterologous functional domain comprises a histone methyltransferase. In some embodiments, the homologous or heterologous functional domain comprises a histone domain. In some embodiments, the homologous or heterologous functional domain is selected from one, two, three, or more of the following: a transcriptional repressor, a DNA methyltransferase, a histone methyltransferase, and a histone domain.

In some embodiments, the transcriptional repressor is randomly selected from: Kruppel-associated Box (KRAB), Enhancer of Zeste Homolog 2 (EZH2), Zinc Finger Protein 57 (ZFP57), Zinc Finger Protein 445 (ZNF445), Tripartite Motif Containing 28 (TRIM28, also known as KAP1), Methyl-CpG Binding Protein (MeCP, such as MeCP2), Sin3 Interaction Domain (SID), Tandem Repeat of Sin3 Interaction Domain (SID4X), Methyl-CpG Binding Domain Protein 2 (MBD2), Methyl-CpG Binding Domain Protein 3 (MBD3), DNA Methyltransferase 1 (DNMT1), DNA Methyltransferase 3 Alpha (DNMT3A), DNA Methyltransferase 3 Beta (DNMT3B), RE1-Silencing Transcription Factor (REST), Neuron-Restrictive Silencer Factor (NRSF, alias for REST), TGF-β-Inducible Early Gene (TIEG, also known as KLF10), Corepressor of REST (CoREST), G9a (Euchromatic Histone-Lysine N-Methyltransferase 2, also known as EHMT2), Suppressor of Variegation 3-9 Homolog 1 (SUV39H1), SET Domain, Bifurcated 1 (SETDB1), Histone Deacetylase 1 (HDAC1), Histone Deacetylase 2 (HDAC2), Histone Deacetylase 3 (HDAC3), Silencing Mediator for Retinoid and Thyroid Hormone Receptors (SMRT), Nuclear Receptor Corepressor (NCoR), ERG-Associated Protein with SET Domain (ESET, also referred to as SETDB1), Zinc Finger and BTB Domain Containing 33 (ZBTB33, also known as KAISO), Viral Oncogene Homolog of Thyroid Hormone Receptor (v-ERB-A), Transducin Beta-Like 1 (TBL1), Transducin Beta-Like Related 1 (TBLR1), Lysine-Specific Histone Demethylase 1A (LSD1, also known as KDM1A), C-terminal Binding Protein 1 (CtBP1), C-terminal Binding Protein 2 (CtBP2), Arabidopsis Histone Deacetylase 2A (AtHD2A), BCL6 Corepressor (BCOR), Mesoderm Induction Early Response 1 (MIER1), Zinc Finger Protein 10 (ZNF10), Zinc Finger Protein 91 (ZNF91), Zinc Finger Protein 809 (ZFP809), Bromo Adjacent Homology Domain Containing 1 (BAHD1), Retinoblastoma Binding Protein 4 (RBBP4), Retinoblastoma Binding Protein 7 (RBBP7), Heterochromatin Protein 1 Alpha (HP1α, also known as CBX5), Heterochromatin Protein 1 Beta (HP1β, also known as CBX1), Chromobox Protein Homolog 3 (CBX3), Chromobox Protein Homolog 7 (CBX7), PR Domain Zinc Finger Protein 1 (PRDM1, also known as BLIMP-1), PR Domain Zinc Finger Protein 14 (PRDM14), Nuclear Receptor Corepressor 2 (NCOR2, also known as SMRT), Metastasis Associated 1 (MTA1), Metastasis Associated 2 (MTA2), Metastasis Associated 3 (MTA3), Chromodomain Helicase DNA Binding Protein 4 (CHD4), and Lysine Demethylase 5B, also known as JARID1B (KDM5B).

In some embodiments, the DNA methyltransferase is selected from DNMT3A, DNMT3B, DNMT3L, DRM, UHRF1, DNMT1, dim-2, M.Sssl, M.Pvull (N4C), DMS3, and T4Dam (N6C).

In some embodiments, the DNA methyltransferase is selected from DNA (Cytosine-5)-Methyltransferase 1 (DNMT1), DNA (Cytosine-5)-Methyltransferase 3 Alpha (DNMT3A), DNA (Cytosine-5)-Methyltransferase 3 Beta (DNMT3B), DNA (Cytosine-5)-Methyltransferase 3-Like (DNMT3L), tRNA Aspartic Acid Methyltransferase 1 (DNMT2), Ubiquitin-like with PHD and RING Finger Domains 1 (UHRF1), Ubiquitin-like with PHD and RING Finger Domains 2 (UHRF2), Helicase, Lymphoid Specific (HELLS, also known as LSH), Cell Division Cycle Associated 7 (CDCA7), Nuclear Protein 95 (NP95, the obsolete name of UHRF1), tRNA aspartic acid methyltransferase 1 (TRDMT1, alias for DNMT2), DNA (Cytosine-5)-Methyltransferase 3C (DNMT3C, a new type of DNA methyltransferase unique to mice, used for transposon silencing in male germ cells), and KIAA1429 (also known as VIRMA, which regulates m6A but may be related to methylation regulation). In some embodiments, the DNA methyltransferase is selected from Methyltransferase 1 (MET1, functionally analogous to mammalian DNMT1), Chromomethylase 3 (CMT3, mediating CHG site methylation), Chromomethylase 2 (CMT2, mediating CHH methylation), Domains Rearranged Methyltransferase 1 (DRM1), and Domains Rearranged Methyltransferase 2 (DRM2, functionally analogous to mammalian DNMT3, mediating de novo methylation). In some embodiments, the DNA methyltransferase is selected from Repeat-Induced Point mutation defective protein (RID, involved in RIP mutation and DNA methylation) and Methyltransferase associated with sexual cycle 1 (Masc1). In some embodiments, the DNA methyltransferase is selected from CpG-specific DNA methyltransferase from Spiroplasma species (M.SssI, intended to be used as a model enzyme for specifically methylation of CpG sites), DNA adenine methyltransferase (Dam, an adenine methyltransferase for methylation of GATC sites, commonly used in prokaryotic regulation research), and DNA cytosine methyltransferase (Dcm, a cytosine methyltransferase for specifically methylation of the CCWGG sequence).

In some embodiments, the histone domain is selected from a histone H3 domain, a histone H2 domain, and a histone H4 domain. In some embodiments, the histone domain is selected from a histone H3 tail domain, a histone H2 tail domain, and a histone H4 tail domain.

In some embodiments of the present disclosure, the subcellular positioning signal is selected from a nuclear localization signal, a nuclear export signal, a mitochondrial localization signal, and a chloroplast localization signal.

In some embodiments, the fusion protein or conjugate comprises 1, 2, 3, 4, 5, 6, 7, 8, 9, or more homologous or heterologous functional domains, and the functional domains are identical or different.

In some embodiments, the fusion protein or conjugate connects 0, 1, 2, 3, 4, 5, 6, 7, 8, or more functional domains at the N-terminal and/or C-terminal of the Cas12 protein.

In some embodiments, the fusion protein comprises 1, 2, 3, 4, or more nuclear positioning signals.

In some embodiments, the fusion protein is used to achieve base editing, such as in conjunction with the guide polynucleotide to achieve the base editing. In some embodiments, the fusion protein comprises the nuclear positioning signal and the deaminase domain.

In some embodiments, the fusion protein comprises the nuclear positioning signal, a cytidine deaminase domain, and optionally 1 or 2 UGI domains. The fusion protein is used to achieve C→T base editing of the target nucleic acid.

In some embodiments, the fusion protein comprises the nuclear positioning signal and the adenosine deaminase domain. The fusion protein is used to achieve A→G base editing of the target nucleic acid.

In some embodiments, the fusion protein comprises the nuclear positioning signal, the cytidine deaminase domain, and the adenosine deaminase domain. In some embodiments, the fusion protein comprises 1, 2, or 3 nuclear positioning signals, and the deaminase domain. In some embodiments, the fusion protein comprises the UGI domain. In some embodiments, the fusion protein comprises 1, 2, or 3 nuclear positioning signals, the deaminase domain, and 1 or 2 UGI domains.

In some embodiments, the fusion protein is used to achieve the transcriptional activation of a specific target gene, such as in conjunction with the guide polynucleotide for achieving the transcriptional activation of the specific target gene. In some embodiments, the fusion protein comprises the nuclear positioning signal and the transcriptional activation domain.

In some embodiments, the fusion protein is used to achieve the transcriptional repression of a specific target gene, such as in conjunction with the guide polynucleotide for achieving the transcriptional repression of the specific target gene. In some embodiments, the fusion protein comprises the nuclear positioning signal and the transcriptional repression domain.

In some embodiments, the fusion protein is used to achieve methylation of a specific target sequence, such as in conjunction with the guide polynucleotide for achieving methylation of the specific target sequence. In some embodiments, the fusion protein comprises the nuclear positioning signal and the DNA methylation domain.

In some embodiments, the fusion protein is used to achieve demethylation of a specific target sequence, such as in conjunction with the guide polynucleotide for achieving demethylation of the specific target sequence. In some embodiments, the fusion protein comprises the nuclear positioning signal and the DNA demethylation domain.

In some embodiments, the nuclease domain comprises a polypeptide with an ssDNA cleavage activity and/or a polypeptide with a dsDNA cleavage activity.

In some embodiments, the nuclease domain comprises a polypeptide with an ssDNA cleavage activity.

In some embodiments, the nuclease domain comprises a polypeptide with a dsDNA cleavage activity.

In some embodiments, the Cas12 protein or inactivated Cas12 mutant is directly or indirectly linked to the homologous or heterologous functional domain.

In some embodiments, the direct linkage is a covalent linkage, and the indirect linkage is a linkage through an amino acid linker or a non-amino acid linker.

In some embodiments, the homologous or heterologous functional domain is fused or conjugated at the N-terminal, C-terminal, or internally with respect to the Cas12 protein or inactivated Cas12 mutant.

In the present disclosure, the fusion protein is obtained by connecting (1) to (2) through a peptide linker or directly connecting (1) to (2); and the conjugate is obtained by connecting (1) to (2) through a non-peptide chemical bond.

In some embodiments, the PAM sequence recognized by the fusion protein or the conjugate is the same as the PAM sequence recognized by the Cas12 protein.

In some embodiments, the PAM sequence (5'→3') recognized by the fusion protein or conjugate is selected from any one or more of the following: A, C, T, G, TA, TC, GN, AA, AG, TG, AN, GG, CG, TN, NT, NG, GT, NA, CC, AC, GC, AT, CT, GA, TT, CN, NC, CA, NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG, NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, and NNGC.

N is A, T, C, or G.

In some embodiments, a PAM sequence recognized by the fusion protein is 5'-TTN-3'.

In some embodiments, a PAM sequence recognized by the fusion protein is 5'-TTNC-3'.

In some embodiments, a PAM sequence recognized by the fusion protein is 5'-WTN-3'.

In some embodiments, a PAM sequence recognized by the fusion protein is 5'-ATN-3'.

In some embodiments, a PAM sequence recognized by the fusion protein is 5'-TTN-3'.

In some embodiments, a PAM sequence recognized by the fusion protein is 5'-TTNC-3'.

In some embodiments, a PAM sequence recognized by the fusion protein is 5'-WTN-3'.

In some embodiments, a PAM sequence recognized by the fusion protein is 5'-ATN-3'.

Some embodiments of the present disclosure provide an isolated nucleic acid. The nucleic acid encodes the Cas12 protein, the inactivated Cas12 mutant, or the fusion protein or conjugate as described herein.

In some embodiments of the present disclosure, the nucleic acid encodes the Cas12 protein or the fusion protein as described herein.

In some embodiments, the nucleic acid is codon optimized for expression in cells.

In some embodiments, the nucleic acid is codon optimized for expression in a eukaryote, a mammal such as a human or non-human mammal, a plant, an insect, a bird, a reptile, a rodent (e.g., a mouse, a rat), a fish, a worm/nematode, or a yeast.

Some embodiments of the present disclosure provide a CRISPR-Cas12 system. In some embodiments, the CRISPR-Cas12 system comprises:
a. the Cas12 protein, the inactivated Cas12 mutant, the fusion protein or conjugate, or the isolated nucleic acid as described herein; and
b. a guide polynucleotide, or a polynucleotide sequence encoding the guide polynucleotide.

The Cas12 protein, the inactivated Cas12 mutant, or the fusion protein or conjugate forms a complex with the guide polynucleotide; and the guide polynucleotide comprises a guide sequence engineered to guide a sequence-specific binding of the complex to the target nucleic acid.

The isolated nucleic acid encodes the Cas12 protein, the inactivated Cas12 mutant, or the fusion protein or conjugate as described herein.

In some embodiments, the guide polynucleotide comprises a DR sequence linked to a guide sequence.

In some embodiments, the DR sequence has at least 50% sequence identity to the sequence shown in any one of SEQ ID NO: 54-583 or 704.

In some embodiments, the guide polynucleotide comprises a DR sequence linked to a guide sequence. Further, in some embodiments, the DR sequence has at least 50% sequence identity to the sequence shown in any one of SEQ ID NO: 54-583 or 704. In some embodiments, the DR sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in any one of SEQ ID NO: 54-583 or 704. Further, in some embodiments, the DR sequence comprises or is the sequence shown in any one of SEQ ID NO: 54-583 or 704.

In some embodiments, the guide sequence comprises 15-60 nucleotides. In some embodiments, the guide sequence comprises 15-50 nucleotides. In some embodiments, the guide sequence comprises 15-40 nucleotides. In some embodiments, the guide sequence comprises 15-35 nucleotides. In some embodiments, the guide sequence comprises 15-30 nucleotides. In some embodiments, the guide sequence comprises 15-25 nucleotides. In some embodiments, the guide sequence comprises 18-25 nucleotides. In some embodiments, the guide sequence comprises 20-25 nucleotides. In some embodiments, the guide sequence comprises 18-22 nucleotides. In some embodiments, the guide sequence comprises 20-22 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, or 40 nucleotides.

In some embodiments, the guide sequence hybridizes to the target nucleic acid, and the guide sequence is 90%-100% complementary to the target nucleic acid.

In some embodiments, the guide sequence hybridizes to the target nucleic acid.

In some embodiments, the guide sequence hybridizes to the target nucleic acid, and the guide sequence is mismatched to the target nucleic acid by no more than one nucleotide.

In some embodiments, the DR sequence comprises 15-100 nucleotides. In some embodiments, the DR sequence comprises 15-90 nucleotides. In some embodiments, the DR sequence comprises 15-80 nucleotides. In some embodiments, the DR sequence comprises 15-70 nucleotides. In some embodiments, the DR sequence comprises 15-60 nucleotides. In some embodiments, the guide sequence comprises 15-50 nucleotides. In some embodiments, the guide sequence comprises 15-40 nucleotides. In some embodiments, the guide sequence comprises 20-40 nucleotides. In some embodiments, the guide sequence comprises 20-30 nucleotides. In some embodiments, the guide sequence comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 nucleotides.

In some embodiments, the guide sequence is located at the 3' end of the DR sequence.

In some embodiments, the guide sequence is located at the 5' end of the DR sequence.

In some embodiments, the guide polynucleotide further comprises the tracrRNA.

In some embodiments of the present disclosure, the tracrRNA sequence has at least 50% sequence identity to the sequence shown in any one of SEQ ID NO: 584-695. In some embodiments of the present disclosure, the tracrRNA sequence has at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to the sequence shown in any one of SEQ ID NO: 584-695.

In some embodiments, the tracrRNA is complementarily paired with the DR sequence. In general, the complementary pairing is complementary pairing for partial bases. In some embodiments, the tracrRNA interacts with the DR sequence.

In some embodiments, the tracrRNA sequence is linked to the DR sequence. In some embodiments, the tracrRNA sequence is linked to the DR sequence by a nucleotide sequence. In some embodiments, the tracrRNA sequence is linked to the DR sequence by the nucleotide sequence including 1-10 nucleotides. In some embodiments, the tracrRNA sequence is linked to the DR sequence by the nucleotide sequence including 1, 2, 3, 4, 5, 6, 7, 8, 9, or 10 nucleotides. In some embodiments, the tracrRNA sequence is linked to the DR sequence by the nucleotide sequence including 4 nucleotides. In some embodiments, the tracrRNA sequence is linked to the DR sequence by a 5'-GAAA-3' sequence.

In some embodiments, the tracrRNA sequence is located at the 3' end of the DR sequence.

In some embodiments, the tracrRNA sequence is located at the 5' end of the DR sequence.

In some embodiments, the tracrRNA comprises 10-200 nucleotides. In some embodiments, the tracrRNA comprises 10-190, 10-180, 10-170, 10-160, 10-150, 10-140, 10 -130, 10-120, 10-110, 10-100, 10-90, 10-80, 10-70, 10-60, 10-50, 10-40, 10-30, 10-20, 20-100, 30-100, 40-100, 20-90, 20-80, 20-70, 20-60, 20-50, or 30-50 nucleotides. In some embodiments, the tracrRNA comprises 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 nucleotides.

In some embodiments, the guide polynucleotide is the guide polynucleotide as described herein.

In some embodiments, the target nucleic acid is DNA or RNA. In some embodiments, dsDNA or ssDNA.

In some embodiments, the DNA is the eukaryotic DNA. In some embodiments, the eukaryotic DNA is non-human mammalian DNA, non-human primate DNA, human DNA, plant DNA, insect DNA, bird DNA, reptile DNA, rodent DNA, fish DNA, worm/nematode DNA, or yeast DNA.

In some embodiments, the target nucleic acid is a disease or a condition-related gene or a signaling biochemical pathway-related gene, or the target nucleic acid is a reporter gene. For example, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.

In some embodiments, the target nucleic acid is a gene as listed in Table 27.

In some embodiments, the target nucleic acid is a disease or disorder related gene, the disease or disorder being selected from: hemophilia A, Best yolk-like macular dystrophy, B-cell acute lymphoblastic leukemia, hemophilia B, CDKL5 deficiency, CLN2 disease, Niemann-Pick disease type C, Dravet syndrome, FOXG1 syndrome, GM1ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5 deficiency, X-linked myotubular myopathy, X-linked retinoschisis, X-linked retinitis pigmentosa, α1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Bietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1, spinal muscular atrophy, Tay-Sachs disease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase deficiency, Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune deficiency, heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin (ATTR) amyloidosis, alpha-1-antitrypsin deficiency (AATD) liver disease, and AATD lung disease.

In some embodiments, genes associated with ATTR amyloidosis comprise, but are not limited to, *ATTR.*

Genes associated with Leber hereditary optic neuropathy comprise, but are not limited to, *MT-ND4.*

Genes associated with the AATD liver disease comprise, but are not limited to, *AATD.*

Genes associated with the AATD lung disease comprise, but are not limited to, *AATD.*

Genes associated with the graft-versus-host disease comprise, but are not limited to, thymidine kinase genes.

Genes associated with hereditary retinal dystrophy comprise, but are not limited to, *RPE65.*

Genes associated with spinal muscular atrophy comprise, but are not limited to, *SMN1.*

Genes associated with osteoarthritis comprise, but are not limited to, *TGF-β1.*

Genes associated with hemophilia A comprise, but are not limited to, *factor VIII.*

Genes associated with hemophilia B comprise, but are not limited to, *factor IX*

Genes associated with cystic fibrosis comprise, but are not limited to, *CFTR.*

Genes associated with Parkinson's disease comprise, but are not limited to, *Gad1, Gad2, PTBP1, KEAP1, RE1, Amigo1, Gprc5c, Let-7a, Pnky, LRRK2, SNCA, GBA, miR-92b, miR-9, miR-124, miR-181, HMGB1, TRIM72, GPNMB,* and *REST.*

Genes associated with Usher syndrome comprise, but are not limited to, *USH2A.*

Genes associated with α-thalassemia, β-thalassemia, and sickle cell disease comprise, but are not limited to, *BCL11A, HBG, HBA,* and *HBB.*

Genes associated with pulmonary hypertension comprise, but are not limited to, *eNOS.*

Genes associated with Stargardt's disease comprise, but are not limited to, *ABCA4.*

Genes associated with age-related macular degeneration comprise, but are not limited to, *VEGFA, VEGFR, IL17, Kir7.1, LCN-2, IRAK-M, CD59, LTA4H, GPX4, GLS1, PAPP-A, cGAS, STING, mTOR, GCN2, Nrf2, Ang 2, CTGF,* complement C3, complement *C5, CHFR4b, DOCK6, CTSS, ELN,* and *FGF2.*

Genes associated with glaucoma comprise, but are not limited to, *AQP1, ADRB2, NMNTA2, NRP1, Hrh1, Anxa2, OPA1, Cx43, ANGPTL7, MYOC, ROCK1, ROCK2, TIMP1, TIMP2, TIMP3, TIMP4,* carbonic anhydrase *CA2,* carbonic anhydrase *CA4,* and carbonic anhydrase *CA12.*

Genes associated with idiopathic pulmonary fibrosis comprise, but are not limited to, *CTGF.*

Genes associated with hyperlipidemia comprise, but are not limited to, *PCSK9.*

Genes associated with Alzheimer's disease comprise, but are not limited to, *NGF.*

Genes associated with coronary heart disease comprise, but are not limited to, *VEGFA* and *bFGF.*

Genes associated with chronic nephrogenic anemia comprise, but are not limited to, *EPO.*

Genes associated with congenital amaurosis comprise, but are not limited to, *RPE65.*

Genes associated with retinitis pigmentosa comprise, but are not limited to, *PDE6B.*

Genes associated with phenylketonuria comprise, but are not limited to, *PAH.*

Genes associated with epilepsy comprise, but are not limited to, *GAT1.*

Some embodiments of the present disclosure provide a vector system. The vector system comprises one or more recombinant vectors. The recombinant vectors comprise the isolated nucleic acid or the CRISPR-Cas12 system as described herein.

In some embodiments, the recombinant vector further comprises a regulatory sequence.

In some embodiments, the vector system comprises one or more recombinant vectors comprising a polynucleotide sequence encoding the Cas12 protein, the inactivated Cas12 mutant, or the fusion proteins or conjugate as described herein and a polynucleotide sequence encoding the guide polynucleotide.

In some embodiments, the polynucleotide sequence encoding the Cas12 protein, the inactivated Cas12 mutant, or the fusion protein or conjugate is operably linked to the regulatory sequence 1.

In some embodiments, the polynucleotide sequence encoding the guide polynucleotide is operably linked to the regulatory sequence 2.

Further, in some embodiments, the regulatory sequence 1 is the same as or different from the regulatory sequence 2.

In some embodiments, the regulatory sequence is optionally selected from one or more of: a promoter, an enhancer, an internal ribosome entry site, and a transcription termination signal. The promoter comprises a constitutive promoter, an inducible promoter, a broad-spectrum promoter, or a tissue-specific promoter, and/or the transcriptional termination signal comprises a polyadenylation signal or a poly-U sequence.

In some embodiments, a scaffold of the one or more recombinant vectors is an adeno-associated virus vector, a lentiviral vector, or a virus-like particle.

In some embodiment of the present disclosure, when the scaffold is the adeno-associated virus vector, the adeno-associated virus vector is a recombinant adeno-associated virus vector of serotype AAV1, AAV2, AAV4, AAV5, AAV6, AAV7, AAVrh74, AAV8, AAV9, AAV10, AAV11, AAV12, or AAV13; when the scaffold is the lentiviral vector, the lentiviral vector is pseudotyped with an envelope protein; in some embodiments, the isolated nucleic acid is linked to an aptamer sequence; and when the scaffold is the virus-like particle, the isolated nucleic acid is linked to a gene encoding a gag protein.

Some embodiments of the present disclosure provide a delivery system. The delivery system comprises (1) a delivery tool, and (2) the Cas12 protein, the guide polynucleotide, the inactivated Cas12 mutant, the fusion protein or conjugate, the isolated nucleic acid, the CRISPR-Cas12 system, or the vector system as described herein.

In some embodiments, the delivery tool is a virus, a lipid nanoparticle, a nanoparticle, a liposome, an exosome, a microbubble, or a gene gun.

In some embodiments, the delivery tool is the lipid nanoparticle comprising the guide polynucleotide and mRNA encoding the Cas12 protein, the inactivated Cas12 mutant, or the fusion protein or conjugate.

Some embodiments of the present disclosure provide a cell comprising the Cas12 protein, the guide polynucleotide, the inactivated Cas12 mutant, the fusion protein or conjugate, the isolated nucleic acid, the CRISPR-Cas12 system, or the vector system as described herein.

In some embodiments of the present disclosure, the cell is a prokaryotic cell.

In some embodiments of the present disclosure, the cell is a eukaryotic cell.

In some embodiments of the present disclosure, the eukaryotic cell is a mammalian cell.

Some embodiments of the present disclosure provide a pharmaceutical composition, wherein the pharmaceutical composition comprises the Cas12 protein, the guide polynucleotide, the inactivated Cas12 mutant, the fusion protein or conjugate, the nucleic acid, the CRISPR-Cas12 system, the vector system, the delivery system, or the cell as described herein.

In some embodiments, the pharmaceutical composition further comprises pharmaceutically acceptable excipients.

Some embodiments of the present disclosure provide a kit, wherein the kit comprises the Cas12 protein, the guide polynucleotide, the inactivated Cas12 mutant, the fusion protein or conjugate, the isolated nucleic acid, the CRISPR-Cas12 system, the vector system, the delivery system, or the cell as described herein.

In some embodiments, the kit further comprises a cut buffer. The cut buffer is any buffer known in the art suitable for cleaving the target nucleic acid by the Cas12 protein.

Some embodiments of the present disclosure provide a use of the Cas12 protein, the guide polynucleotide, the inactivated Cas12 mutant, the fusion protein or conjugate, the nucleic acid, the CRISPR-Cas12 system, the vector system, the delivery system, the cell, the pharmaceutical composition, or the kit as described herein in preparing a reagent or medicament for diagnosing, treating, or preventing a disease or disorder associated with a target nucleic acid.

In some embodiments, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease. In some embodiments, the reagent or medicament is used to: cleave one or more target nucleic acid molecules or introduce nicks into the one or more target nucleic acid molecules, activate or upregulate an expression of the one or more target nucleic acid molecules, activate or inhibit transcription of the one or more target nucleic acid molecules, inactivate the one or more target nucleic acid molecules, visualize, label, or detect the one or more target nucleic acid molecules, bind the one or more target nucleic acid molecules, transport the one or more target nucleic acid molecules, and mask the one or more target nucleic acid molecules.

In some embodiments, the target nucleic acid is optionally selected from the genes as listed in Table 27, and the disease or disorder is the disease or disorder as listed in Table 27.

In some embodiments, the disease or disorder is selected from: hemophilia A, Best yolk-like macular dystrophy, B-cell acute lymphoblastic leukemia, hemophilia B, CDKL5deficiency, CLN2 disease, Niemann-Pick disease type C, Dravet syndrome, FOXG1syndrome, GM1 ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5deficiency, X-linked myotubular myopathy, X-linked retinoschisis, X-linked retinitis pigmentosa, α1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Bietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1,spinal muscular atrophy, Tay-Sachs disease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase deficiency, Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune deficiency, heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin (ATTR) amyloidosis, alpha-1-antitrypsin deficiency (AATD) liver disease, and AATD lung disease.

In some embodiments, genes associated with the ATTR amyloidosis comprise, but is not limited to, *ATTR.*

Genes associated with the Leber hereditary optic neuropathy comprise, but are not limited to, *MT-ND4.*

Genes associated with the AATD liver disease comprise, but are not limited to, *AATD.*

the genes associated with the AATD lung disease comprise, but are not limited to, *AATD.*

Genes associated with the graft-versus-host disease comprise, but are not limited to, thymidine kinase genes.

Genes associated with the hereditary retinal dystrophy comprise, but are not limited to, *RPE65.*

Genes associated with the spinal muscular atrophy comprise, but are not limited to, *SMN1.*

Genes associated with the osteoarthritis comprise, but are not limited to, *TGF-β1.*

Genes associated with the hemophilia A comprise, but are not limited to, *factor VIII.*

Genes associated with the hemophilia B comprise, but are not limited to, *factor IX.*

Genes associated with the cystic fibrosis comprise, but are not limited to, *CFTR.*

Genes associated with the Parkinson's disease comprise, but are not limited to, *Gad1, Gad2, PTBP1, KEAP1, RE1, Amigo1, Gprc5c, Let-7a, Pnky, LRRK2, SNCA, GBA* gene, *miR-92b, miR-9, miR-124, miR-181, HMGB1, TRIM72, GPNMB,* and *REST.*

Genes associated with Usher syndrome comprise, but are not limited to, *USH2A.*

Genes associated with α-thalassemia, β-thalassemia, and sickle cell disease comprise, but are not limited to, *BCL11A, HBG, HBA,* and *HBB.*

Genes associated with the pulmonary hypertension comprise, but are not limited to, *eNOS.*

Genes associated with the Stargardt's disease comprise, but are not limited to, *ABCA4.*

Genes associated with the age-related macular degeneration comprise, but are not limited to, *VEGFA, VEGFR, IL17, Kir7.1, LCN-2, IRAK-M, CD59, LTA4H, GPX4, GLS1, PAPP-A, cGAS, STING, mTOR, GCN2, Nrf2, Ang 2, CTGF,* complement *C3*, complement *C5, CHFR4b, DOCK6, CTSS, ELN,* and *FGF2.*

Genes associated with the glaucoma comprise, but are not limited to, *AQP1, ADRB2, NMNTA2, NRP1, Hrh1, Anxa2, OPA1, Cx43, ANGPTL7, MYOC, ROCK1, ROCK2, TIMP1, TIMP2, TIMP3, TIMP4,* carbonic anhydrase *CA2,* carbonic anhydrase *CA4,* and carbonic anhydrase *CA12.*

Genes associated with the idiopathic pulmonary fibrosis comprise, but are not limited to, *CTGF.*

Genes associated with cardiovascular diseases such as hyperlipidemia comprise, but are not limited to, *PCSK9*(Proprotein Convertase Subtilisin/Kexin Type 9).

Genes associated with the Alzheimer's disease comprise, but are not limited to, *NGF.*

Genes associated with the coronary heart disease comprise, but are not limited to, *VEGFA* and *bFGF.*

Genes associated with the chronic nephrogenic anemia comprise, but are not limited to, *EPO.*

Genes associated with the congenital amaurosis comprise, but are not limited to, *RPE65.*

Genes associated with the retinitis pigmentosa comprise, but are not limited to, *PDE6B.*

Genes associated with the phenylketonuria comprise, but are not limited to, *PAH.*

Genes associated with the epilepsy comprise, but are not limited to, *GAT1.*

Some embodiments of the present disclosure provide a method for detecting, binding, or cleaving a target nucleic acid, comprising: using the Cas12 protein, the guide polynucleotide, the inactivated Cas12 mutant, the fusion protein or conjugate, the nucleic acid, the CRISPR-Cas12 system, the vector system, the delivery system, the cell, the pharmaceutical composition, or the kit as described herein to contact the target nucleic acid.

In some embodiments, the method is for non-diagnostic and/or non-therapeutic purposes; and/or the fusion protein or conjugate comprises a detectable marker, such as a marker detectable by fluorescence, DNA blotting, or FISH.

In some embodiments, when the method is for cleaving the target nucleic acid, the method further comprises performing a cleavage reaction using a cut buffer. The cut buffer may be any buffer known in the art suitable for cleaving the target nucleic acid by the Cas12 protein.

Some embodiments of the present disclosure provide is a method for altering a cell state, comprising using the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein to contact the cell to alter a cell state.

In some embodiments of the present disclosure, the method results in one or more of: an increase or decrease in an expression of a specific gene, an induction of cellular senescence in vitro or in vivo, an induction of cellular cycle arrest in vitro or in vivo, a cellular growth promotion and/or a cellular growth inhibition in vitro or in vivo, an induction of anergy in vitro or in vivo, an induction of apoptosis in vitro or in vivo, and an induction of necrosis in vitro or in vivo.

In some embodiments, the method is for non-diagnostic and/or non-therapeutic purposes.

Some embodiments of the present disclosure provide a method for diagnosing, treating, or preventing a disease or disorder associated with a target nucleic acid, comprising applying the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit as described herein to a sample from a subject in need or the subject in need.

In some embodiments, the target nucleic acid is optionally selected from genes as listed in Table 27, and the disease or disorder is the disease or disorder as listed in Table 27.

In some embodiments, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.

Some embodiments of the present disclosure provide a use of the Cas12 protein, the guide polynucleotide, the inactivated Cas12 mutant, the fusion proteins or conjugate, the isolated nucleic acid, the CRISPR-Cas12 system, the vector system, the delivery system, the cell, the pharmaceutical composition, or the kit as described herein in diagnosing, treating, or preventing a disease or disorder associated with the target nucleic acid.

In some embodiments, the target nucleic acid is optionally selected from genes as listed in Table 27, and the disease or disorder is the disease or disorder as listed in Table 27.

In some embodiments, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.

On the basis of conforming to the common knowledge in the field, the above preferred conditions may be arbitrarily combined, thereby obtaining the preferred embodiments of the present disclosure.

### BRIEF DESCRIPTION OF THE DRAWINGS

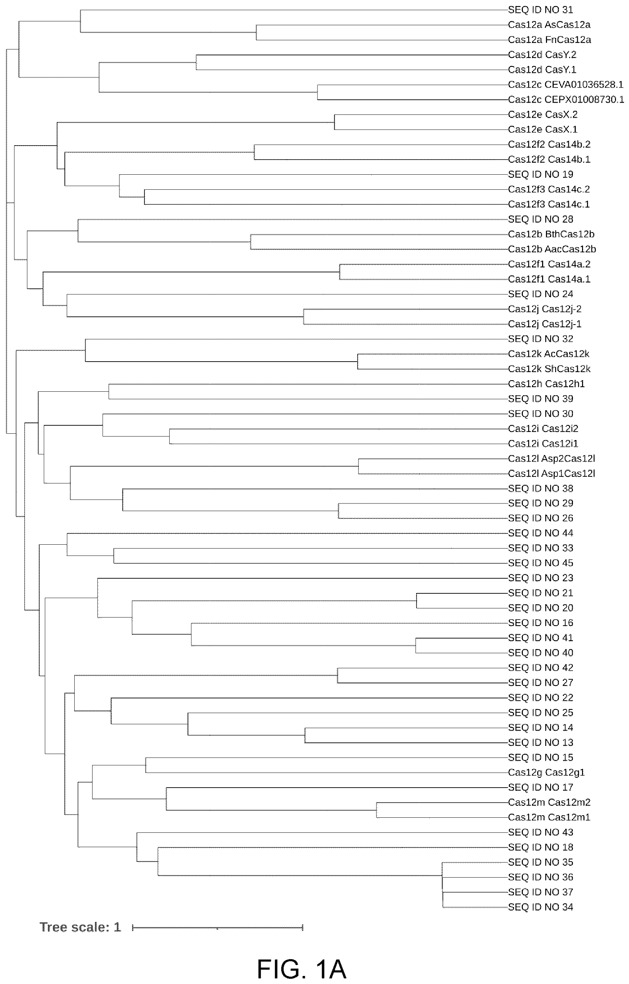

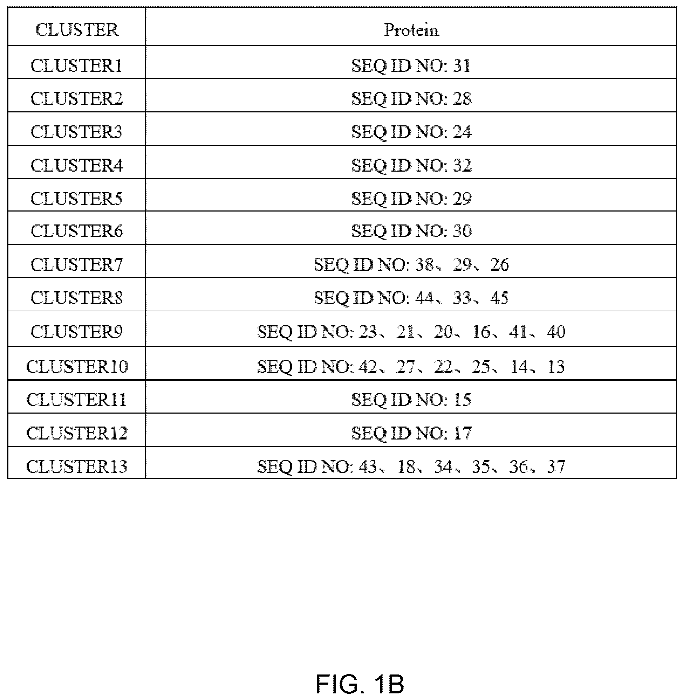

FIG. 1A is a diagram illustrating an evolutionary relationship of Cas proteins and known Cas12 isoform proteins (performing sequence alignment using MAFFT, then constructing an evolutionary tree using FastTree) according to embodiments of the present disclosure, and FIG. 1B shows the proteins according to some embodiments of the present disclosure;
   In the evolutionary tree, some proteins of the present disclosure form a separate and distinctly separated branch (a different cluster [CLUSTER]) compared to the known Cas12 isoform proteins, i.e., the proteins of the present disclosure are not mixed with the known Cas12 isoform proteins; and evalue of the alignment between these proteins in the present disclosure (FIG. 1B) and the existing Cas12 HMM Profile model is greater than 1e-5. These proteins of the present disclosure comprise CLUSTER1-CLUSTER13 proteins shown in FIG. 1B. Overall, this suggests that these Cas proteins may be novel subgroups, e.g., new Cas12 isoform proteins;
FIG. 2 is an SDS-PAGE electrophoresis pattern of a C12-279 recombinant protein according to some embodiment of the present disclosure;
FIG. 3 shows a PAM library for in vitro cleavage by Cas12 protein according to some embodiments of the present disclosure, and sequences in FIG. 3 are shown in SEQ ID NO: 878-881;
FIG. 4 shows a motif identified after C12-279-sgRNA targeting a 7nt random sequence according to some embodiments of the present disclosure;
FIG. 5 shows a motif identified after C12-279-sgRNA-Rev targeting a 7nt random sequence according to some embodiments of the present disclosure;
FIG. 6 shows fragments of plasmids containing 7nt random sequences in a plasmid elimination assay according to some embodiments of the present disclosure, and the sequences in FIG. 6 are shown in SEQ ID NO: 720 and SEQ ID NO: 882-886;
FIG. 7 shows a motif identified in the plasmid elimination assay for C12-279 according to some embodiments of the present disclosure;
FIG. 8 shows a partial indel result generated after C12-279 editing TTR genes according to some embodiments of the present disclosure, and the sequences in the FIG. 8 are shown in SEQ ID NO: 722 and SEQ ID NO: 887-916;
FIG. 9 shows that a CI1062732 protein (SEQ ID NO: 46) with dozens of amino acid residues deletion at an N-terminal of the C12-279 protein (SEQ ID NO: 696), in combination with a gRNA containing the DR sequence (GTAATGCGTCTCCCATTGACGCC) (SEQ ID NO: 529), targets a 7nt random sequence plasmid library in bacteria, grabbing a "false" PAM motif of 5'-TTNC-3.
FIG. 10 is a diagram of a secondary structure of a "false" DR sequence according to some embodiments of the present disclosure, hypothesizing that the 3'-end base C of the identified "false" PAM motif TTNC is caused by an extra C at the 3' end of the DR, and the sequences in FIG. 10 are shown in SEQ ID NO: 917 and SEQ ID NO: 918.
FIG. 11 is an SDS-PAGE electrophoresis pattern of a C12-101-07 recombinant protein (126 KDa) according to some embodiments of the present disclosure;
FIG.12 shows a motif identified after C12-101-07-sgRNA targeting a 7nt random sequence according to some embodiments of the present disclosure;
FIG. 13 shows a motif identified after C12-101-07-sgRNA-Rev targeting a 7nt random sequence according to some embodiments of the present disclosure;
FIG. 14 shows a motif identified in a plasmid elimination assay for C12-101-09 according to some embodiments of the present disclosure;
FIG. 15A shows fragments of pCDH-CMV-EGFP-reporter3-EF1-Puro plasmid according to some embodiments of the present disclosure; the sequences in FIG. 15A are shown in SEQ ID NO: 919-921; FIG. 15B shows fragments of a plasmid library with a PAM sequence of NAAN according to some embodiments of the present disclosure; and the sequences in FIG. 15B are shown in SEQ ID NO: 922-924;
FIG. 16 shows the editing efficiency of different C12-279 mutants in NAAN cells, expressed as multiples of the editing efficiency of C12-279, according to some embodiments of the present disclosure;
FIG. 17 shows the editing efficiency of different C12-279 mutants in NAAN cells, expressed as multiples of the editing efficiency of C12-279, according to some embodiments of the present disclosure;

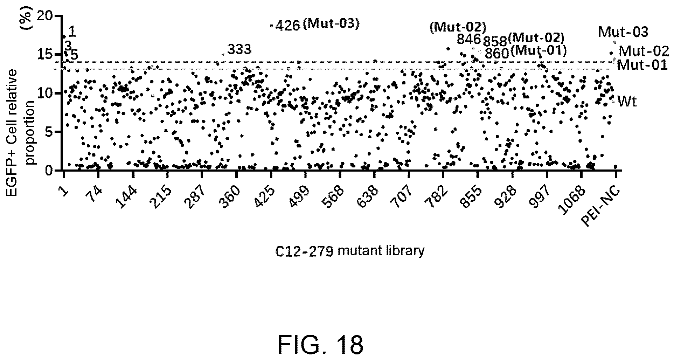

FIG. 18 shows editing efficiency test results of different mutants targeting a reporter system according to some embodiments of the present disclosure, where Wt denotes a wild type C12-279; the marked number n indicates that the amino acid residue at position n is mutated to arginine R; Mut-01 is a mutant C12-279-pCDH-05 (Q186R mutant), Mut-02 is a mutant C12-279-pCDH-28 (double point mutation of Q186R and D352R), and Mut-03 is a mutant C12-279-pCDH-35 (triple point mutation of G184R, Q186R, and D352R); a lower dashed line indicates a 50% increase in editing efficiency compared to the wild type C12-279; an upper dashed line indicates an editing efficiency equal to the editing efficiency of Mut-02;
FIG. 19 shows editing efficiency test results of different multipoint mutants in the reporter system according to some embodiments of the present disclosure; where Mut-02-1-5-426-858-860 denotes a multipoint mutant obtained by additionally introducing mutations at positions 1, 5, 426, 858, and 860 (all mutate to arginine R) based on the Mut-02 mutant, i.e., the multipoint mutant contains the following mutations: amino acid residues at positions 1, 5, 186, 352, 426, 858, and 860 are all mutated to R; and other mutants are similar;

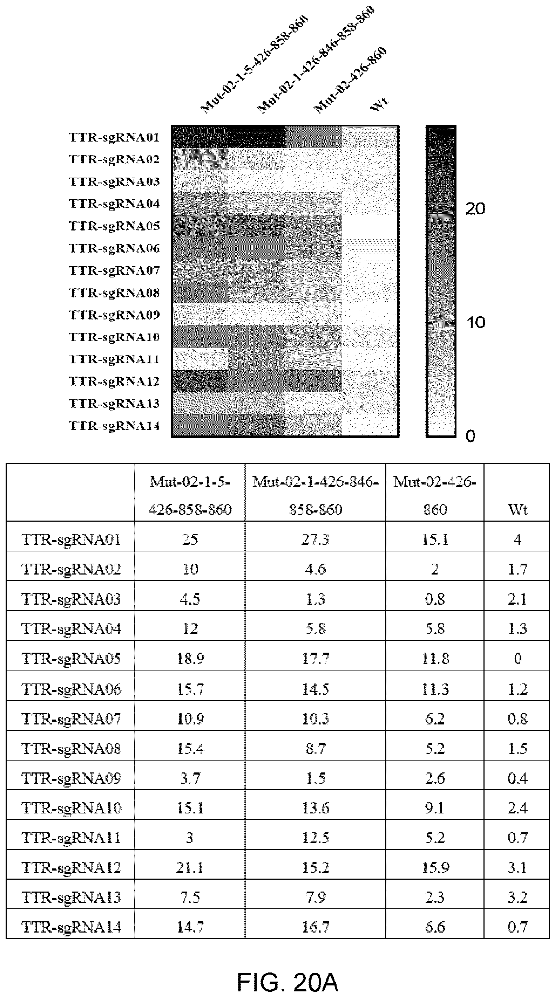

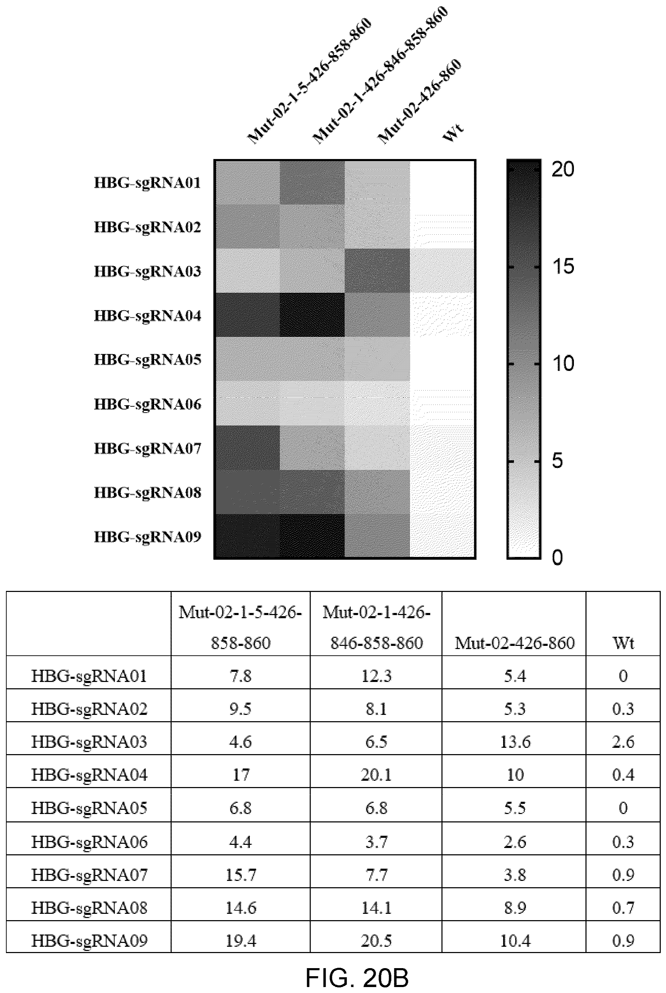

FIG. 20A shows an efficiency of multipoint mutants combined with different gRNAs in editing *TTR* gene according to some embodiments of the present disclosure; FIG. 20B shows an efficiency of multipoint mutants combined with different gRNAs in editing *HBG* gene according to some embodiments of the present disclosure; where Mut-02-1-5-426-858-860 denotes a multipoint mutant obtained by additionally introducing mutations at positions 1, 5, 426, 858, and 860 (all mutated to arginine R) based on the Mut-02 mutant, i.e., the multipoint mutant contains the following mutations: all amino acid residues at positions 1, 5, 186, 352, 426, 858, and 860 are mutated to R; and other mutants are similar;
FIG. 21 shows editing activity test results of dCas12-279 according to some embodiments of the present disclosure; where Mut-02-426-860 denotes a multipoint mutant obtained by additionally introducing 426R and 860R mutations based on the Mut-02 mutant; Mut-02-426-860-D651A denotes a multipoint mutant obtained by additionally introducing 426R, 860R, and 651A mutations based on the Mut-02 mutant; and other mutants are similar;
FIG. 22 shows NGS sequencing results after the coding mRNA of the mutant Mut-02-1-5-426-858-860 is combined with the modified gRNA (C279-dmTTR01-02) for electroporation of HEK293 cells and editing of *TTR* gene, with an editing efficiency up to 92.18%, according to some embodiments of the present disclosure; and the sequences in FIG. 22 are shown as SEQ ID NO: 925-974;
FIG. 23 shows a PAM recognized by C12-279 mutant Mut-02-1-426-846-858-860 according to some embodiments of the present disclosure; and
FIG. 24 is a schematic diagram illustrating a structure of C12-279 according to some embodiments of the present disclosure.

### DETAILED DESCRIPTION

In the present disclosure, scientific and technical terms used herein have the meanings commonly understood by those of skill in the art unless otherwise indicated. Additionally, the procedures involving molecular genetics, nucleic acid chemistry, chemistry, molecular biology, biochemistry, cell culture, microbiology, cell biology, genomics, and recombinant DNA, as used herein, are all standard techniques widely employed in their respective fields. At the same time, for better understanding of the present disclosure, definitions and explanations of relevant terms are provided below.

In the present disclosure, the term "several" refers to a quantity greater than or equal to 2. In the present disclosure, the term "multiple" refers to a quantity greater than or equal to 2.

In the present disclosure, depending on the context, the term "cleavage" refers to cutting of a main chain of a polynucleotide chain; and non-limiting examples include complete cleavage of a single-stranded DNA, cleavage of one strand of a double-stranded DNA, or cleavage of both strands of a double-stranded DNA.

In the present disclosure, depending on the context, the term "modification" refers to other forms of chemical reactions of nucleic acid strands other than "cleavage". It includes, but is not limited to, base substitution, addition, and/or deletion, as well as methylation and demethylation of nucleic acid strands. Non-limiting examples include base substitution on a target nucleic acid strand through single-base editing (e.g., the Cas12 of the present disclosure is fused with a deaminase domain and combined with gRNA), such as A→G, C→T, T→C, or G→A nucleotide mutations, as well as other types of nucleotide mutations (e.g., A→T, C→G, T→A, G→C, etc.). Other examples include base substitution, addition, or deletion through Prime editing technology (e.g., the Cas12 of the present disclosure is fused with a reverse transcriptase and combined with pegRNA), or base substitution, addition, or deletion through homology-directed repair (HDR) (e.g., the Cas12 of the present disclosure is combined with gRNA and a donor template). In addition, the Cas12 of the present disclosure is also fused with a DNA methyltransferase or a DNA demethylase and combined with gRNA for targeted modification.

In the present disclosure, depending on the context, the term "modulating an expression of a target nucleic acid" refers to modulation of the transcription of the target nucleic acid. Non-limiting examples include enhancing or suppressing the transcription of the target nucleic acid using CRISPRa or CRISPRi technologies, by means of a transcriptional activation or repression domain fused to Cas12.

In the present disclosure, letters in amino acid sequences denote single-letter abbreviations for amino acids well known in the art, as described in J. Biol. Chem, 243, p3558 (1968*),* Alanine: Ala-A, Arginine: Arg-R, Aspartic acid: Asp-D, Cysteine: Cys-C, Glutamine: Gln-Q, Glutamic acid: Glu-E, Histidine: His-H, Glycine: Gly-G, Asparagine: Asn-N, Tyrosine: Tyr-Y, Proline: Pro-P, Serine: Ser-S, Methionine: Met-M, Lysine: Lys-K, Valine: Val-V, Isoleucine: Ile-I, Phenylalanine: Phe-F, Leucine: Leu-L, Tryptophan: Trp-W, and Threonine: Thr-T.

In the present disclosure, the term "amino acid difference" refers to the difference of amino acid residues at specific positions in the protein's amino acid sequence, including substitution, addition, or deletion.

In the present disclosure, a mutation of an amino acid residue at a specific position refers to the substitution, addition, or deletion of the amino acid residue at that position.

It is well known to those skilled in the art that in proteins or peptides, two adjacent amino acids each lose an OH or H through dehydration condensation to form a peptide bond, and each amino acid exists in the form of an amino acid residue. Thus, in the present disclosure, the terms "amino acid" and "amino acid residue" refer to the same meaning. Further, in the present disclosure, to simplify the expression, the amino acid residue before the substitution is retained before the position of the amino acid residue; the letter before the position indicates the original amino acid residue, and the letter after the position indicates the substituted amino acid residue. For example, "S211" represents that the original amino acid residue at position 211 is S, and when it is substituted with R, it may be expressed as "S211R".

In the present disclosure, the symbol "+" is sometimes used to connect one amino acid mutation on each side, indicating that both point mutations are present simultaneously in a single mutant; if two or more point mutations are connected by two or more "+", it represents that these point mutations are present simultaneously.

In the present disclosure, if an amino acid is substituted, it refers to that it is substituted with another amino acid residue different from the original amino acid residue. If the original amino acid is a positively charged amino acid and is substituted with a positively charged amino acid, it refers to that it is substituted with another positively charged amino acid residue different from the original one. For example, if an original amino acid residue is R and is substituted with a positively charged amino acid, it refers to that it is substituted with H or K.

In the present disclosure, when referring to an "RNA sequence", "T" in the sequence is used interchangeably with "U". When referring to a "guide sequence", "T" in the sequence is used interchangeably with "U". When referring to a "direct repeat (DR) sequence", "T" in the sequence is used interchangeably with "U".

In the present disclosure, when referring to the numbering of a Cas protein, C12-n and Cas12-n refer to the same protein. For example, Cas12-279 and C12-279 are used interchangeably.

### Sequence identity

As used herein, the term "identity" refers to a sequence matching degree between two polypeptides or between two nucleic acids. The terms "identity", "percent identity", and "sequence identity" are used interchangeably. When a given position in two compared sequences are occupied by the same base or amino acid monomeric subunit (for example, if the same position in each of two DNA molecules is occupied by adenine, or the same position in each of two polypeptides is occupied by lysine), the molecules are considered to be identical at that position. The percent identity between two sequences is calculated by a function: the number of matching positions shared by the two sequences/the total number of compared positions×100%. For example, if there are 6 matching positions in 10 positions of two sequences, then the two sequences have 60% sequence identity. Typically, the alignment is performed by aligning the two sequences to generate the maximum sequence identity. Such alignment may be performed by using published and commercially available alignment algorithms and programs, including but not limited to CLUSTER Ω, MAFFT, Probcons, T-Coffee, Probalign, and BLAST, which may be reasonably selected and used by one of ordinary skill in the art. Those skilled in the art can determine appropriate parameters for sequence alignment, including any algorithm required to achieve an optimal or best alignment for the full length of the compared sequences, as well as any algorithm required to achieve an optimal or best alignment for the local region of the compared sequences.

### CRISPR-Cas12 system

As used herein, the terms "clustered regularly interspaced short palindromic repeats (CRISPR)-CRISPR-Cas system" or "CRISPR system" are used interchangeably and have the meanings commonly understood by those of skill in the art, which generally comprise transcription products or other elements associated with the expression of a CRISPR-associated (Cas) gene or transcription products or other elements capable of guiding the activity of the Cas gene. Such transcription products or other elements may comprise sequences encoding Cas effector proteins and guide polynucleotides.

*Zhang Feng* et al. discovered Cas12a in 2015, categorized as type V in the Class II CRISPR-Cas system. After detailed studies of subtype V-A (Cas12a), *Zhang Feng* et al. reported Cas12b (C2C1) in 2015. In 2017, *Burstein* et al. reported the Cas12e (CasX) nuclease. In 2019, *Winston X. Yan* et al. reported the newly discovered type V Cas effector proteins Cas12c, Cas12h, Cas12i, and Cas12g in detail by bioinformatics analysis.

In some embodiments, a Cas12 protein as described herein refers to a protein having an amino acid sequence, the amino acid sequence having at least 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% sequence identity to any one of sequences shown in SEQ ID NO: 1-53, SEQ ID NO: 696, or SEQ ID NO: 728. When the CRISPR-Cas12 system comprises a fusion protein or a conjugate comprising the Cas12 protein and a functional domain, a percent sequence identity between the Cas12 portion of the fusion protein or the conjugate and a reference sequence is calculated.

In the present disclosure, the CRISPR-Cas12 system comprises the Cas12 protein with the amino acid sequence having at least 50% sequence identity to any one of sequences shown in SEQ ID NO: 1-53, SEQ ID NO: 696, and SEQ ID NO: 728, or a nucleic acid encoding the Cas12 protein; and a guide polynucleotide or a nucleic acid encoding the guide polynucleotide; the guide polynucleotide comprises a DR sequence linked to a guide sequence, the guide sequence is engineered to hybridize with a target nucleic acid, and the guide polynucleotide is capable of forming a complex with the Cas12 protein and guiding the sequence-specific binding of the complex to the target nucleic acid.

### Guide polynucleotide

As used herein, the term "guide polynucleotide" refers to a molecule in a CRISPR-Cas system that forms a complex with the Cas protein and guides the complex to a target sequence. Typically, the guide polynucleotide comprises a scaffold sequence that is linked to a guide sequence, and the guide sequence may hybridize to the target sequence. Typically, the scaffold sequence comprises a DR sequence, and sometimes, the scaffold sequence comprises a tracrRNA sequence. In some embodiments, the guide polynucleotide does not comprise a tracrRNA sequence. In some embodiments, the guide polynucleotide comprises a tracrRNA sequence.

In some embodiments, the guide polynucleotide of the CRISPR-Cas12 system is a guide RNA. In some embodiments, the guide polynucleotide is a chemically modified guide polynucleotide. In some embodiments, the guide polynucleotide comprises at least one chemically modified nucleotide.

In some embodiments, the chemically modified nucleotide comprises a base-modified nucleotide, a phosphate-modified nucleotide, and a ribose-modified nucleotide.

In some embodiments, the base-modified nucleotide is selected from nucleotides containing non-natural bases.

In some embodiments, the phosphate-modified nucleotide is selected from an aminophosphate nucleotide, a phosphorothioate nucleotide, a dithiophosphate nucleotide, a methylphosphonate nucleotide, a 5'-phosphate nucleotide, an alkyl phosphate nucleotide, and a borane phosphate nucleotide.

In some embodiments, the ribose-modified nucleotide is selected from a deoxynucleotide, a 3'-terminal deoxythymidine (dT) nucleotide, a 2'-O-methyl-modified nucleotide, a 2'-fluoro-modified nucleotide, a 2'-deoxy-modified nucleotide, a 2'-amino-modified nucleotide, a 2'-O-allyl-modified nucleotide, a 2'-C-alkyl-modified nucleotide, a 2'-hydroxy-modified nucleotide, a 2'-methoxyethyl-modified nucleotide, a 2'-O-alkyl-modified nucleotide, and a morpholino nucleotide.

In some embodiments, the base-modified nucleotide is selected from nucleotides containing non-natural bases, for example, 5-methylcytosine, 5-hydroxymethylcytosine, pseudouridine, 2,6-diaminopurine, 2-thiopurine, 7-methylguanosine, 8-bromoguanosine, 5-iodouracil, 5-bromouracil, 5-propargyluracil, 5-methyluracil, 1-methylpseudouridine, N6-methyladenosine, N6-methylthioadenosine, 2-aminopurine, isocytosine, isoguanine, and isoguanine. In some embodiments, the phosphate-modified nucleotide is selected from an aminophosphate nucleotide, a phosphorothioate nucleotide, a phosphorodithioate nucleotide, a methylphosphonate nucleotide, a 5'-phosphorylated nucleotide, an alkylphosphate nucleotide, a boranephosphonate nucleotide, a phosphoroselenoate nucleotide, a fluorophosphonate nucleotide, an allylphosphate nucleotide, a benzylphosphate nucleotide, a cyanophosphonate nucleotide, and a sulfonate-modified nucleotide. In some embodiments, the ribose-modified nucleotide is selected from deoxynucleotide, 3'-deoxythymidine nucleotide, 2'-O-methyl nucleotide, 2'-fluoro nucleotide, 2'-deoxy nucleotide, 2'-amino nucleotide, 2'-O-allyl nucleotide, 2'-C-alkyl nucleotide, 2'-hydroxy nucleotide, 2'-O-methoxyethyl (MOE) nucleotide, 2'-O-alkyl nucleotide, morpholino nucleotide (PMO), locked nucleic acid (LNA), thio-sugar nucleotide, 2'-O-methoxy nucleotide, 4'-methyl nucleotide, 3'-O-alkyl nucleotide, 3'-amino nucleotide, 4'-thionucleotide, cyclo-nucleotide, peptide nucleic acid (PNA), β-deoxyinosine nucleotide, 2'-fluoro-deoxynucleotide, 2'-protected nucleotide (for stabilizing CRISPR RNP), methylated ribose-modified nucleotide, and hydrophobic-tailed nucleotide (for cell membrane penetration).

In some embodiments, the guide polynucleotide comprises a chemically modified nucleotide at a 5' end and/or a 3' end.

In some embodiments, the guide polynucleotide comprises a deoxynucleotide at the 5' end; and the deoxynucleotide has a length in a range of 10 to 25 nt, e.g., 14 nt or 25 nt.

In some embodiments, the guide polynucleotide comprises a nucleotide with phosphorothioate group and 2'-O-methyl modifications at the 5' end or the 3' end.

In some embodiments, the guide polynucleotide comprises at least one guide sequence (or referred to as a spacer sequence) linked to at least one DR sequence. In some embodiments, the guide sequence is located at the 3' end of the DR sequence. In some embodiments, the guide sequence is located at the 5' end of the DR sequence.

In some embodiments, the tracrRNA sequence is linked to the DR sequence.

In some embodiments, the tracrRNA sequence is located at the 5' end or 3' end of the DR sequence. In some embodiments, the tracrRNA sequence is located at the 5' end of the DR sequence. In some embodiments, the tracrRNA sequence is located at the 3' end of the DR sequence.

In some embodiments, a nucleotide sequence of the guide polynucleotide comprises the tracrRNA, the DR sequence, and the guide sequence in order from the 5' end to the 3' end.

In some embodiments, the nucleotide sequence of the guide polynucleotide comprises the tracrRNA, a linker sequence, the DR sequence, and the guide sequence in order from the 5' end to the 3' end.

In some embodiments, the nucleotide sequence of the guide polynucleotide comprises the tracrRNA, a loop sequence, the DR sequence, and the guide sequence in order from the 5' end to the 3' end.

In some embodiments, a structure of the guide polynucleotide is as follows: 5'-tracrRNA-loop sequence-DR sequence-guide sequence-3'.

In some embodiments, the tracrRNA and the DR sequence of the guide polynucleotide are linked by a nucleotide sequence.

In some embodiments, the tracrRNA sequence is linked to the DR sequence by a nucleotide sequence including 1, 2, 3, 4, 5, 6, 7, 8, 9, or 10 nucleotides. In some embodiments, the tracrRNA sequence is linked to the DR sequence by a nucleotide sequence including 4 nucleotides. In some embodiments, the tracrRNA sequence is linked to the DR sequence by a 5'-GAAA-3' sequence.

In some embodiments, the guide sequence is sufficiently complementary to a target nucleic acid to hybridize with the target nucleic acid and to guide sequence-specific binding of a CRISPR-Cas12 complex to the target nucleic acid. In some embodiments, the guide sequence has 100% complementarity with the target nucleic acid, but the guide sequence may also have less than 100% complementarity with the target nucleic acid, for example, at least 80%, at least 85%, at least 90%, at least 95%, at least 98%, or at least 99% complementarity.

In some embodiments, the guide sequence is engineered to hybridize to the target nucleic acid and is mismatched to the target nucleic acid by no more than two nucleotides. In some embodiments, the guide sequence is engineered to hybridize to the target nucleic acid and is mismatched to the target nucleic acid by no more than one nucleotide. In some embodiments, the guide sequence is engineered to hybridize to the target nucleic acid and is not mismatched or is mismatched to the target nucleic acid.

In some embodiments of the present disclosure, the guide sequence has at least 50%, at least 55 %, at least 60 %, at least 65 %, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% sequence identity to any one of sequences shown in SEQ ID NO: 722, SEQ ID NO: 761-782, and SEQ ID NO: 825-877. In some embodiments of the present disclosure, the guide sequence is shown in any one of SEQ ID NO: 722, SEQ ID NO: 761-782, and SEQ ID NO: 825-877.

In some embodiments, the CRISPR-Cas12 system comprises at least 2, at least 3, at least 4, at least 5, at least 10, or at least 20 different guide polynucleotides. In some embodiments, the guide polynucleotide targets at least 2, at least 3, at least 4, at least 5, at least 10, or at least 20 different target nucleic acid molecules, or targets at least 2, at least 3, at least 4, at least 5, at least 10, or at least 20 different regions of one or more target nucleic acid molecules.

In some embodiments, the guide polynucleotide comprises a constant DR sequence located upstream of a variable guide sequence. In some embodiments, a plurality of guide polynucleotides is a portion of an array, which may be a portion of a vector, such as a viral vector or plasmid. For example, a guide array that comprises a sequence: DR sequence-spacer-DR sequence-spacer-DR sequence-spacer-...-DR sequence-spacer may comprise a plurality of unique unprocessed guide polynucleotides (one for each DR sequence-spacer or space -DR sequence). Once introduced into a cell or cell-free system, the array is processed by the Cas12 protein into several individual mature guide polynucleotides. This allows for multiplexing, such as delivering a plurality of guide polynucleotides into the cell or system to target a plurality of target nucleic acids or a plurality of regions within a single target nucleic acid.

The ability of the guide polynucleotide to guide the sequence-specific binding of the complex (a CRISPR complex) to the target nucleic acid may be assessed by any suitable assay. For example, components of the CRISPR system sufficient to form the complex (the CRISPR complex), including a guide polynucleotide to be tested, may be delivered to a host cell containing the corresponding target nucleic acid molecules, such as by transfection with a vector encoding the components of the CRISPR complex, followed by assessment of preferential cleavage within a target sequence. Similarly, cleavage of the target nucleic acid sequence may be assessed in vitro by providing the target nucleic acid and the components of the CRISPR complex including the guide polynucleotide to be tested and a control guide polynucleotide different from the guide polynucleotide to be tested, and then comparing the ability of the guide polynucleotide to be tested and the control guide polynucleotide to bind the target nucleic acid or the rate of the guide polynucleotide to be tested and the control guide polynucleotide to cleave the target nucleic acid. The ability of the CRISPR complex to cleave or bind the target nucleic acid may also be assessed by the manner described above.

### Cas12 mutant

As described herein, when referring to "a position corresponding to a sequence shown in SEQ ID NO: XX" or a similar textual description, the position may be determined by amino acid sequence alignment. Typically, the alignment is made when two sequences are aligned to produce a maximum sequence identity. Such an alignment may be performed by using published and commercially available alignment algorithms and programs such as, but not limited to, Clustal Ω, MAFFT, Probcons, T-Coffee, Probalign, and BLAST, which may be reasonably selected by one of ordinary skill in the art. One skilled in the art can determine appropriate parameters for sequence alignment, including any algorithm needed to achieve an optimal or best alignment for the full length of the compared sequences, as well as any algorithm required to achieve an optimal or best local alignment for the local region of the compared sequences.

In some embodiments, the corresponding position is determined by performing an online sequence alignment of an amino acid sequence of the Cas12 protein with any one of sequences shown in SEQ ID NO: 1-53, SEQ ID NO: 696, and SEQ ID NO: 728 using the MAFFT version 7 tool (https://mafft.cbrc.jp/alignment/server/index.html), with the following parameters: G-INS-i (Very slow; recommended for <200 sequences with global homology; 2 iterative cycles only), Try to align gappy regions anyway, Scoring matrix for amino acid sequences-BLOSUM62, Gap opening penalty 1.53, Offset value 0.0, and Mafft-homologs-Use UniRef50 (more comprehensive and requires longer search time).

In some embodiments, the corresponding position is determined by performing an online sequence alignment of the amino acid sequence of the Cas12 protein with the sequence shown in SEQ ID NO: 696 using the MAFFT version 7 tool (https://mafft.cbrc.jp/alignment/server/index.html), with the following parameters: G-INS-i (very slow; recommended for <200 sequences with global homology; 2 iterative cycles only), Try to align gappy regions anyway, Scoring matrix for amino acid sequences-BLOSUM62, Gap opening penalty 1.53, Offset value 0.0, Mafft-homologs-Use UniRef50 (more comprehensive and requires longer search time).

In some embodiments, the Cas12 protein herein comprises one or more mutations, e.g., a single amino acid insertion, a single amino acid deletion, a single amino acid substitution, or any combination thereof compared to the Cas12 protein with a sequence shown in any one of SEQ ID NO: 1-53, SEQ ID NO: 696, and SEQ ID NO: 728. In some embodiments, compared to the Cas12 protein with the sequence shown in any one of SEQ ID NO: 1-53, SEQ ID NO: 696, and SEQ ID NO: 728, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions), but retains the ability to bind to a target nucleic acid molecule that is complementary to a guide sequence of a guide polynucleotide, and/or the ability to process an RNA transcript containing a guide sequence into a guide polynucleotide molecule. In some embodiments, compared to the Cas12 protein with the sequence shown in any one of SEQ ID NO: 1-53, SEQ ID NO: 696, and SEQ ID NO: 728, the Cas12 protein comprises 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, or 130 amino acid changes (e.g., insertions, deletions, or substitutions), but retains the ability to bind a target nucleic acid molecule that is complementary to the guide sequence of the guide polynucleotide.

One type of modification or mutation comprises replacing an amino acid residue with an amino acid having similar biochemical properties, i.e., a conservative substitution. Usually, the conservative substitution has little or no effect on the activity of the resulting protein or peptide. For example, the conservation substitution refers to a substitution of an amino acid residue in the Cas12 protein that does not substantially affect the binding between the Cas12 protein and a target nucleic acid molecule that is complementary to a guide sequence of a gRNA molecule, and/or the process of processing a guide array RNA transcript into gRNA molecules.

More substantial changes may be introduced by using fewer conservative substitutions, e.g., by selecting residues that differ more significantly in maintaining the following effects: (a) the polypeptide backbone structure in the region where the substitution occurs, such as a helical or folded conformation; (b) the charge or hydrophobicity of the region interacted with the target site; or (c) the bulk of the amino acid side chain. The substitutions that are generally expected to produce the greatest changes in peptide function are (a): a substitution between hydrophilic residues (e.g., serine or threonine) and hydrophobic residues (e.g., leucine, isoleucine, phenylalanine, valine, or alanine); (b) a substitution between cysteine or proline and any other residue; (c) a substitution between residues with a positively charged side chain (e.g., lysine, arginine, or histidine) and residues with a negatively charged residue (e.g., glutamic acid or aspartic acid); or (d) a substitution between a residue having a bulky side chain (e.g., phenylalanine) and a residue not having a side chain (e.g., glycine).

### Cas12 active fragment

In the present disclosure, the Cas12 protein may comprise only a WED-I domain, a Helical-I1 domain, a PI domain, a Helical-I2 domain, a Helical-II domain, a WED-II domain, a Ruvc-I domain, a Helical-III domain, a BH domain, a Ruvc-II domain, a Nuc domain, and/or a Ruvc-III domain.

The Cas12 protein described herein, in addition to including the domains described above, may also comprise domains of the Cas12 proteins in the prior art, which together form a complete structure of the Cas12 protein to fulfill the function of the Cas12 protein described in the present disclosure. The function comprises, but is not limited to, retaining the ability of the Cas12 protein to form a complex with a gRNA, retaining the ability of the Cas12 protein to form a complex with a gRNA and target a target nucleic acid, retaining the ability of the complex formed by the Cas12 protein with the gRNA to target and modulate the expression of the target nucleic acid, retaining the ability of the complex formed by the Cas12 protein with the gRNA to target and cleave a single strand or double strands of a target nucleic acid, retaining the ability of the Cas12 protein to bind a target nucleic acid molecule that is complementary to a guide sequence of a guide polynucleotide, and/or retaining the ability to process RNA transcripts containing the guide sequence into guide polynucleotide molecules.

### Inactivated Cas12 mutant

By inactivating the RuvC domain of Cas12 through introducing point mutations, the Cas12 protein loses its endonuclease activity, resulting in a dCas12 that can only bind to a target gene under the mediation of the guide polynucleotide but does not possess the function of cleaving DNA.

Point mutations may also be introduced to partially inactivate the RuvC domain of Cas12, resulting in a nickase Cas12 (nCas12), which can bind to a target gene and cleave only one strand of the double-stranded nucleic acid under the mediation of the guide polynucleotide, while leaving the other strand intact.

Accordingly, the dCas12 or the nCas12 may be fused or conjugated with other domains (including, but not limited to, deaminase domains, transcriptional activation domains, transcriptional repression domains, methylation domains, demethylation domains, histone acetylation domains, and histone deacetylation domains), and guided to a target sequence of a target nucleic acid by the guide polynucleotide, to exert corresponding functions through the other domains. For example, the conversion of cytosine (C) to thymine (T) in the target nucleic acid is achieved by deamination of the cytosine base; the conversion of adenine (A) to guanine (G) is achieved by deamination of the adenine base; the transcriptional repression of the target nucleic acid is achieved using the transcriptional repression domain KRAB; and the transcriptional activation is promoted using the transcriptional activation domain VP64.

### Functional domain

In some embodiments, the Cas12 protein or the inactivated Cas12 mutant is covalently linked or fused to a homologous or heterologous functional domain.

In some embodiments, the functional domain has an enzyme activity that modifies a target nucleic acid sequence; the enzyme activity comprising a nuclease activity, a methyltransferase activity, a demethylase activity, a DNA repair activity, a DNA damage activity, a deamination activity, a dismutase activity, an alkylation activity, a depurination activity, an oxidation activity, a pyrimidine dimer formation activity, an integrase activity, a transposase activity, a recombinase activity, a polymerase activity, a ligase activity, a helicase activity, a photolyase activity, a glycosylase activity, a deglycosylation activity, an acetyltransferase activity, a deacetylase activity, a kinase activity, a phosphatase activity, a ubiquitin ligase activity, a deubiquitination activity, an adenylylation activity, a deadenylation activity, a SUMOylating activity, a deSUMOylating activity, a myristoylation activity, and/or a demyristoylation activity.

In some embodiments, the functional domain is selected from one or more of the following: a nuclease (e.g., FokI), a methyltransferase, a demethylase, a DNA repair enzyme, a DNA damage enzyme, a deaminase, a dismutase, an alkylase, a depurinase, an oxidase, a pyrimidine dimer-forming enzyme, an integrase, a transposase, a recombinase, a polymerase, a ligase, a helicase, a photolyase, a glycosylase, a deglycosylase, an acetyltransferase, a deacetylase, a kinase, a phosphatase, a ubiquitin ligase, a deubiquitinating enzyme, an adenylylase, a deadenylase, a SUMOylating enzyme, a deSUMOylating enzyme, a myristoylase, and/or a demyristoylase.

In some embodiments, the functional domain is selected from one, two, three, four, or more of the following: a subcellular positioning signal, a DNA binding domain, a protease domain, a transcriptional activation domain, a transcriptional repression domain, a nuclease domain, a deaminase domain, a uracil DNA glycosylase domain (UDG), a uracil DNA glycosylase inhibitory domain (UGI), a methylase, a demethylase, a transcription release factor, a histone acetylase domain, a histone deacetylase domain, a DNA ligase, an affinity tag, a reporter tag, an affinity domain, and a reporter domain.

In some embodiments of the present disclosure, the deaminase domain is selected from the following: APOBEC1, APOBEC3A, APOBEC3B, APOBEC3C, APOBEC3D, APOBEC3F, an activation-induced cytidine deaminase (AID), cytidine deaminase (CDA) from lamprey, and engineered mutants of adenosine deaminase (TadA) that act on DNA.

In some embodiments, the transcriptional activation domain is selected from the following: P65, VPR, VP16, VP64, VTR1, VTR2, VTR3, p65, MyoD1, HSF1, RTA, SET7/9, and a histone acetyltransferase. In some embodiments, the transcriptional activation domain is selected from the following: the sequence ETFSDLWKL from p53 TAD1, the sequence DDIEQWFTE from p53 TAD2, the sequence SDIMDFVLK from MLL, the sequence DLLDFSMMF from E2A, the sequence ETLDFSLVT from Rtg3, the sequence RKILNDLSS from CREB, the sequence EAILAELKK from CREBaB6, the sequence DDWQYLNS from Gli3, the sequence DDVYNYLFD from Gal4, the sequence DLFDYDFLV from Oaf1, the sequence DFFDYDLLF from Pip2, the sequence EDLYSILWS from Pdr1, and the sequence TDLYHTLWN from Pdr3.

In some embodiments, the transcriptional repression domain is selected from: KRAB domain of KOX1, KRAB domain of KAP-1, MAD, FKHR, EGR-1, ERD, SID, a tandem repeat of SID (e.g., SID4X), KRAB domain of TIEG, v-ERB-A, MBD2, MBD3, TRa, a histone methyltransferase, a histone deacetylase (HDAC), a nuclear hormone receptor (e.g., an estrogen receptor or a thyroid hormone receptor), members of the DNMT family (e.g., DNMT1, DNMT3A, DNMT3B), the KRAB domain of MeCP2, ROM2, and AtHD2A.

In some embodiments, the transcriptional repression domain is a KRAB domain. In some embodiments, the transcriptional repression domain is a KRAB domain from a KOX1 protein.

In some embodiments, the nuclease domain is selected from the following: FokI, a polypeptide with single-stranded DNA (ssDNA) cleavage activity, or a polypeptide with double-stranded DNA (dsDNA) cleavage activity.

In some embodiments, the methylase domain is selected from a DNA methylase, including, but not limited to, DNMT1, DNMT3a, and DNMT3b.

In some embodiments, the demethylase is selected from TET1CD, TET1, ROS1, DME, DML2, and DML3.

Methylation and demethylation are recognized in the field as important modes of epigenetic gene modulation.

In some embodiments, the homologous or heterologous functional domain refers to a sequence tag useful for the solubility, purification, or detection of the fusion protein or conjugate. Suitable protein tag sequences are provided in the present disclosure, which include, but are not limited to, a biotin carboxylase carrier protein (BCCP) tag, a myc tag, a calmodulin tag, a FLAG tag, a hemagglutinin (HA) tag, a polyhistidine tag (also known as His tag), a maltose-binding protein (MBP) tag, a nus tag, and a glutathione-S-transferase (GST) tag, a green fluorescent protein (GFP) tag, a thioredoxin tag, a S-tag, a Softag (e.g., Softag 1, Softag 3), a strep-tag, a biotin ligase tag, a FIAsH tag, a V5 tag, and a SBP tag. Additional suitable sequences are apparent to those of ordinary skill in the art.

In some embodiments of the present disclosure, a single-base editor is constructed by fusing a Mut-02-1-426-846-858-860-D651A-E891A-D1082A mutant with a deaminase domain and a nuclear localization signal (NLS). In some embodiments of the present disclosure, a single-base editor is constructed by fusing a Mut-02-1-426-846-858-860-D651A-E891A-D1082A mutant with an APOBEC3A domain and an SV40 NLS.

In some embodiments of the present disclosure, a transcriptional repression epigenetic editor is constructed by fusing a Mut-02-1-426-846-858-860-D651A-E891A-D1082A mutant with a KRAB domain and an SV40 NLS. In some embodiments of the present disclosure, a transcriptional activation epigenetic editor is constructed by fusing a Mut-02-1-426-846-858-860-D651A-E891A-D1082A mutant with a VP64 domain and an SV40 NLS.

### Subcellular localization signal

In some embodiments, the Cas12 protein is fused to at least one type of homologous or heterologous subcellular localization signal. In some embodiments, the Cas12 protein is fused to at least one homologous or heterologous subcellular localization signal. Exemplarily, the subcellular localization signal comprises an organelle localization signal, such as a nuclear localization signal (NLS), a nuclear export signal (NES), or a mitochondrial localization signal.

Non-limiting examples of NLS include NLS sequences derived from: an NLS of SV40 large T antigen having the amino acid sequence PKKKKRKV (SEQ ID NO: 738); an NLS of a nucleoplasmic protein (e.g., a sequence KRPAATKKKAGQAKKKKK, SEQ ID NO: 739); an NLS of c-myc having the amino acid sequence PAAKRVKLD (SEQ ID NO: 740) or the amino acid sequence RQRRNELKRSP (SEQ ID NO: 741); an NLS of hRNPA1 M9 having the amino acid sequence NQSSNFGPMKGGGNFGGRSSGPYGGGGGQYFAKPRNQGGY (SEQ ID NO: 742); a sequence RMRIZFKNKGKDTAELRRRRVEVSVELRKKAKKDEQILKRRNV (SEQ ID NO: 743) derived from an IBB domain; a sequence VSRKRPRP (SEQ ID NO: 744) and a sequence PPKKARED (SEQ ID NO: 745) of the rhabdomyosarcoma T-protein; a sequence PQPKKKPL of human p53 (SEQ ID NO: 746); a sequence SALIKKKKKKMAP (SEQ ID NO: 747) of mouse c-ablIV; a sequence DRLRR (SEQ ID NO: 748) and a sequence PKQKKRK (SEQ ID NO: 749) of influenza virus NS1; and a sequence RKLKKKKKKKL (SEQ ID NO: 750) of hepatitis virus delta antigen; a sequence REKKKKFLKRR (SEQ ID NO: 751) of mouse Mx1 protein; a sequence KRKGDEVDGVDEVAKKKSKK (SEQ ID NO: 752) of human poly(ADP-ribose) polymerase; and a sequence RKCLQAGMNLEARKTKKK (SEQ ID NO: 753) of steroid hormone receptor. In some embodiments, the nuclear localization sequence has sufficient strength to drive the accumulation of the fusion protein or conjugate described herein within the nucleus of a eukaryotic cell to a detectable level. In summary, the strength of the nuclear localization activity may be derived from a count of the NLS, one or more specific used NLSs, or any combination of these factors. The accumulation within the nucleus may be detected using any suitable technique. For example, a detectable marker may be fused to the Cas protein to allow visualization of its intracellular location, such as in combination with detection methods of nuclear location (e.g., nucleus-specific dyes such as DAPI)). As another example, the cell nucleus may be isolated from the cell, and its contents are subsequently analyzed using any appropriate method for detecting protein, including but not limited to immunohistochemistry, western blotting, or enzymatic activity assays. As another example, the accumulation within the nucleus may also be indirectly determined, for example, by assessing the effect of the formation of a nucleic acid-targeting complex (e.g., measuring DNA or RNA cleavage or mutation at a target sequence, or measuring changes in gene expression activity resulting from the formation of a DNA-targeting complex or a RNA-targeting complex and/or the activity of a DNA-targeting Cas protein or a RNA-targeting Cas protein), compared with a control group that is not exposed to a nucleic acid-targeting Cas protein or complex, or exposed to a nucleic acid-targeting Cas protein lacking one or more NLSs.

### Vector system

Some embodiments of the present disclosure relate to a vector system comprising the CRISPR-Cas12 system described herein. The vector system comprises one or more recombinant vectors, and the recombinant vector comprises a polynucleotide sequence encoding the Cas12 protein and a polynucleotide sequence encoding the guide polynucleotide.

In some embodiments, the vector system comprises at least one plasmid or viral recombinant vector (e.g., retrovirus, lentivirus, adenovirus, adeno-associated virus, or herpes simplex virus). In some embodiments, the polynucleotide sequence encoding the Cas12 protein and the polynucleotide sequence encoding the guide polynucleotide are located at the same recombinant vector. In some embodiments, the polynucleotide sequence encoding the Cas12 protein and the polynucleotide sequence encoding the guide polynucleotide are located at a plurality of recombinant vectors.

In some embodiments, the polynucleotide sequence encoding the Cas12 protein and/or the polynucleotide sequence encoding the guide polynucleotide is operably linked to a regulatory sequence (also known as a regulatory element). The regulatory element comprises a promoter, an enhancer, an internal ribosome entry site (IRES), and other expression control elements (e.g., a transcriptional termination signal such as a polyadenylation signal and a poly-U sequence). The regulatory element comprises an element that enables constitutive expression of the nucleotide sequence in many types of host cell types, as well as an element that restrict expression to specific host cells (e.g., a tissue-specific regulatory sequence). A tissue-specific promoter can be directly expressed primarily in the desired tissue of interest, e.g., muscle, neurons, bone, skin, blood, specific organs (e.g., liver, pancreas), or specific cell types (e.g., lymphocytes). The regulatory element may also guide expression in a time-dependent manner, e.g., in a cell-cycle-dependent or developmental-stage-dependent manner, which may or may not also be tissue-type specific or cell-type specific. In some embodiments, the regulatory element is enhancer elements, such as a WPRE, a CMV enhancer, an R-U5 segment in the LTR of HTLV-1, an SV40 enhancer, or an intronic sequence between exons 2 and 3 of the rabbit β-globin.

In some embodiments, the recombinant vector comprises a polymerase III (pol III) promoter (e.g., a U6 promoter and an H1 promoter), a polymerase II (pol II) promoter (e.g., the retroviral Rous sarcoma virus (RSV) long terminal repeat (LTR) promoter (optionally with an RSV enhancer), a cytomegalovirus (CMV) promoter (optionally with a CMV enhancer), an SV40 promoter, a dihydrofolate reductase promoter, a β-actin promoter, a phosphoglycerol kinase (PGK) promoter, or an EF1α promoter), or both a pol III promoter and a pol II promoter.

In some embodiments, the promoter is a constitutive promoter, which is continuously active and not modulated by external signals or molecules. Suitable constitutive promoters include, but are not limited to, CMV, RSV, SV40, EF1α, CAG, and β-actin promoters. In some embodiments, the promoter is an inducible promoter modulated by an external signal or molecule (e.g., a transcription factor).

In some embodiments, the promoter is a tissue-specific promoter, which may be used to drive tissue-specific expression of the Cas12 protein. Suitable muscle-specific promoters include, but are not limited to, CK8, MHCK7, a myoglobin (Mb) promoter, a desmin promoter, a muscle creatine kinase (MCK) promoter and mutants thereof, and an SPc5-12 synthesis promoter. Suitable immune cell-specific promoters include, but are not limited to, a B29 promoter (B cells), a CD14 promoter (monocytes), a CD43 promoter (leukocytes and platelets), a CD68 (macrophages) promoter, and an SV40/CD43 promoter (leukocytes and platelets). Suitable blood cell-specific promoters include, but are not limited to, a CD43 promoter (leukocytes and platelets), a CD45 promoter (hematopoietic cells), INF-β (hematopoietic cells), a WASP promoter (hematopoietic cells), an SV40/CD43 promoter (leukocytes and platelets), and an SV40/CD45 promoter (hematopoietic cells). Suitable pancreas-specific promoters include, but are not limited to, an elastase-1 promoter. Suitable endothelial cell-specific promoters include, but are not limited to, a Fit-1 promoter and an ICAM-2 promoter. Suitable neuronal tissue/cell-specific promoters include, but are not limited to, a GFAP promoter (astrocytes), an SYN1 promoter (neurons), and NSE/RU5' (mature neurons). Suitable kidney-specific promoters include, but are not limited to, a NphsI promoter (podocytes). Suitable bone-specific promoters include, but are not limited to, an OG-2 promoter (osteoblasts, dentinogenic cells). Suitable lung-specific promoters include, but are not limited to, an SP-B promoter (lung). Suitable liver-specific promoters include, but are not limited to, an SV40/Alb promoter. Suitable heart-specific promoters include, but are not limited to, α-MHC. In some embodiments, the tissue-specific promoter is selected from liver-specific promoters such as an albumin (ALB) promoter, an alpha-fetoprotein (AFP) promoter, a transthyretin (TTR) promoter, a hepatocyte nuclear factor 4 alpha (HNF4α) promoter, an apolipoprotein B (APOB) promoter, a carbamoyl-phosphate synthase 1 (CPS1) promoter, and a coagulation factor VII promoter (F7 promoter); a hematopoietic stem/progenitor cell (HSC)-specific promoter such as a cluster of differentiation 34 (CD34) promoter, a stem cell leukemia (SCL) promoter, a KIT proto-oncogene (c-Kit) promoter, a GATA binding protein 2 (GATA2) promoter, a LIM domain only 2 (LM02) promoter, and a runt-related transcription factor 1 (RUNX1) promoter; a nerve-specific promoter such as a synapsin I promoter, a neuron-specific enolase (NES) promoter, a tyrosine hydroxylase (TH) promoter, a glial fibrillary acidic protein (GFAP) promoter, and a myelin basic protein (MBP) promoter; muscle-specific promoters such as a muscle creatine kinase (MCK) promoter, a desmin promoter, an alpha-myosin heavy chain (α-MHC) promoter, and a myogenin promoter; immune/hematopoietic lineage-specific promoters such as a cluster of differentiation 19 (CD19) promoter, a cluster of differentiation 3 epsilon (CD3ε) promoter, a CD8 promoter, a Integrin alpha M (CD11b) promoter, an interleukin-2 (IL-2) promoter, and a lymphocyte-specific protein tyrosine kinase (Lck) promoter; lung-specific promoters such as a surfactant protein C (SP-C) promoter, a clara cell 10-kDa protein (CC10) promoter, and a forkhead box protein J1 (FOXJ1) promoter; heart-specific promoters such as an alpha-myosin heavy chain (α-MHC) promoter, a cardiac troponin T (cTnT) promoter, a myosin light chain 2 ventricular (MLC-2v) promoter; and kidney-specific promoters such as a nephrin (NPHS1) promoter and an aquaporin 2 (AQP2) promoter.

In some embodiments, the tissue-specific promoter is selected from pancreatic/islet β-cell-specific promoters such as an insulin (INS) promoter, a pancreatic and duodenal homeobox 1 (PDX1) promoter, and a glucose transporter type 2 (GLUT2) promoter; and an intestinal-specific promoter such as a villin promoter, a mucin-2 (MUC2) promoter, and a fatty acid binding protein 2 (FABP2) promoter.

### Adeno-associated virus (AAV) vector

Some embodiments of the present disclosure relate to an AAV vector comprising the CRISPR-Cas12 system, and the AAV vector comprises DNA encoding the Cas12 protein and the guide polynucleotide.

Delivery of the CRISPR-Cas system via the AAV vector was described in Maeder et al., Nature Medicine 25:229-233 (2019*).* It clinically demonstrated the safety and efficacy of subretinal delivery of AAV. Localized delivery via subretinal injection, the natural tropism of AAV5 for photoreceptor cells, and the use of the photoreceptor-specific GRK1 promoter were all employed to restrict expression of the CRISPR/Cas system to the therapeutic target tissue and cell types. The entire contents of this reference are incorporated herein by reference. In some embodiments, the AAV vector comprises a ssDNA genome that comprises coding sequences for the Cas12 protein and a guide polynucleotide flanked by inverted terminal repeats (ITRs).

In some embodiments, the CRISPR-Cas12 system is packaged into the AAV vector, such as AAV1, AAV2, AAV3, AAV4, AAVS, AAV6, AAV7, AAV8, AAV9, or AAVrh74. In some embodiments, the CRISPR-Cas12 system described herein is packaged into the AAV vector including an engineered capsid with tissue tropism, such as an engineered muscle-tropic capsid. Taboada et al., Cell 184:4919-4938 (2021*)* described the engineering of tissue-tropic AAV capsids through directed evolution and identifying a class of capsids containing an RGD motif, and systemic injection of MyoAAV enables efficient transduction of muscle tissue in non-human primates. The entire contents of this reference are incorporated herein by reference.

### Lipid nanoparticle

Some embodiments of the present disclosure relate to a lipid nanoparticle (LNP) comprising the CRISPR-Cas12 system, and the LNP comprises the guide polynucleotide and mRNA encoding the Cas12 protein as described herein.

The LNP delivery of the CRISPR-Cas system was described in Gillmore et al., N. Engl. J. Med. 385:493-502 (2021*).* The LNP is composed of four lipids, including a proprietary ionizable lipid LP000001, DSPC, cholesterol, and DMG-PEG2k. An LNP suspension is formulated in an aqueous buffer including Tris, NaCl, and sucrose at pH of 7.4. The entire contents of this reference are incorporated herein by reference. In some embodiments, in addition to RNA payload (Cas12 mRNA and a guide polynucleotide), the LNP further comprises four components: a cationic or ionizable lipid, cholesterol, a helper lipid, and a PEG-lipid. In some embodiments, the cationic or ionizable lipid comprises cKK-E12, C12-200, ALC-0315, DLin-MC3-DMA, DLin-KC2-DMA, FTT5, Moderna SM-102, and Intellia LP01. In some embodiments, the PEG-lipid comprises PEG-2000-C-DMG, PEG-2000-DMG, or ALC-0159. In some embodiments, the helper lipid comprises DSPC. The components of LNP were described in Panuska et al., Nature Reviews Genetics 23:265-280 (2022*).* FDA-approved LNP comprises mutants of four basic components: a cationic or ionizable lipid, cholesterol, a helper lipid, and polyethylene glycol (PEG) lipid. The entire contents of this reference are incorporated herein by reference.

### Lentiviral vector

Some embodiments of the present disclosure relate to a lentiviral vector comprising the CRISPR-Cas12 system described herein, and the lentiviral vector comprises the guide polynucleotide and mRNA encoding the Cas12 protein described herein. In some embodiments, the lentiviral vector is pseudotyped with homologous or heterologous envelope proteins such as VSV-G. In some embodiments, the mRNA encoding the Cas12 protein is linked to an aptamer sequence.

### Ribonucleoprotein (RNP) complex

Some embodiments of the present disclosure relate to a RNP complex comprising the CRISPR-Cas12 system, and the RNP complex is formed by the guide polynucleotide and the Cas12 protein described herein. In some embodiments, the RNP complex may be delivered into eukaryotic cells, mammalian cells, or human cells via microinjection or electroporation. In certain embodiments, the RNP complex may be packaged into virus-like particles and delivered in vivo to mammalian or human subjects.

### Virus-like particle (VLP)

Some embodiments of the present disclosure relate to a VLP comprising the CRISPR-Cas12 system, and the VLP comprises the guide polynucleotide and the Cas12 protein described herein, or the RNP complex formed by the guide polynucleotide and the Cas12 protein.

The development and application of DNA-free virus-like particles (eVLPs) for efficient packaging and delivery of base editors or Cas9 ribonucleoproteins was described in Banskota et al., Cell 185(2):250-265 (2022*).* Mangeot et al., Nature Communications 10(1):1-15 (2019*)* revealed engineered murine leukemia virus-like particles (Nanoblades) loaded with Cas9-sgRNA ribonucleoproteins to induce efficient genome editing in cell lines and primary cells (including human induced pluripotent stem cells, human hematopoietic stem cells, and mouse bone marrow cells). Campbell et al., Molecular Therapy 27:151-163 (2019*)* revealed specialized extracellular vesicles called "gesicles" to efficiently yet transiently deliver Cas9 ribonucleoproteins targeting the HIV long terminal repeat (LTR) sequence. Gesicles are produced by expressing vesicular stomatitis virus glycoprotein and packaging proteins (as their cargo), thus eliminating the need for transgenic delivery and enabling more precise control over Cas9 expression. Mangeot et al., Molecular Therapy 19(9):1656-1666 (2011*)* revealed that overexpression of the vesicular stomatitis virus glycoprotein (VSV-G) in human cells induces the release of fusogenic vesicles (named gesicles). Biochemical and functional studies showed that glial cells incorporate proteins from producer cells and can deliver them to recipient cells. This protein transduction method enables the direct transfer of cytoplasmic, nuclear, or surface proteins in target cells. These references all describe engineered VLPs, the entire contents of each of which are incorporated herein by reference.

In some embodiments, the engineered VLP is pseudotyped with homologous or heterologous envelope proteins such as VSV-G. In some embodiments, the Cas12 protein is fused to a gag protein (e.g., MLV gag) via a cleavable linker, and cleavage of the linker in the target cell exposes a nuclear localization signal (NLS) located between the linker and the Cas12 protein. In some embodiments, the fusion protein or conjugate comprises (e.g., from the 5' end to the 3' end) the gag protein (e.g., MLV gag), one or more nuclear export signals (NES), a cleavable linker, one or more NLS, and Cas12, as described in Banskota et al., Cell 185(2):250-265 (2022*).*

In some embodiments, the Cas12 protein is fused to a first dimerization domain that is capable of dimerizing or heterodimerizing with a second dimerization domain fused to a membrane protein, and the presence of a ligand promotes such dimerization and facilitates the enrichment of the Cas12 protein or the fusion protein or conjugate thereof into the VLP, as described in Campbell et al., Molecular Therapy 27:151-163 (2019*).*

### Cell

Some embodiments of the present disclosure relate to a cell comprising the CRISPR-Cas12 system described herein. The cell (e.g., used to generate a cell-free system) may be prokaryotic or eukaryotic. For example, the cell comprises, but is not limited to, bacteria, archaea, plant, fungi, yeast, insect, and mammalian cell, such as *Lactobacillus, Lactococcus, Bacillus*(e.g.*, B. subtilis), Escherichia* (e.g., *Escherichia coli*)*, Clostridium, Saccharomyces or Pichia* (e.g., *Saccharomyces cerevisiae* or *Pichia pastoris*)*, Kluyveromyces lactis, Salmonella typhimurium,* Drosophila cells, *Caenorhabditis elegans* cell, *Xenopus laevis* cell, SF9 cells, C129 cells, HEK293 cells, *Neurospora,* and immortalized mammalian cell line (e.g., HeLa, bone marrow cell line, and lymphoid cell line).

In some embodiments, the cell is a prokaryotic cell, such as a bacterial cell (e.g., *Escherichia coli*)*.* In some embodiments, the cell is a eukaryotic cell, such as a mammalian or human cell. In some embodiments, the cell is a primary eukaryotic cell, a stem cell, a tumor/cancer cell, a circulating tumor cell (CTC), a blood cell (e.g., T cell, B cell, NK cell, regulatory T cell (Treg), etc.), a hematopoietic stem cell, a specialized immune cell (e.g., tumor-infiltrating lymphocyte or tumor-suppressive lymphocyte), or a stromal cell in the tumor microenvironment (e.g., cancer-associated fibroblast). In some embodiments, the cell is a brain or neuronal cell of the central or peripheral nervous system (e.g., neuron, astrocyte, microglial cell, retinal ganglion cell, rod or cone cell).

### Target nucleic acid or target DNA

In some embodiments, the target nucleic acid is a target DNA.

The CRISPR-Cas12 system described herein may be used to target one or more target nucleic acid molecules, such as target nucleic acid molecules present in biological samples or environmental samples (e.g., soil, air, or water samples).

In some embodiments of the present disclosure, the target nucleic acid is a gene associated with a disease or disorder. In some embodiments, the target nucleic acid is a disease-associated gene. In some embodiments, the disease-associated gene is a pathogenic gene that directly causes the disease. In some embodiments, the disease-associated gene is an aberrant gene that directly causes the disease or a gene exhibiting abnormal expression. For example, the gene undergoes deleterious mutations, leading to occurrence of disease. As another example, the gene may be overexpressed or underexpressed, resulting in occurrence of disease. In some embodiments, overexpression of the gene leads to disease. In some embodiments, underexpression of the gene leads to disease. In some embodiments, the overexpression of the gene is associated with the occurrence of disease. In some embodiments, the underexpression of the gene is associated with the occurrence of disease.

In some embodiments of the present disclosure, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.

In some embodiments of the present disclosure, the target nucleic acid is selected from any one of the genes listed in Table 27, and the disease or disorder is listed in Table 27. Table 27 shows target nucleic acids and a disease or disorder corresponding to each target nucleic acid.

In some embodiments of the present disclosure, the disease or disorder is selected from: hemophilia A, Best yolk-like macular dystrophy, B-cell acute lymphoblastic leukemia, hemophilia B, CDKL5 deficiency, CLN2 disease, Niemann-Pick disease type C, Dravet syndrome, FOXG1 syndrome, GM1 ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5 deficiency, X-linked myotubular myopathy, X-linked retinoschisis, X-linked retinitis pigmentosa, α1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Bietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1, spinal muscular atrophy, Tay-Sachs diesease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase deficiency, Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune deficiency, heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin (ATTR) amyloidosis, alpha-1-antitrypsin deficiency (AATD) liver disease, and AATD lung disease.

Genes associated with ATTR amyloidosis comprise, but are not limited to, *ATTR.*

Genes associated with Leber hereditary optic neuropathy comprise, but are not limited to, *MT-ND4.*

Genes associated with AATD liver disease comprise, but are not limited to, *AATD.*

Genes associated with AATD lung disease comprise, but are not limited to, *AATD.*

Genes associated with the graft-versus-host disease comprise, but are not limited to, thymidine kinase genes.

Genes associated with hereditary retinal dystrophy comprise, but are not limited to, *RPE65.*

Genes associated with spinal muscular atrophy comprise, but are not limited to, *SMN1.*

Genes associated with osteoarthritis comprise, but are not limited to, *TGF-β1.*

Genes associated with hemophilia A comprise, but are not limited to, *factor VIII.*

Genes associated with hemophilia B comprise, but are not limited to, *factor IX.*

Genes associated with cystic fibrosis comprise, but are not limited to, *CFTR.*

Genes associated with Parkinson's disease comprise, but are not limited to, *Gad1, Gad2, PTBP1, KEAP1, RE1, Amigo1, Gprc5c, Let-7a, Pnky, LRRK2, SNCA, GBA, miR-92b, miR-9, miR-124, miR-181, HMGB1, TRIM72, GPNMB,* and *REST.*

Genes associated with Usher syndrome comprise, but are not limited to, *USH2A.*

Genes associated with α-thalassemia, β-thalassemia, and the sickle cell disease comprise, but are not limited to, *BCL11A, HBG, HBA,* and *HBB.*

Genes associated with pulmonary hypertension comprise, but are not limited to, *eNOS.*

Genes associated with Stargardt's disease comprise, but are not limited to, *ABCA4.*

Genes associated with age-related macular degeneration comprise, but are not limited to, *VEGFA, VEGFR, IL17, Kir7.1, LCN-2, IRAK-M, CD59, LTA4H, GPX4, GLS1, PAPP-A, cGAS, STING, mTOR, GCN2, Nrf2, Ang 2, CTGF,* Complement *C3,* Complement *C5, CHFR4b, DOCK6, CTSS, ELN,* and *FGF2.*

Genes associated with glaucoma comprise, but are not limited to, *AQP1, ADRB2, NMNTA2, NRP1, Hrh1, Anxa2, OPA1, Cx43, ANGPTL7, MYOC, ROCK1, ROCK2, TIMP1, TIMP2, TIMP3, TIMP4,* carbonic anhydrase *CA2,* carbonic anhydrase *CA4,* and carbonic anhydrase *CA12.*

Genes associated with idiopathic pulmonary fibrosis comprise, but are not limited to, *CTGF.*

Genes associated with hyperlipidemia comprise, but are not limited to, *PCSK9.*

Genes associated with Alzheimer's disease comprise, but are not limited to, *NGF.*

Genes associated with coronary heart disease comprise, but are not limited to, *VEGFA* and *bFGF.*

Genes associated with chronic nephrogenic anemia related comprise, but are not limited to, *EPO.*

Genes associated with Leber's congenital amaurosis comprise, but are not limited to, *RPE65.*

Genes associated with retinitis pigmentosa comprise, but are not limited to, *PDE6B.*

Genes associated with phenylketonuria comprise, but are not limited to, *PAH.*

Genes associated with epilepsy comprise, but are not limited to, *GAT1.*

In some embodiments of the present disclosure, a sequence of the target nucleic acid is as shown in any one of sequences shown in SEQ ID NO: 761-782.

Non-limiting examples of the target nucleic acid also include target nucleic acids disclosed in United States Provisional Patent Application No. 61/736,527, filed on December 12, 2012, United States Provisional Patent Application No. 61/748,427, filed on January 2, 2013, and International Application No. PCT/US2013/074667, filed on December 12, 2013, the entire contents of each of which are incorporated herein by reference.

In some embodiments, the target nucleic acid is a reporter gene. Examples of the reporter gene include, but are not limited to, glutathione-S-transferase (GST), horseradish peroxidase (HRP), chloramphenicol acetyltransferase (CAT), β-galactosidase, β-glucuronidase, luciferase, green fluorescent protein (GFP), HcRed, DsRed, cyan fluorescent protein (CFP), yellow fluorescent protein (YFP), and autofluorescent protein including blue fluorescent protein (BFP).

### Use for treatment or prevention of disease

Some embodiments of the present disclosure relate to a pharmaceutical composition comprising the Cas12 protein, the guide polynucleotide, the inactivated Cas12 mutant, the fusion protein or conjugate, the isolated nucleic acid, the CRISPR-Cas12 system, the vector system, the delivery system, or the cell, which are all described in the present disclosure. For example, the pharmaceutical composition may comprise an AAV vector encoding the Cas12 protein or the inactivated Cas12 mutant and the guide polynucleotide. For example, the pharmaceutical composition may comprise a lipid nanoparticle comprising the guide polynucleotide and mRNA encoding the Cas12 protein. For example, the pharmaceutical compositions may comprise a lentiviral vector comprising the guide polynucleotide and the mRNA encoding the Cas12 protein. For example, the pharmaceutical composition may comprise a virus-like particle comprising the guide polynucleotide and the Cas12 protein, or a ribonucleoprotein complex formed by the guide polynucleotide and the Cas12 protein.

Some embodiments of the present disclosure relate to use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit described herein in cleaving or editing a target nucleic acid in a mammalian cell.

Some embodiments of the present disclosure relate to use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit described herein in any of the following: cleaving one or more target nucleic acid molecules or introducing nicks into the one or more target nucleic acid molecules, activating or upmodulating an expression of the one or more target nucleic acid molecules, activating or inhibiting transcription of the one or more target nucleic acid molecules, inactivating the one or more target nucleic acid molecules, visualizing, labeling, or detecting the one or more target nucleic acid molecules, binding the one or more target nucleic acid molecules, transporting the one or more target nucleic acid molecules, and masking the one or more target nucleic acid molecules.

Some embodiments of the present disclosure relate to use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit described herein in modifying one or more target nucleic acid molecules, and the modifying one or more target nucleic acid molecules comprises one or more of: nucleic acid base substitution, nucleic acid base deletion, nucleic acid base insertion, breakage of a target nucleic acid, nucleic acid methylation, and nucleic acid demethylation.

Some embodiments of the present disclosure relate to use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit described herein in diagnosing, treating, or preventing a disease or disorder associated with the target nucleic acid.

Some embodiments of the present disclosure relate to use of the Cas12 protein, the guide polynucleotide, the inactivated Cas12 mutant, the fusion protein or conjugate, the isolated nucleic acid, the CRISPR-Cas12 system, the vector system, the delivery system, the cell, the pharmaceutical composition, or the kit described herein in preparing a medicament for diagnosing, treating, or preventing a disease or disorder associated with the target nucleic acid.

In some embodiments of the present disclosure, the target nucleic acid is optionally selected from genes as listed in Table 27, and the disease or disorder is as listed in Table 27. Table 27 shows target nucleic acids and a disease or disorder corresponding to each target nucleic acid.

In some embodiments of the present disclosure, specific genes, such as those listed in Table 27, are subjected to targeted cleavage by the CRISPR-Cas12 system, thereby preventing, diagnosing, or treating the corresponding disease or disorder in Table 27. After targeted cleavage, indels are introduced through cellular repair, resulting in the knockout of the target gene, thereby suppressing its function.

In some embodiments of the present disclosure, specific genes, such as those listed in Table 27, are subjected to targeted modification by the CRISPR-Cas12 system, thereby preventing, diagnosing, or treating the corresponding disease or disorder in Table 27.

In some embodiments of the present disclosure, an expression of specific genes, such as those listed in Table 27, is subjected to targeted modulation by the CRISPR-Cas12 system, thereby preventing, diagnosing, or treating the corresponding disease or disorder in Table 27.

In some embodiments, the pharmaceutical composition is delivered in vivo to a human subject. The pharmaceutical composition may be delivered by any effective route. Exemplary routes of administration include, but are not limited to, intravenous infusion, intravenous injection, intraperitoneal injection, intramuscular injection, intratumoral injection, subcutaneous injection, intradermal injection, intraventricular injection, intravascular injection, intracerebellar injection, intraocular injection, subretinal injection, intravitreal injection, intracameral injection, intratympanic injection, intranasal injection, and inhalation.

### Diagnostic Application

Some embodiments of the present disclosure relate to an in vitro composition, comprising the CRISPR-Cas12 system described herein and a marked detector DNA that does not hybridize with the guide polynucleotide described herein.

Some embodiments of the present disclosure relate to the use of the CRISPR-Cas12 system in detecting a target nucleic acid in a nucleic acid sample suspected of containing a target nucleic acid.

Some embodiments of the present disclosure relate to the use of the CRISPR-Cas12 system in detecting a target nucleic acid in a nucleic acid sample containing the target nucleic acid.

In some embodiments, the detected target nucleic acid is a target RNA.

In some embodiments, the detected target nucleic acid is a target DNA. In some embodiments, a method for detecting the target DNA comprises fusing a Cas12 protein to a fluorescent protein or other detectable marker and a guide sequence of a guide polynucleotide being specific to the target DNA. The binding of Cas12 to the target DNA may be visualized by microscopy or other imaging manners.

In some embodiments, a method for detecting a target nucleic acid in a cell-free system results in the generation of a detectable marker or enzymatic activity. For example, by using the Cas12 protein, the guide polynucleotide comprising the guide sequence specific to the target DNA, and the detectable marker, the target nucleic acid may be recognized by Cas12. Binding of Cas12 to the target nucleic acid triggers its DNase activity, which results in cleavage of the target nucleic acid and the detectable marker.

In some embodiments, the detectable marker is DNA linked to a fluorescent probe and a quencher. The complete detectable DNA ligates to the fluorescent probe and quencher, suppressing fluorescence. After the detectable DNA is cleaved by Cas12, the fluorescent probe is released from the quencher and exhibits fluorescent activity. This method may be used to determine whether the target DNA is present in lysed cell samples, lysed tissue samples, blood samples, saliva samples, environmental samples (e.g., water, soil, or air samples), or other lysed cell or cell-free samples. This method may also be used to detect pathogens such as viruses or bacteria, or to diagnose disease states such as cancer.

In some embodiments, the detection of the target nucleic acid is conducive to diagnosing a disease and/or pathological condition, or the presence of viral or bacterial infection.

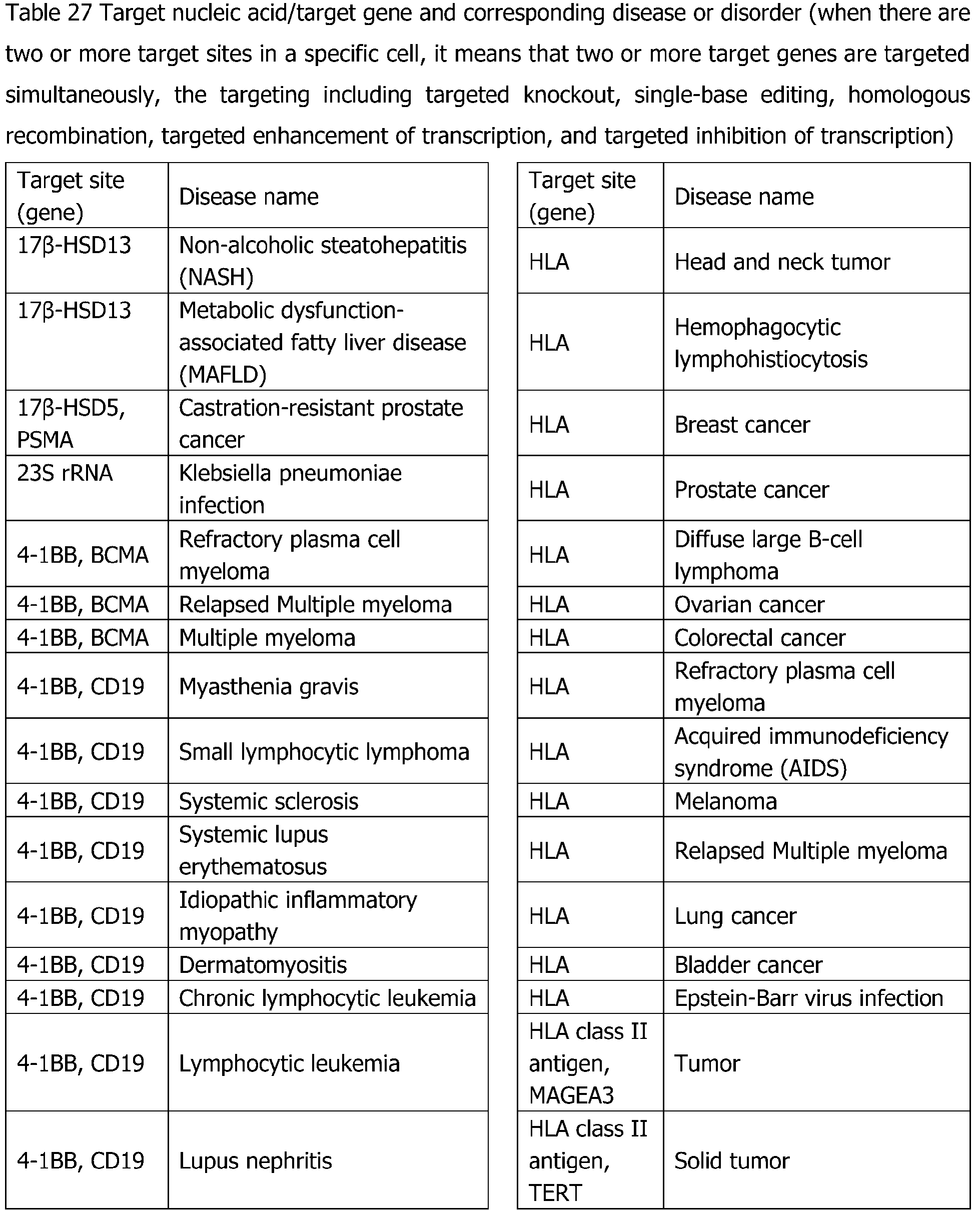

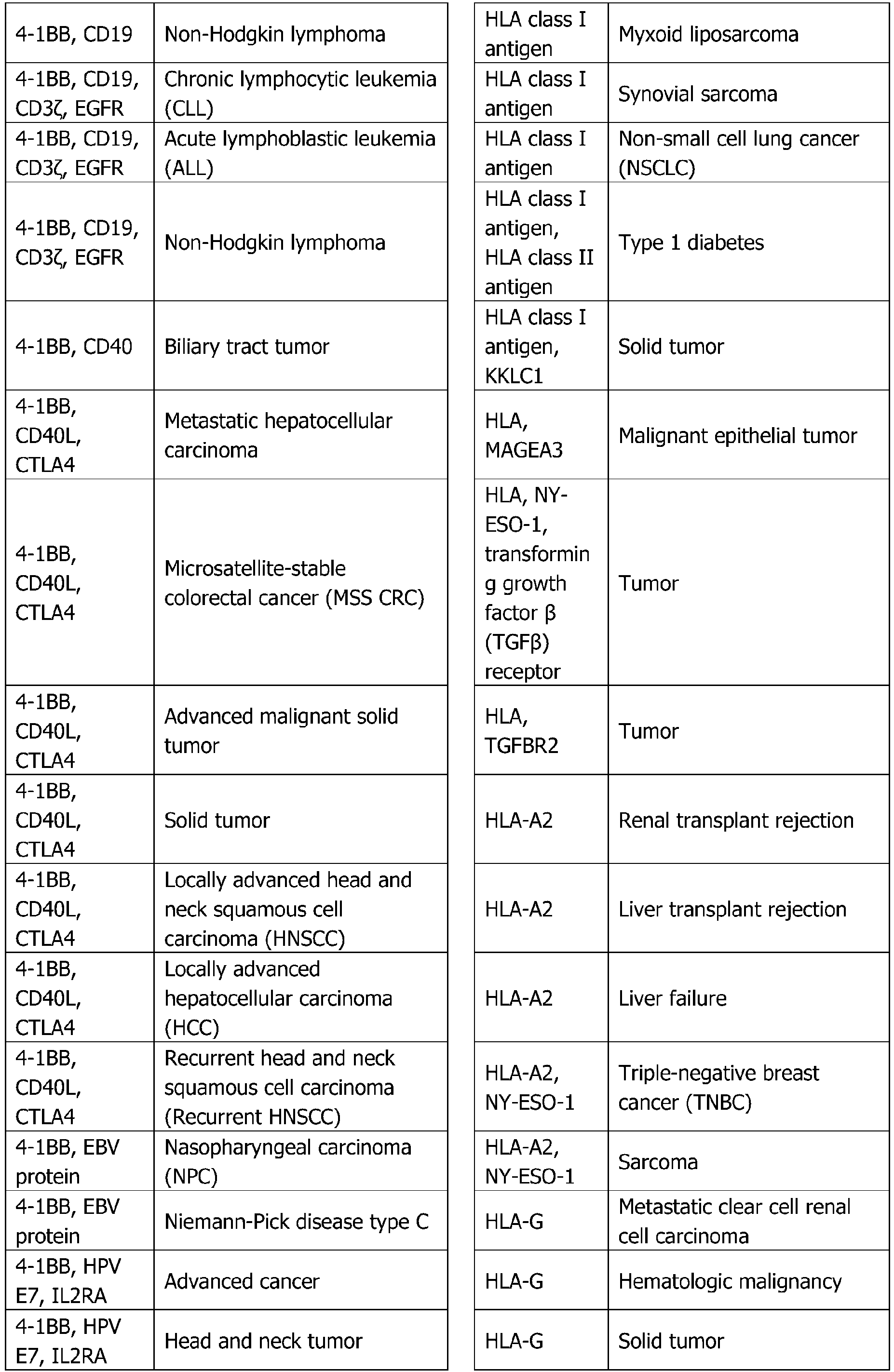

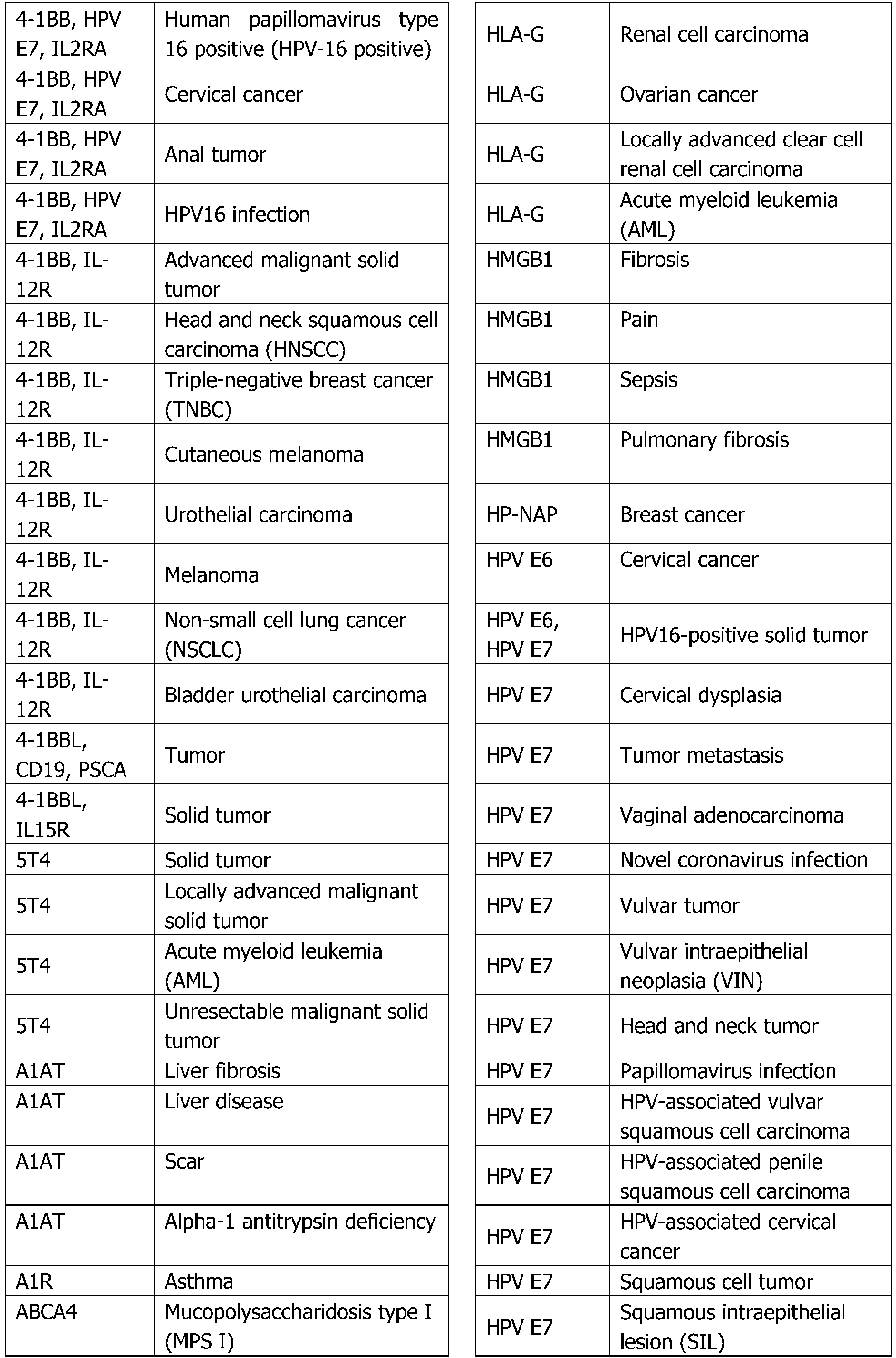

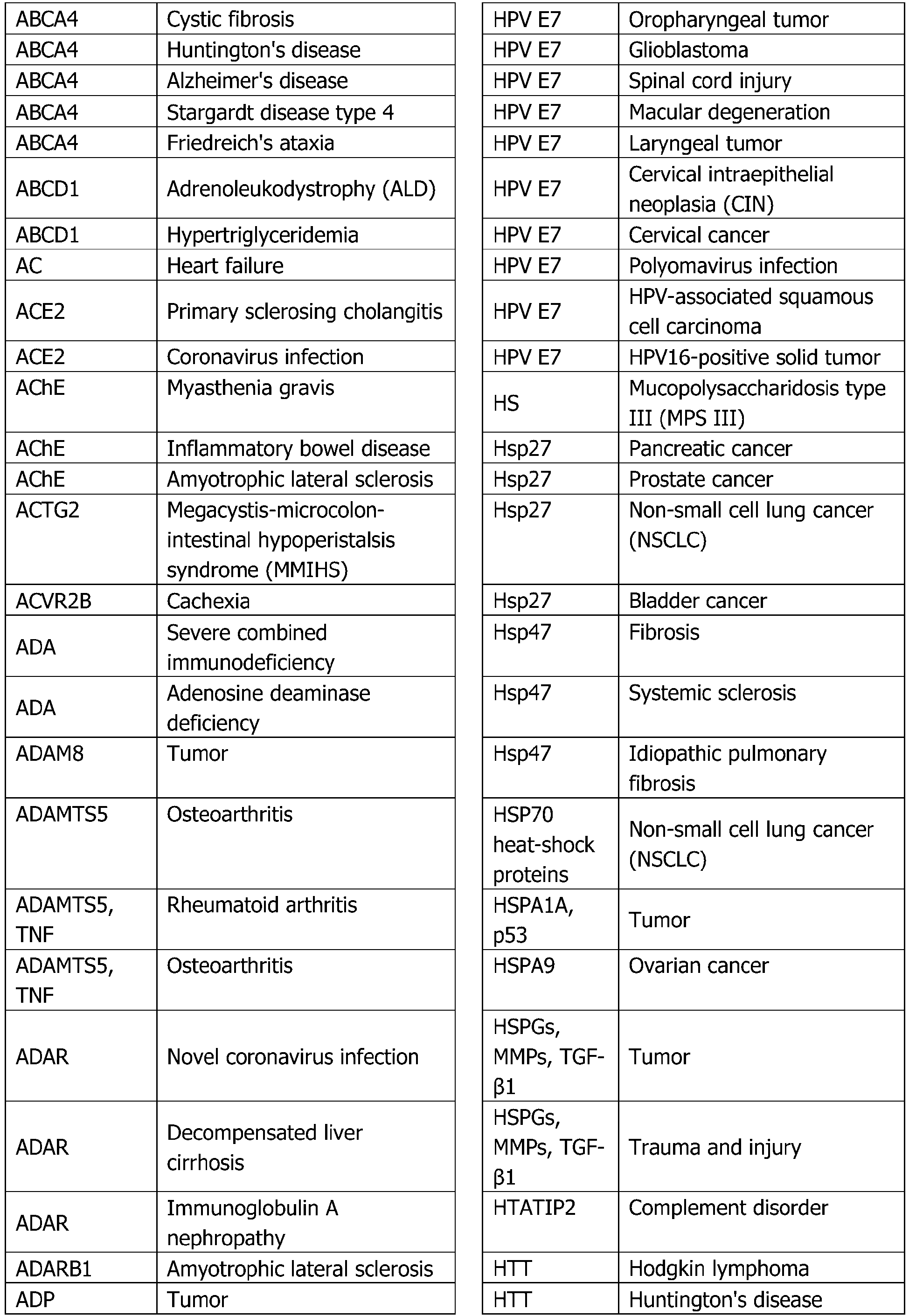

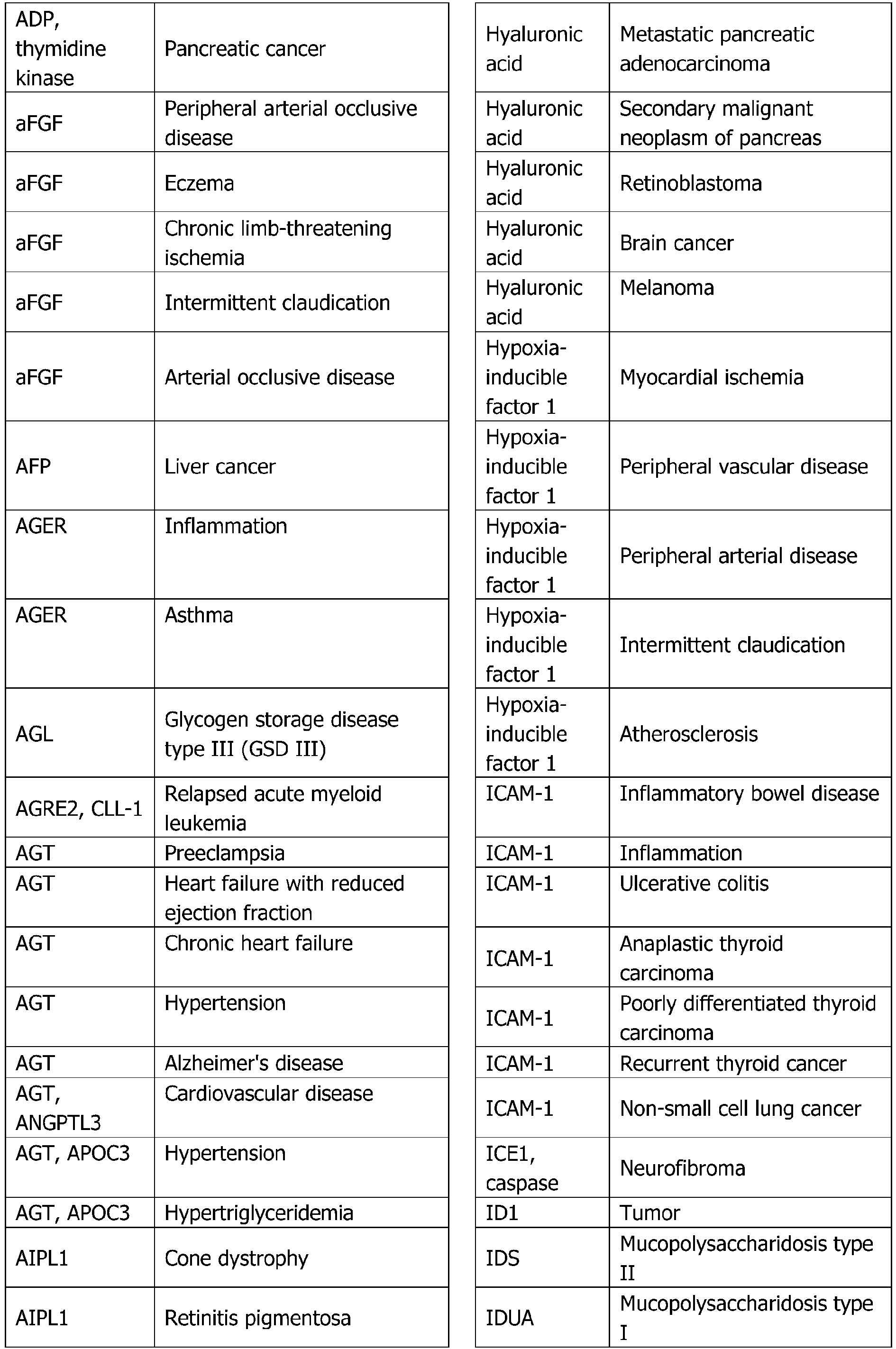

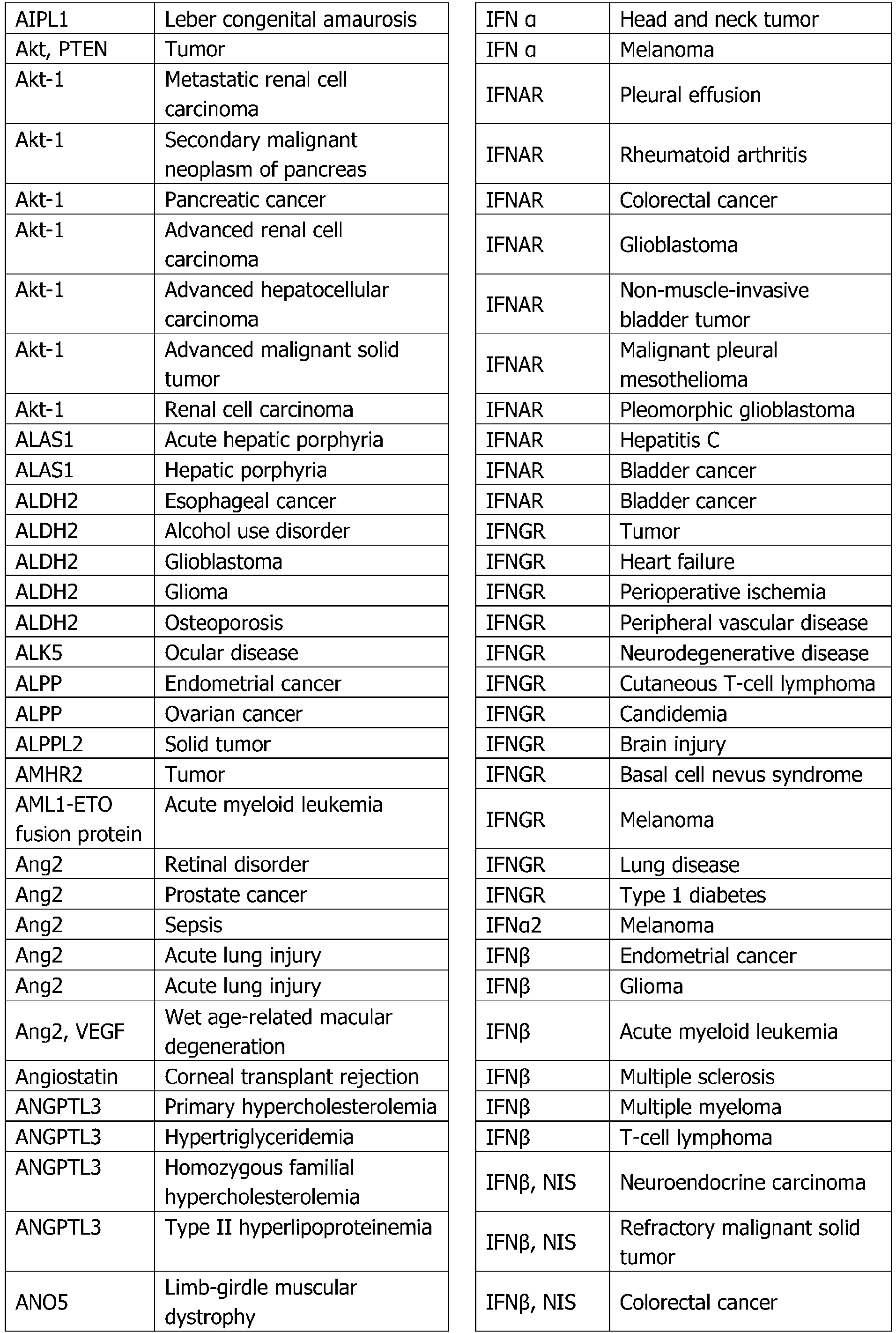

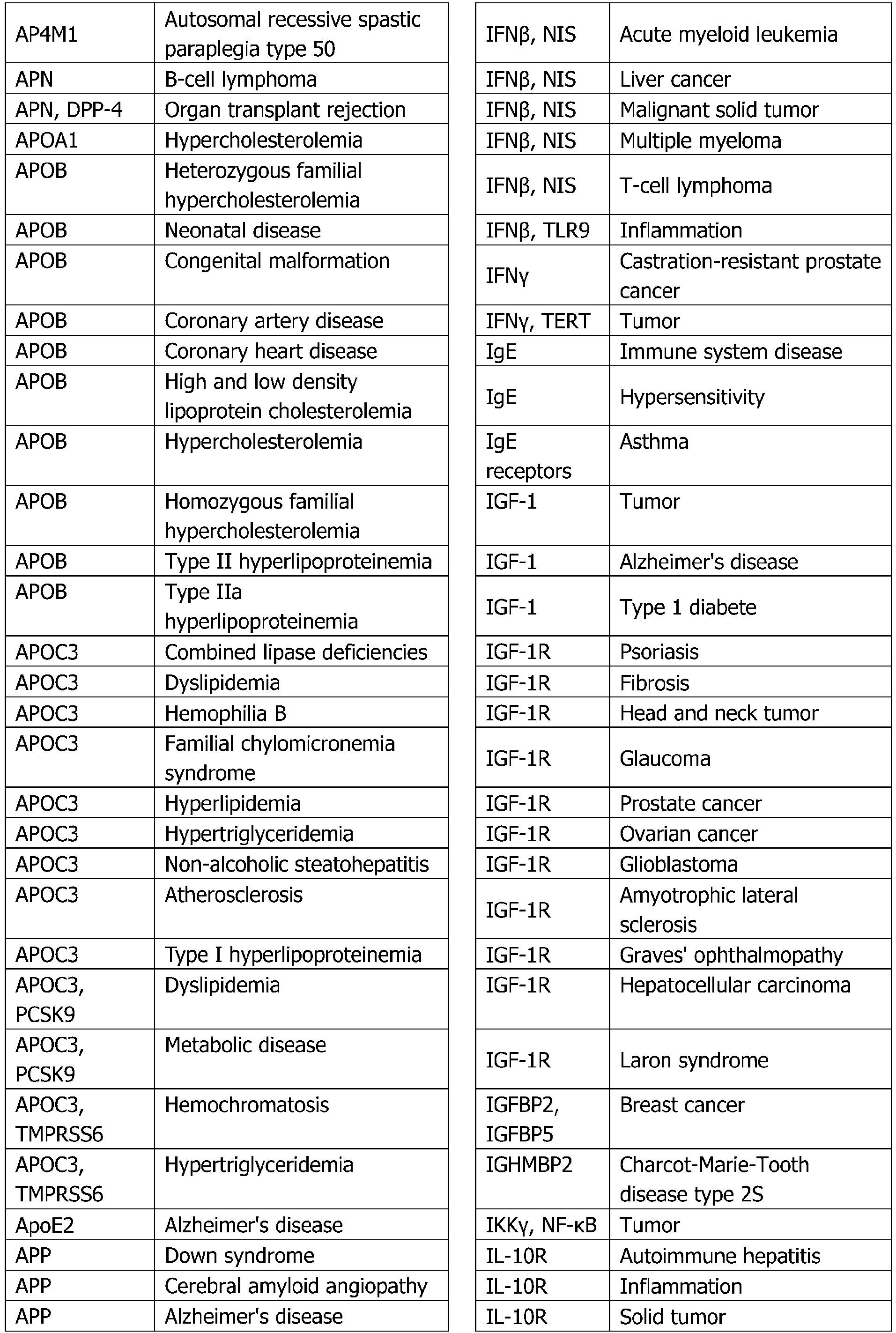

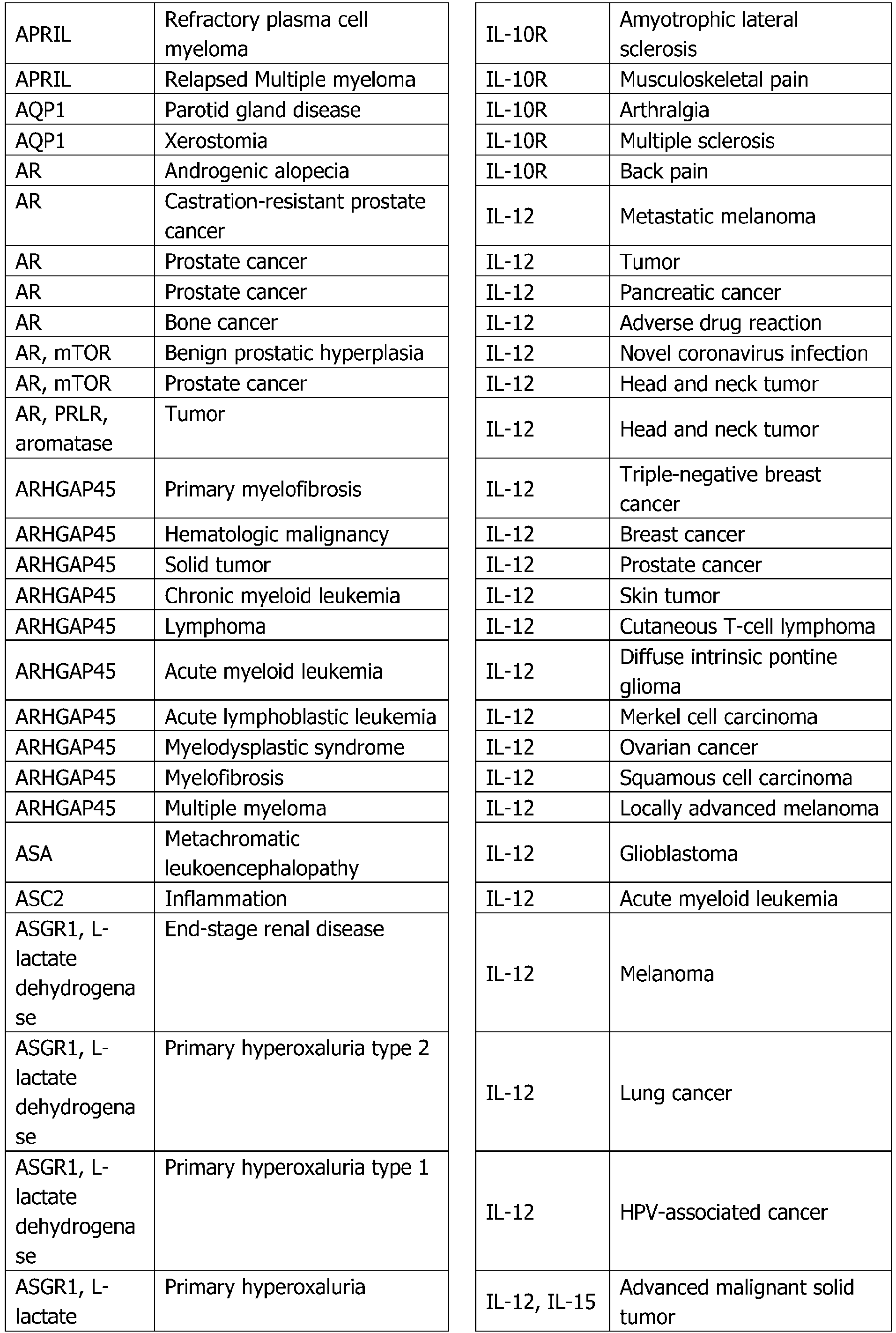

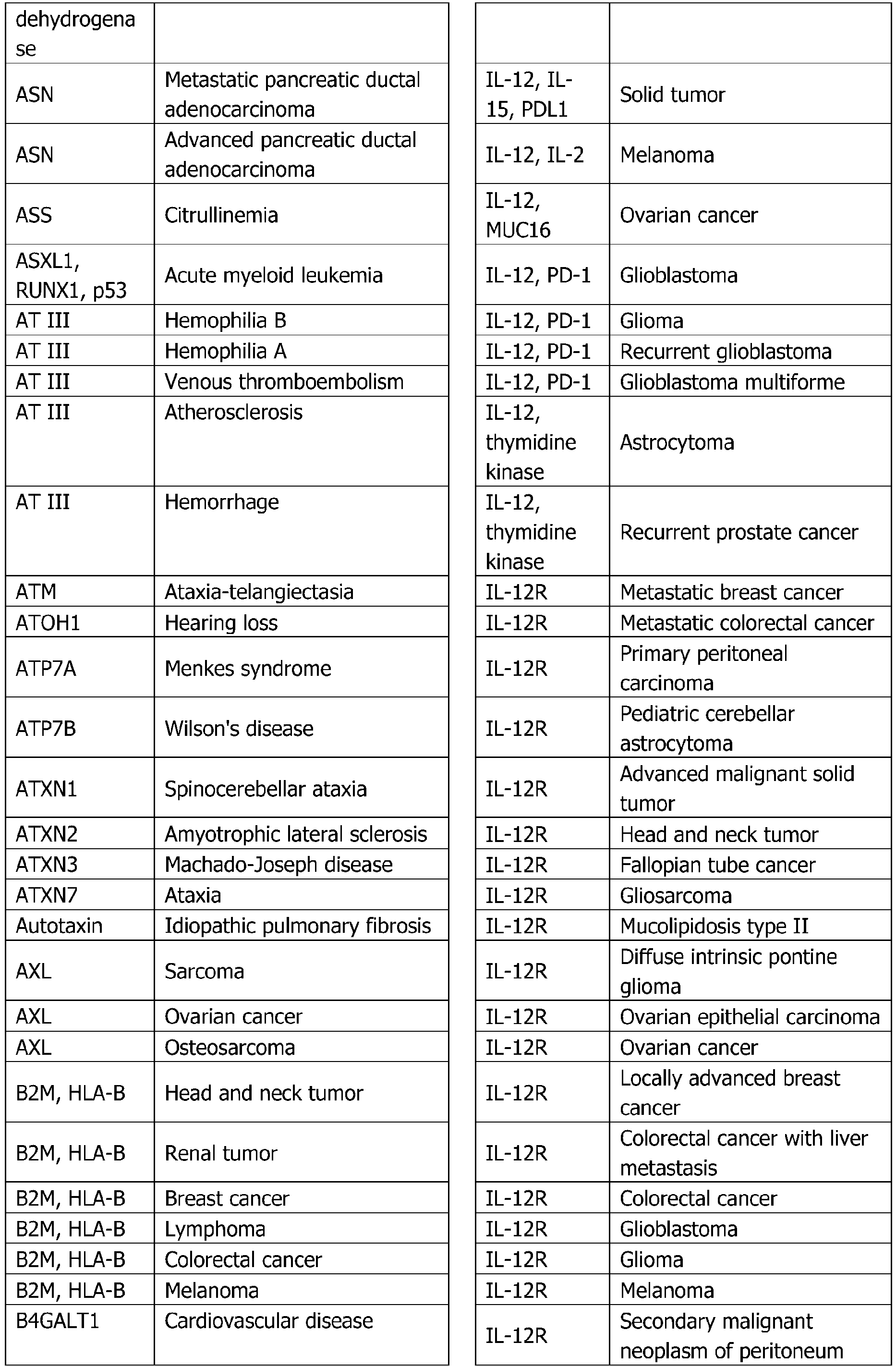

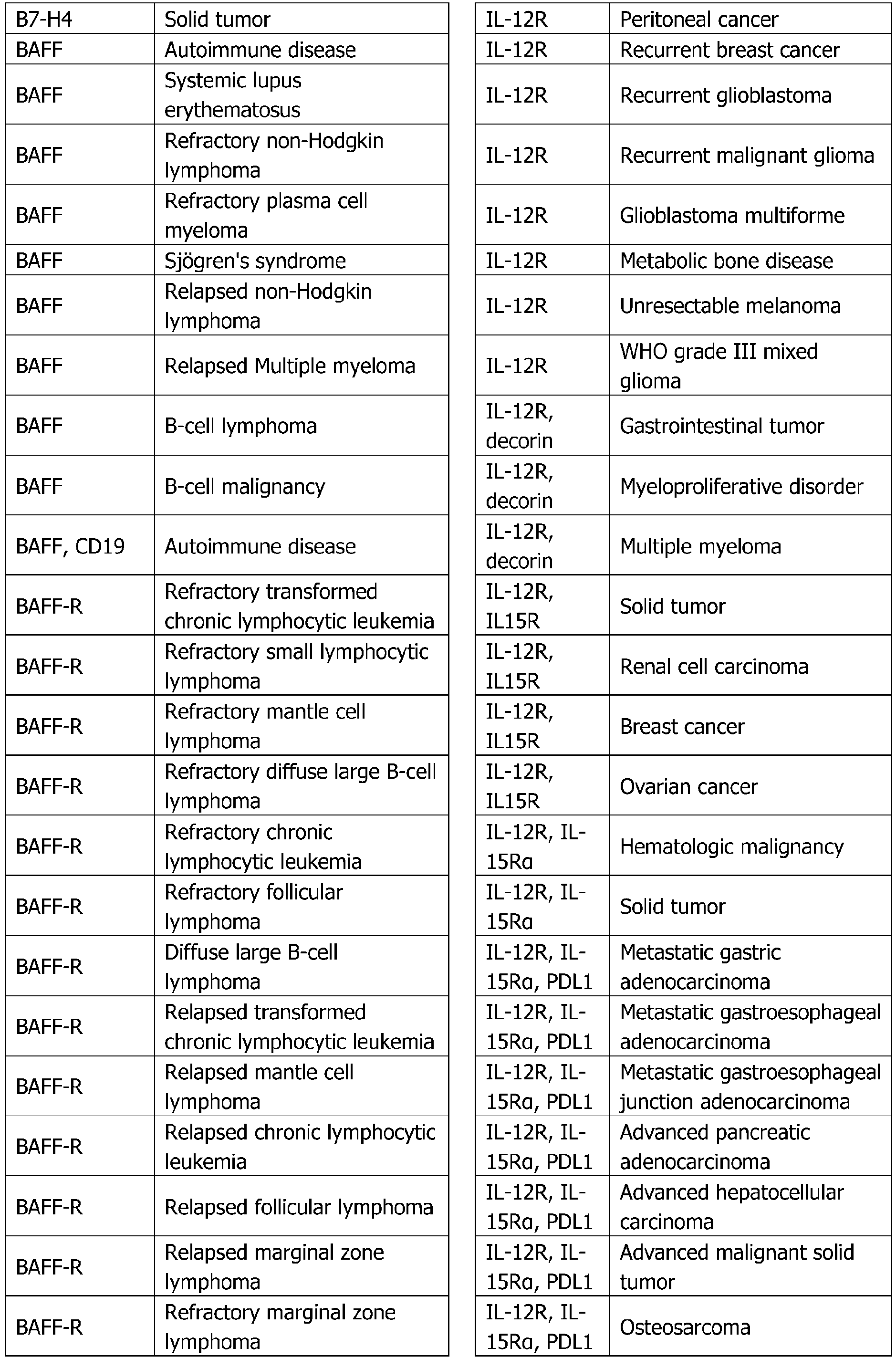

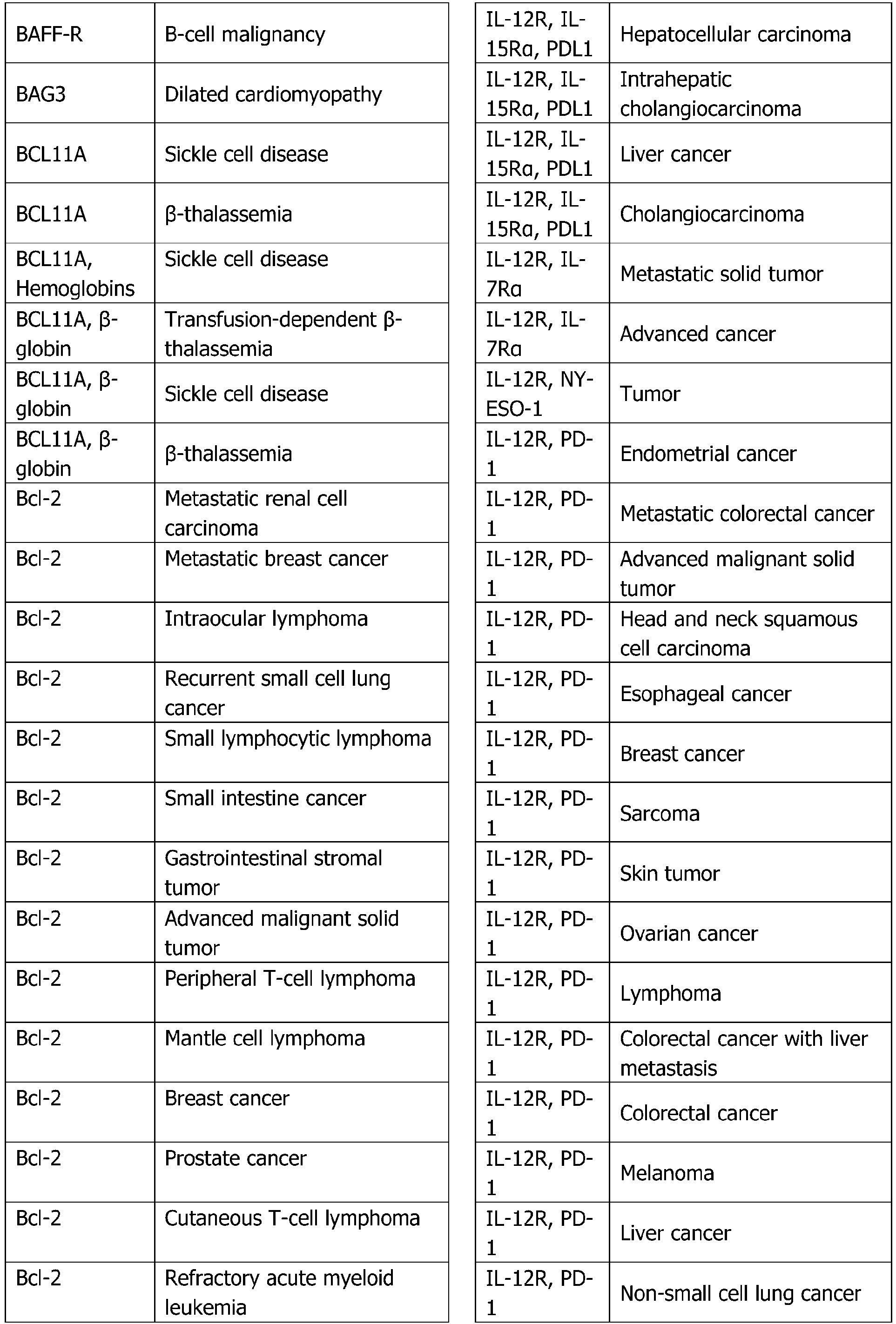

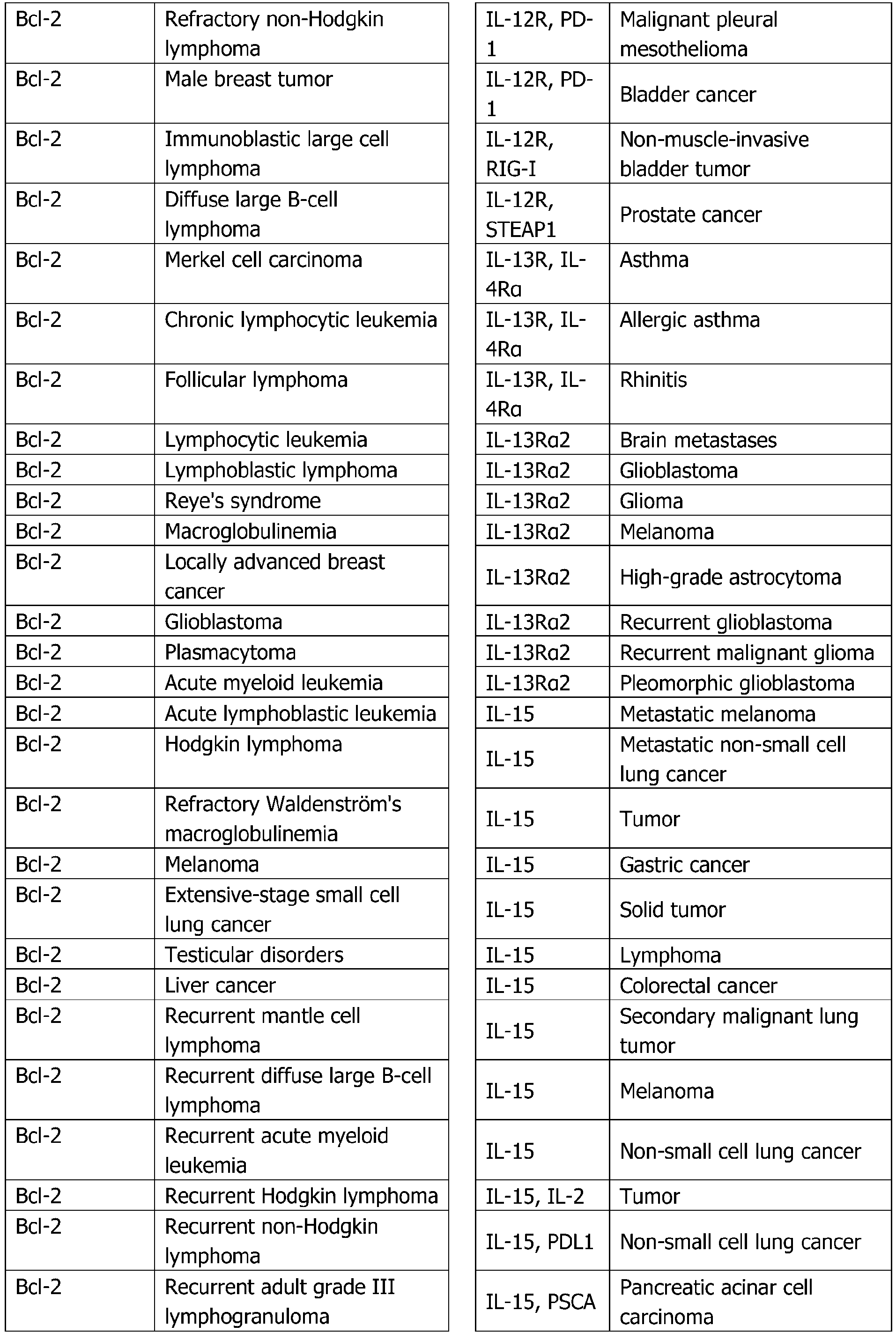

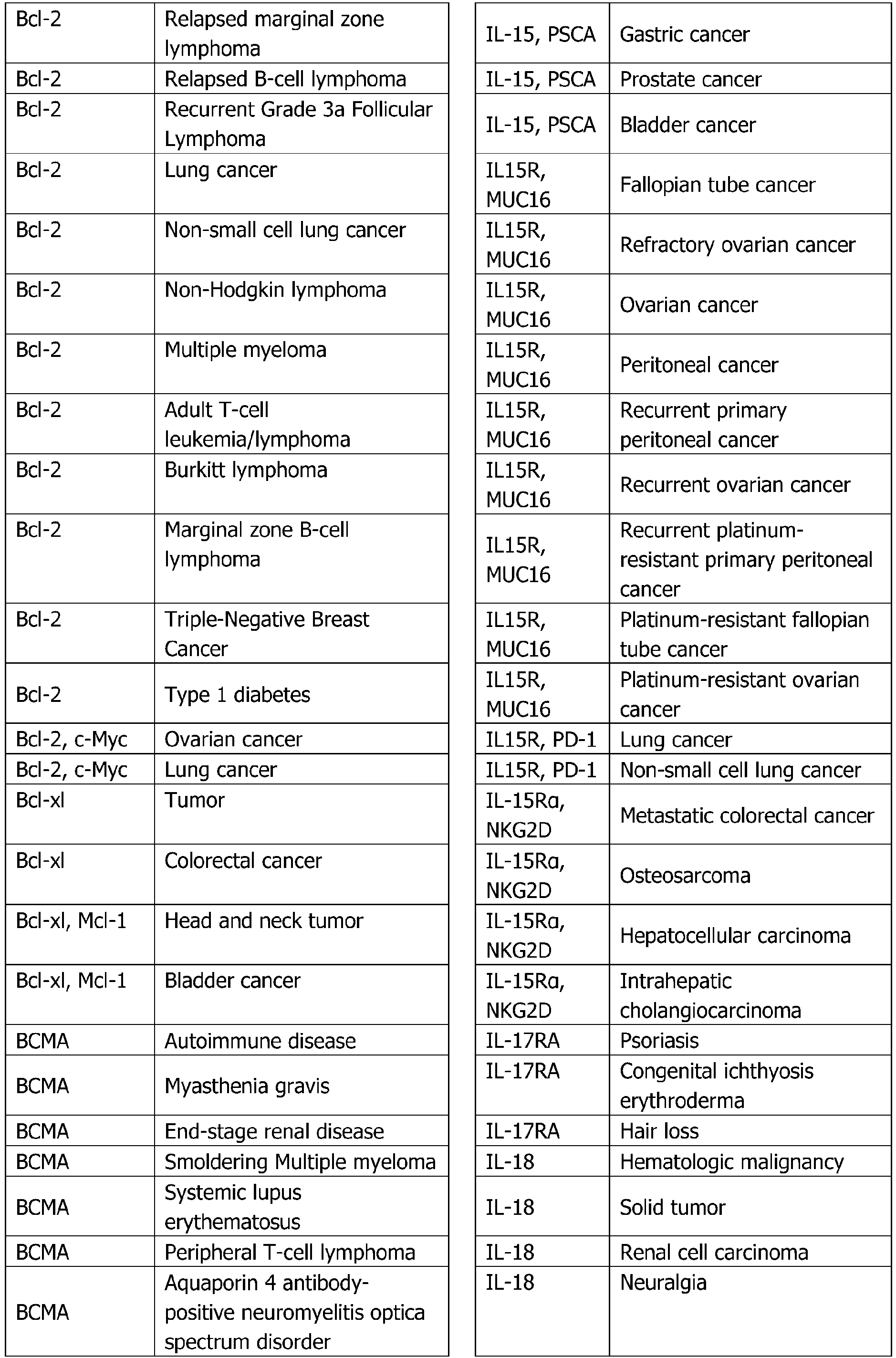

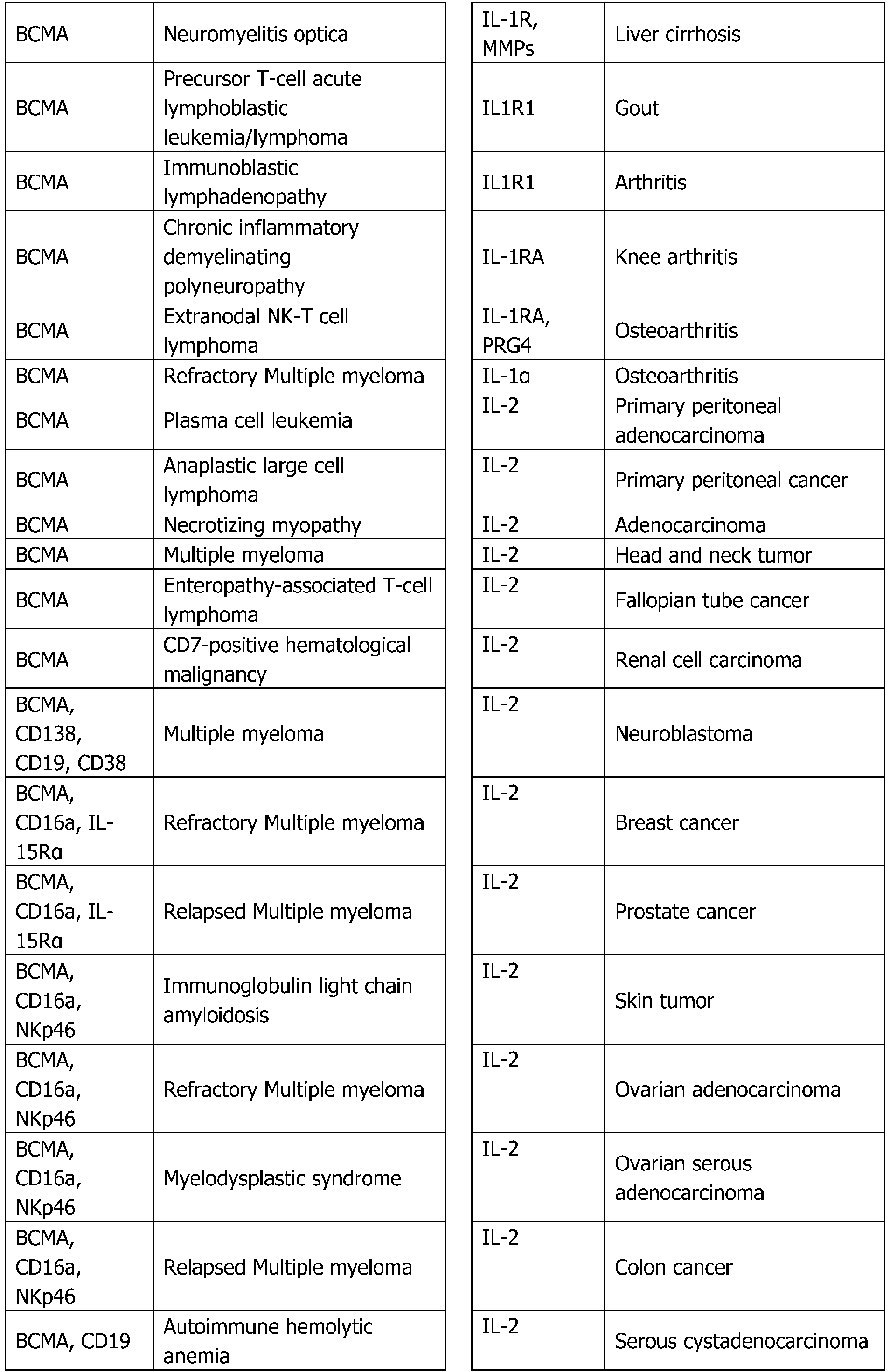

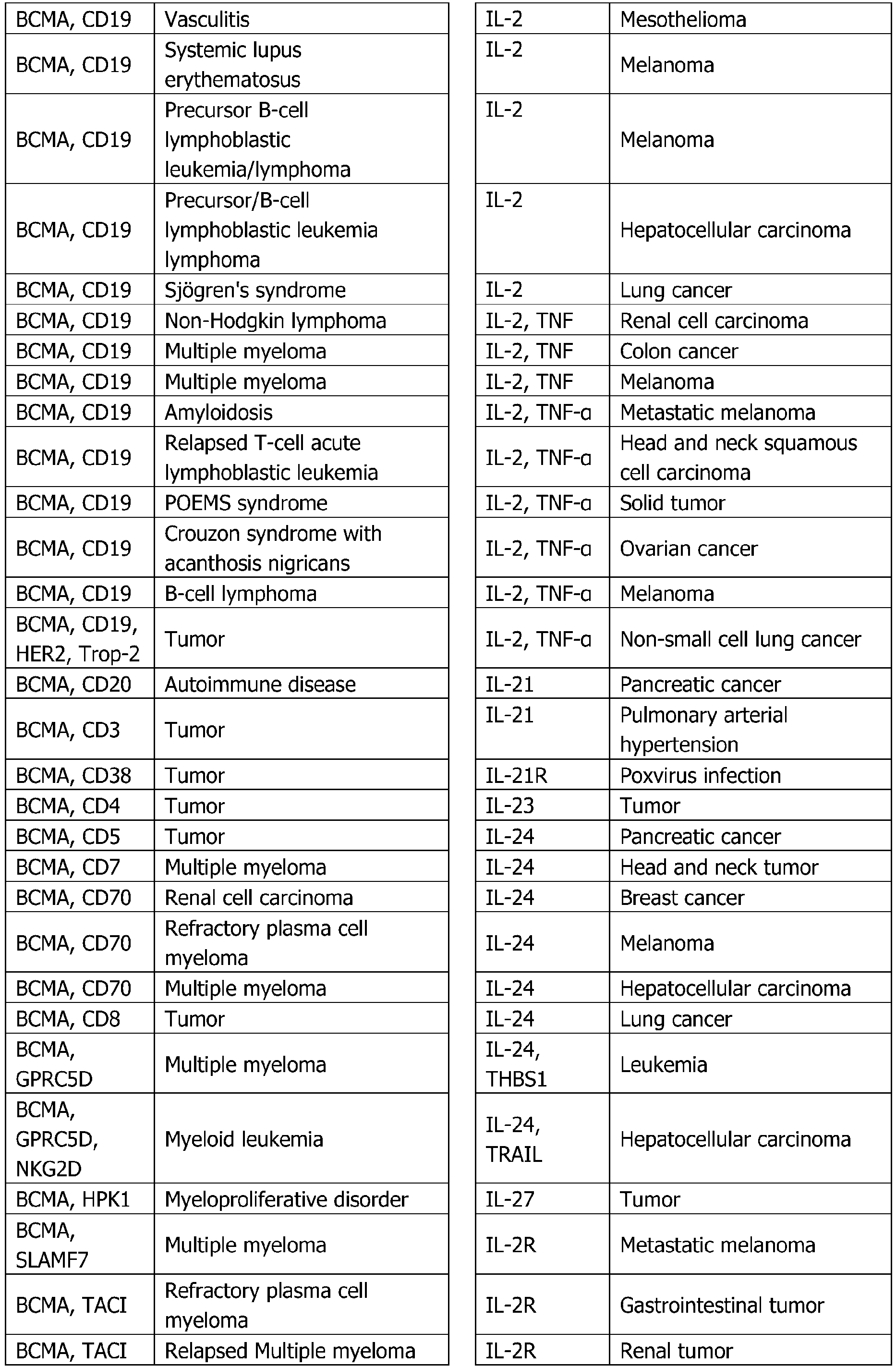

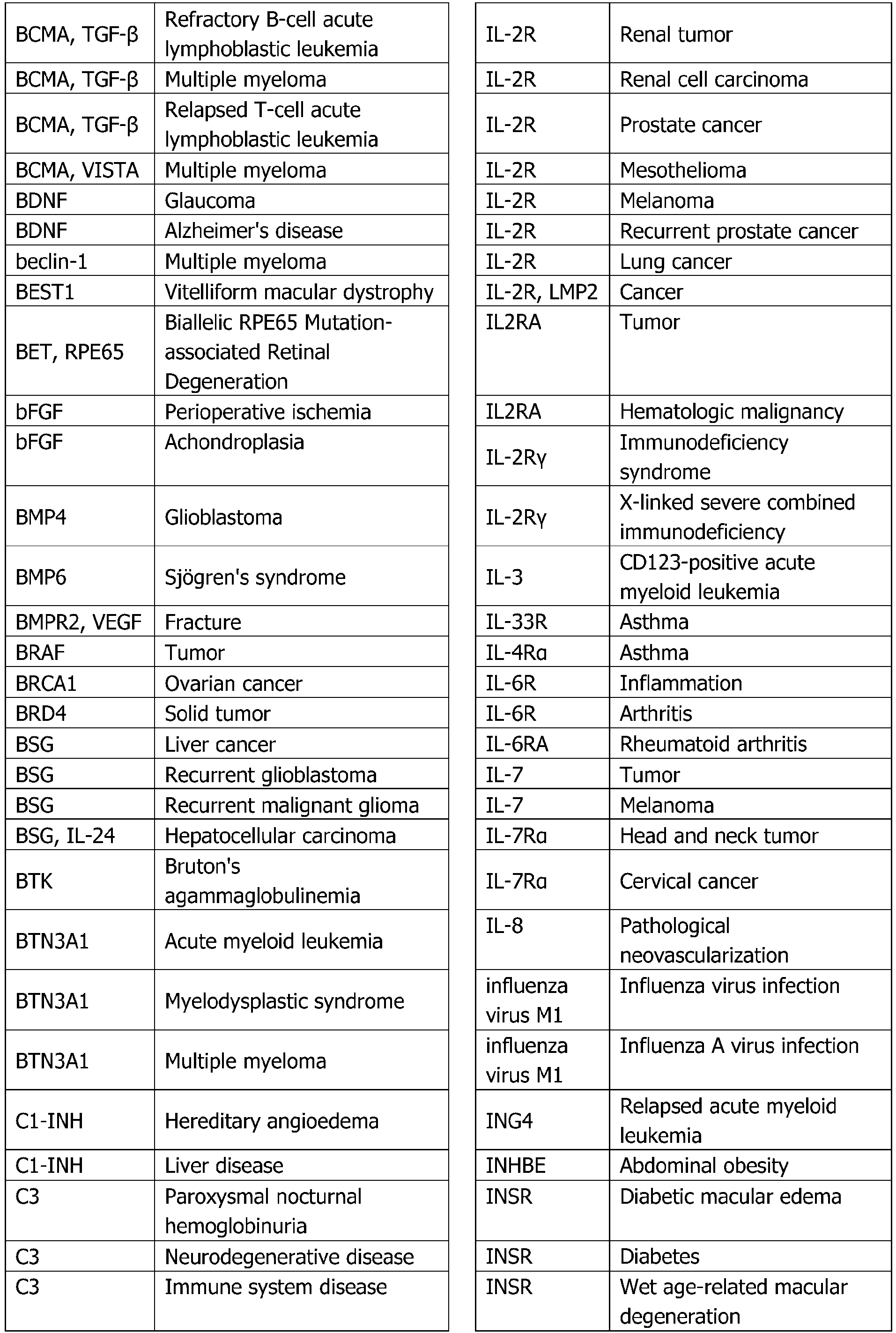

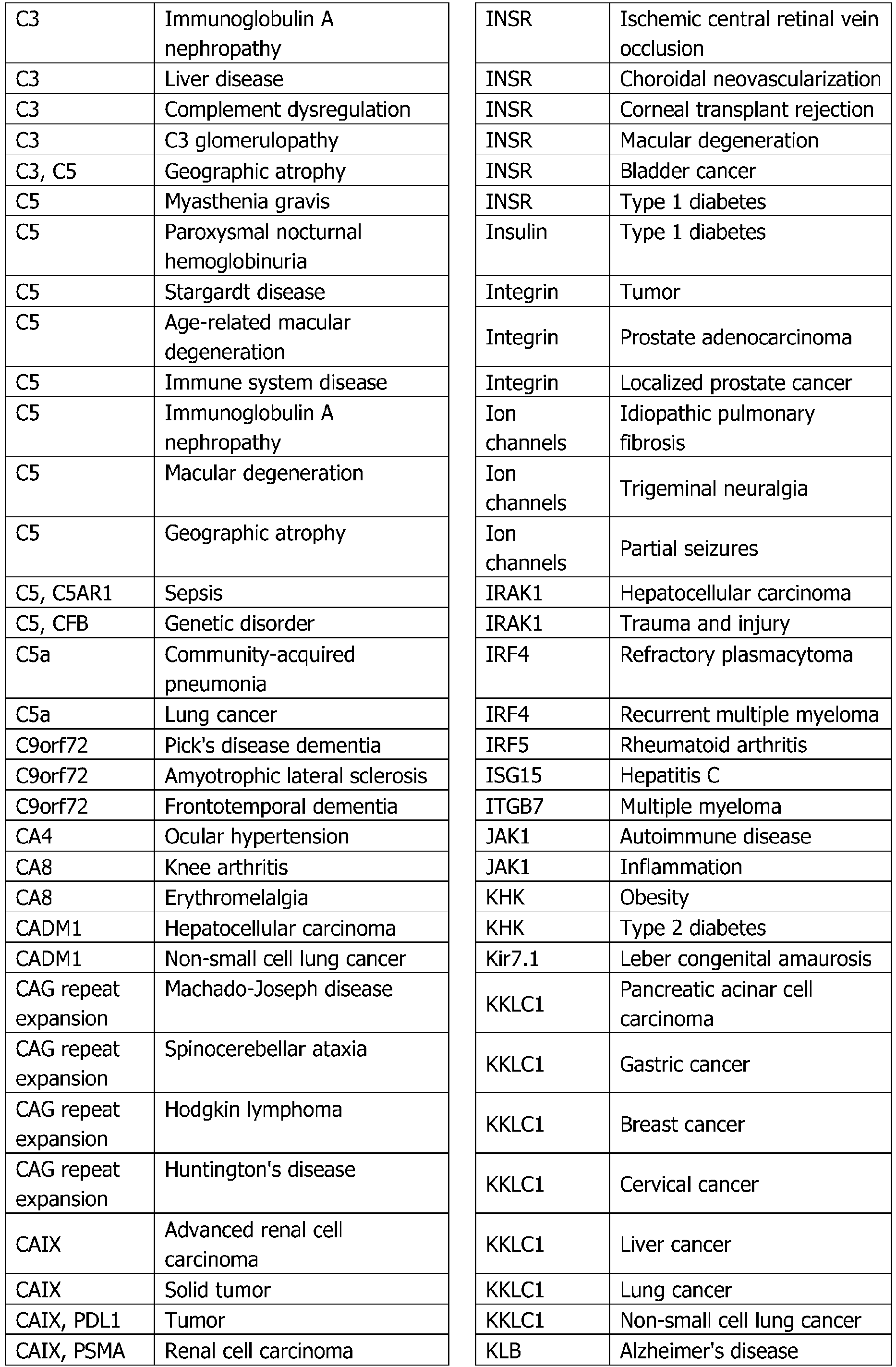

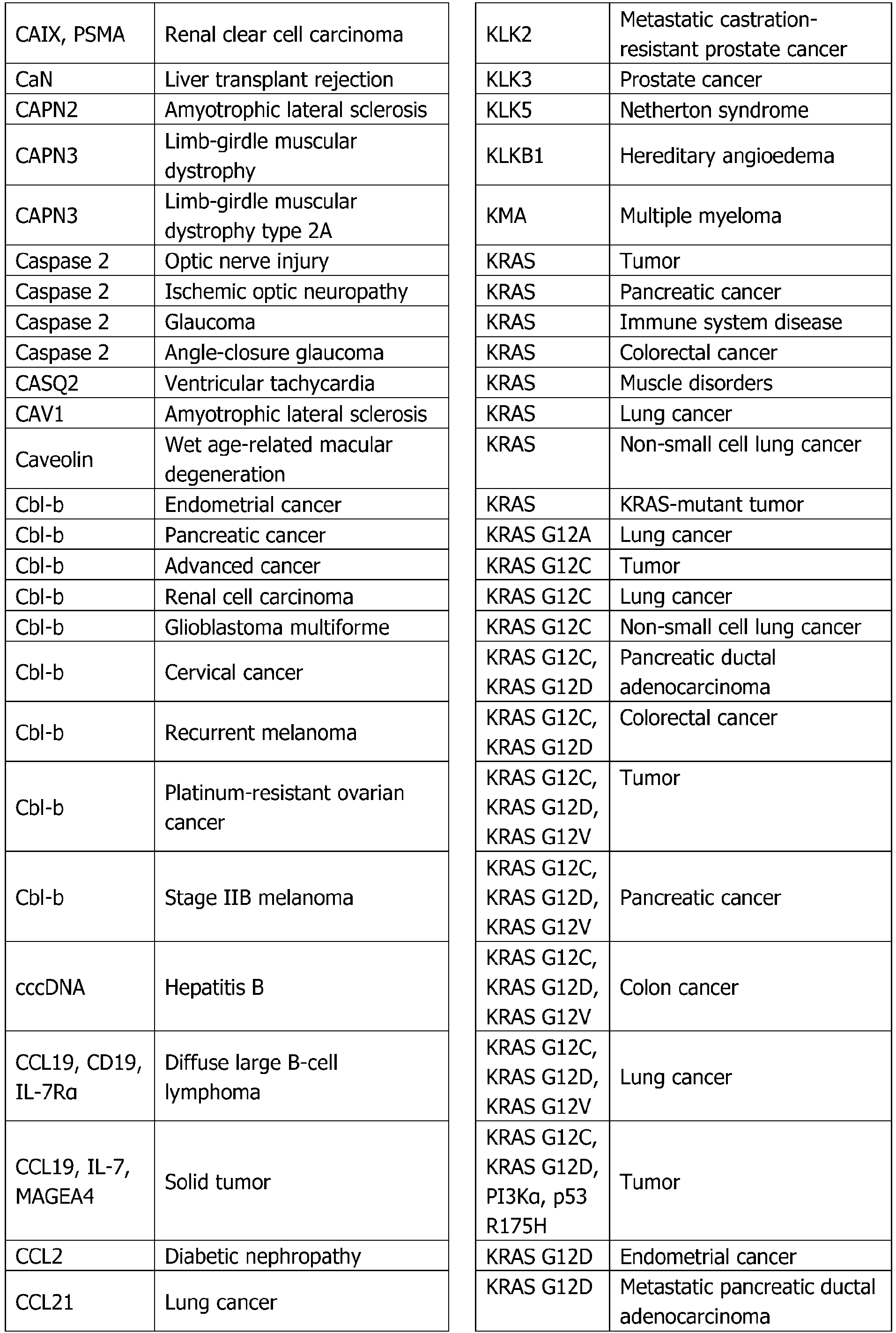

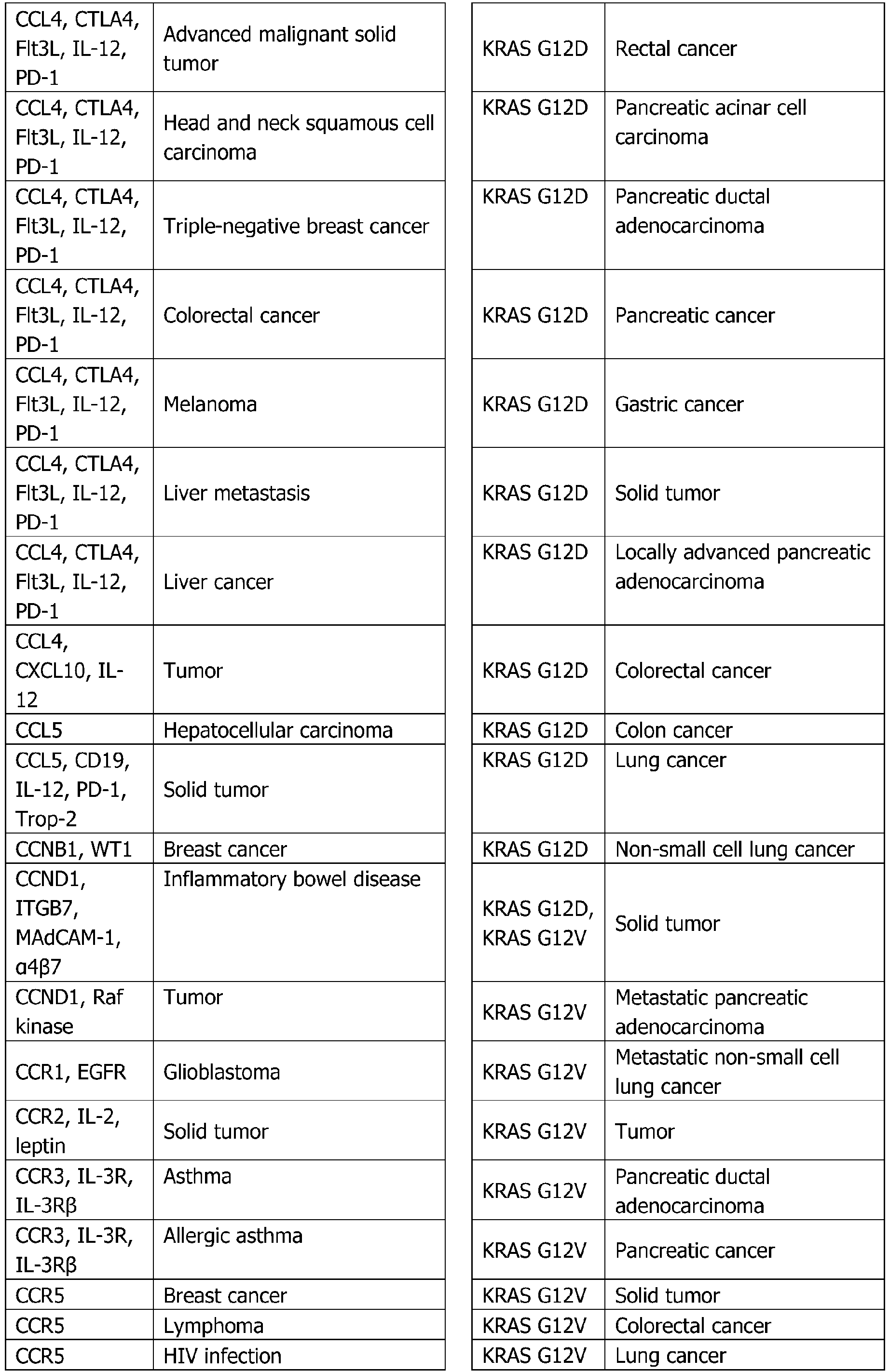

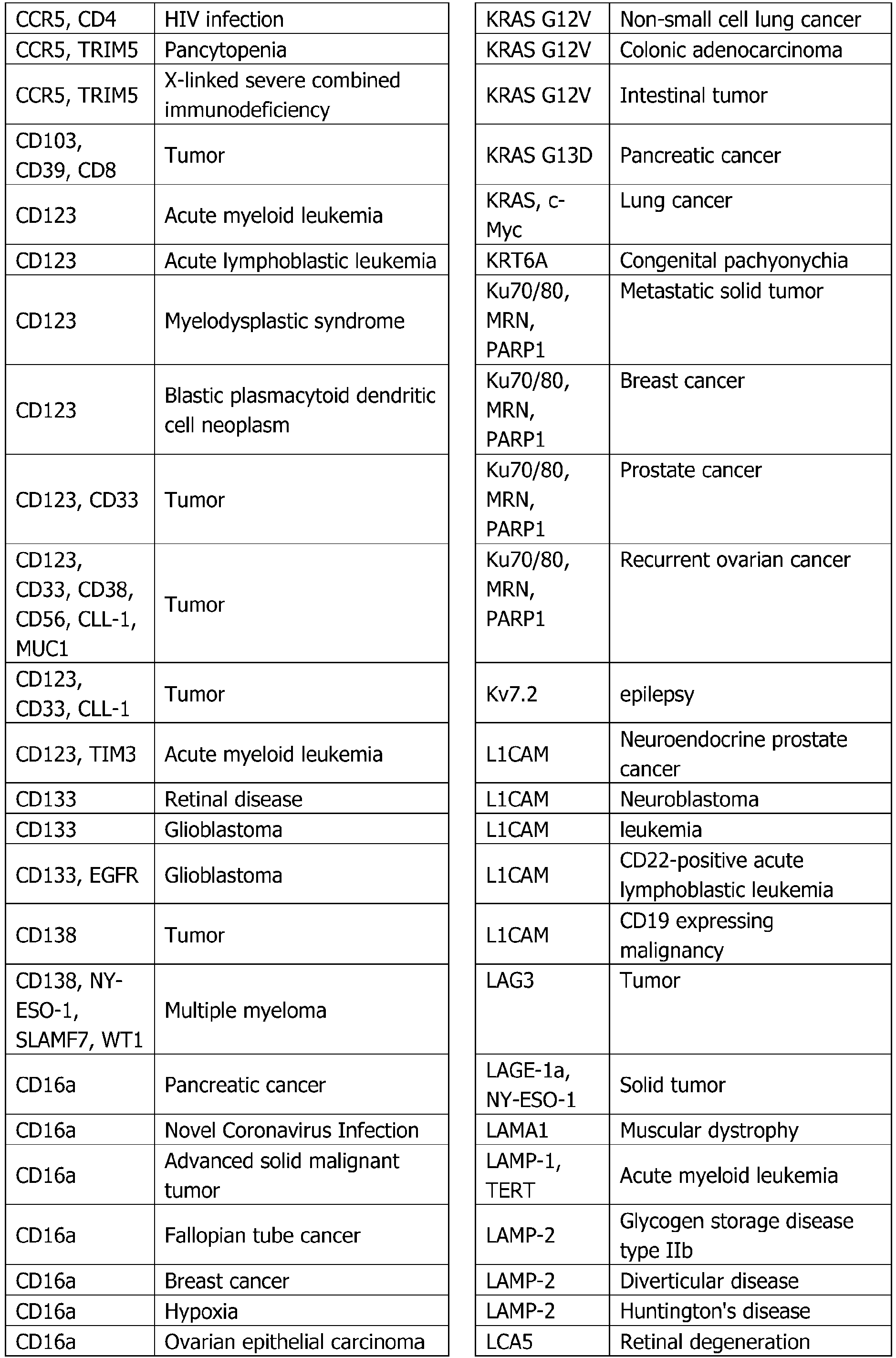

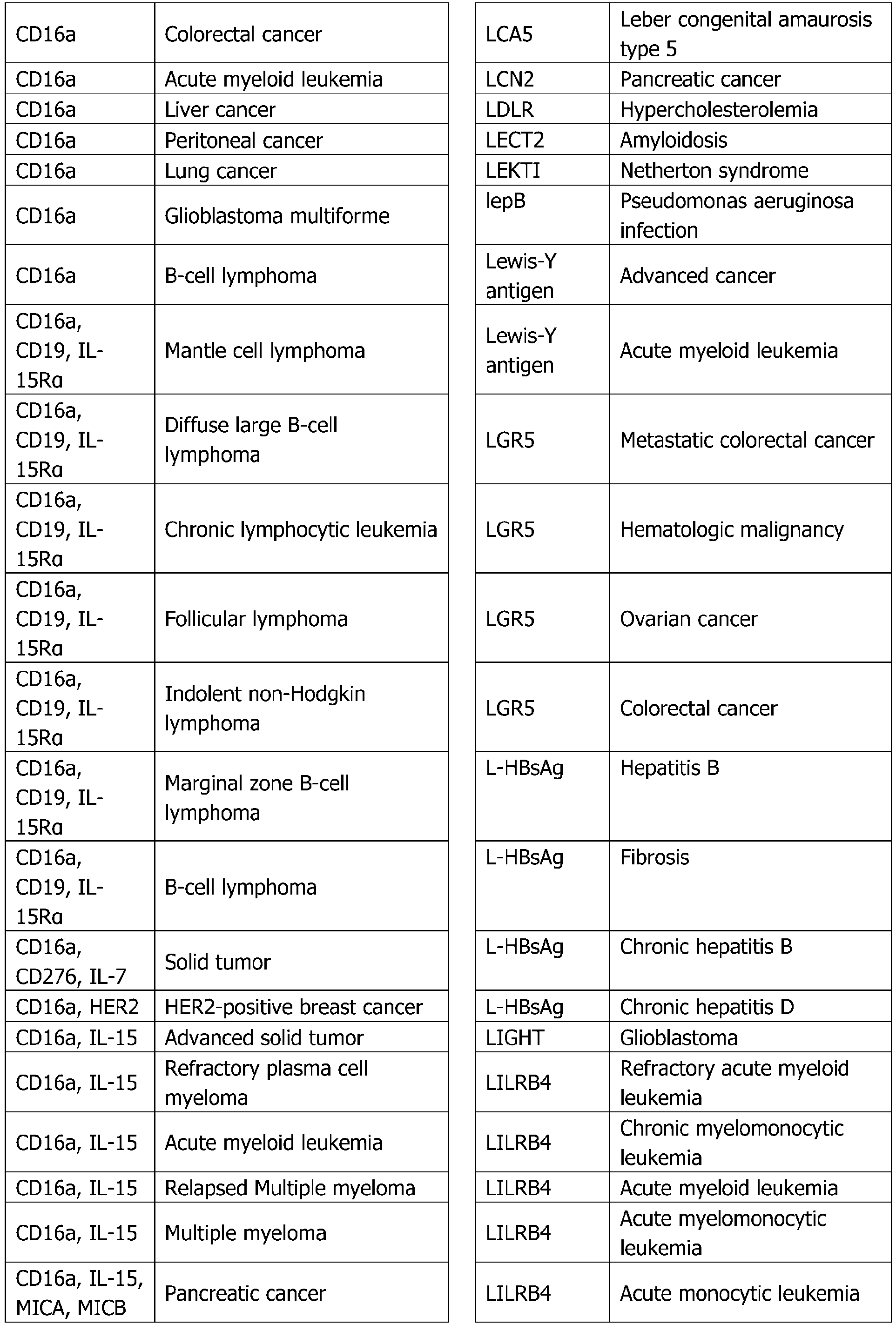

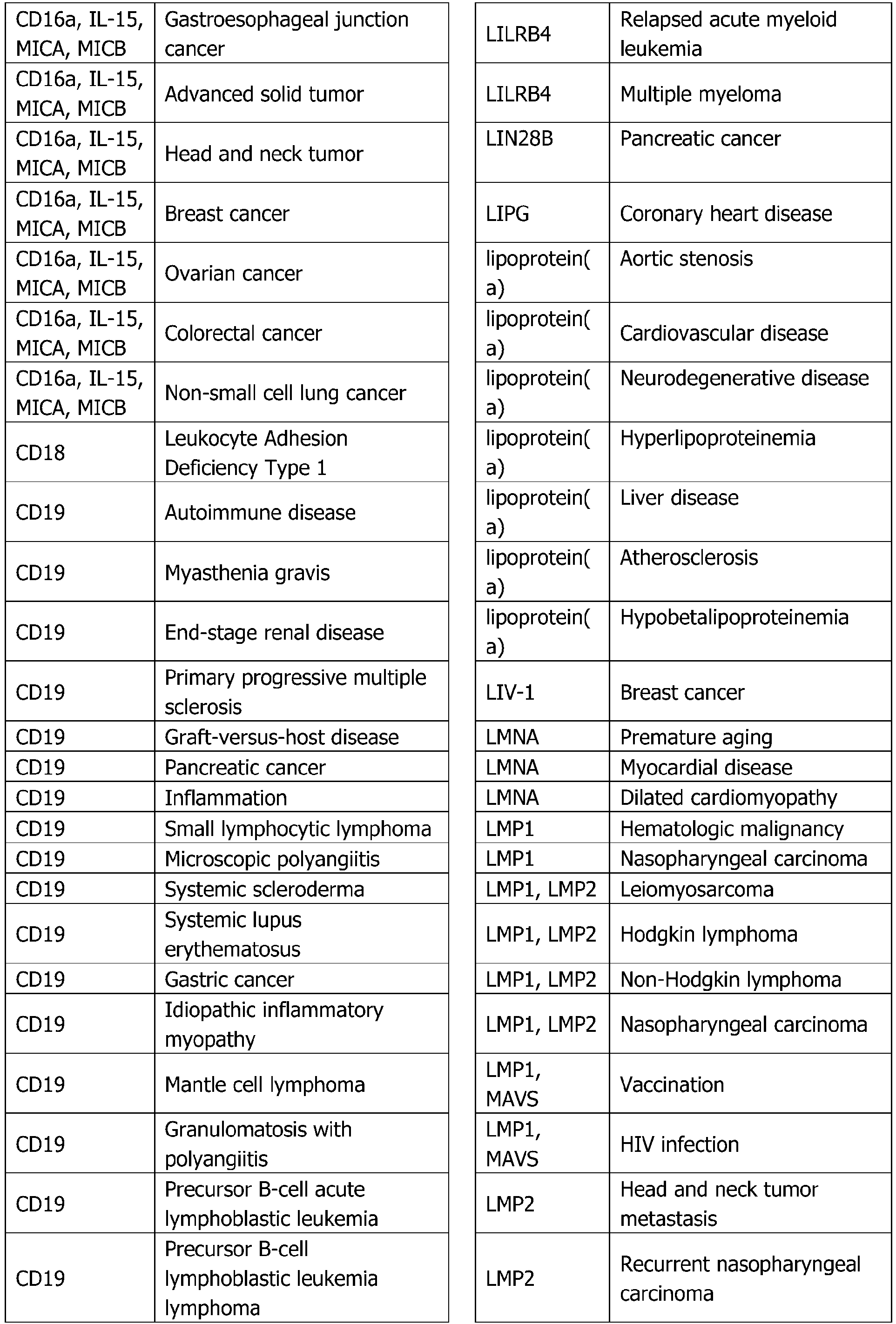

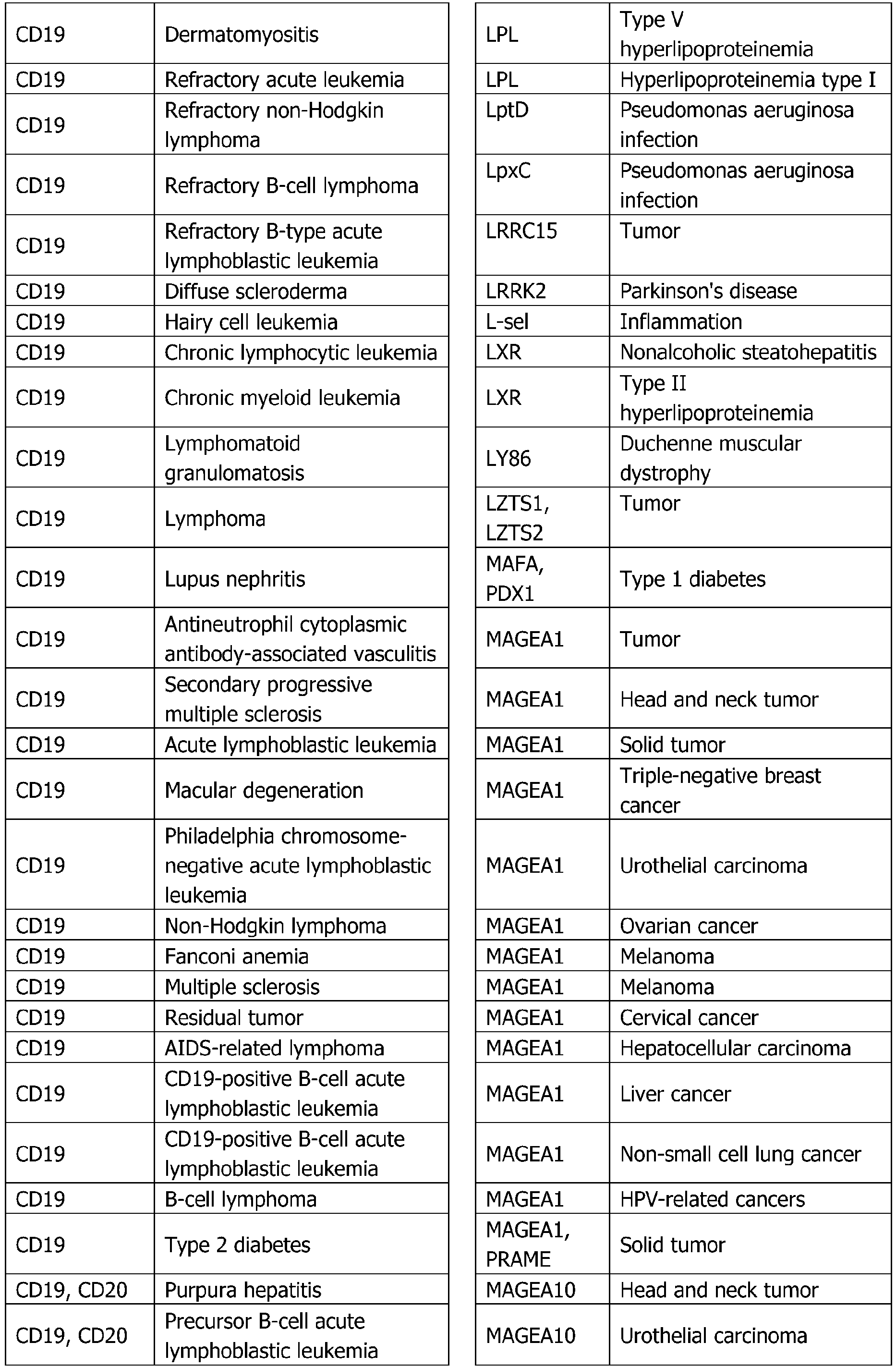

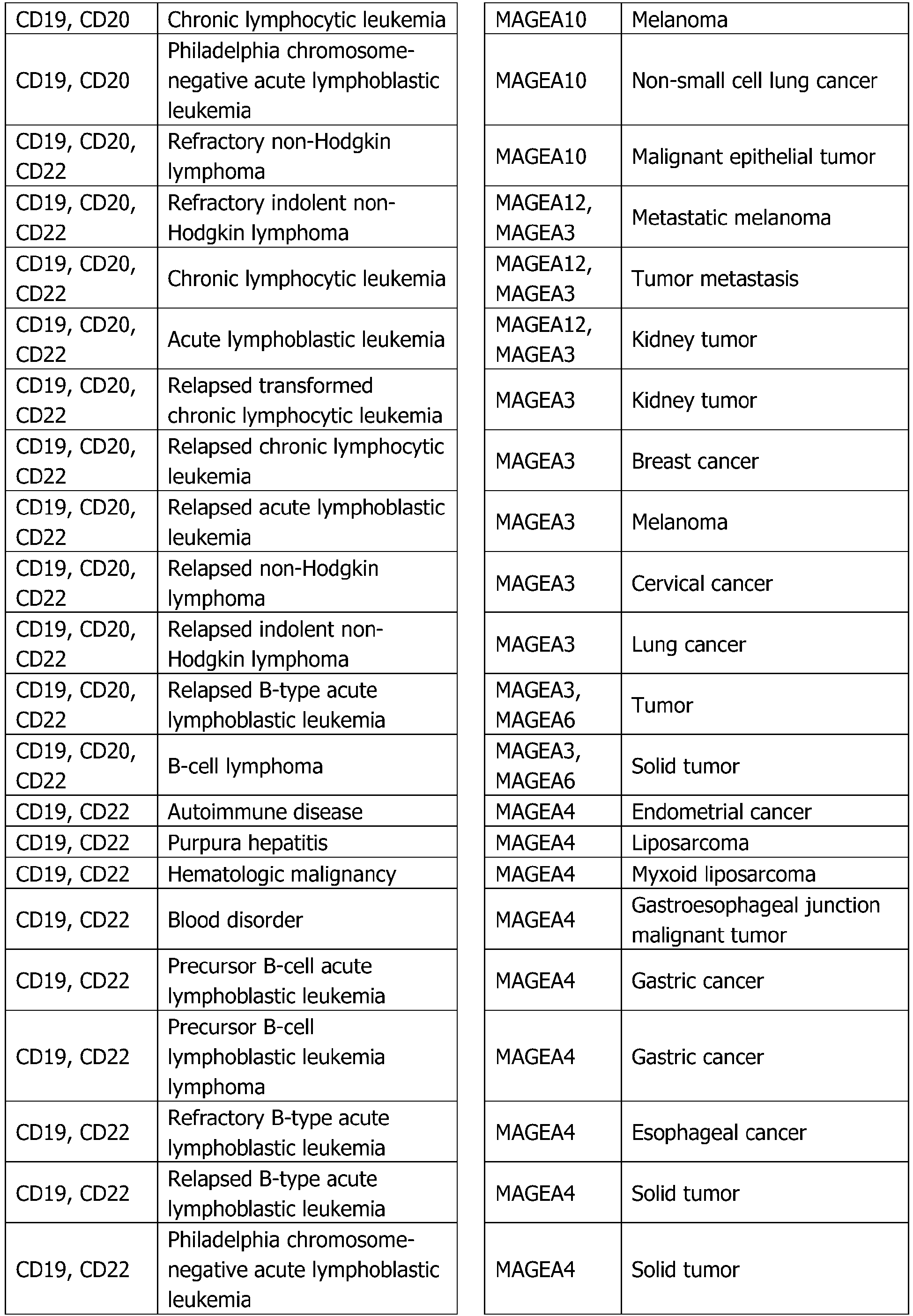

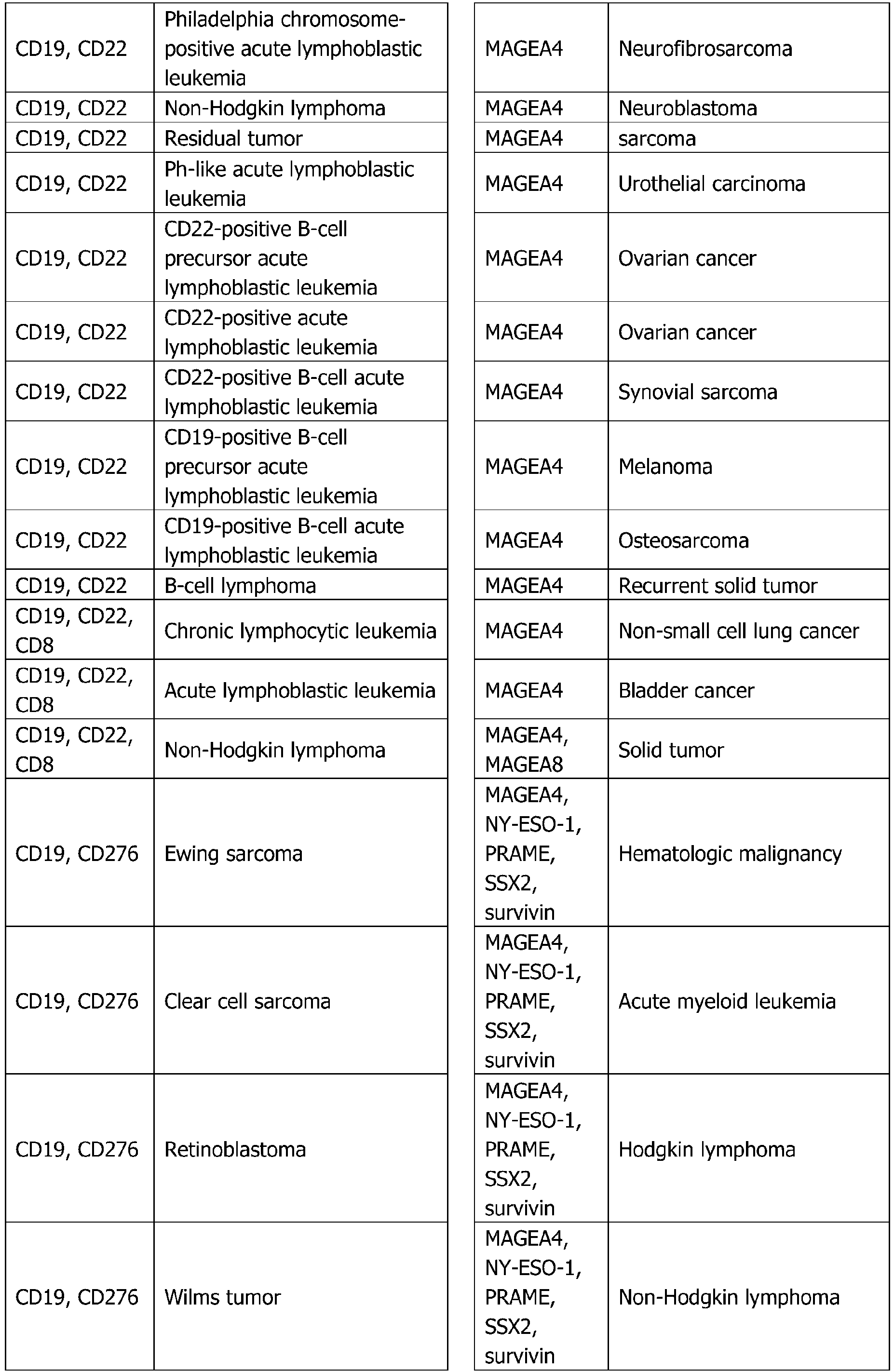

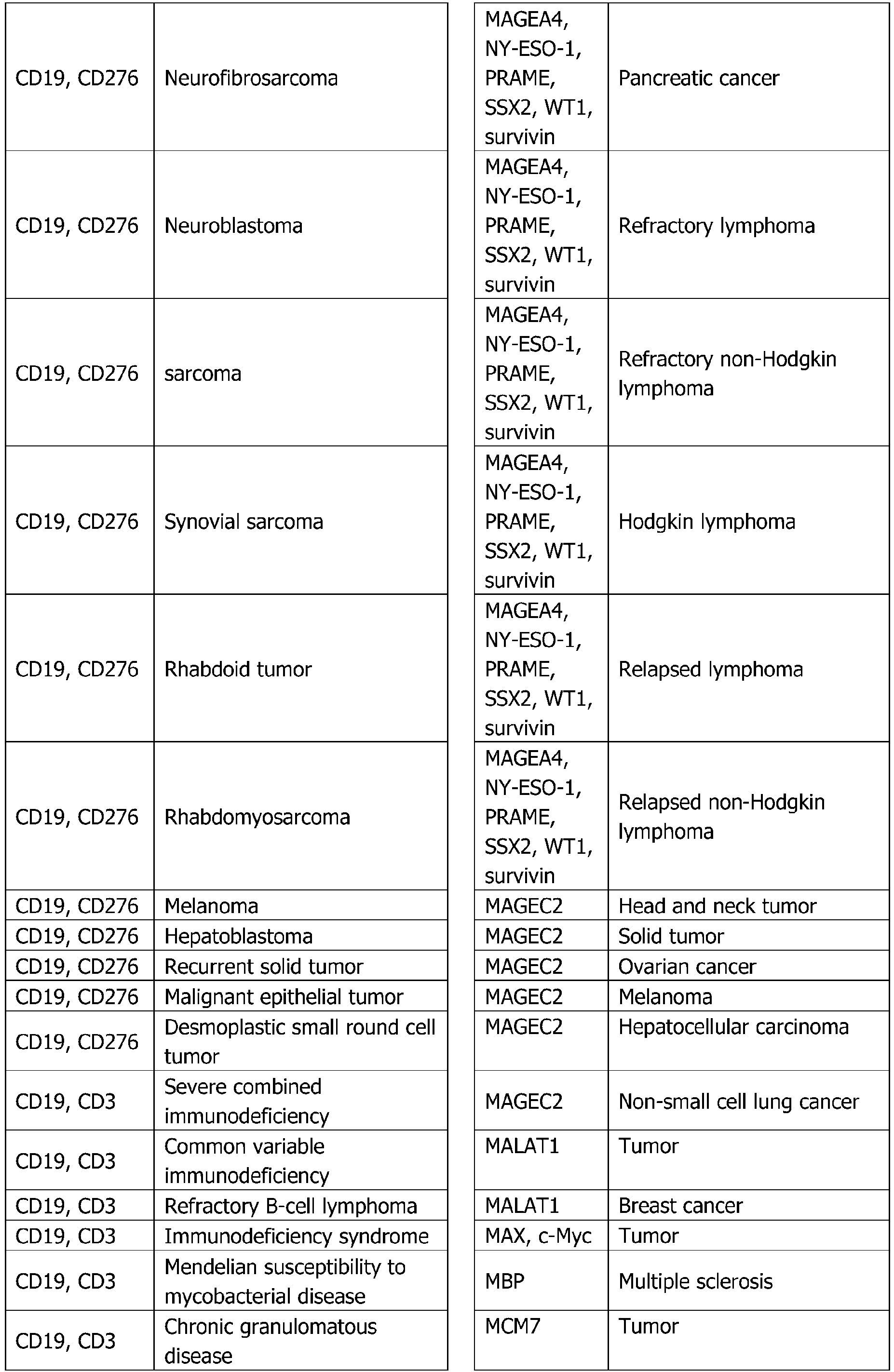

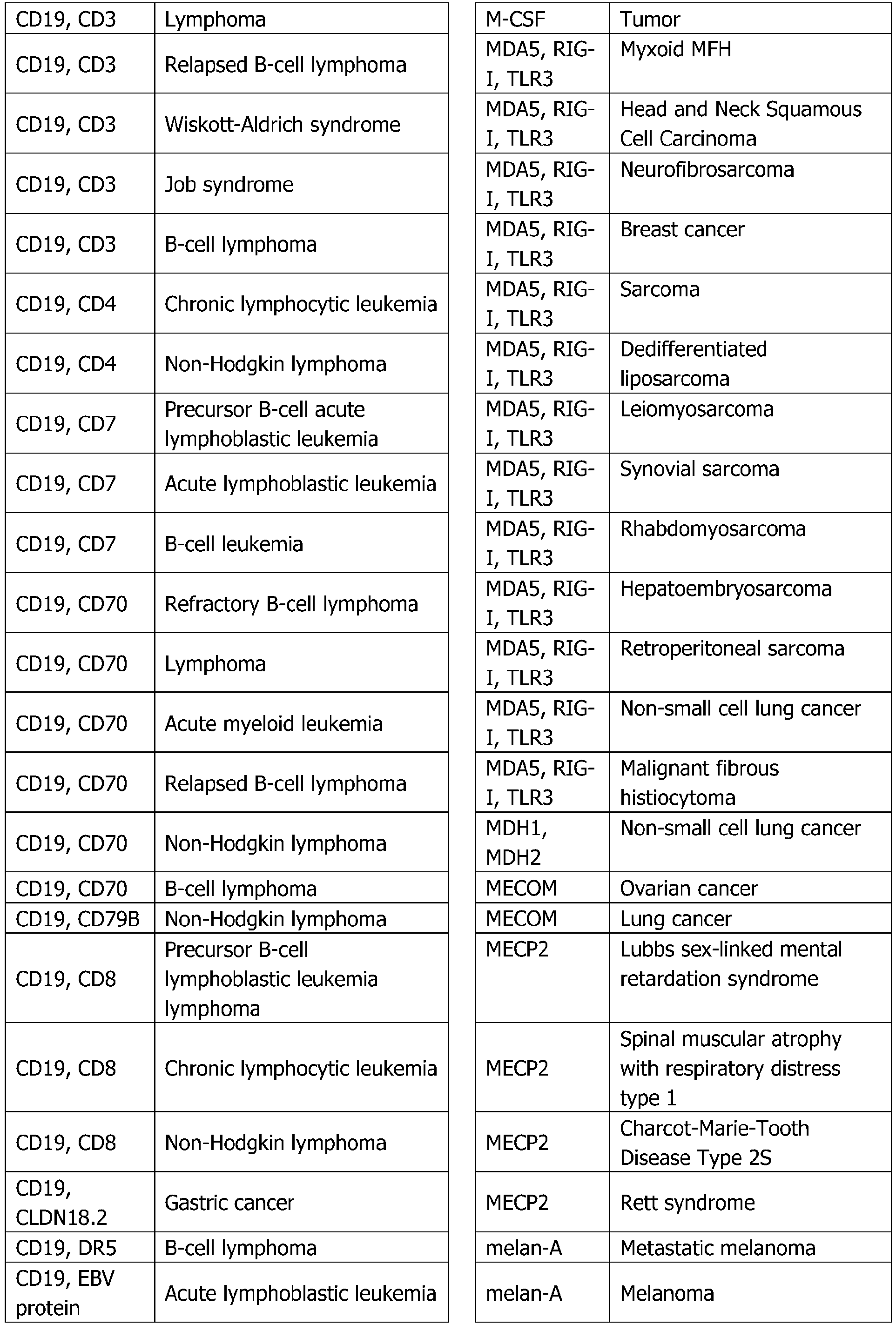

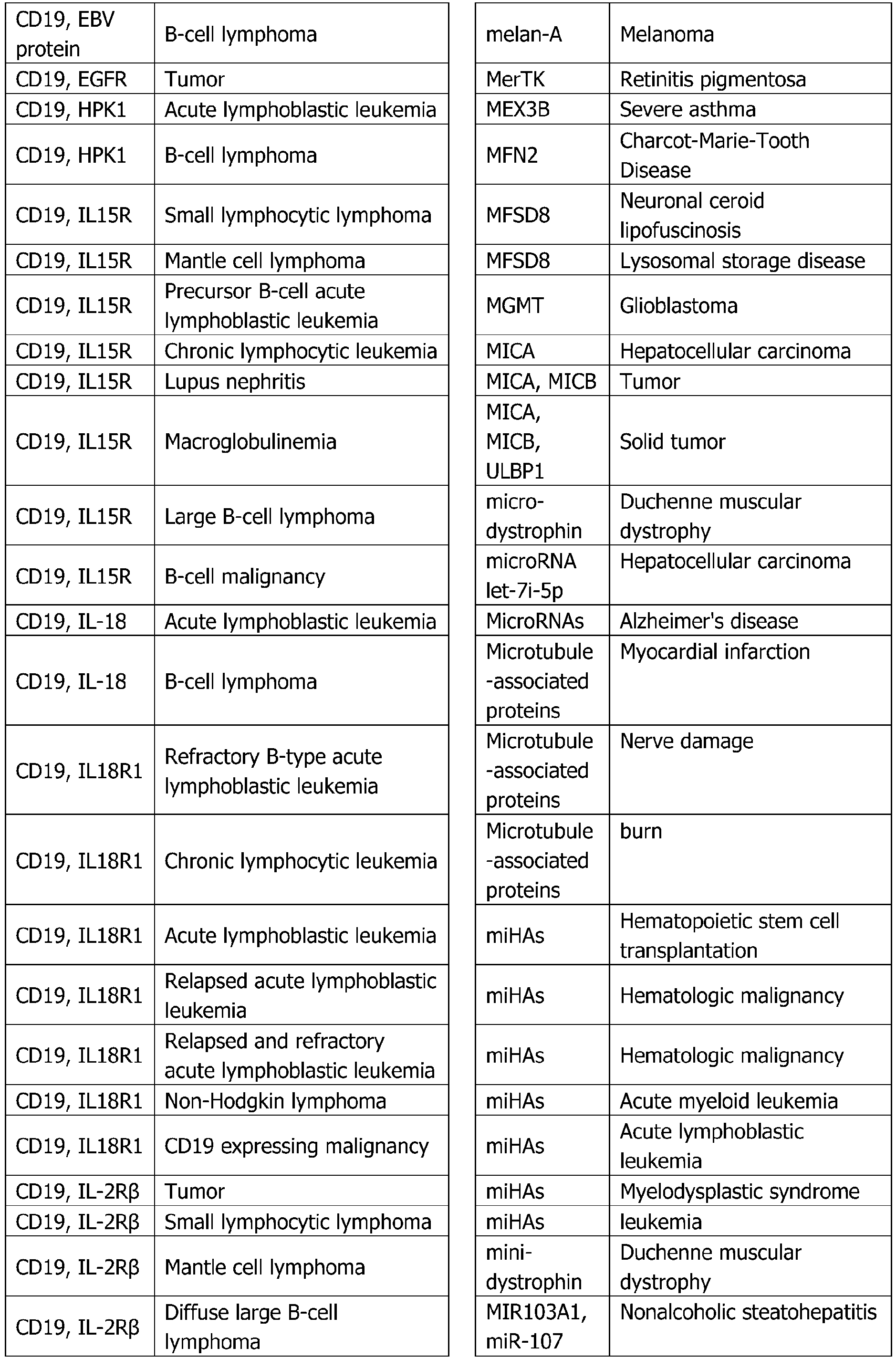

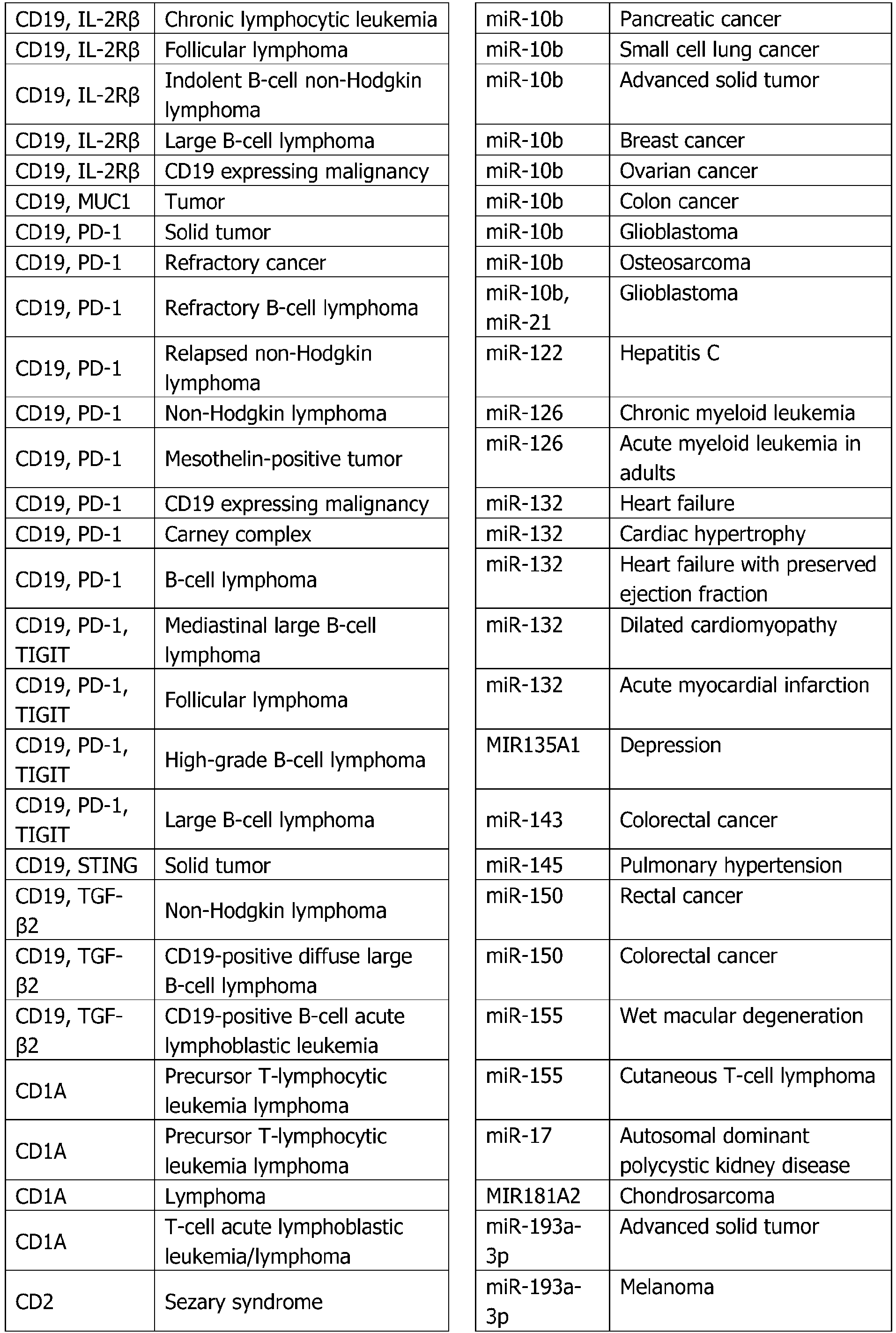

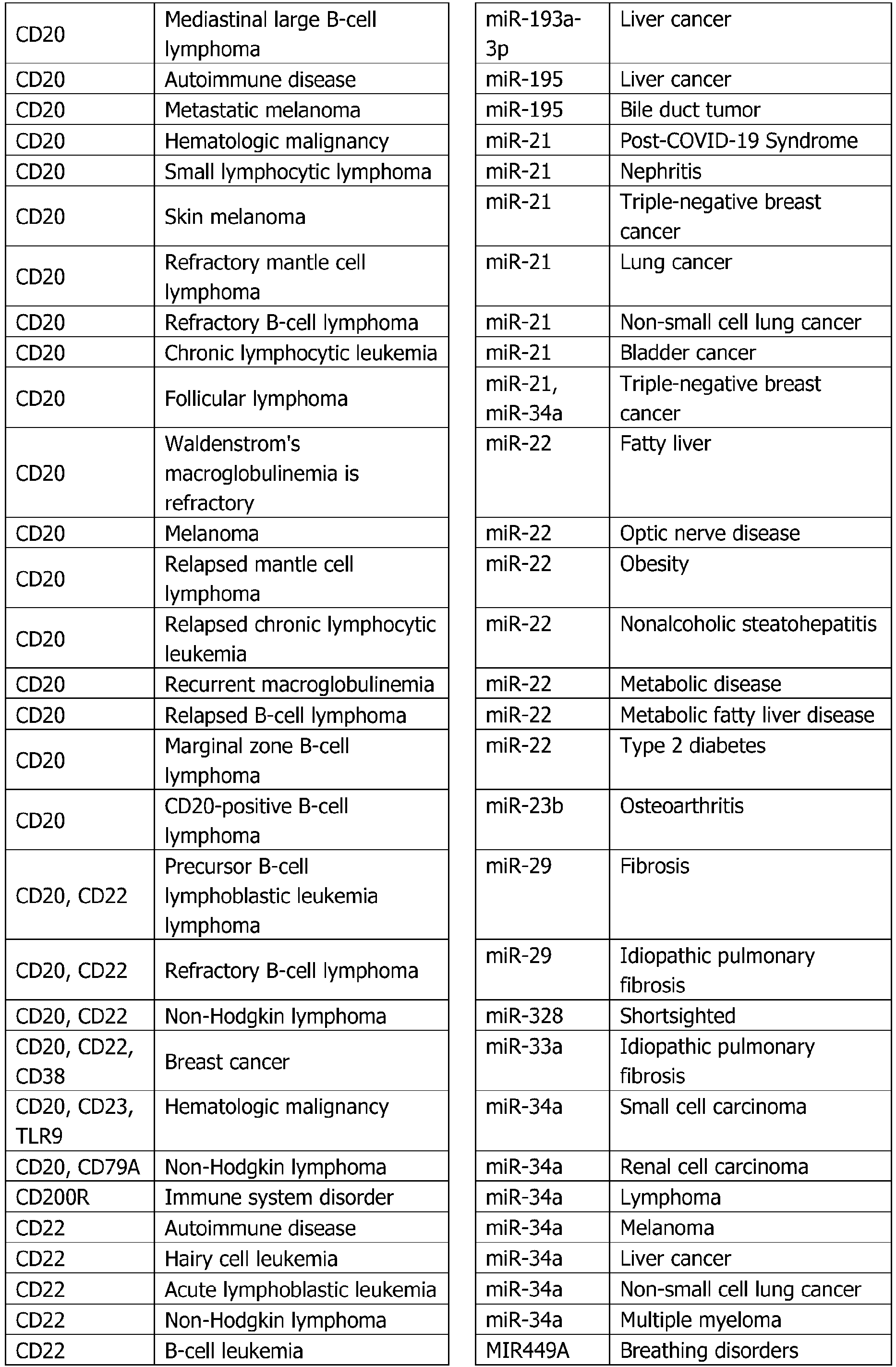

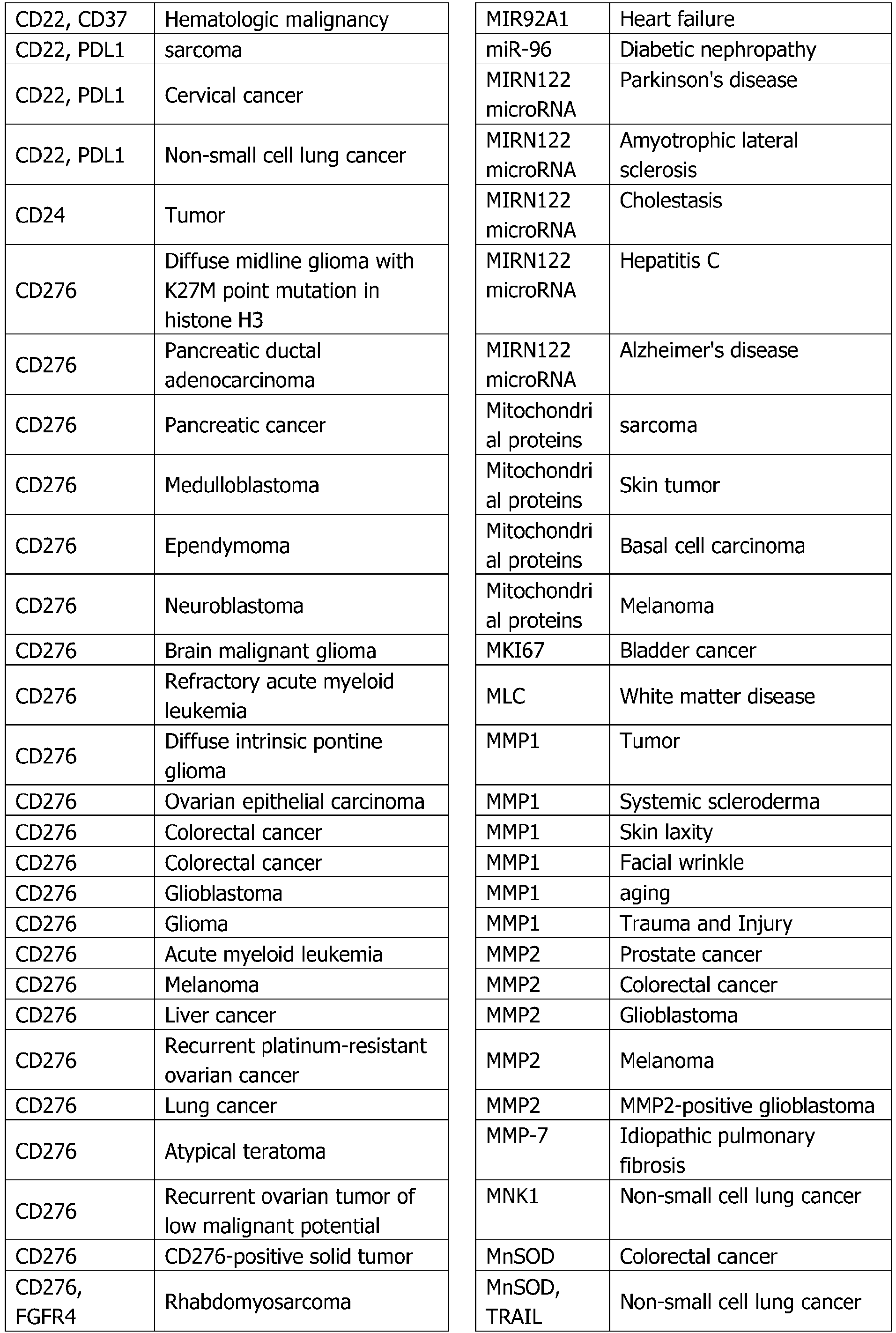

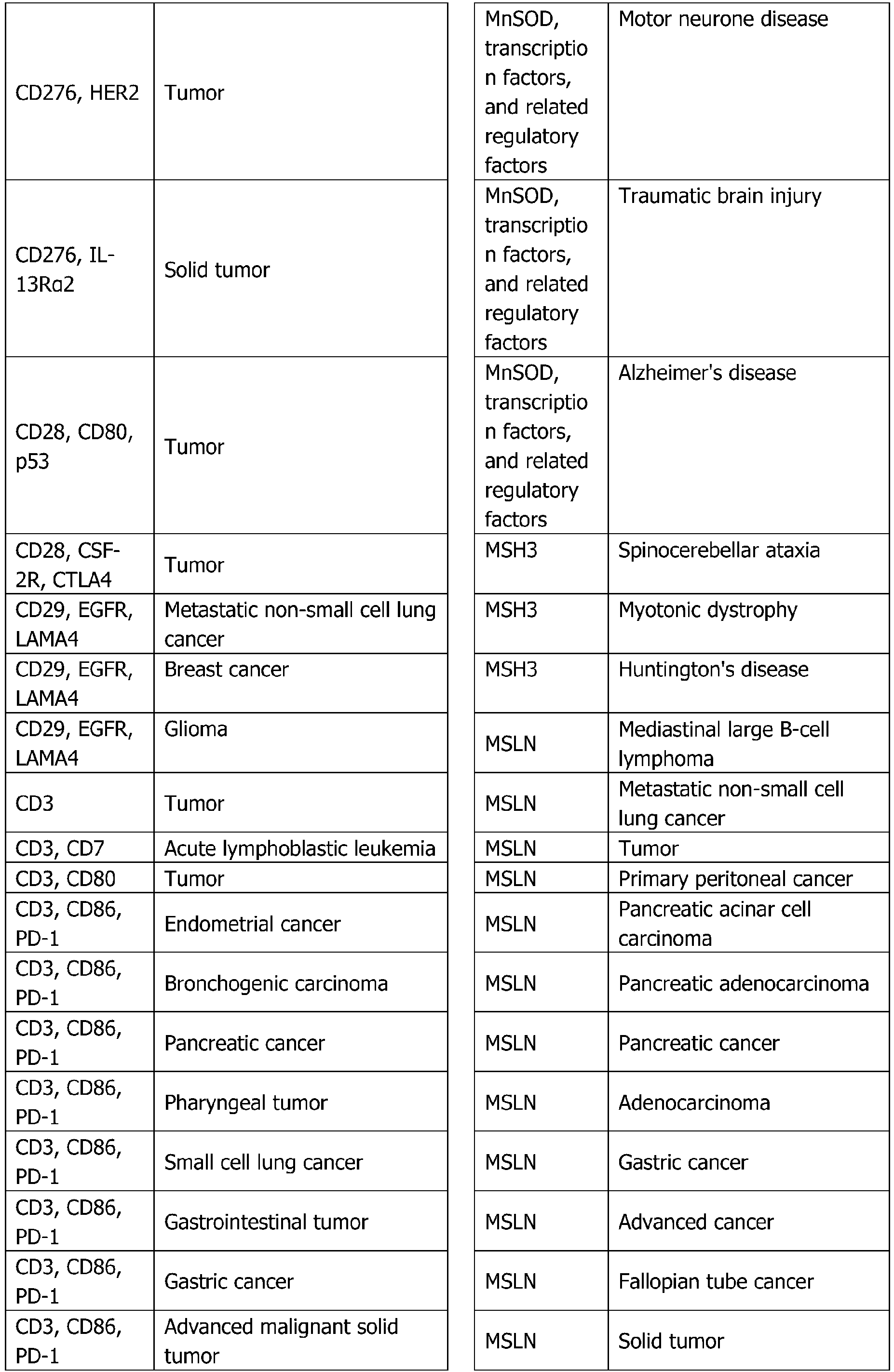

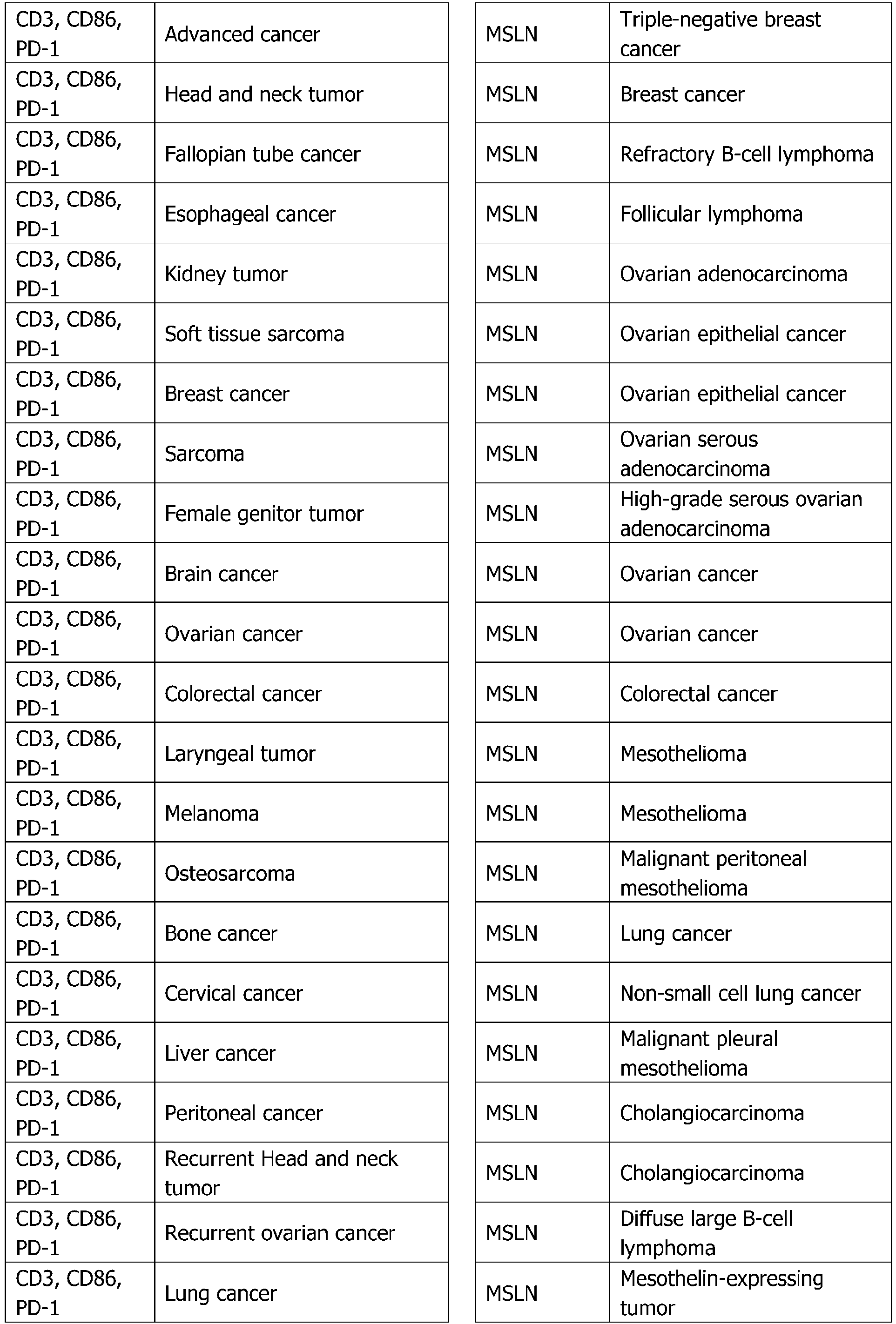

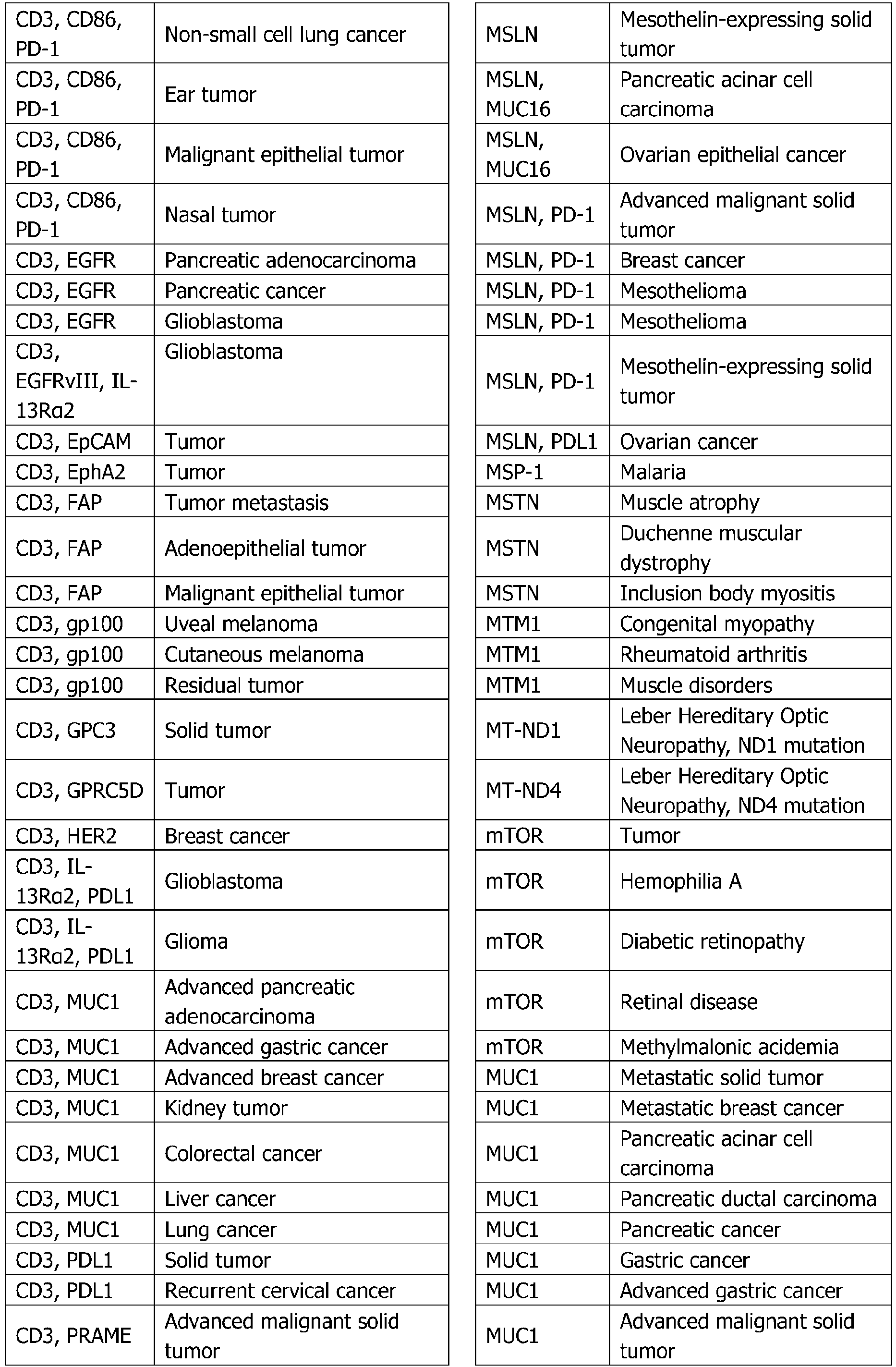

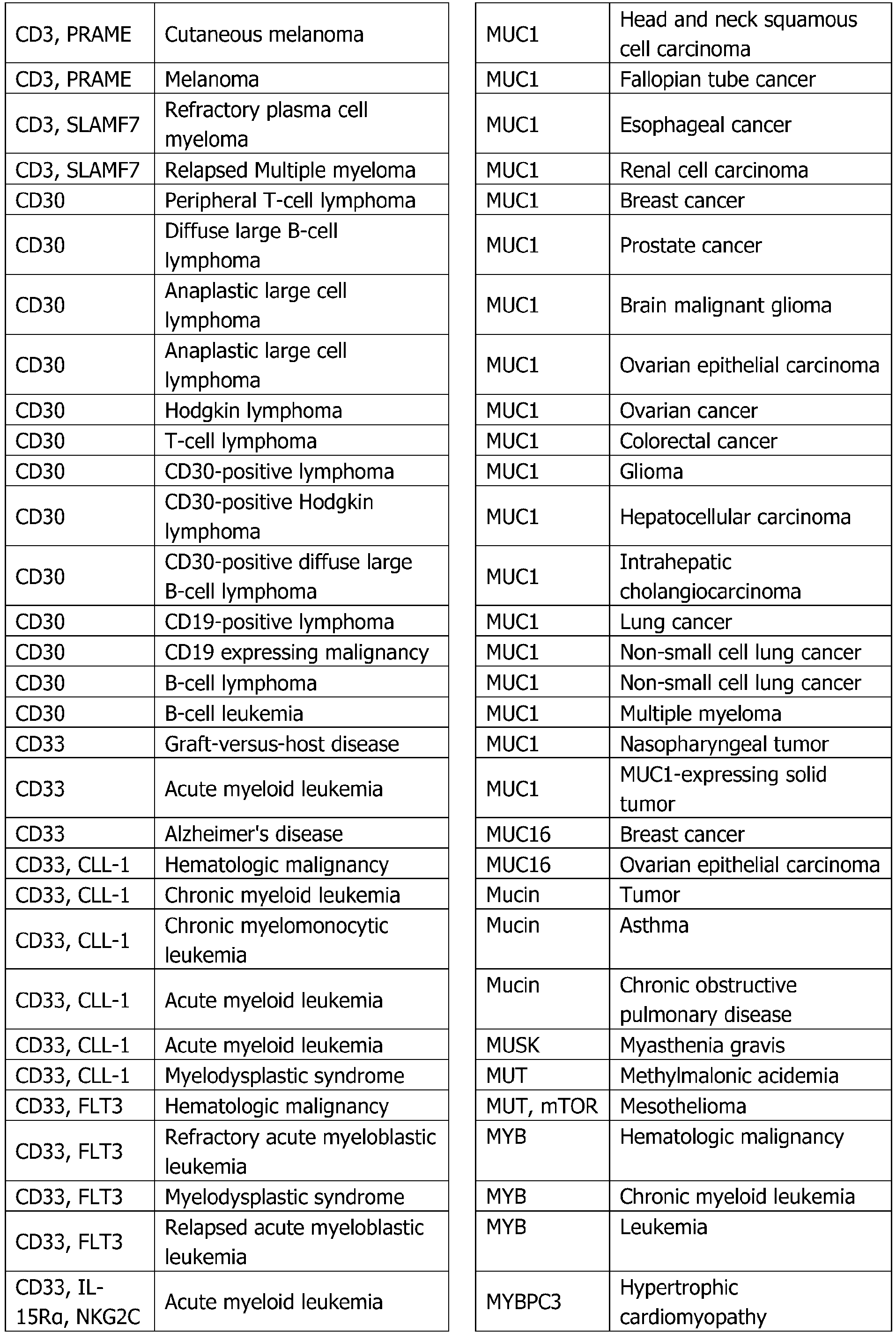

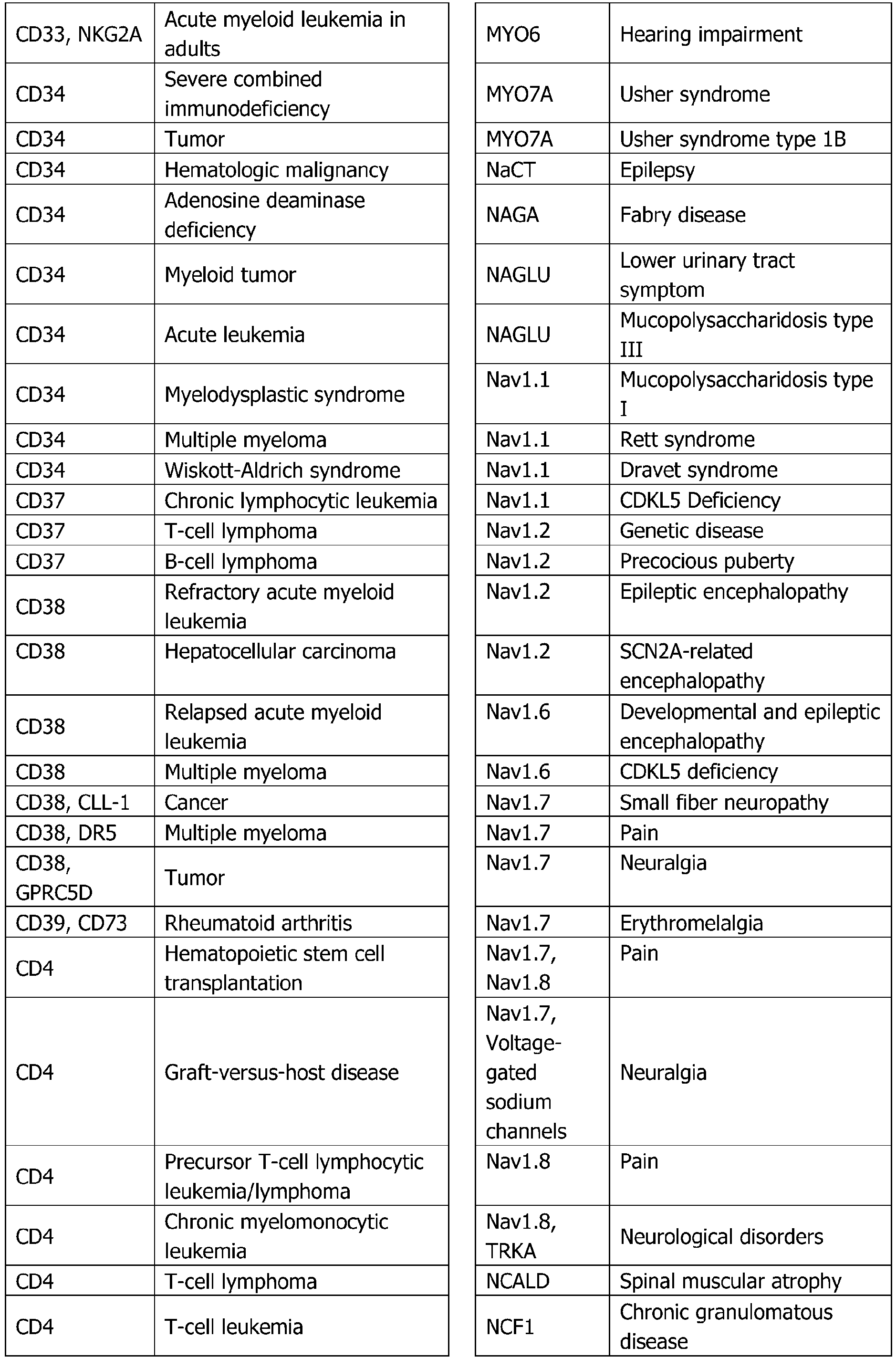

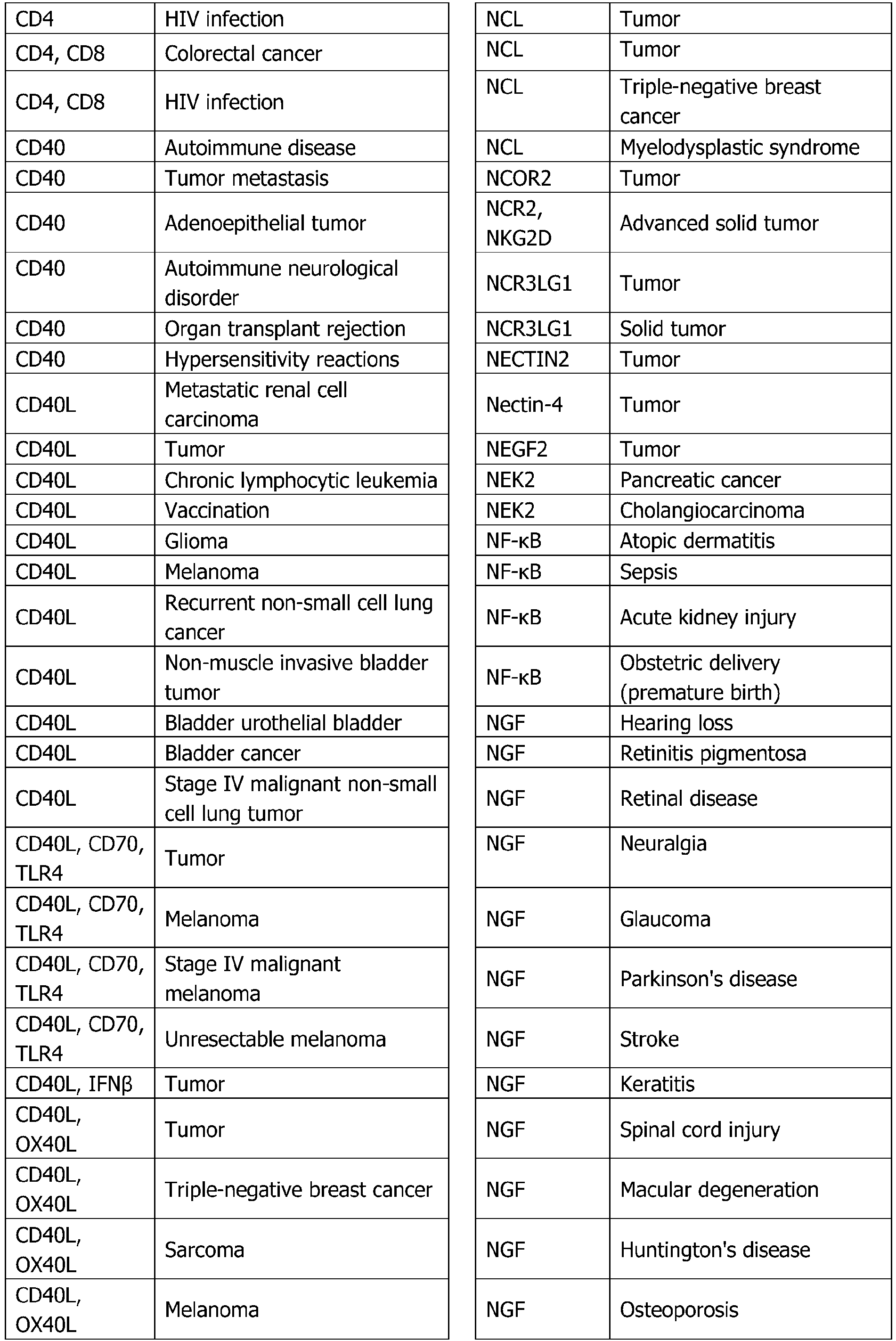

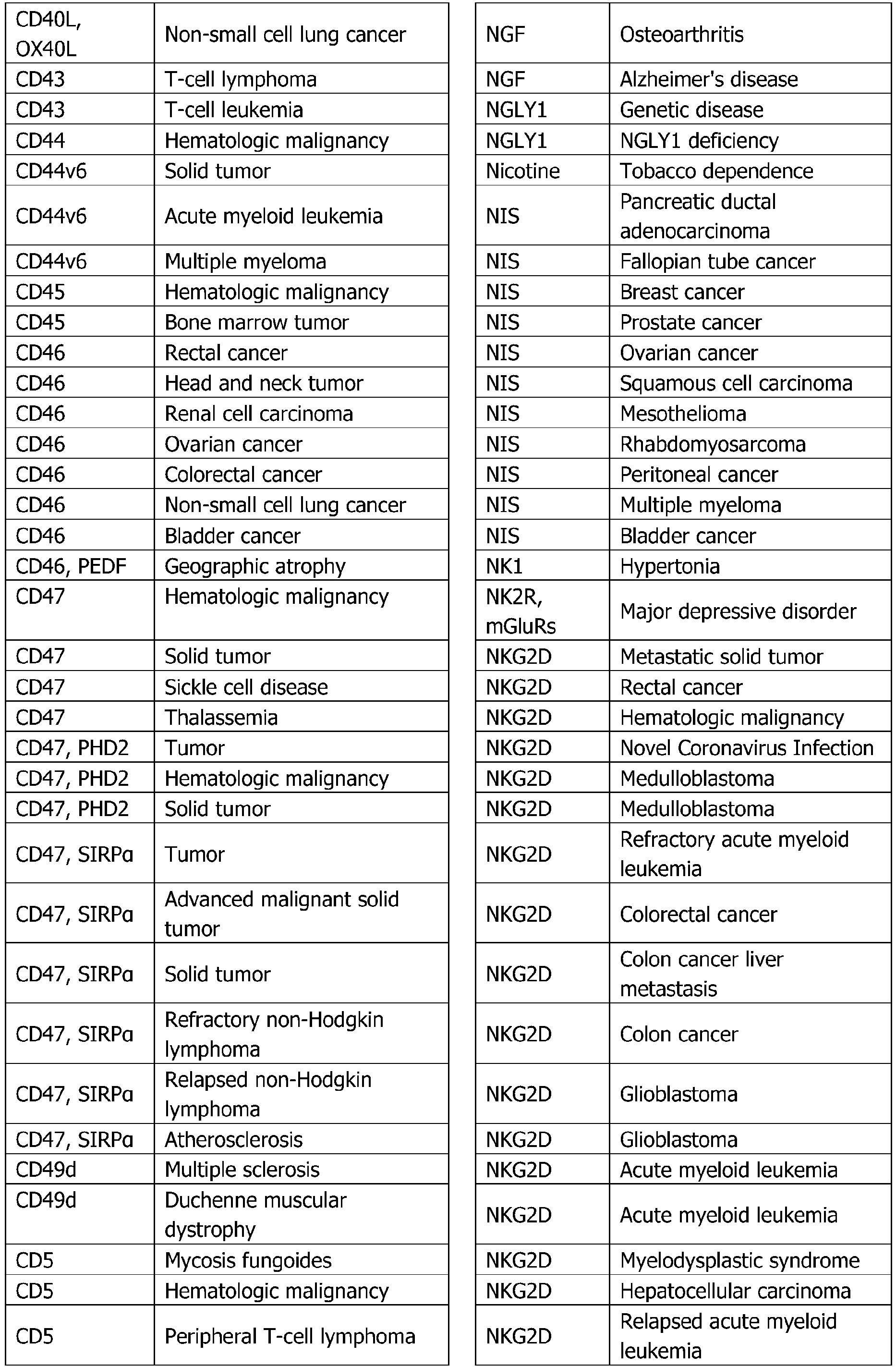

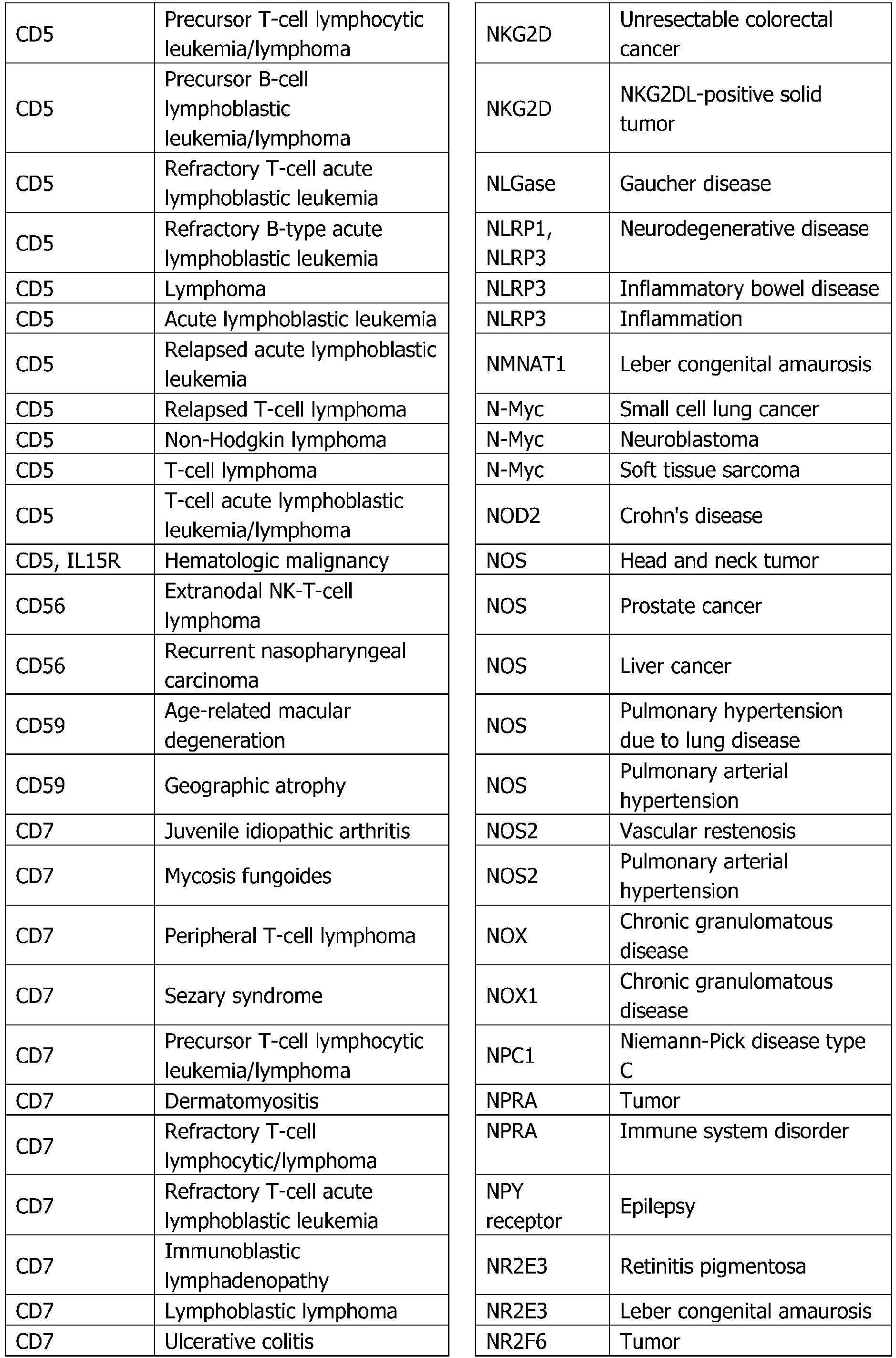

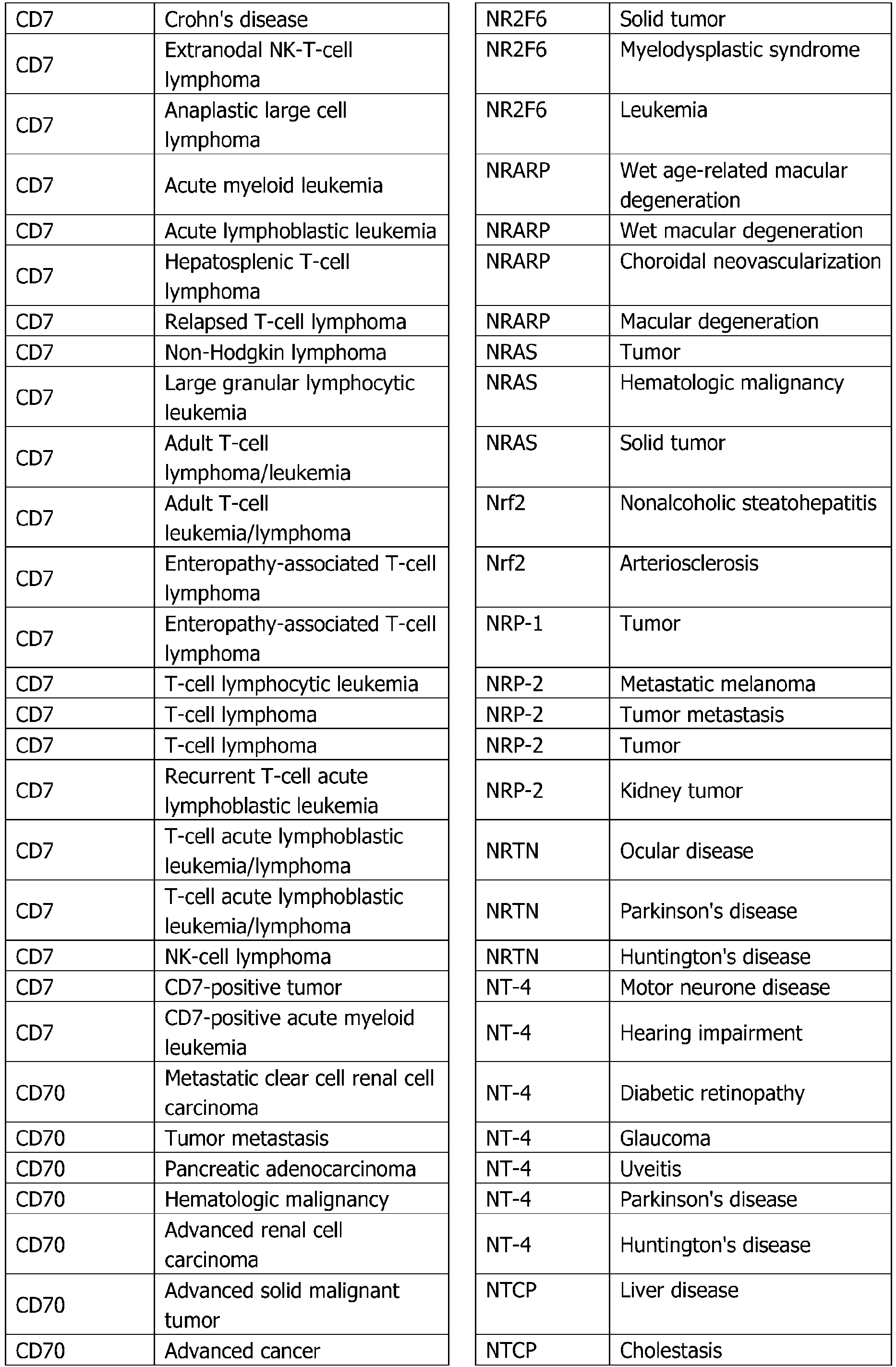

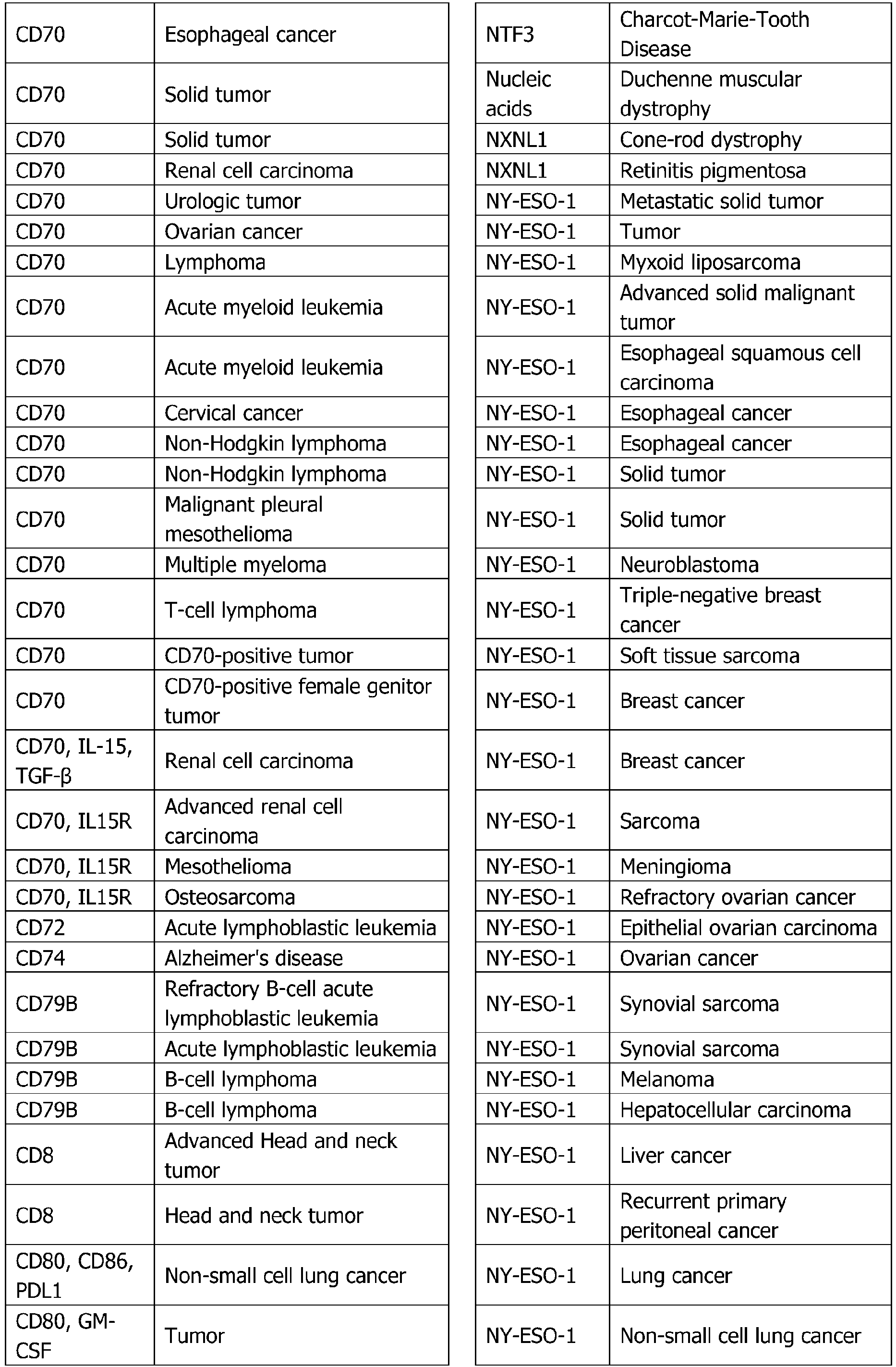

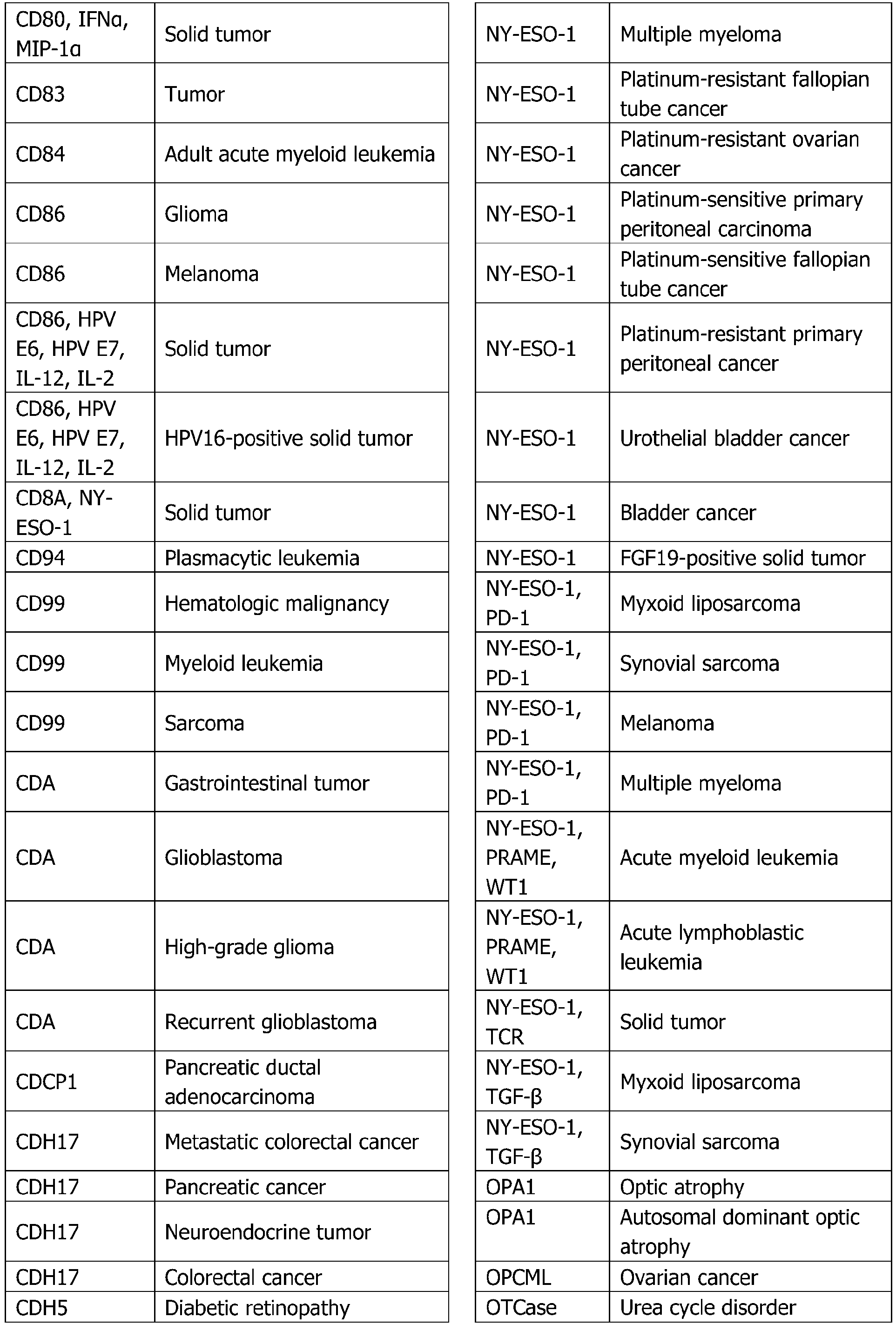

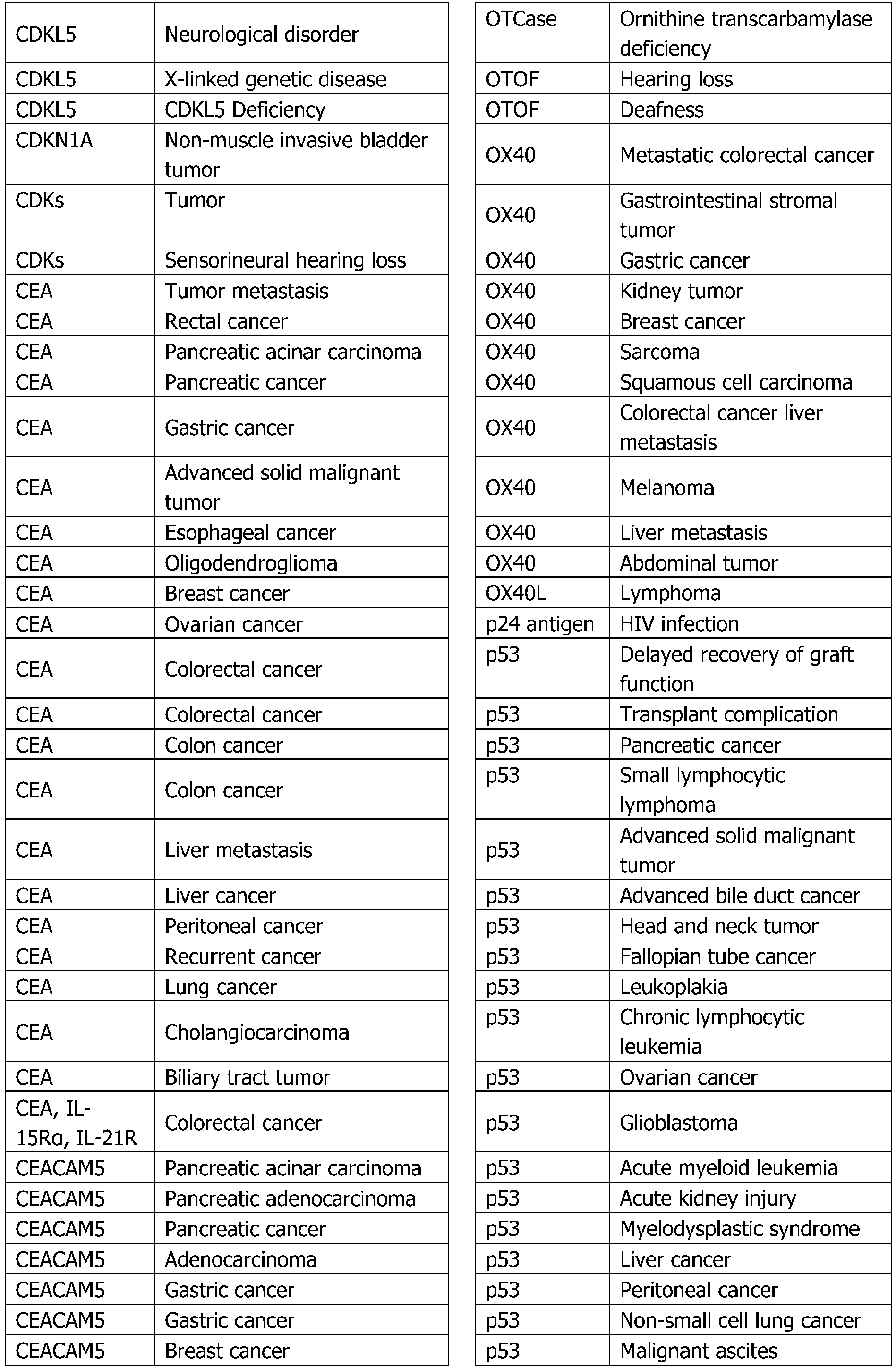

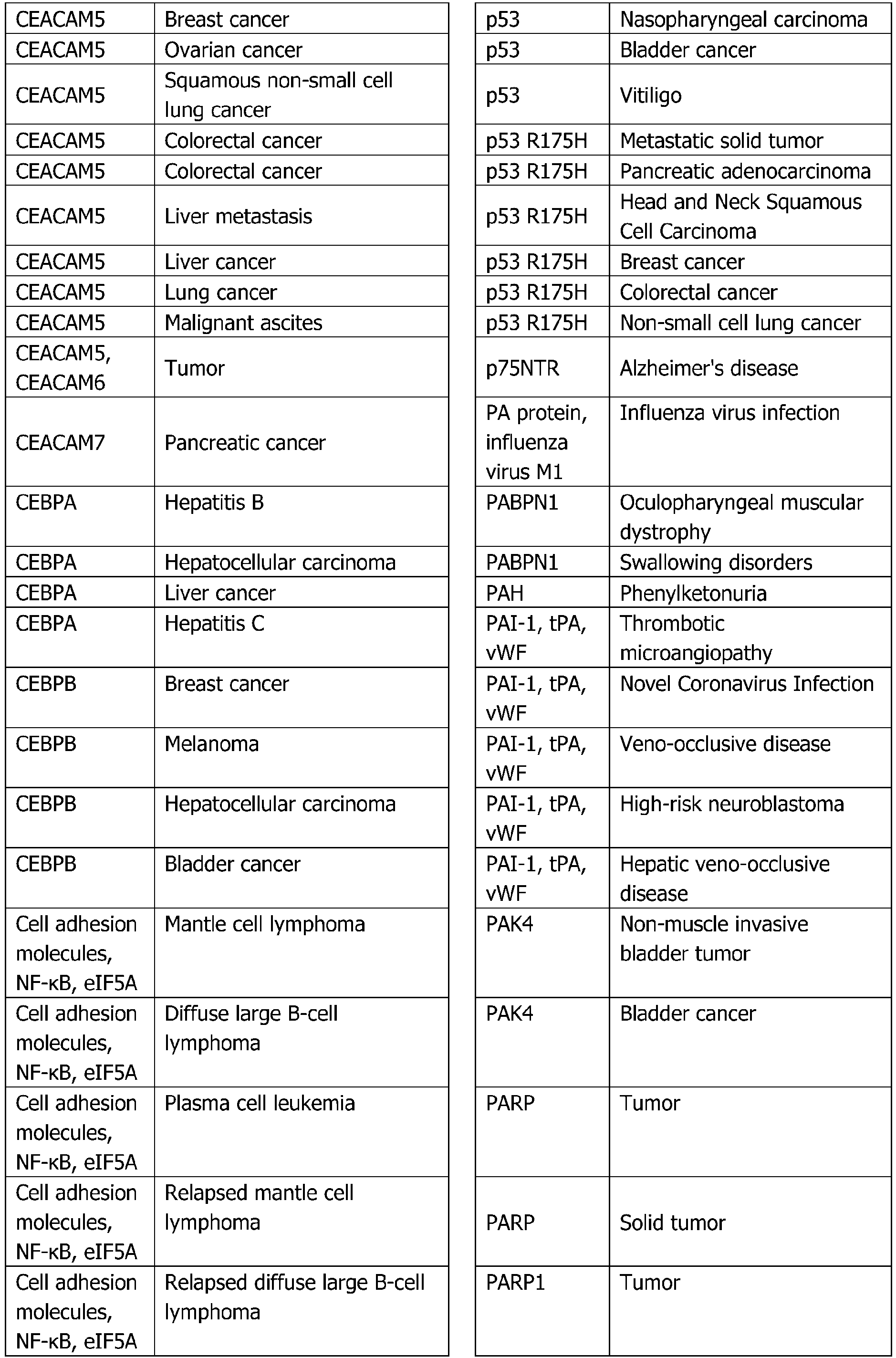

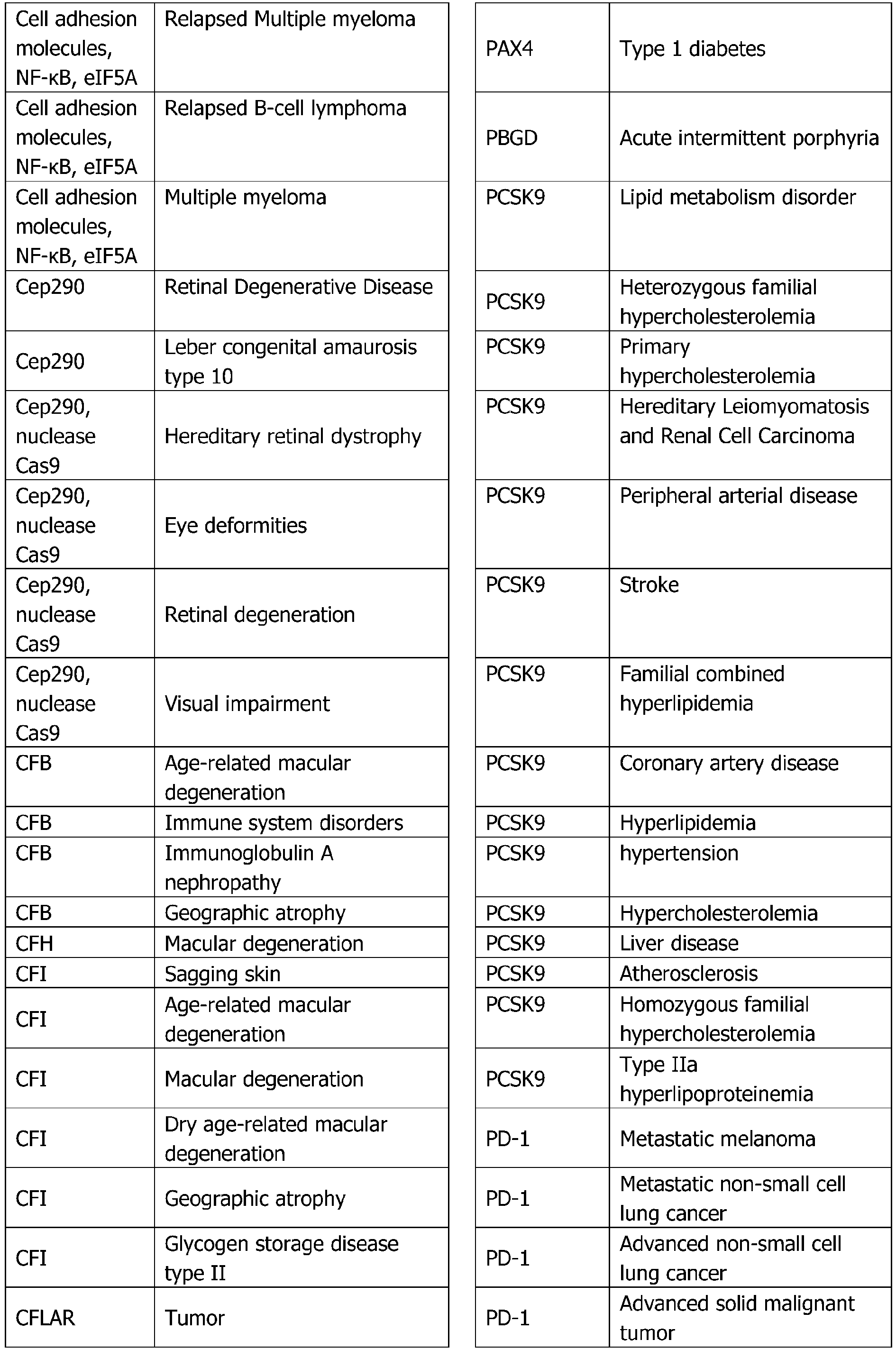

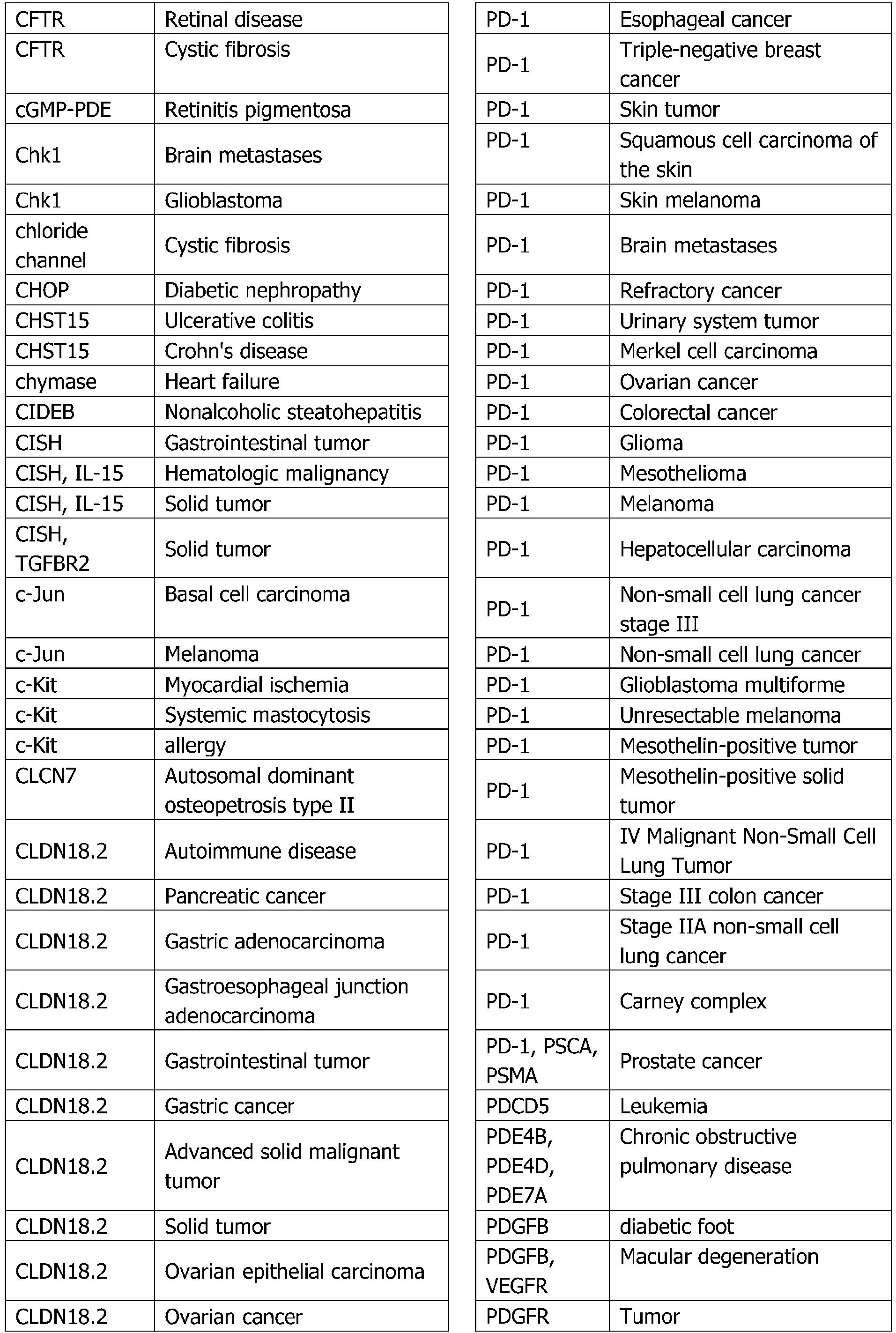

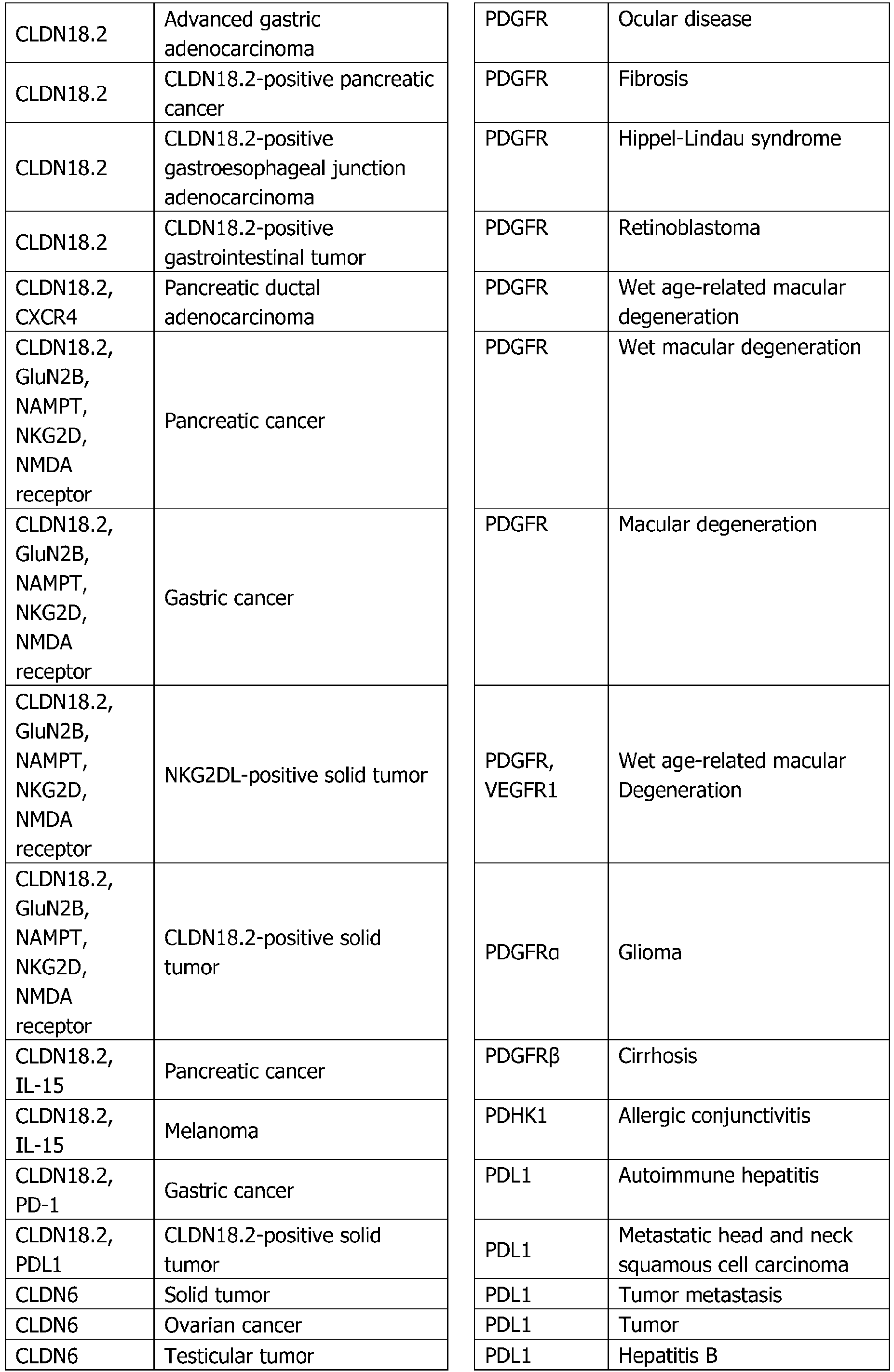

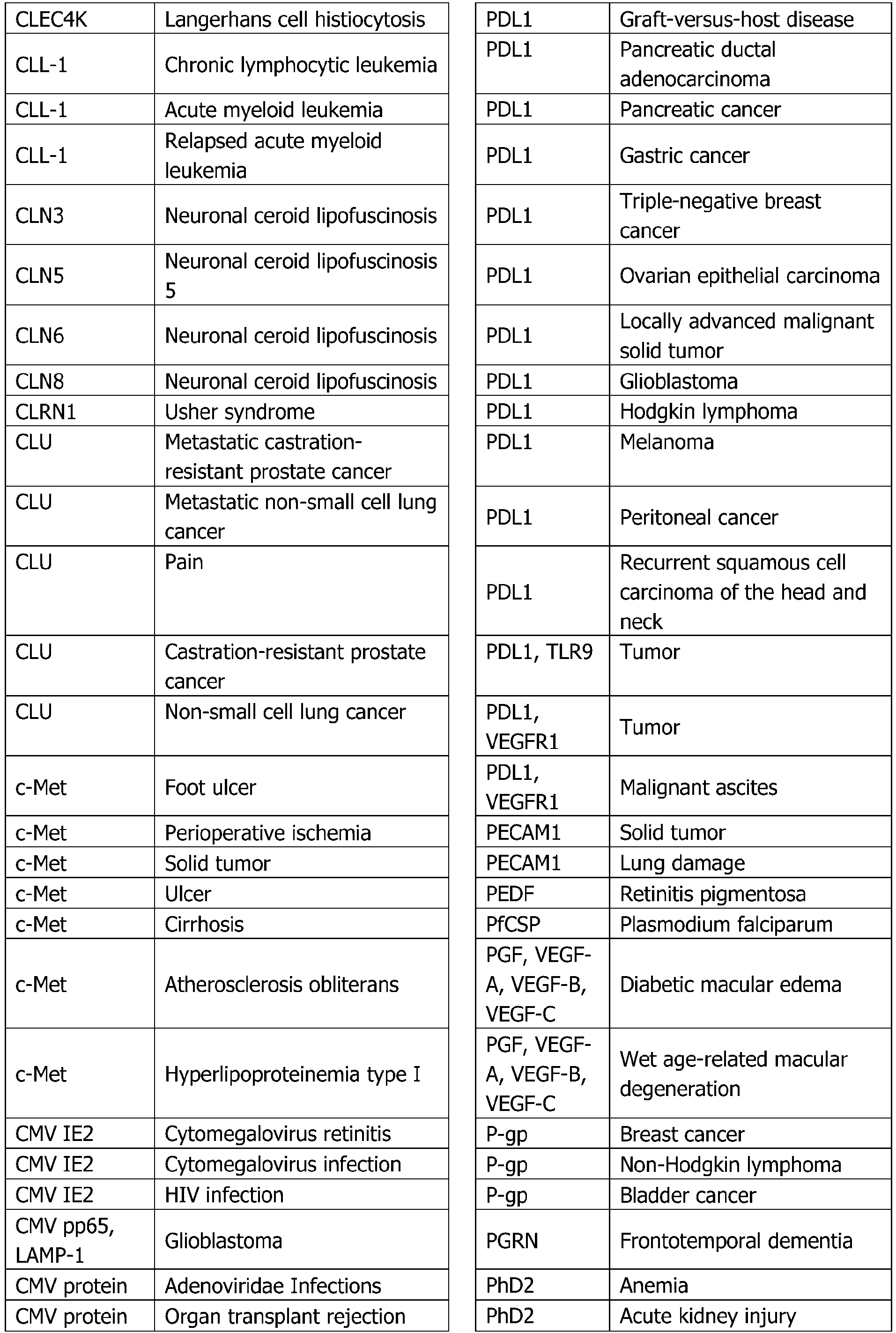

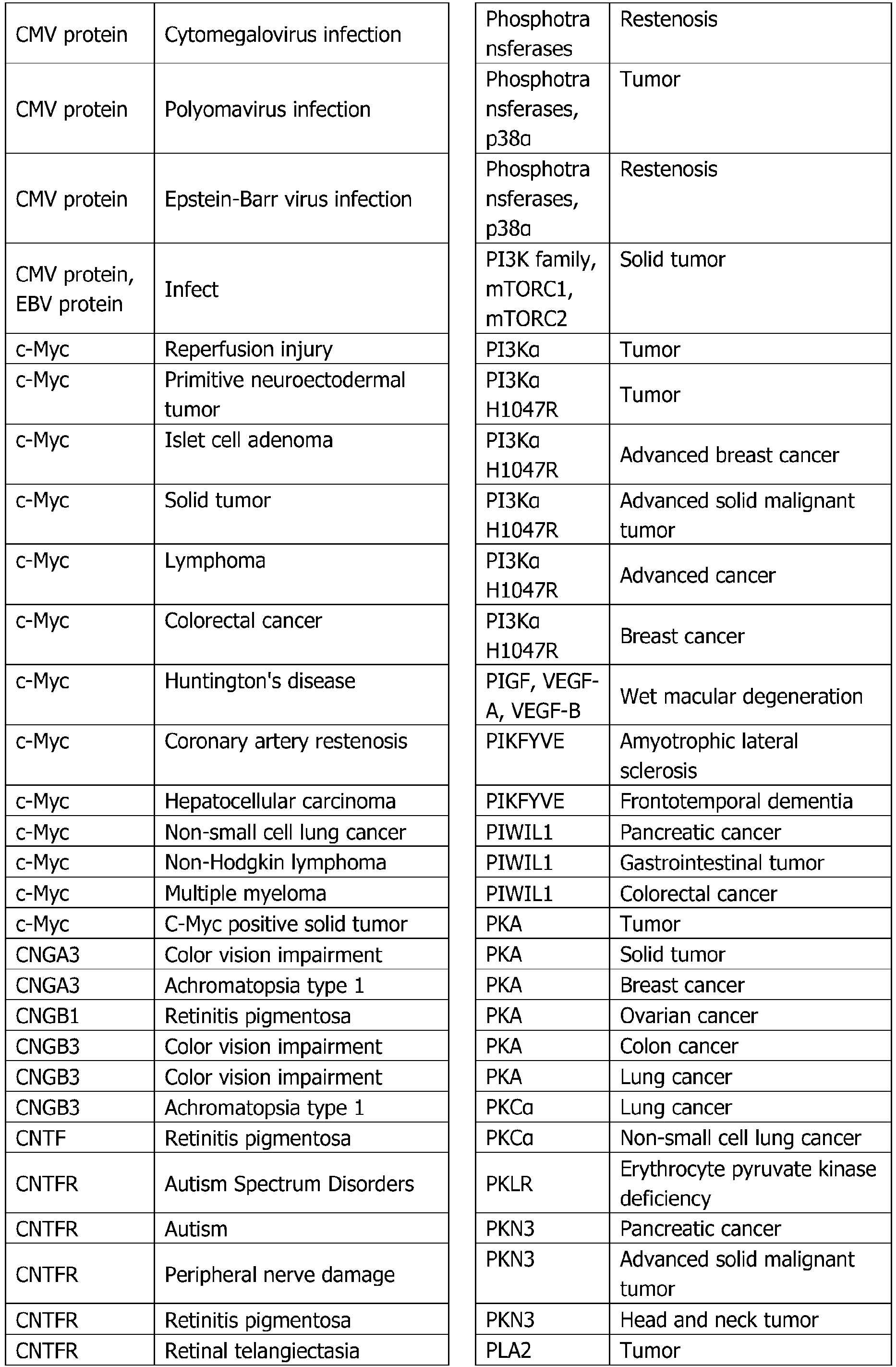

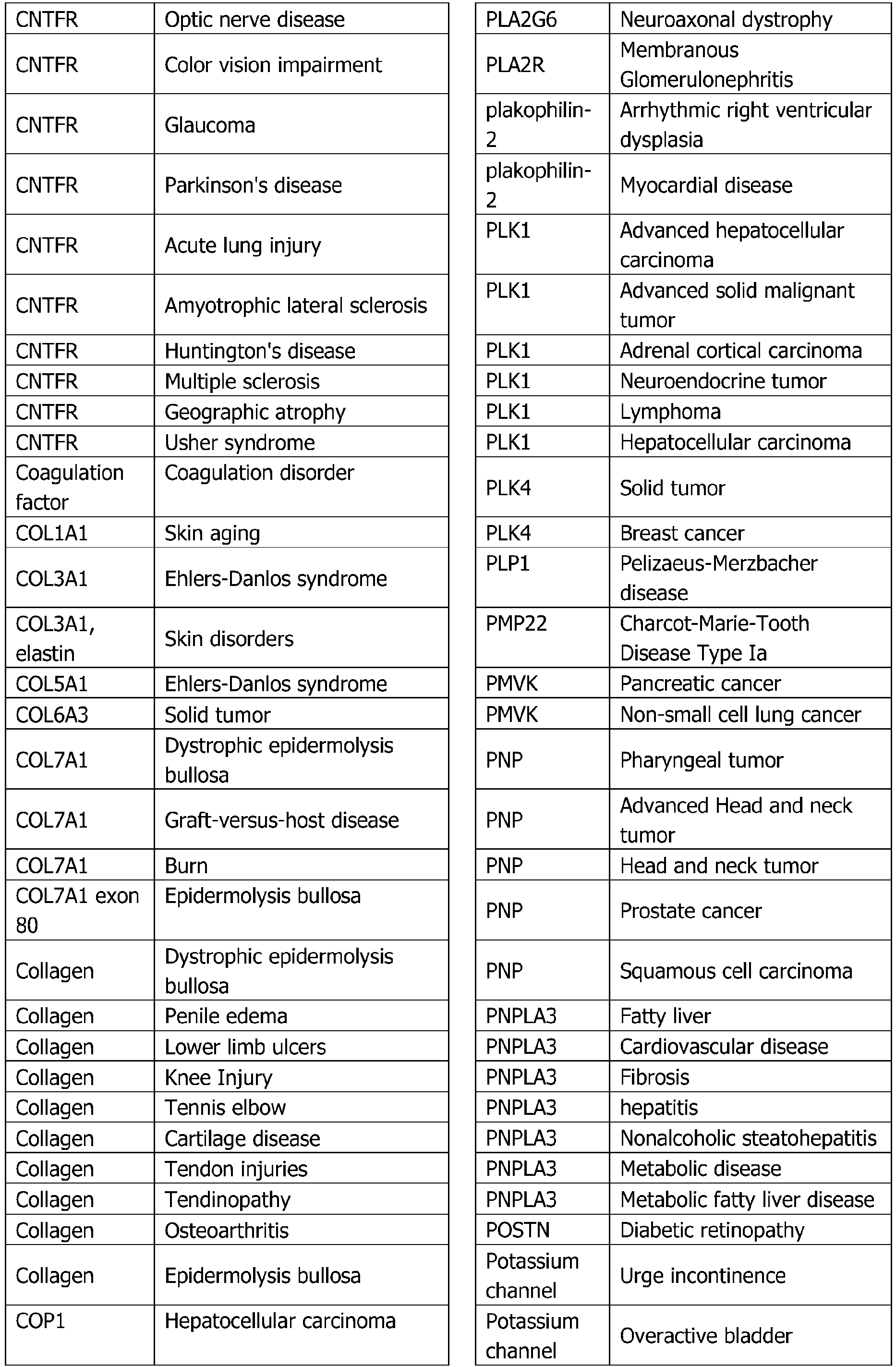

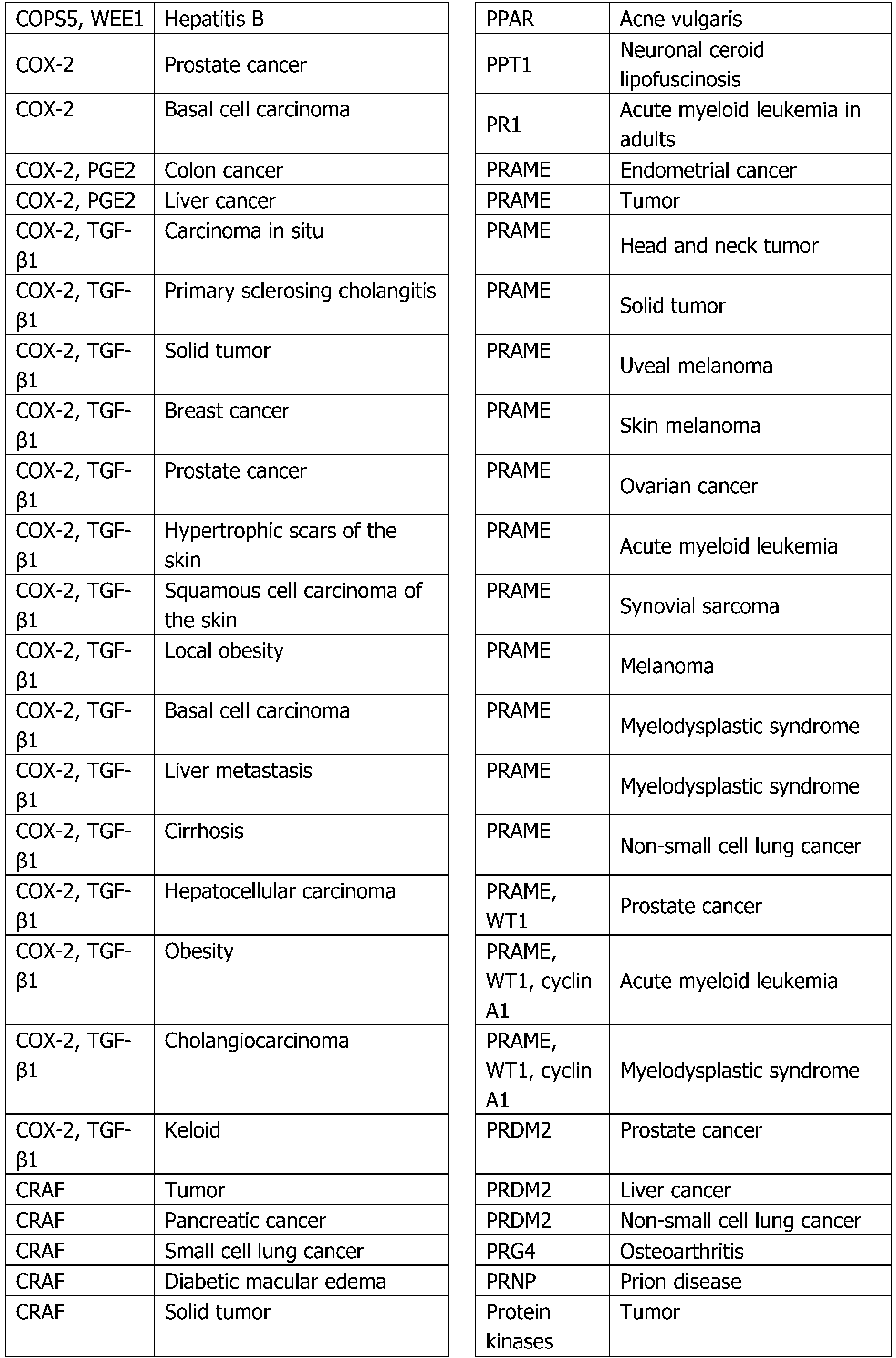

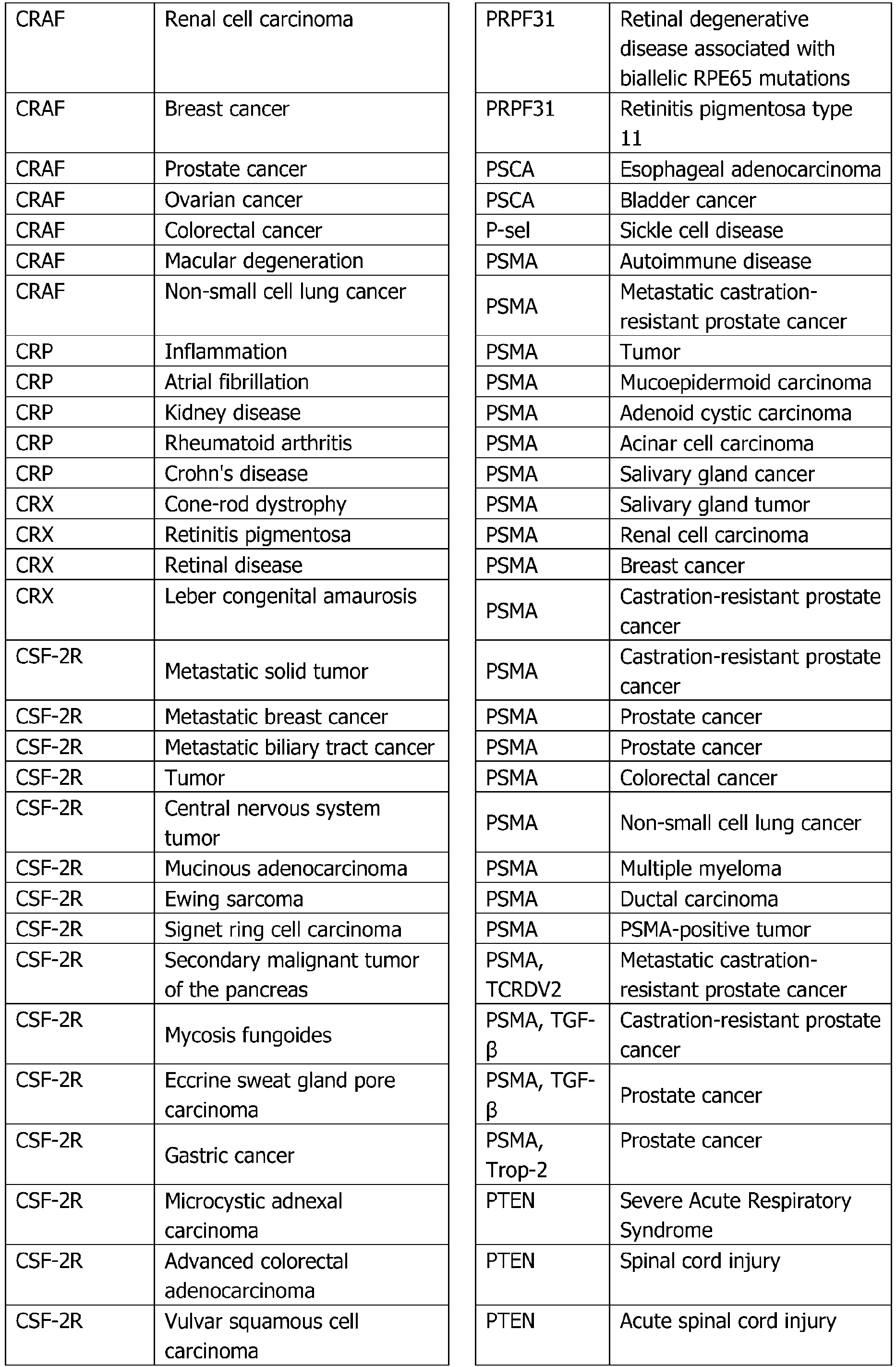

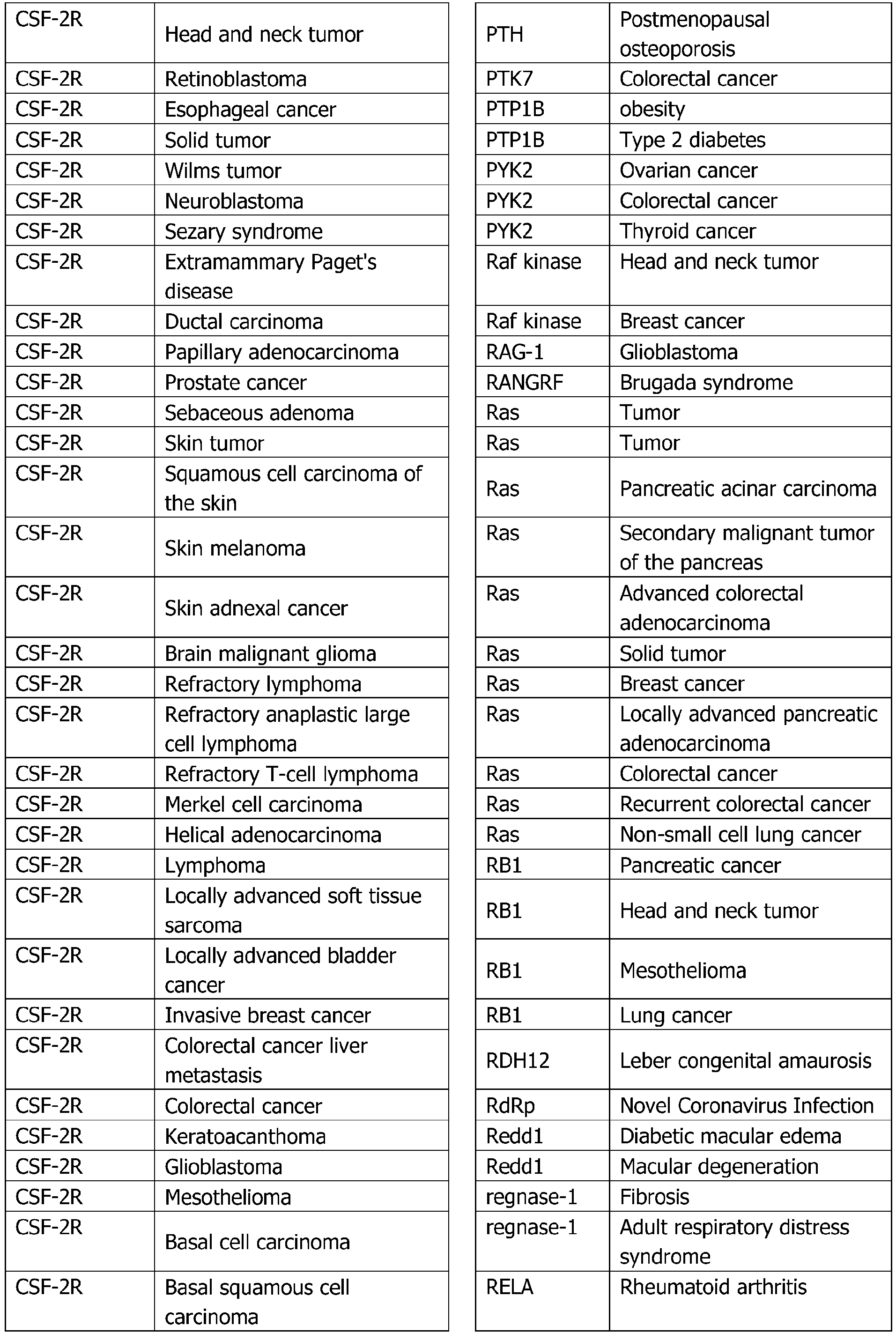

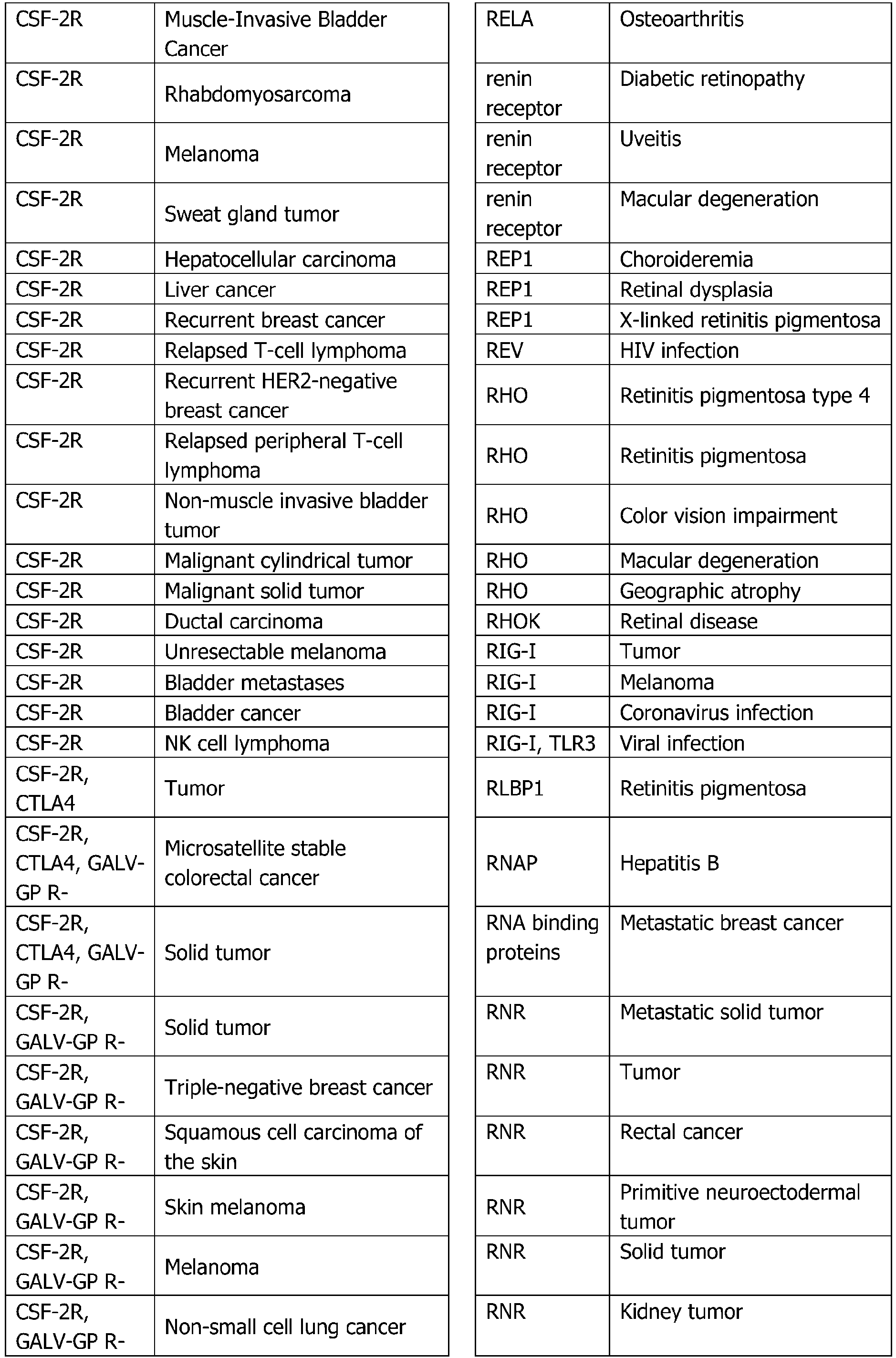

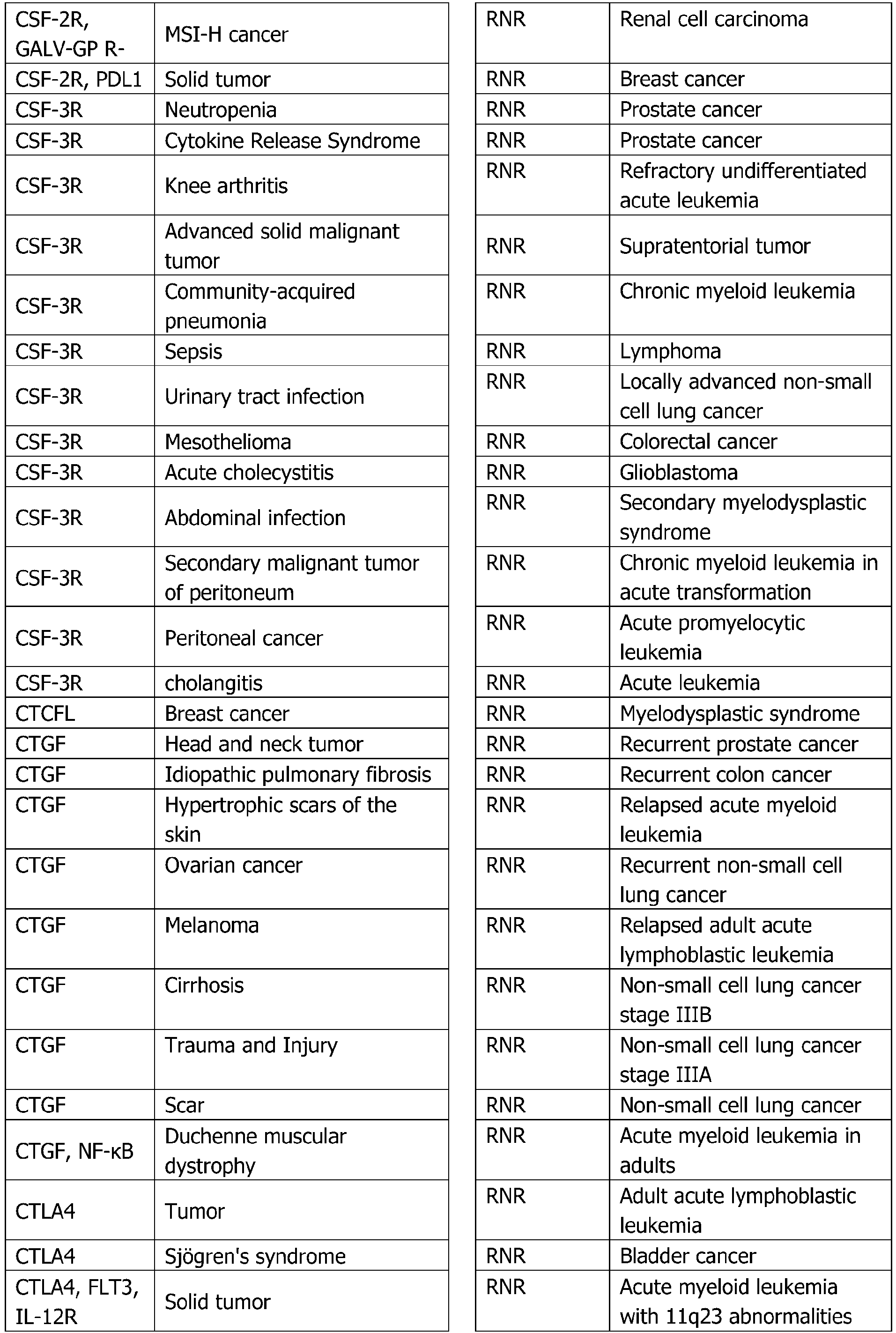

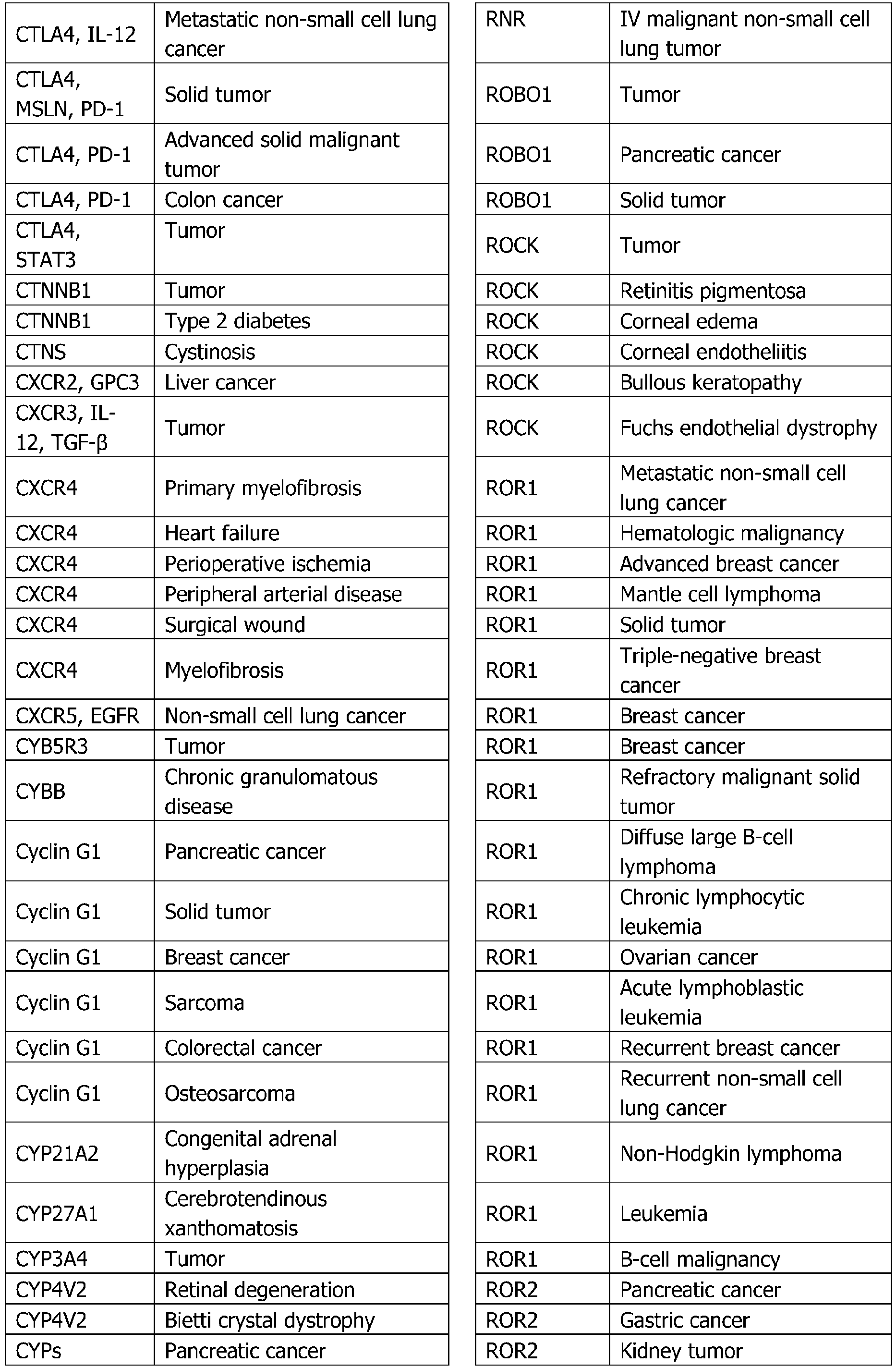

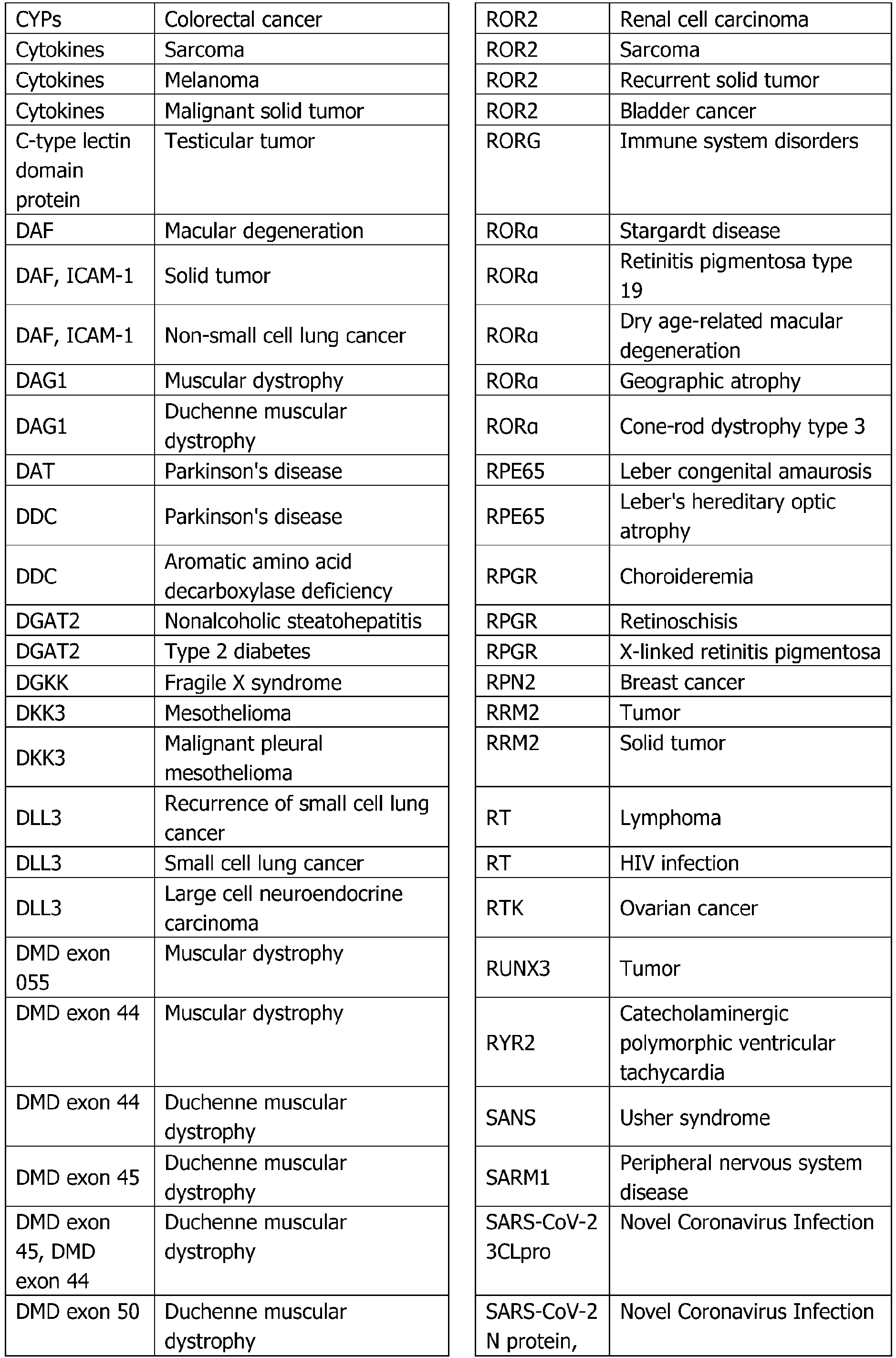

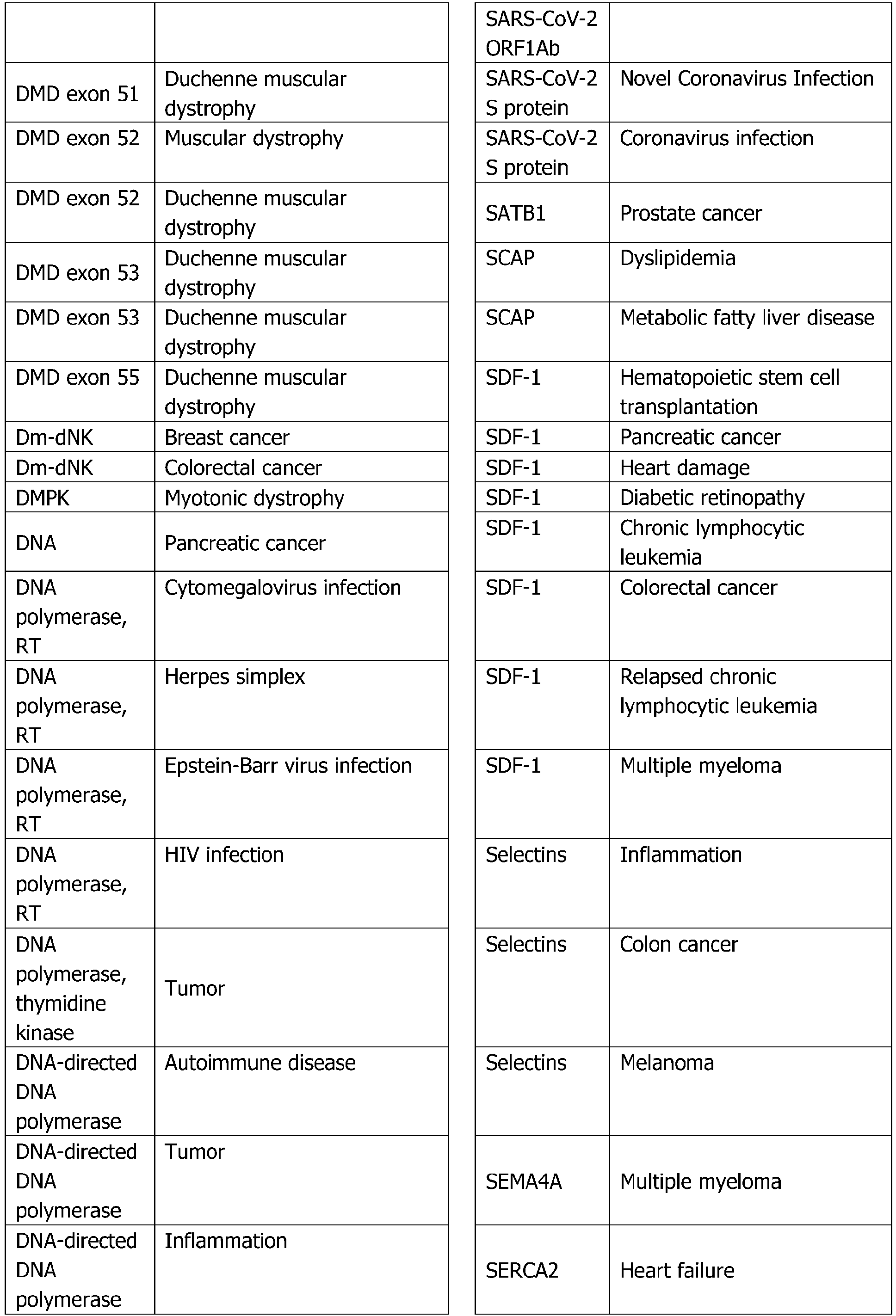

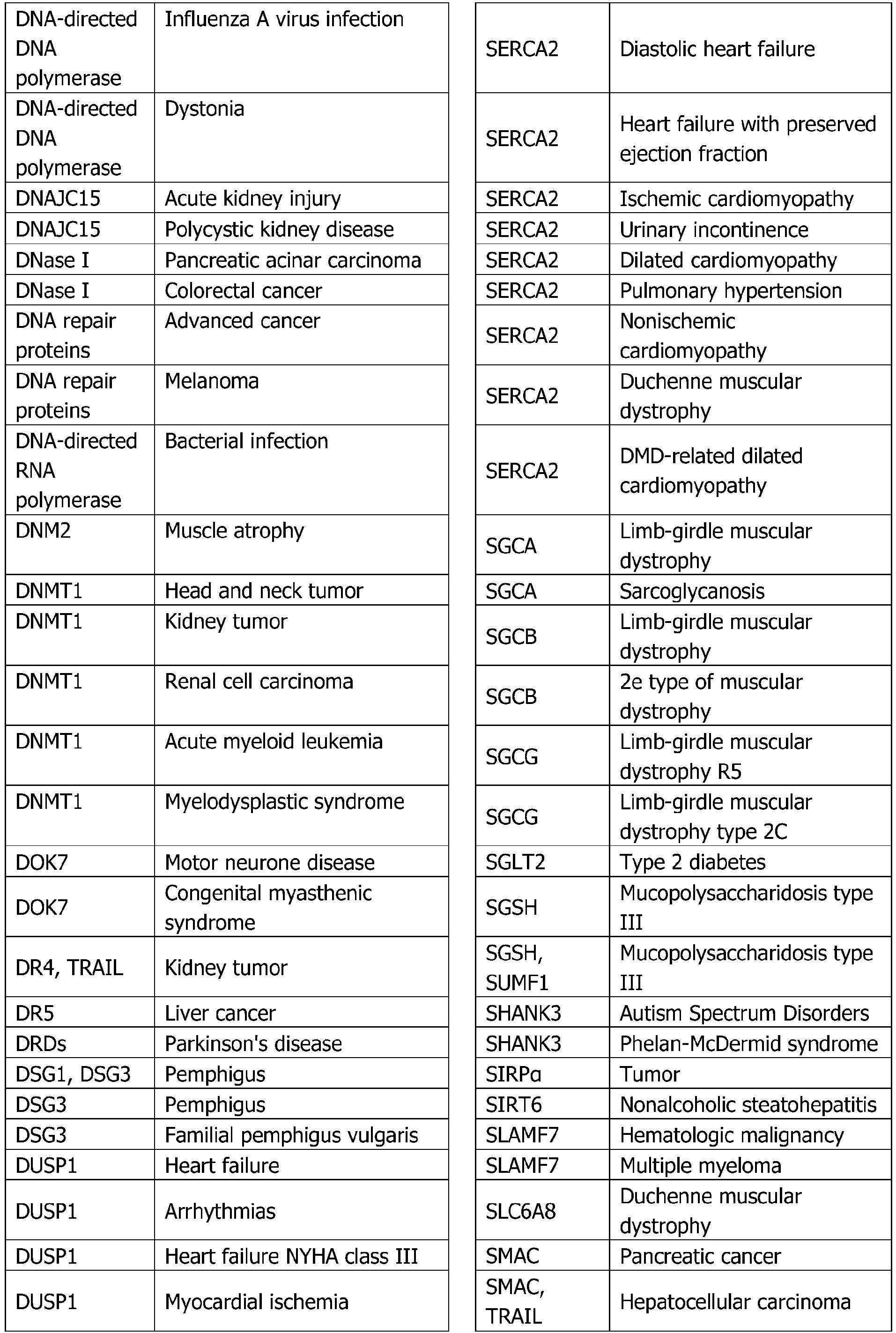

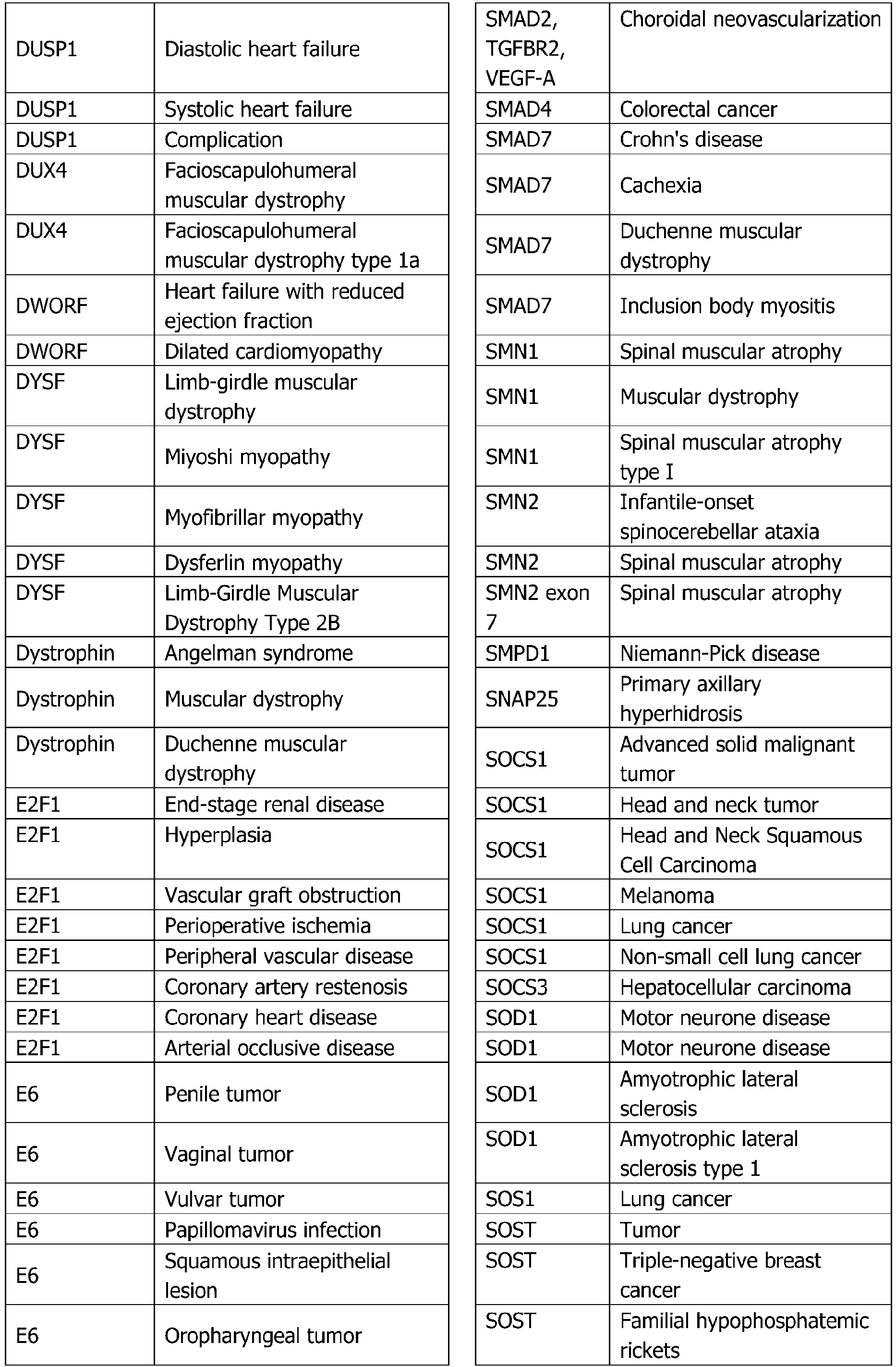

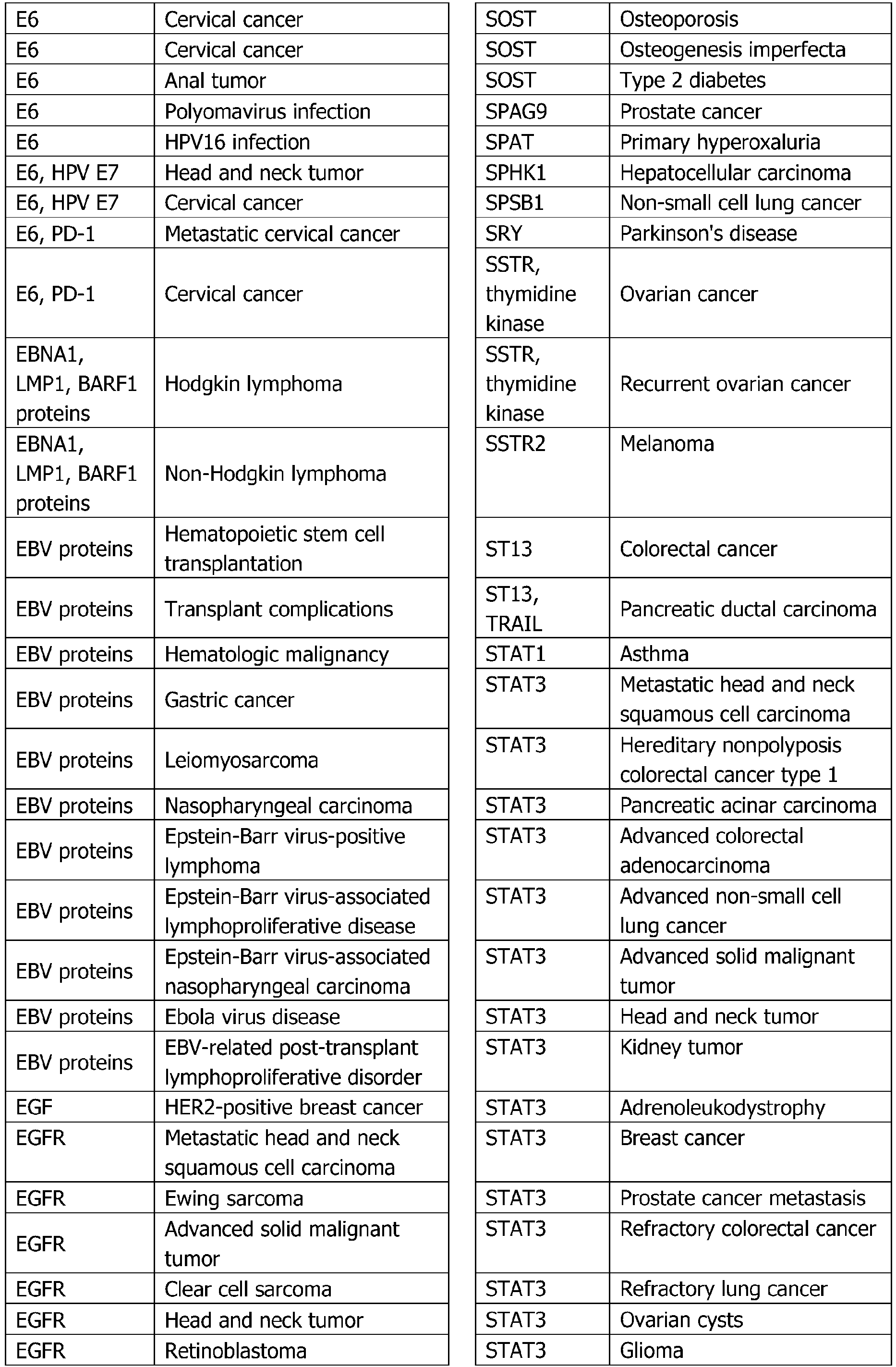

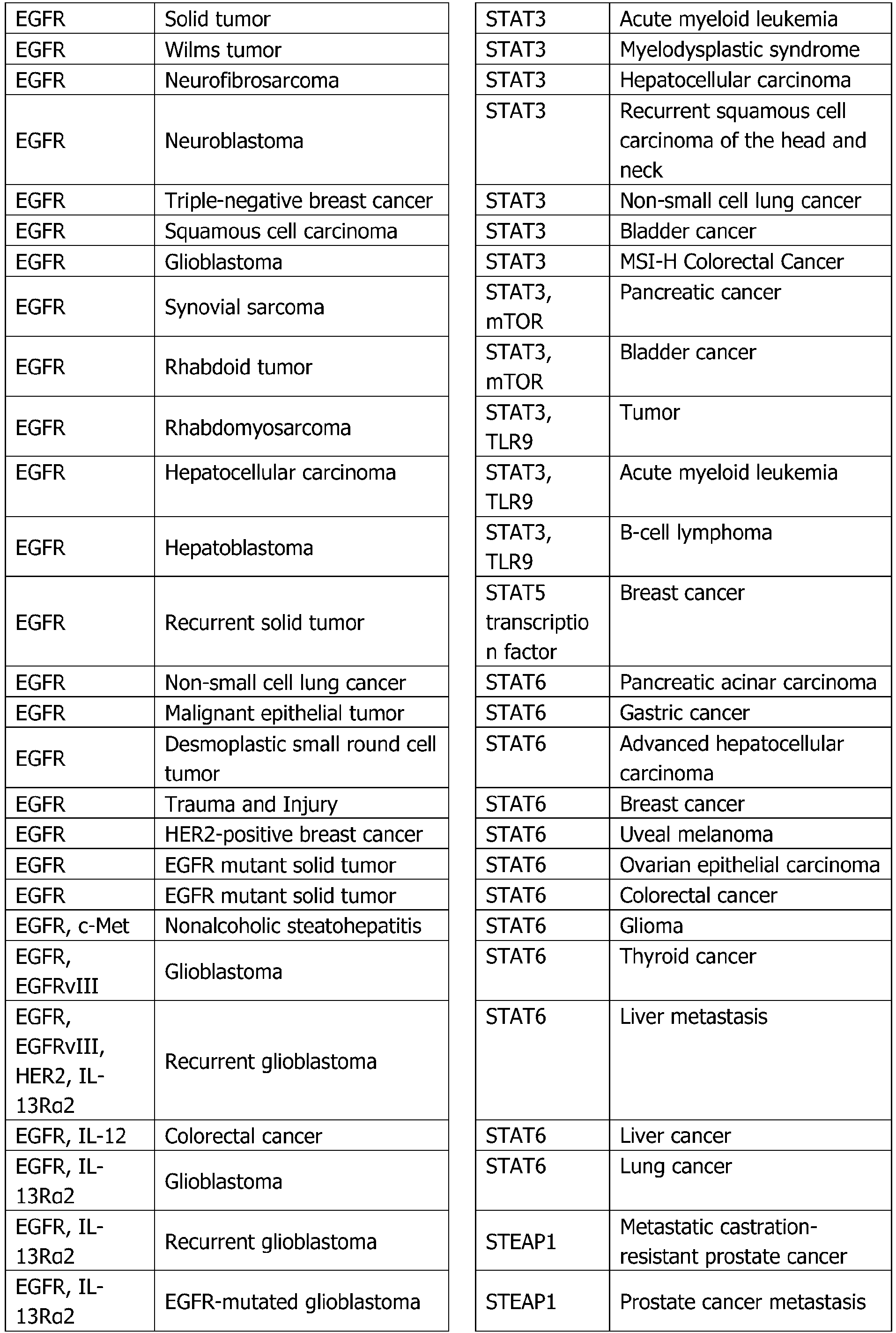

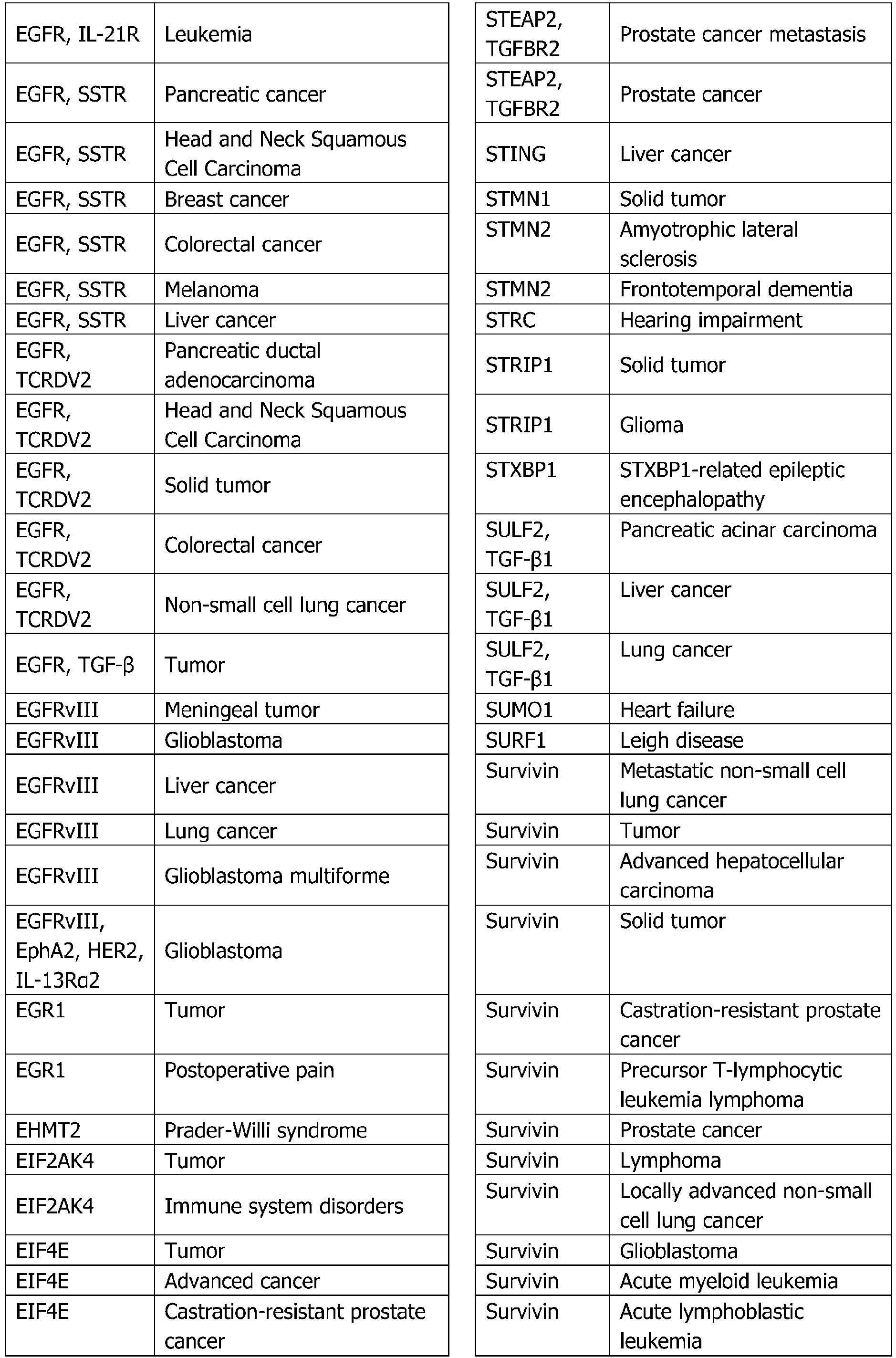

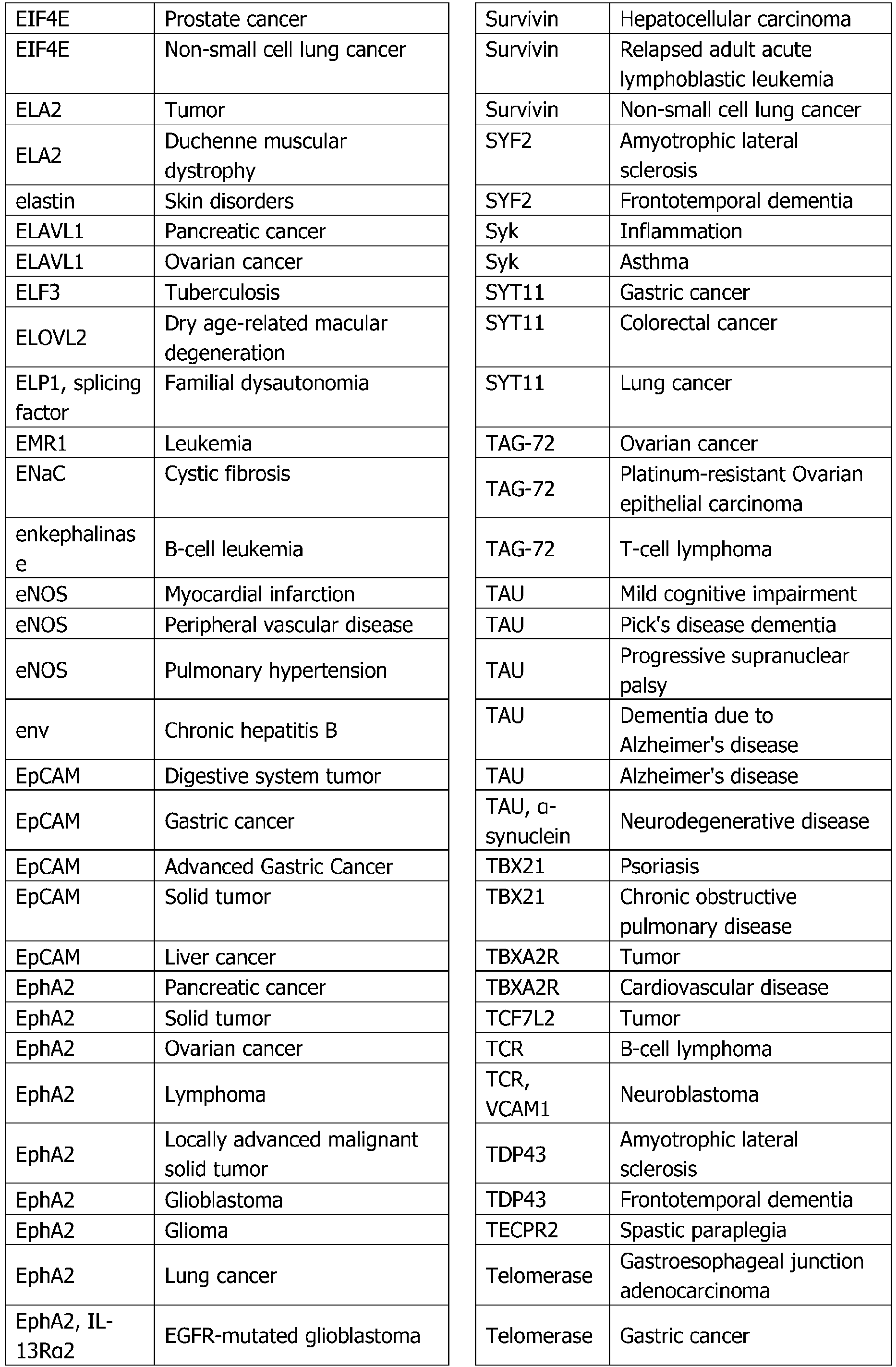

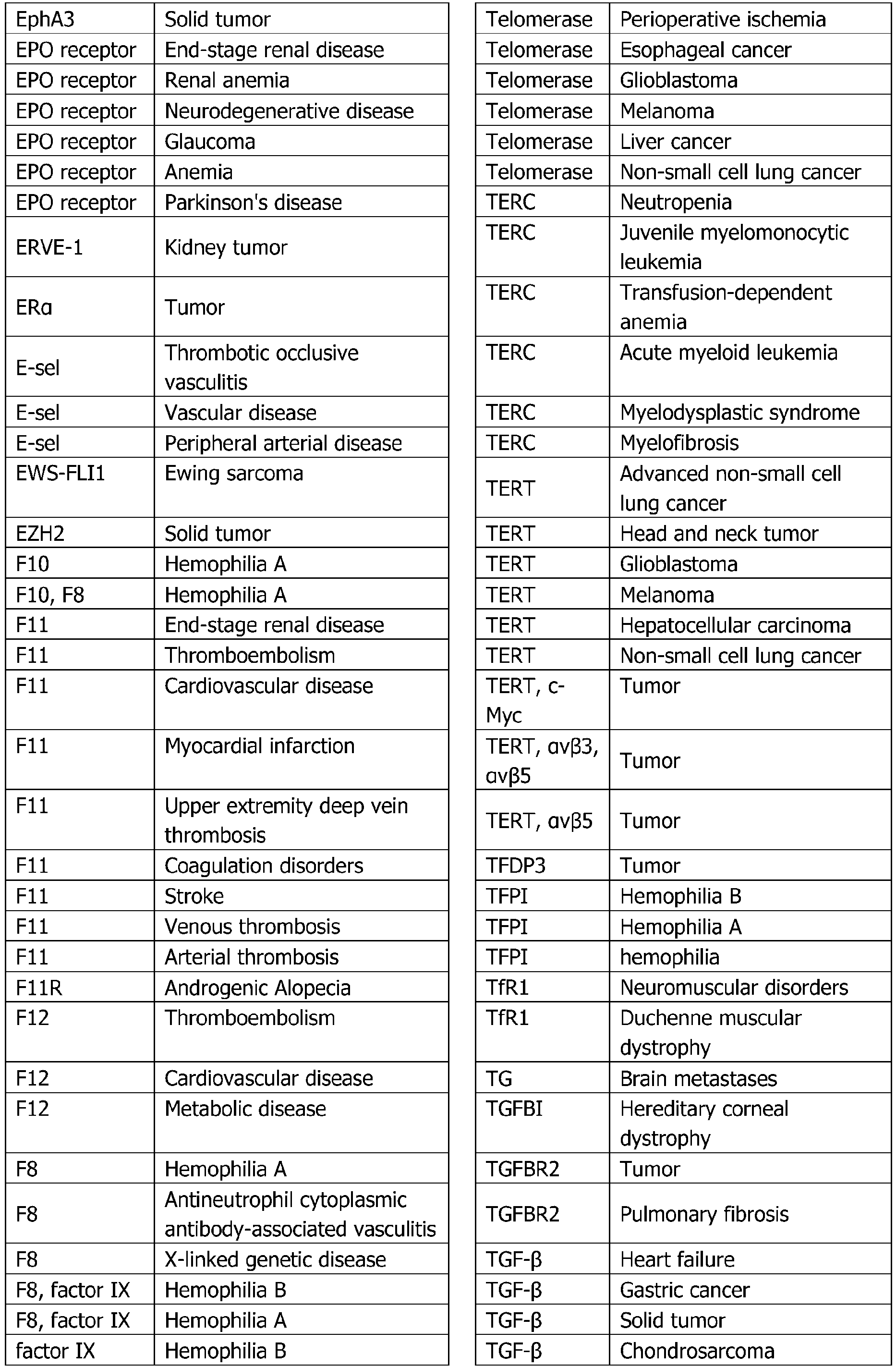

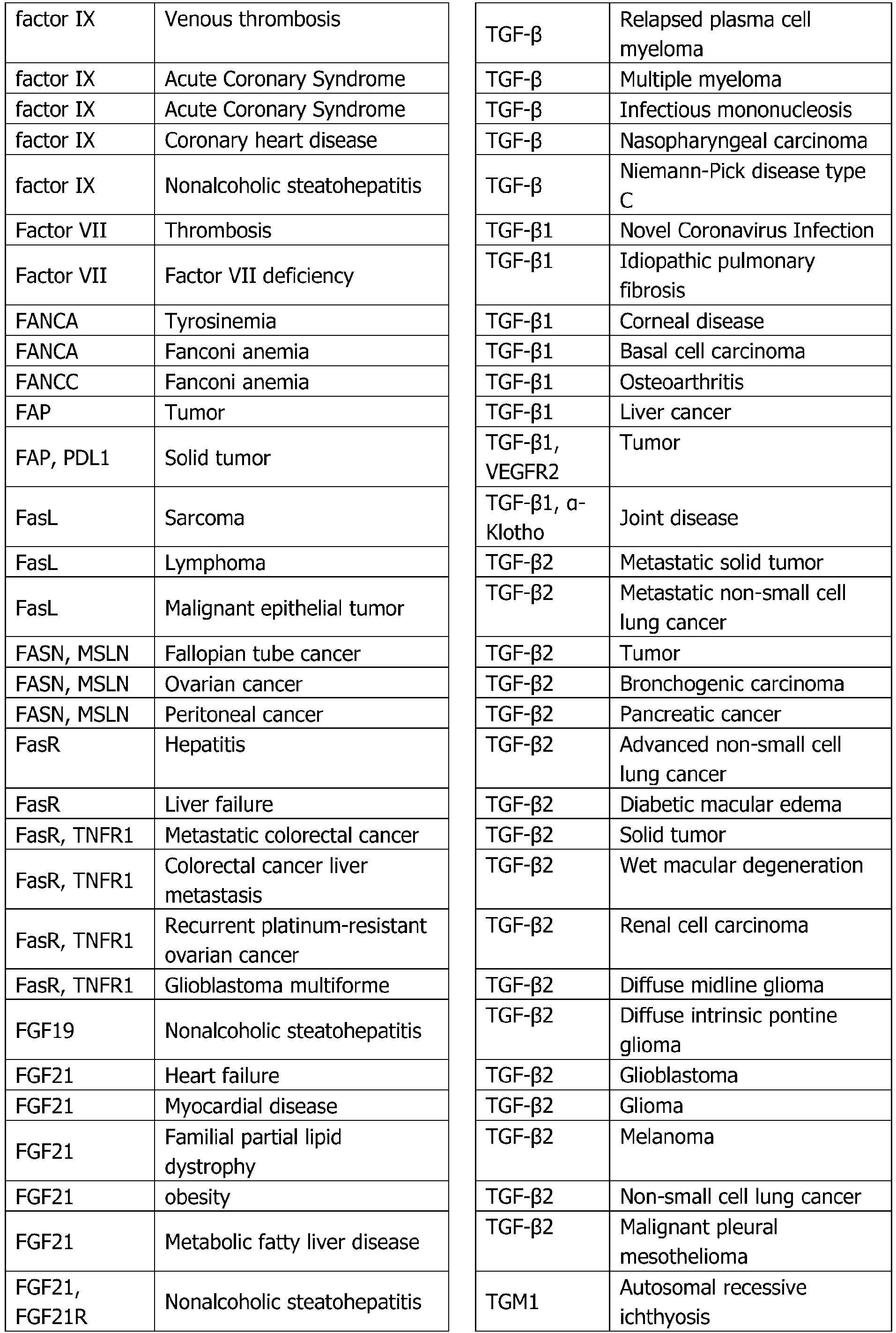

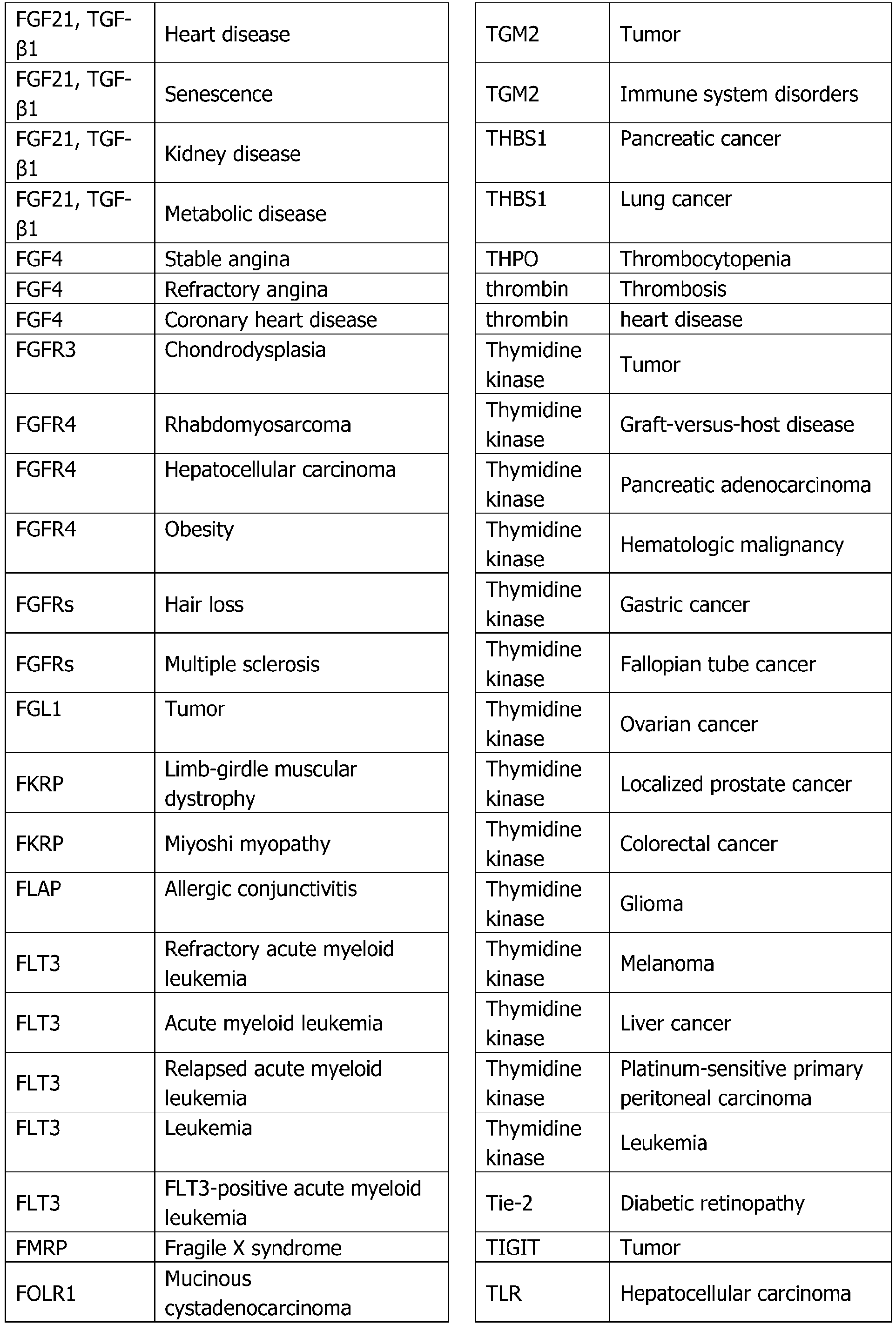

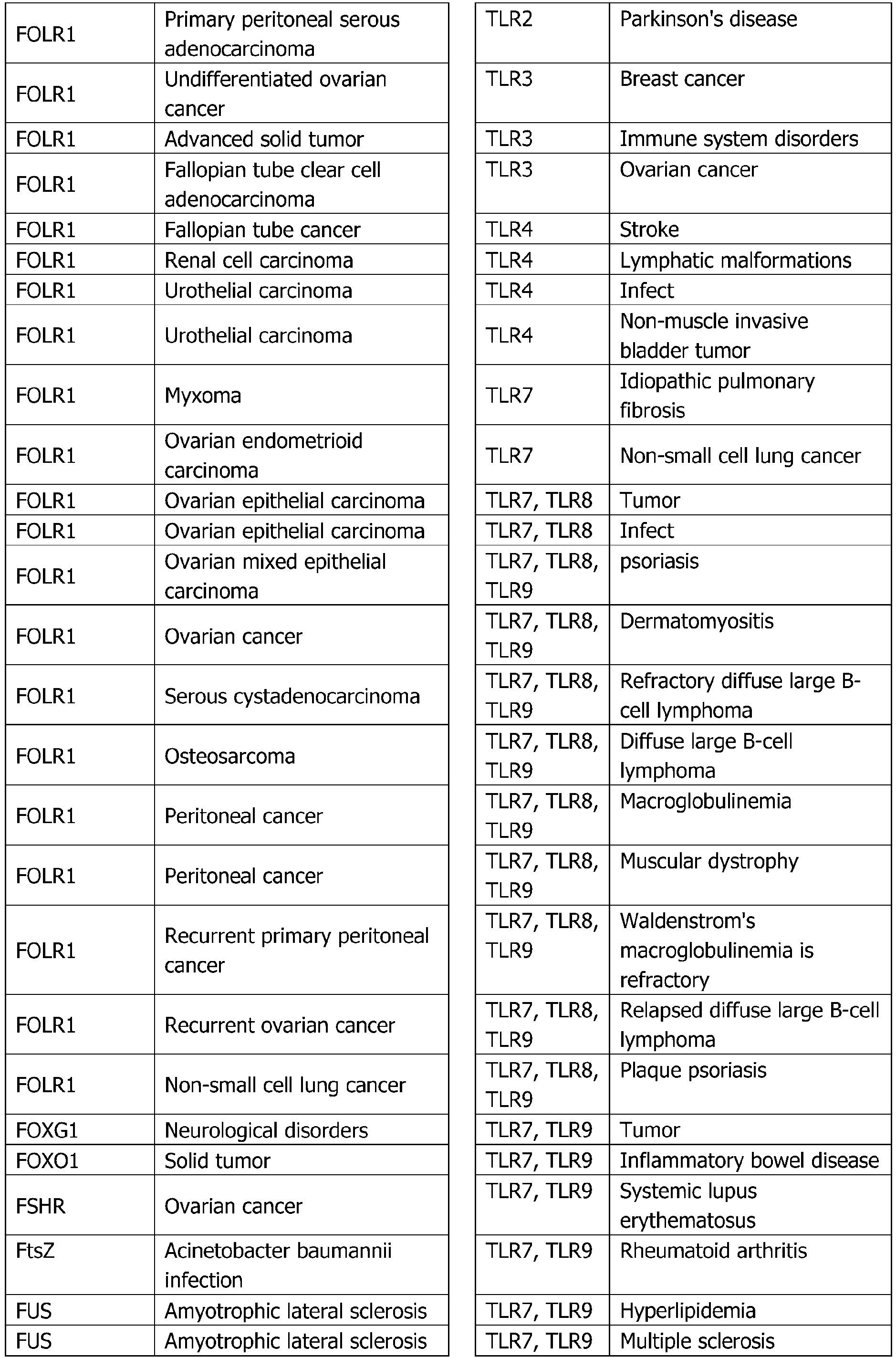

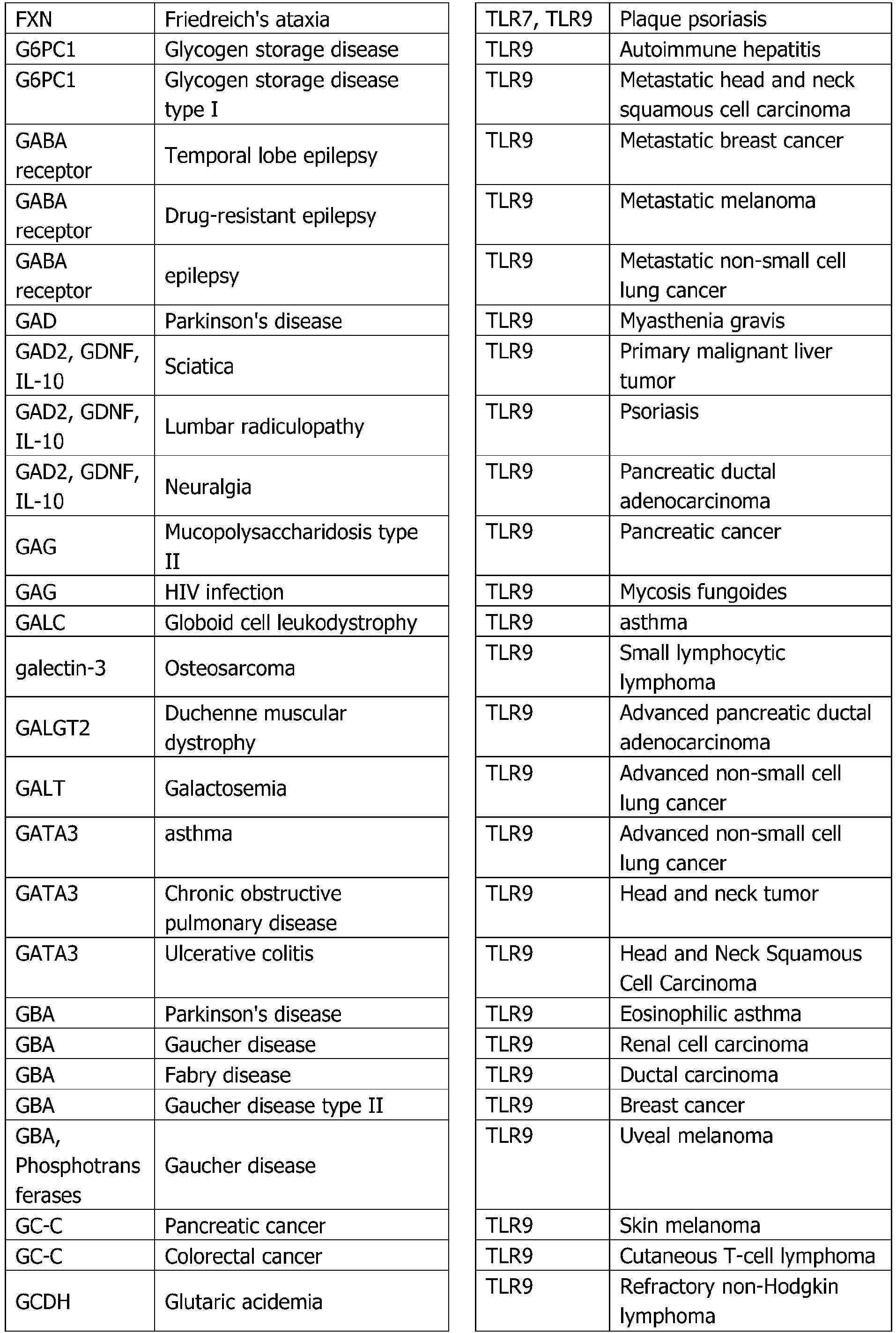

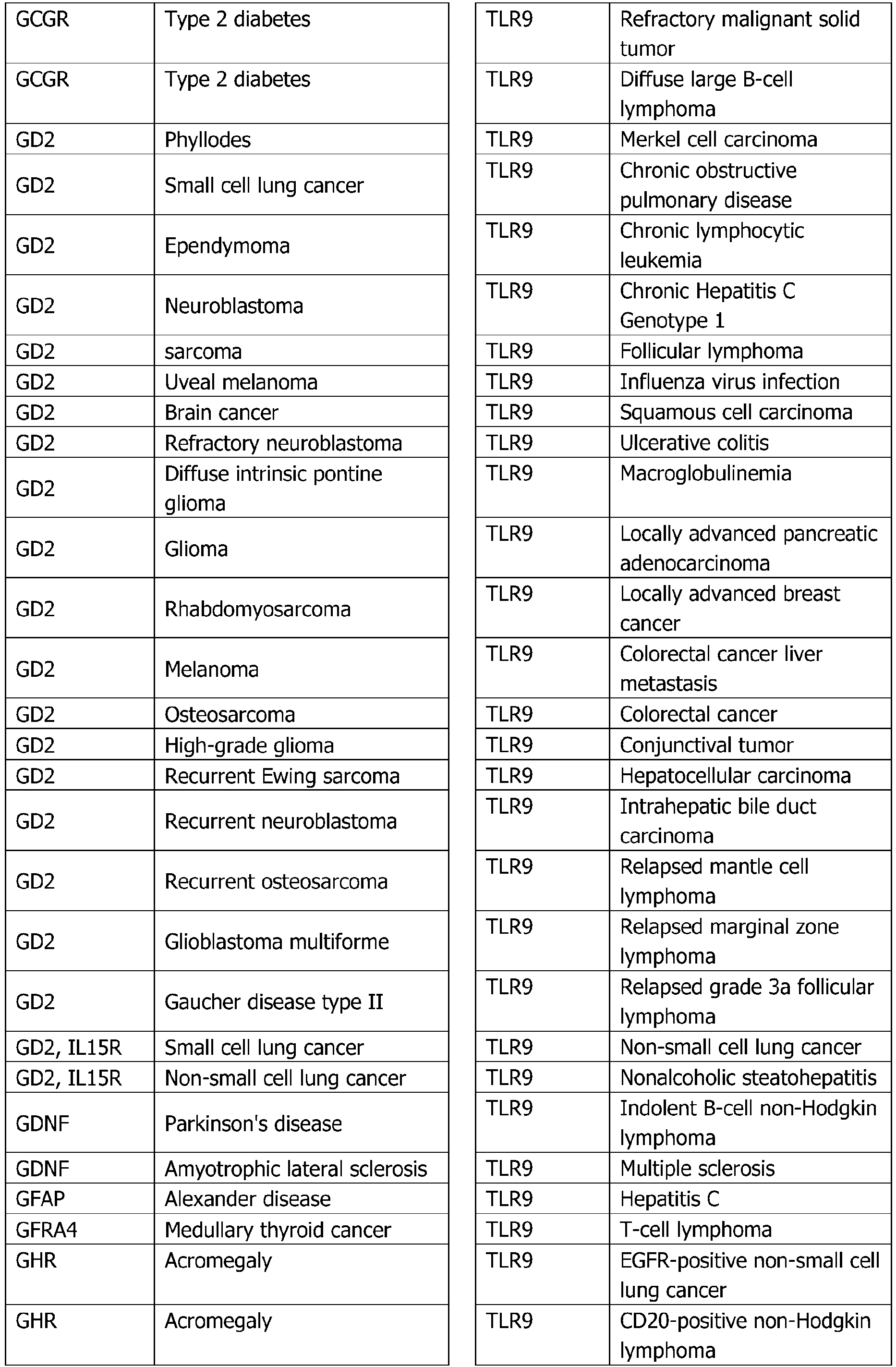

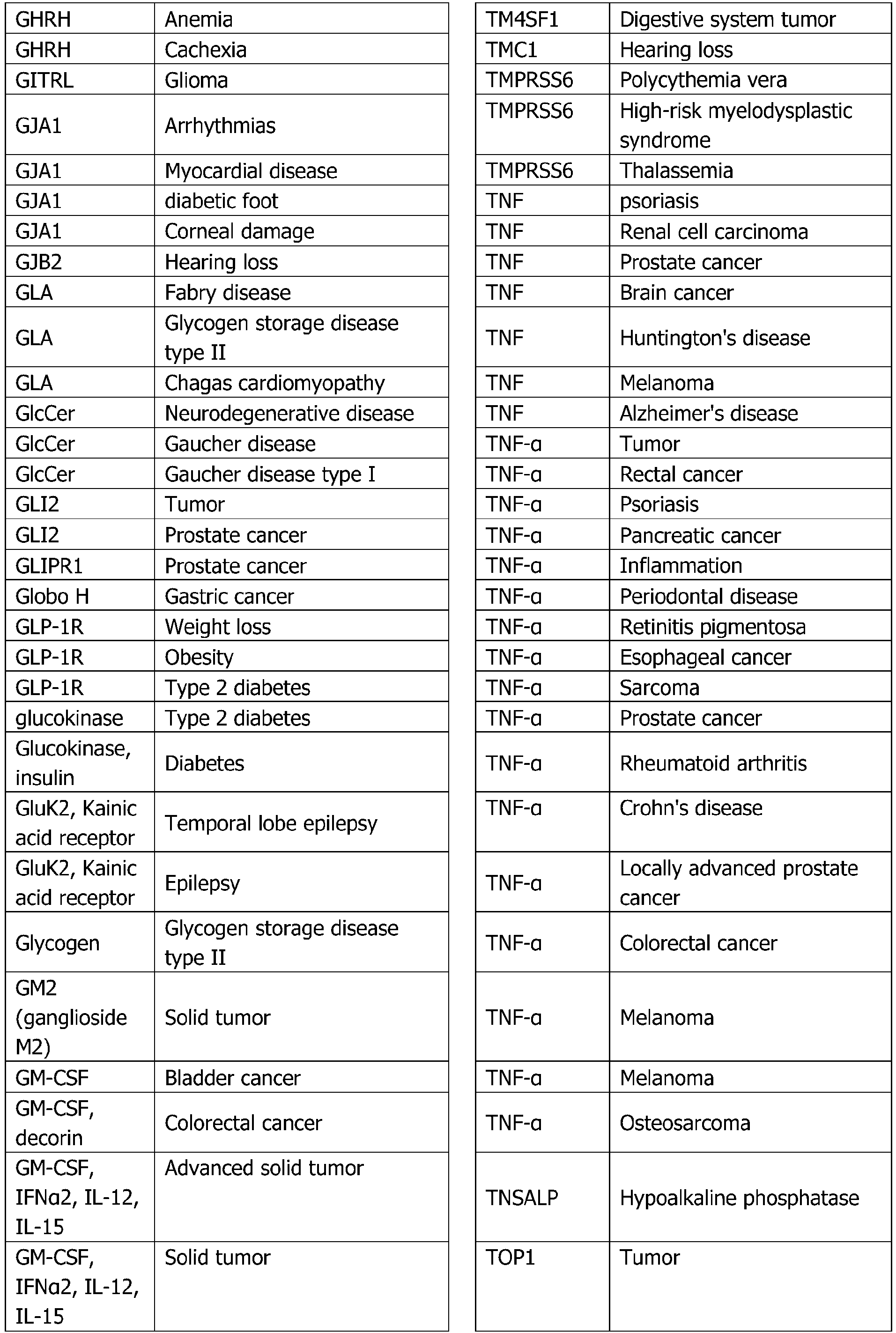

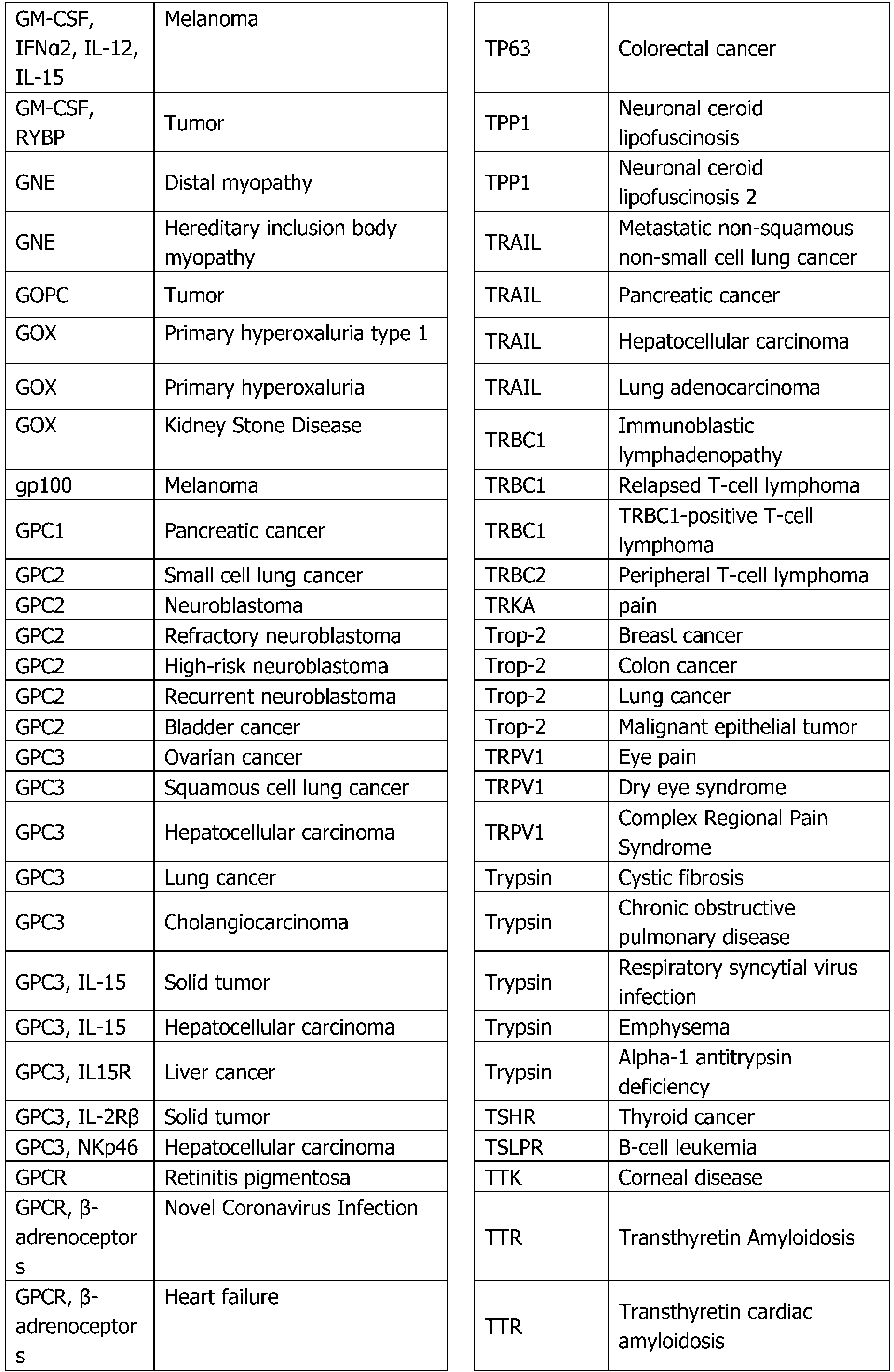

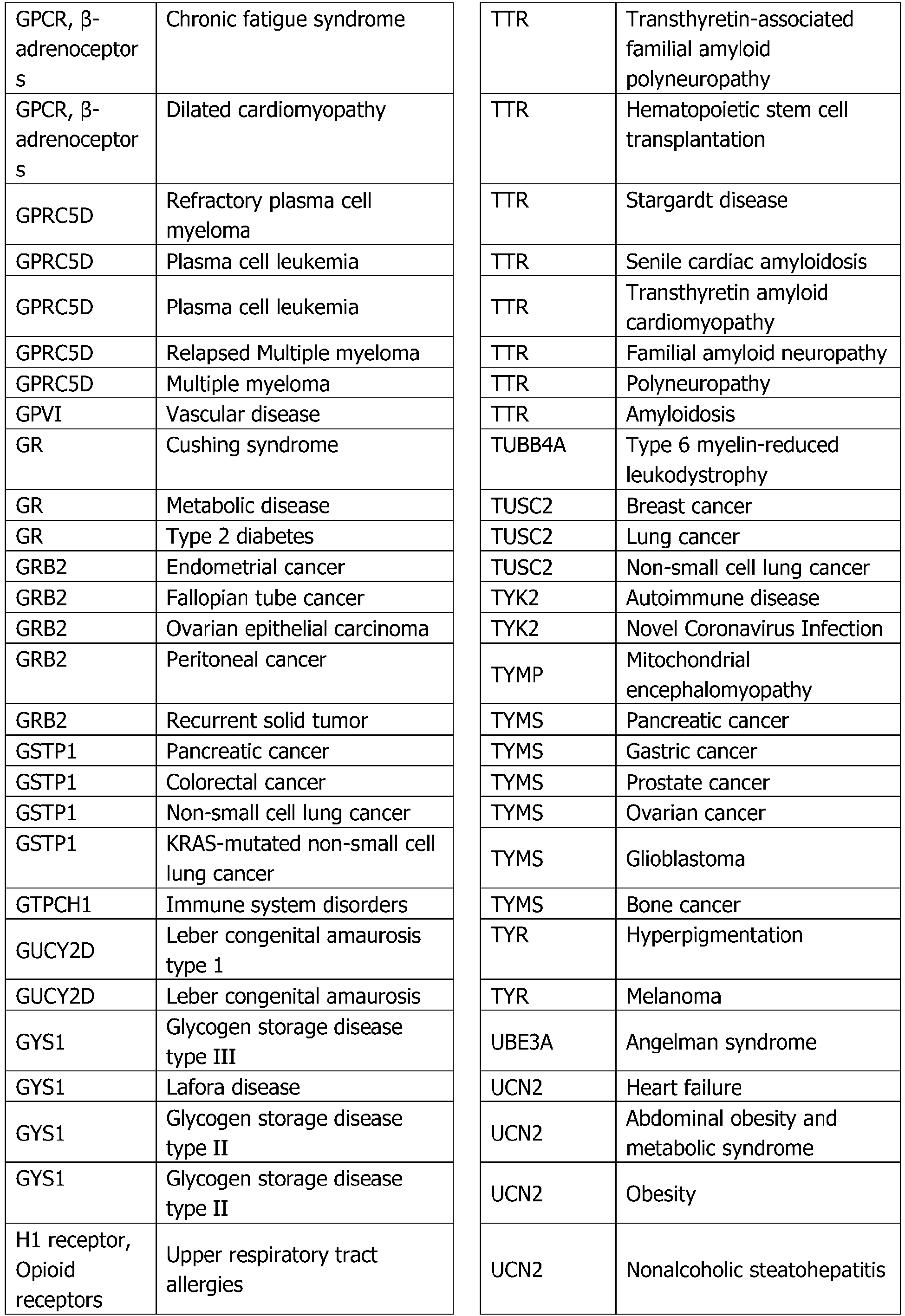

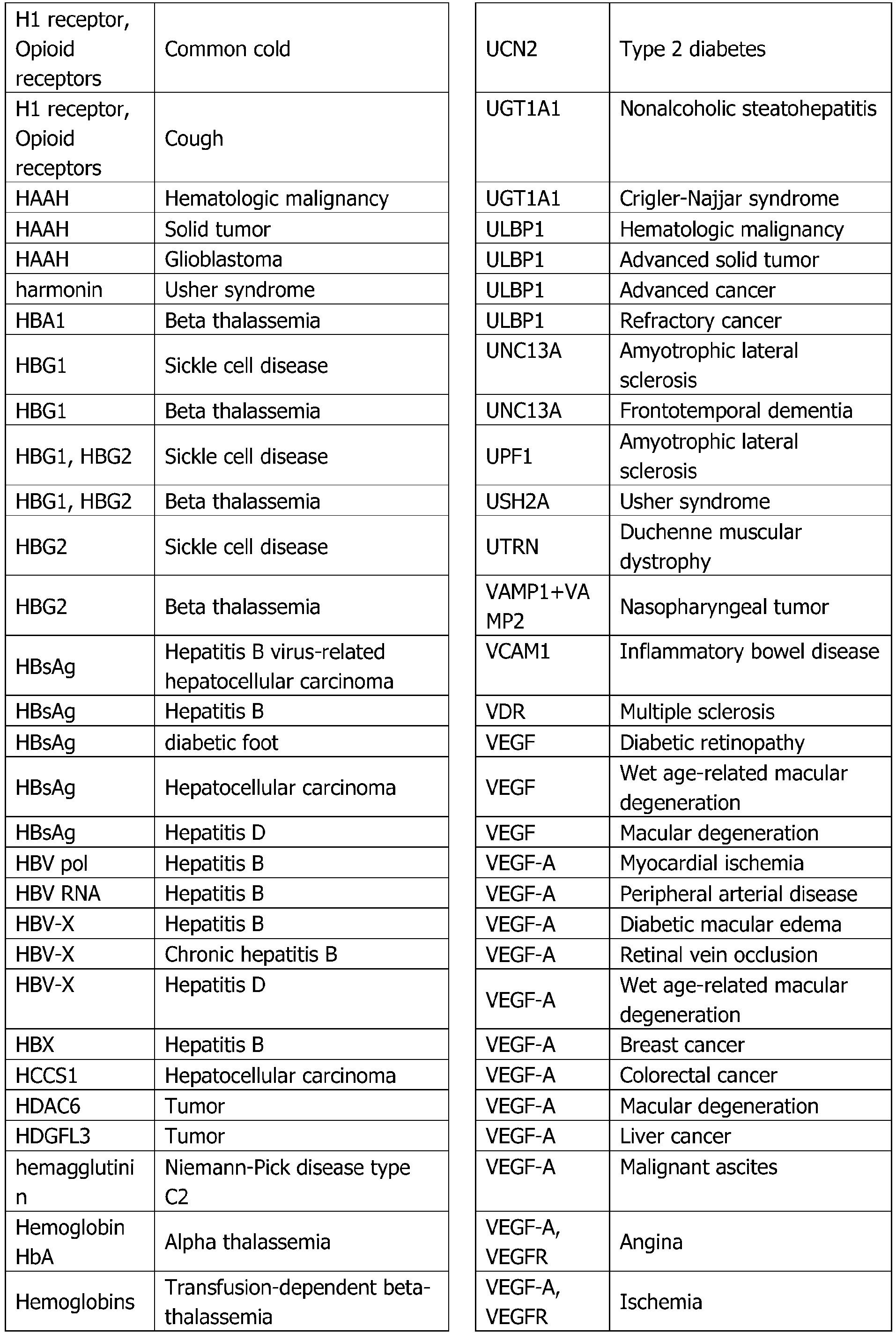

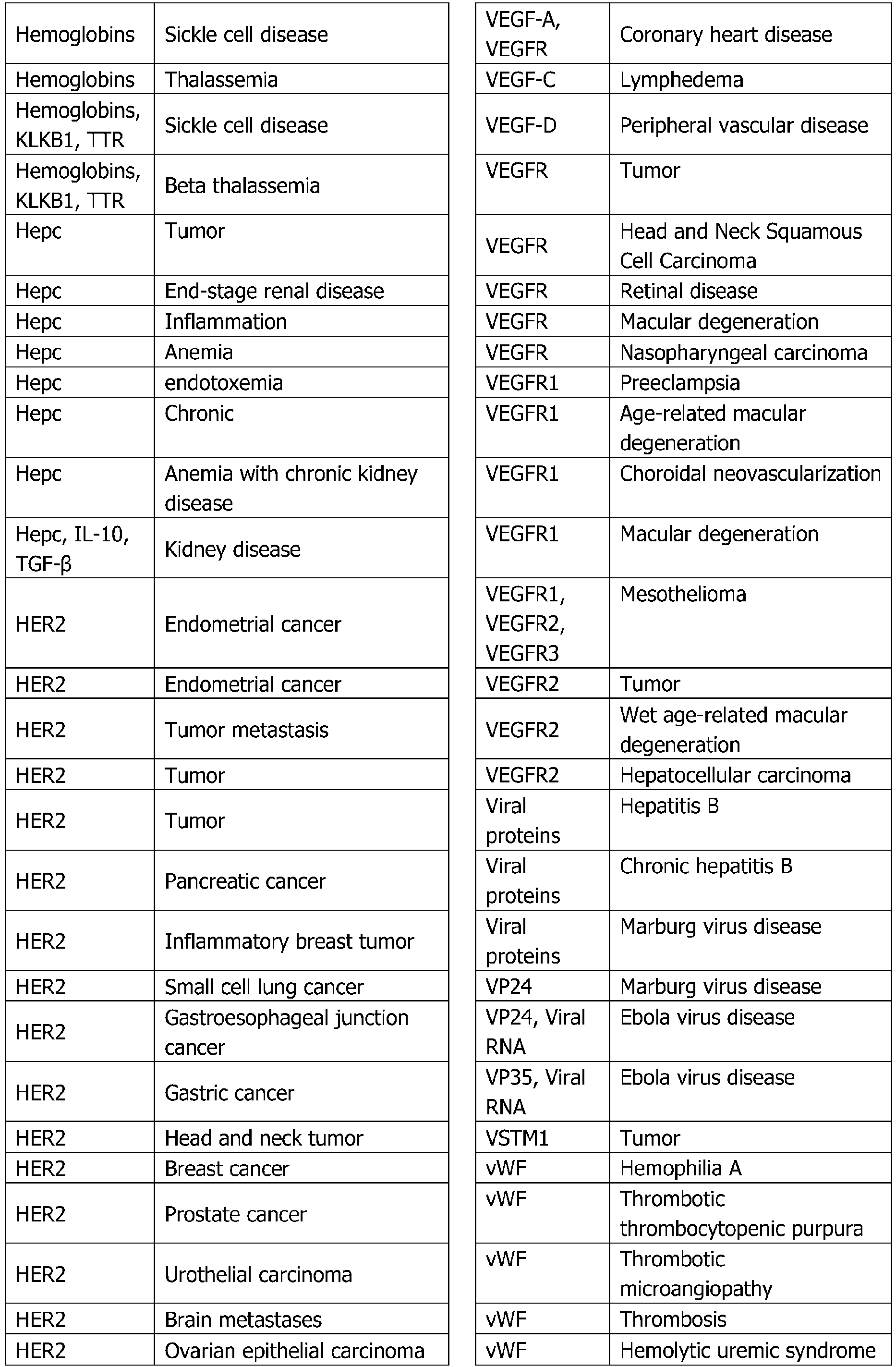

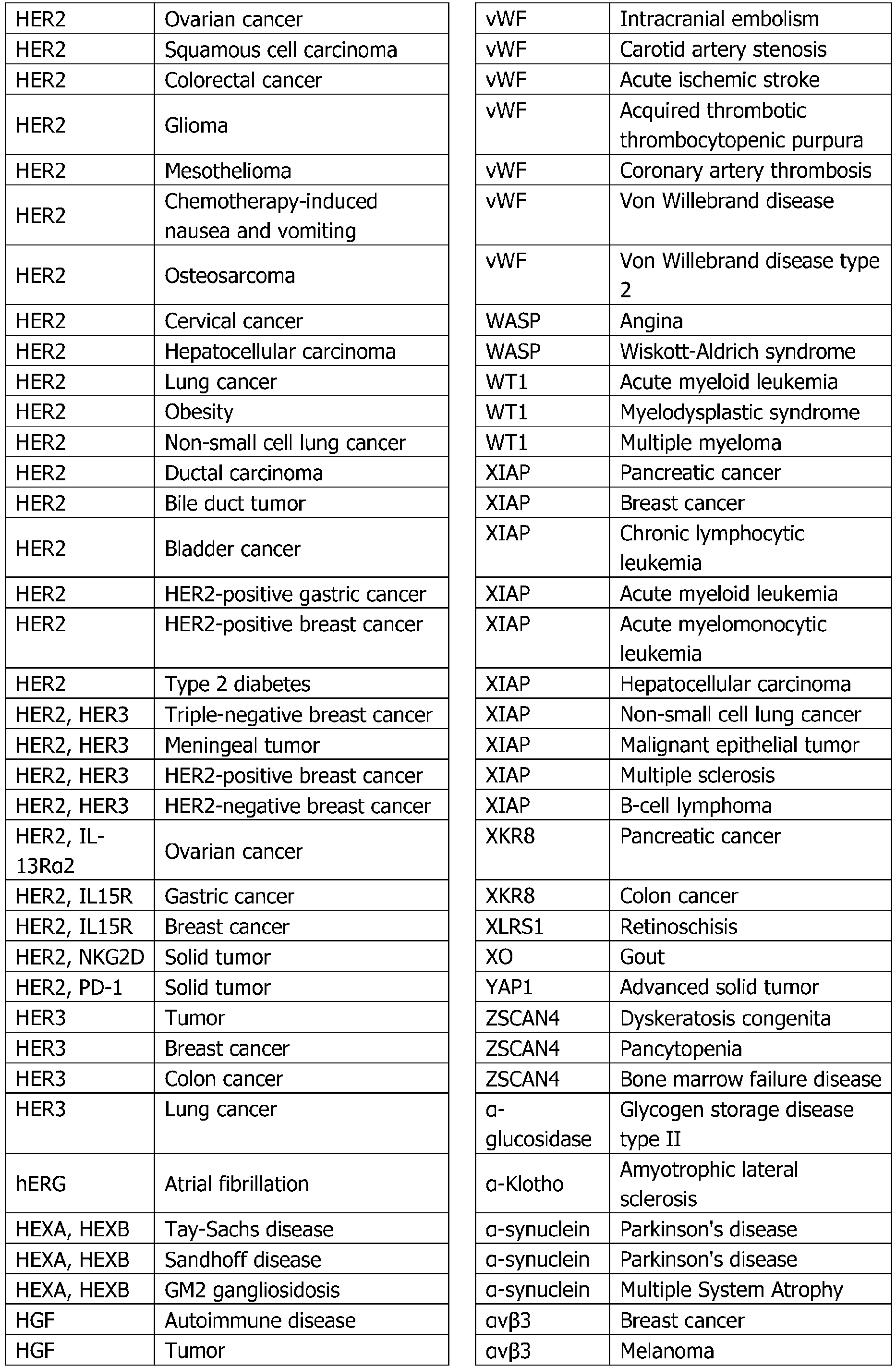

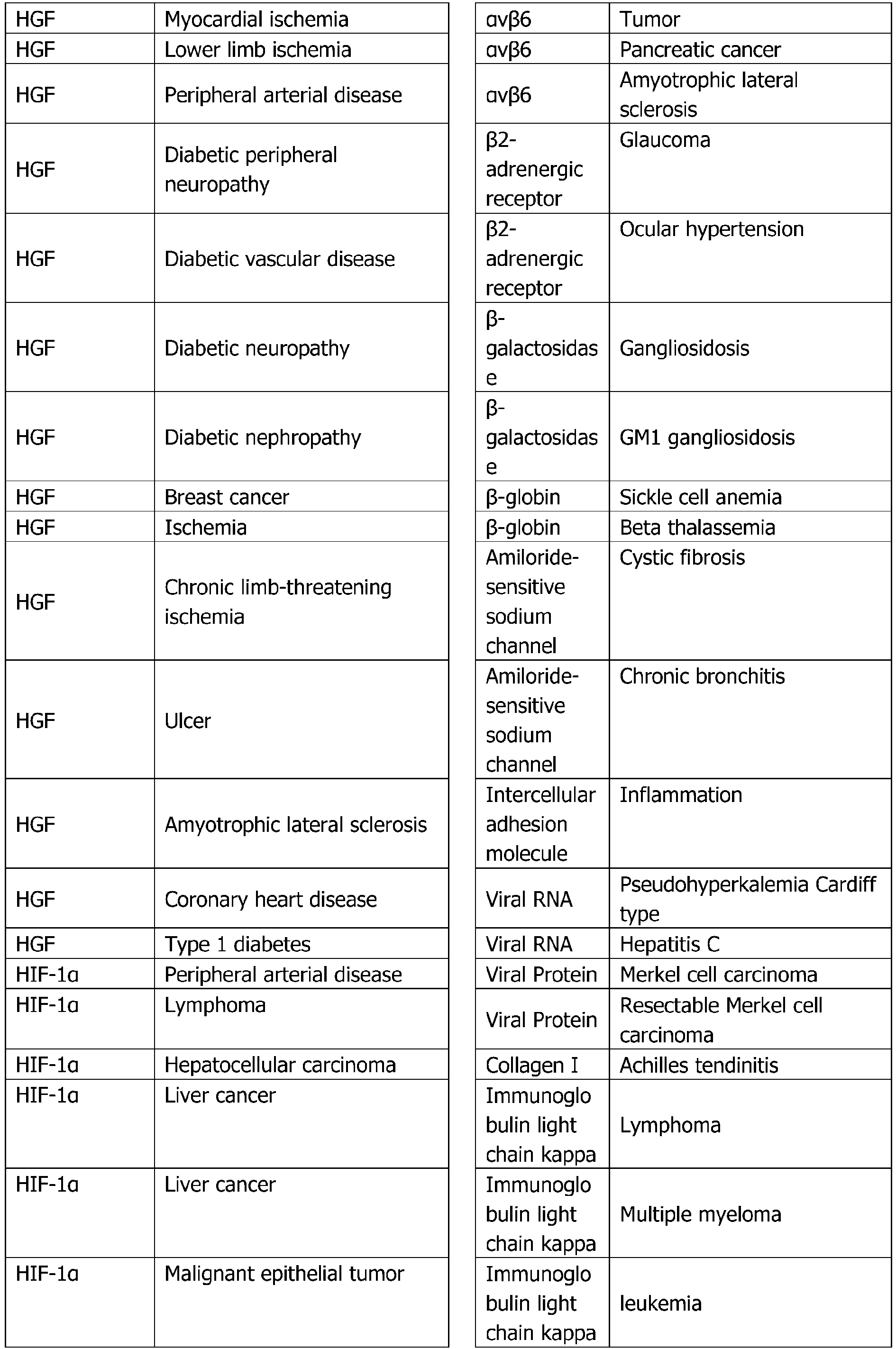

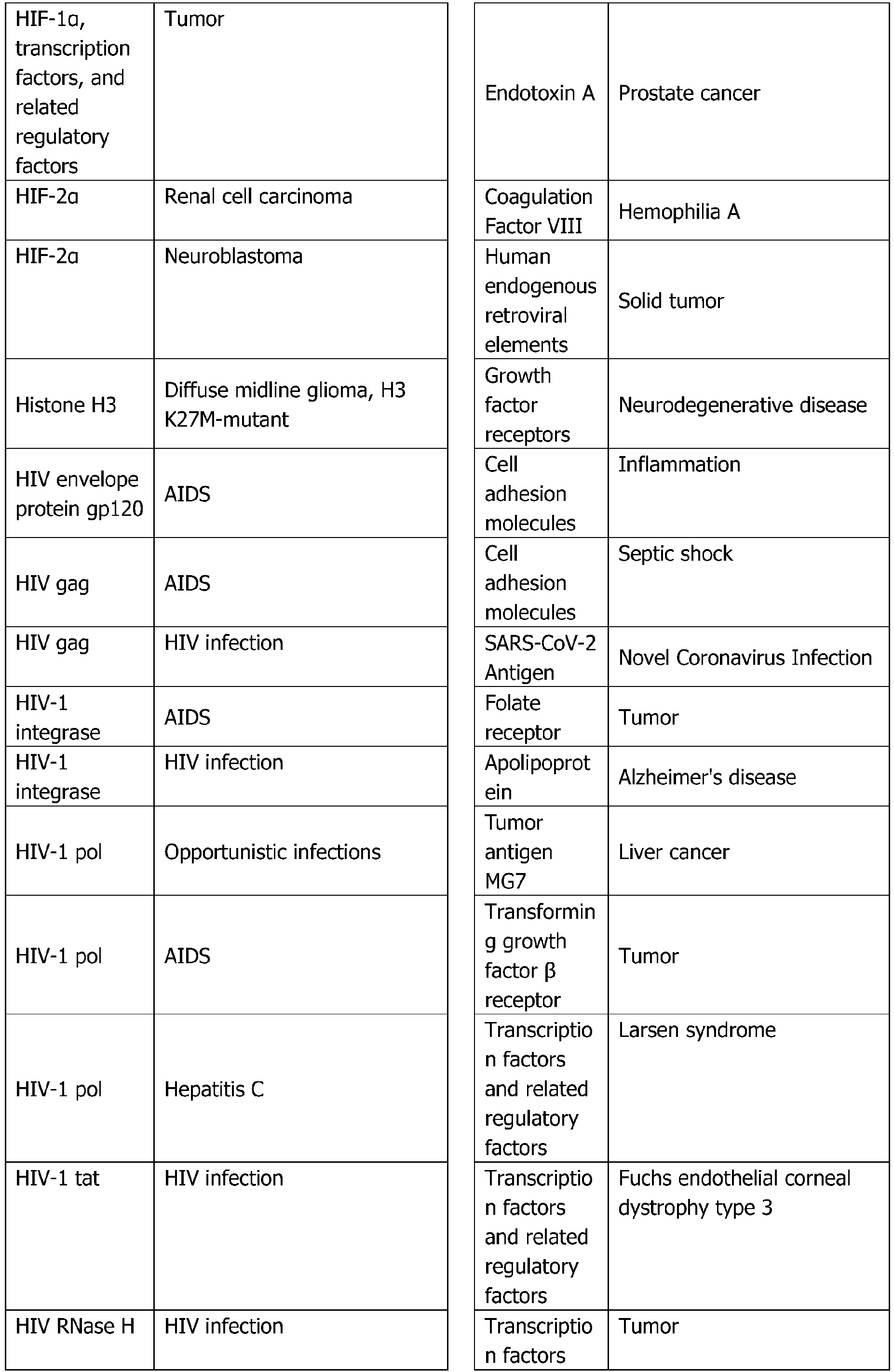

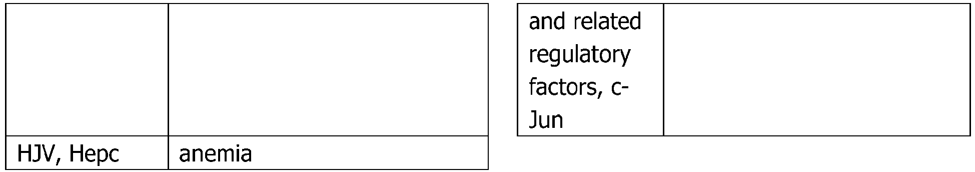

### EXAMPLES

The present disclosure is further described below by way of examples, but the present disclosure is not limited to the scope of the examples. The experimental methods without specific conditions in the following examples are performed according to conventional manners and conditions, or according to product specifications.

### Example 1. Screening C12-279 protein

As shown in FIGs. 1A and 1B, the Cas12i protein C12-279 was finally obtained through screening, analysis, and designing using a complex bioinformatics manner.

An amino acid sequence of the C12-279 protein (SEQ ID NO: 696) is:

IFVVLSVNQFRCNPNAFRQASMKTDDEKKDTAIKSYTSWLLPNDRKEKDMVRSFLALDQGSRFFFDLMQA
WYGGLTPDIIKSVTKERDLVDLWCAIYWFRPMKKELASHPIDRVDLAKTFQRYYGGPASDVVQEYLSASIGE
DCCWNDCRQKYQEFCKNLGVDFTPDLKTLVREKLIAVALNDGSQAAISNLFGNGGKEDRNVKVNICKKILI
ALEKAGKSVKYVRDVQSIILKCANAKDREDFNRIYADKNGRPGTLLLLLDRKGENTYDAEVLKRYLRKTIKSK
NAPLVWNYNQKLFEYIERGIRNLLRRTINYDAWAWGEMWRVGHTPLQTKATRNYHFAQEQLKRHSDIAAA
SQSSHAIAVALNEFFESPFFDGENPFTICPHHLGKNLEKLFKAWADEKNQTDEEDAGETAITDYCAEFKDGF
DREPIRNLLRYVYASLRSKYSAKELIQAAKYNQQFERYQRQKAHPVTPDNQGYTWPEAVIKPSKAERKNRE
NSLDTRIWVSVKLLQKDGTWEKHHIPFYNLRFFEEVYAAASIAEDMPSPTFRNSRFGFKLPKLLNTHAKINK
KKKDGKKDKKAVNAAKREARIYLAAKEGRLPAPSVPLDKLSAVIARVNGKFQVTVPVKFKVNKQPKYPLPQL
GQIILGYDQNLTANHAWALFEVVTEDTPGAHFCRNQYLRVLKTGQVRSITKTKDGQEIDQLSYDGLEYKEY
GEWRKQAKKFASQWYITTKVRKGKETTLLSQPALERFESIEKYKPALYRFNKEYARLLLAVIRRKTLNELEEIR
PEIFRLVEQGFGICRFGSLSLSSFEAIRAAKAVIYGYFSTALKHDHNTPITDDERQAFDPELFGLLNKLETLRQ
NKGEEKINRAANALIRIALENKADFIRGEGDLPTTNKSVKKGQNSRSMDWLARGLFNKICQLAVMHNIIPTA
ANPHSTSHQDPFEHNKDFDDPQPAMKCRYAEFKIEDIGDSVLERLGTSLRNAKAGQTGAYYYKGAQDFLA
HYGLQDIEEELKKCRRGRKANMPCWELQKRLIQKLGSKDAVVYIPMRGGRIYLATHSVTTGAKPIIFNGEEV
WVSNADEIAAVNIGLTIIPTSKKDEEHPDRENDDKAIRKSVRRPRKSGERRSGDKMPATARKT (SEQ ID NO:
696).

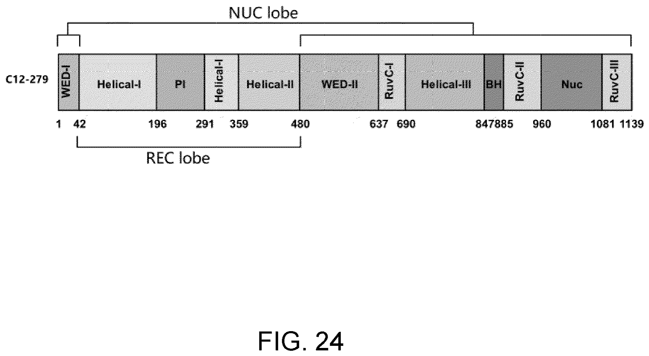

A predicted structure of the C12-279 protein is shown in FIG. 24.

### Example 2. Preparation and purification of C12-279 protein

### 1. Vector Construction

A pET28a vector plasmid was double digested with BamHI and XhoI, and a linearized vector was recovered by agarose gel electrophoresis. Using the prepared pXC12-279-GFPPAM-DR5 plasmid (SEQ ID NO: 697) as a template, DNA fragments containing coding sequences of the C12-279 protein was obtained by PCR amplification with primers ChkCas-pET28-PF1 (SEQ ID NO: 699) and ChkCas-pET28-PR1 (SEQ ID NO: 700). The DNA fragments were inserted into a cloning region of the pET28a vector by homologous recombination (NEB, Gibson Assembly^{®} Master Mix) to construct a recombinant vector C12-279-pET28a-01 (SEQ ID NO: 698). A reaction solution was transformed into Stbl3 competent cells, coated on an LB plate containing kanamycin sulfate, and cultured at 37°C overnight, and clones were picked for sequencing and identification.

Positive clones with a correct sequence were selected and cultured overnight. Plasmids were extracted and transformed into an expression strain Rosetta (DE3), and then coated on the LB plate containing kanamycin sulfate and cultured at 37°C overnight.

### 2. Protein expression

A single clone was selected and inoculated into 5 mL of LB culture medium containing kanamycin sulfate and cultured at 37°C overnight.

The selected single clone was reinoculated into 500 mL of LB culture medium containing kanamycin sulfate at a ratio of 1:100, cultured at 220 rpm and 37°C until OD reached 0.6, added with IPTG to a final concentration of 0.2 mM, and induced at 16°C for 24 h.

After rinsing with 15 mL PBS, the bacteria were collected by centrifugation, added with a lysis buffer for sonication disruption, a supernatant containing the recombinant protein was obtained by centrifuging at 10,000 g for 30 min, and the supernatant was filtered through a 0.45 µm filter membrane before being applied to column purification.

### 3. Protein Purification

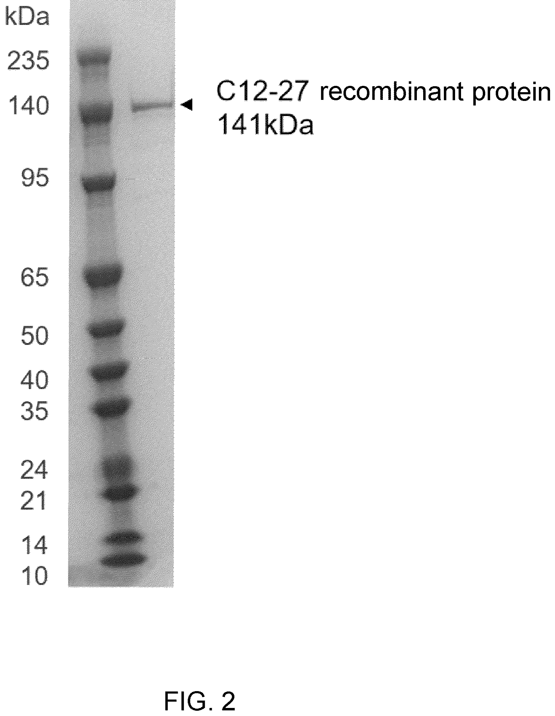

The expressed recombinant protein has 1240 amino acids (aa) and a structure of His tag-NLS-C12-279-SV40 NLS-nucleoplasmin NLS. Using 6×His tag at an N-terminal as purification tags, the C12-279 recombinant protein was obtained by Immobilized Metal Ion Affinity Chromatography (IMAC) (Ni Sepharose 6 Fast Flow, Cytiva) and heparin affinity chromatography (POROS^{™} Heparin 50 µm chromatographic column, Thermo Scientific^{™}). The purified recombinant protein was detected by SDS-PAGE electrophoresis, and results are shown in FIG. 2.

### Example 3. In vitro cleavage of PAM library and grabbing of PAM by C12-279 protein

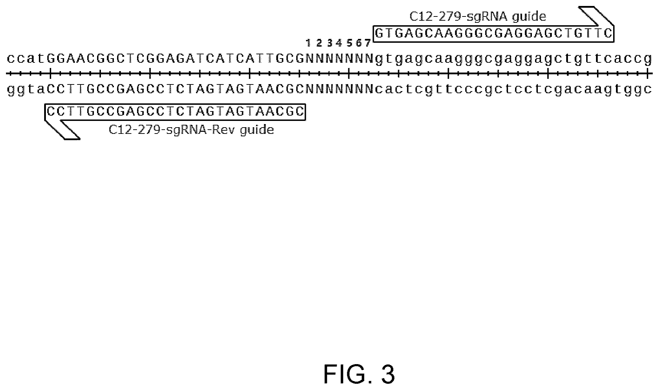

In this example, sgRNA containing a specific guide sequence and the C12-279 recombinant protein prepared and purified as described in Example 2 were mixed to cleave an in vitro cleavage substrate (containing a spacer sequence and a 7nt random sequence) (as shown in FIG. 3). After incubation at 37°C, purification and library construction were performed, followed by NGS sequencing to determine the PAM sequence of C12-279. Specific operations were as follows.

### In vitro cleavage substrate

The designed in vitro cleavage substrate sequence is as follows:

ggagttcagacgtgtgctcttccgatctcagcacaaaaggaaactcaccctaactgtaaagtaattgtgtgttttgagactataaatatgc
atgcgagaaaagccttgtttgccaccatGGAACGGCTCGGAGATCATCATTGCGNNNNNNNgtgagcaagggcgagga
gctgttcaccggggtggtgcccatcctggtcgagctggacggcgacgtaaacggccacaagttcagcgtgtccggcagatcggaaga
gcacacgtctgaactcc (SEQ ID NO: 701).

In the sequence, N represents any one of A, T, C, and G.

A double-stranded DNA containing the above sequence was prepared by PCR amplification and used as the in vitro cleavage substrate.

The cleavage substrate was sent to a sequencing company for PCR-free library construction and NGS sequencing. A complexity and abundance analysis on the PAM library composed of the 7nt random sequence were analyzed. The result is as follows.

Compositions of the four bases A, T, G, and C are basically the same. At the same time, the PAM library composed of the 7nt random sequence contains 4^7=16384 different combinations, all of which (100%) are detected. The complexity and abundance of the PAM library are qualified.

### B. Preparation of sgRNA

The sgRNA (C12-279-sgRNA) containing a specific guide sequence was synthesized by in vitro transcription at 37°C in a system containing T7 RNA transcriptase, four ribonucleotide triphosphates, and a DNA template with a T7 promoter. A transcription product was precipitated and purified with LiCl. The sgRNA sequence is as follows:
>C12-279-sgRNA
   5'-gtaatgcgtctcccattgacgcGTGAGCAAGGGCGAGGAGCTGTTC-3' (SEQ ID NO: 702).
>C12-279-sgRNA-Rev
   5'-gtaatgcgtctcccattgacgcCGCAATGATGATCTCCGAGCCGTTCC-3' (SEQ ID NO: 703).

The sgRNA scaffold sequence (DR sequence) is: 5'-gtaatgcgtctcccattgacgc-3' (SEQ ID NO: 704).

The uppercase bases are the guide sequence of the sgRNA.

### C. NGS library construction and PAM analysis

### 1) PAM library cleavage and T4 DNA Polymerase treatment

Reaction systems containing the C12-279 protein, two different sgRNAs, the in vitro cleavage substrate, and a buffer were prepared (as shown in Table 1) and reacted at 37°C for 3 h and at 75°C for 15 min).

**Table 1. Reaction system for in vitro cleavage reaction**

| Components | Addition amount (µL) |
|---|---|
| 10×Cut Buffer (500 mM Tris-HCl PH 8.0, 2M NaCl, 100mM MgCl₂, 10 mM DTT) | 5 |
| Cleavage substrate (59.5 ng/µL) | 36.5 |
| C12-279- sgRNA or C12-279-sgRNA-Rev | 2.8 µg |
| C12-279 protein (10 mg/mL) | 0.5 |

### 2) T4 DNA Polymerase treatment for blunting cleavage product

The T4 DNA Polymerase (Thermo Scientific) was added to the cleavage product. The specific reaction system was shown in Table 2. After addition, the reaction was performed at 37°C for 20 min and at 85°C for 10 min.

**Table 2. Reaction system for blunting the C12-279 cleavage product**

| Component | Addition amount (µL) |
|---|---|
| C12-279 cleavage product | 50 |
| 5×T4 DNA Polymerase Buffer | 13 |
| T4 DNA Polymerase | 1 |
| dNTP(10mM) | 0.65 |
| ddH₂O | 0.35 |

### 3) 3' end A-tailing and addition of biotin-labeled adapter

a. 78 uL SPRISelect Beads (Beckman COULTER) were added to the T4 DNA Polymerase reaction product and mixed well, the mixture was placed at room temperature for 5 min, the product was moved to a magnetic rack for adsorption for 5 min, the supernatant was transferred to a new 1.5 mL tube, 39 µL SPRISelect Beads (Beckman COULTER) was added and mixed well, and placed at room temperature for 5 min. The product was moved to the magnetic rack for adsorption for 5 min. A supernatant was discarded, then the product was washed twice with 85% ethanol, placed at room temperature for 10 min for air drying, and added with 50 uL ddH₂O for elution.
b. Using the SynplSeq DNA Library Prep Kit for Illumina library prep kit, 3' A-tailing was performed on the product in step a according to the system in Table 3 at 37°C for 10 min, 65°C for 20 min, and 4°C for later use.

**Table 3. 3' A-tailing of C12-279 cleavage product**

| Component | Addition amount (µL) |
|---|---|
| Cleavage products of C12-279 with different sgRNA | 49 |
| Enhancer | 1.5 |
| End Prep Mix | 6 |
| End Prep Buffer 1 | 3 |
| ddH₂O | 0.5 |
| Total | 60 |

c. Adapter 1 (obtained by annealing an upstream primer: 5'Biosg/gttgacatgctggattgagacttcctacactctttccctacgacgctcttccgatc*t (SEQ ID NO: 705) and a downstream primer: gatcggaagagcgtcgtgtagggaaaga gtgtaggaagtctcaatccagcatgtcaac (SEQ ID NO: 706)) was added according to the system in Table 4 and reacted at 20°C for 30 min and at 16°C overnight. The reaction product was purified using SPRISelect Beads.
Biosg is a biotin modification, and "*" represents thiolation.

**Table 4. Reaction system added with Adapter 1**

| Component | Addition amount (µL) |
|---|---|
| Reaction product in step b | 60 |
| Adapter 1 | 2 |
| DNA Ligase | 1.2 |
| 3x Ligation Buffer 1 | 33 |
| ddH₂O | 3.8 |
| Total | 100 |

d. The reaction product was purified using streptavidin-labeled magnetic beads Dynabeads^{®} M-280 Streptavidin (Invitrogen).
e. Recover PCR
The primers in Table 5 were designed, and the Recover PCR reaction was performed using Q5^{®} Hot Start High-Fidelty 2x Master Mix (NEB) according to the system in Table 6 and the reaction program in Table 7.

**Table 5. Recover PCR primers**

| Primer ID | sequence |
|---|---|
| Recovery PCR Forward | ggagttcagacgtgtgctc (SEQ ID NO: 707) |
| Recovery PCR Reverse | gttgacatgctggattgagacttc (SEQ ID NO: 708) |

**Table 6. Recover PCR reaction system**

| Component | Addition amount (µL) |
|---|---|
| Streptavidin-labeled magnetic beads purification product | 22.5 |
| Recovery PCR Forward (10 uM) | 2.5 |
| Recovery PCR Reverse (10 uM) | 2.5 |
| Q5 Hot-Start 2x Master Mix | 22.5 |
| ddH₂O | Up to 50 |

**Table 7. Recover PCR reaction program**

| Reaction temperature | Duration | Number of cycles |
|---|---|---|
| 98°C | 2 min | 1 |
| 98°C | 10 sec | 12 |
| 61°C | 30 sec | |
| 72°C | 2 min | |
| 72°C | 2 min | 1 |
| 4°C | ∞ | |

f. The Recover PCR product was moved to the magnetic rack and adsorbed for 5 min, the supernatant was moved to a new 1.5 mL centrifuge tube, 3 µL of the Recovery PCR product was taken, and 148.5 µL ddH₂O was added to dilute.
g. Index PCR
The primers in Table 8 were selected, and the Index PCR was performed according to the system in Table 9 and the reaction program in Table 10.

**Table 8. Index PCR primers**

| Primer | Sequence |
|---|---|
| IF501 | aatgatacggcgaccaccgagatctacactatagcctacactctttccctacacgacg (SEQ ID NO: 709) |
| IR701 | caagcagaagacggcatacgagatcgagtaatgtgactggagttcagacgtgtgctc (SEQ ID NO: 710) |

**Table 9. Index PCR reaction system**

| Component | Addition amount (µL) |
|---|---|
| Recovery PCR dilution product | 12 |
| IF501(10 uM) | 4 |
| IR701(10 uM) | 4 |
| Q5 Hot-Start 2x Master Mix | 20 |
| Total | 40 |

**Table 10. Index PCR reaction program**

| Reaction temperature | Duration | Count of cycles |
|---|---|---|
| 98°C | 2 min | 1 |
| 98°C | 10 s | 12 |
| 60°C | 30 s | |
| 72°C | 2 min | |
| 72°C | 2 min | 1 |
| 4°C | ∞ | |

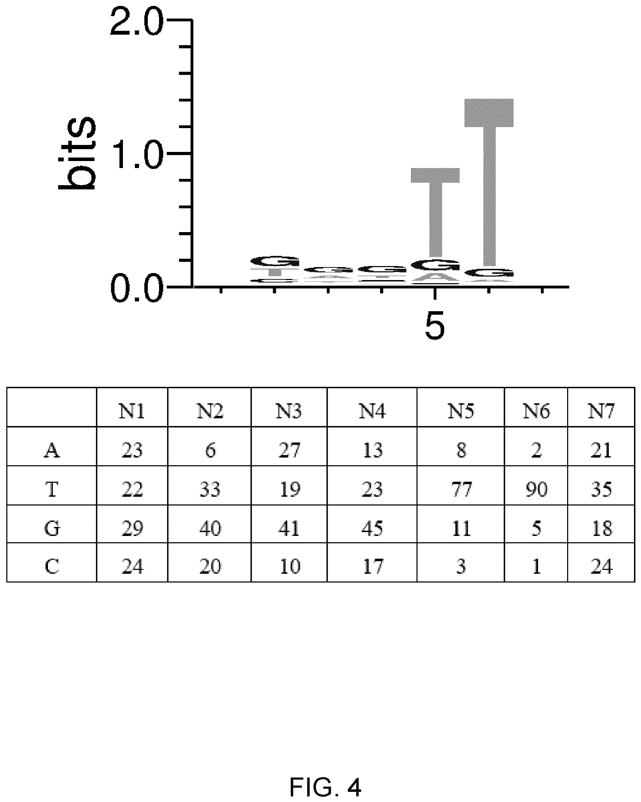

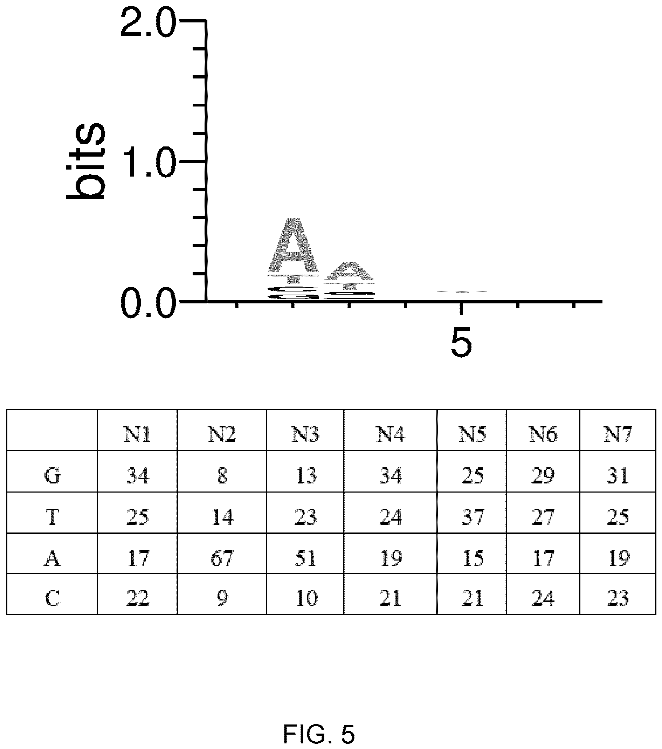

h. 0.7x SPRISelect Beads were added to the Index PCR product for product purification, 38 uL ddH₂O was added for elution, the concentration was measured using Qubit, and then sent for NGS sequencing.
i. Analysis of NGS results: through the NGS sequencing and analysis with WebLogo software by referring to the document *(*A compact Cas9 ortholog from Staphylococcus Auricularis (SauriCas9) expands the DNA targeting scope. PLoS biology, 2020, 18(3), e3000686)*,* the identified motifs were obtained as shown in FIGs. 4 and 5. Both FIGs. 4 and 5 demonstrate that the PAM recognized by the C12-279 is 5'-TTN-3'.

### Example 4. Verification of PAM based on in vivo editing activity of C12-279 protein in bacteria

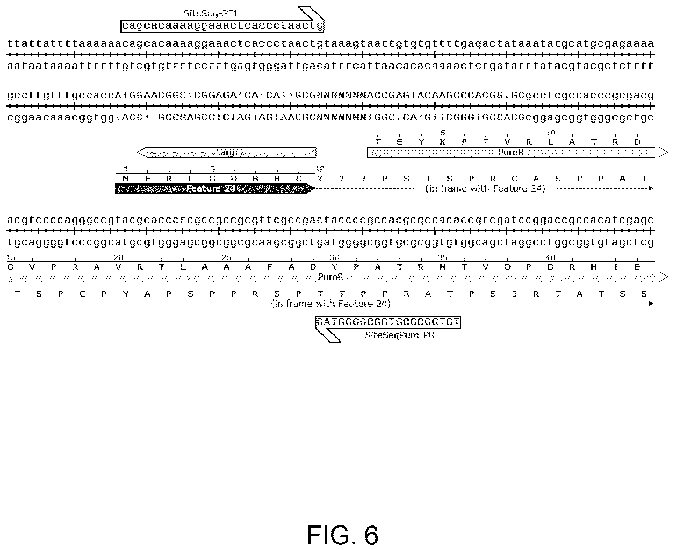

In this example, a plasmid library containing a 7nt random sequence was first constructed, and then a bacterial expression plasmid containing the C12-279 protein coding sequence was constructed. After the expression plasmid was transformed into bacteria to prepare a competent cell, the 7nt random sequence plasmid library was electroporated. If the plasmid in the 7nt random sequence library could be recognized and targeted by C12-279, the plasmid may be removed from the library and the corresponding bacteria could not grow, as shown in FIG. 6.

The specific operations were as follows.

### 1. Construction of 7nt random sequence plasmid library

pLVX-EF1a-BSD vector plasmid (SEQ ID NO: 711) was double-digested by EcoRV and XhoI, and a linearized vector was recovered by agarose gel electrophoresis. Using the prepared pCDH-CMV-EGFP-reporter3-EF1-Puro plasmid (SEQ ID NO: 712) as a template, were used to the DNA fragment containing the coding sequence of the Puro resistance gene was obtained by PCR amplification with primers Puro-PF1 (SEQ ID NO: 714) and Puro-PR1 (SEQ ID NO: 715). The DNA fragment was inserted into the pLVX-EF1a-BSD vector digested with enzymes by homologous recombination (NEB, Gibson Assembly^{®} Master Mix) to construct a recombinant vector pLVX-7NN-Puro library plasmid containing the 7NN random sequence (SEQ ID NO: 713). The reaction solution was transformed into Stbl3 competent cells, coated on an LB plate containing ampicillin, and cultured overnight at 37°C. All colonies were scraped for extracting plasmids.

### 2. Construction of bacterial expression plasmid P15A-C12-279 of C12-279 protein and preparation of competent cells containing the plasmid

### a. Construction of bacterial expression plasmid of C12-279 protein

A P15A-Cas-NC03 vector plasmid (SEQ ID NO: 716) was digested with SalI, and the linearized vector was recovered by agarose gel electrophoresis. Using the prepared pXC12-279-GFPPAM-DR5 plasmid (SEQ ID NO: 697) was used as a template, the DNA fragment encoding the C12-279 protein and the fusion fragment expressing the sgRNA were obtained by PCR with the primers ChkNLS-PF1 (SEQ ID NO: 718) and the synthesized C12-279-sgRNA fragment (SEQ ID NO: 717). The fragments were inserted into the P15A-Cas-NC03 vector digested with enzymes by homologous recombination (NEB, Gibson Assembly^{®} Master Mix) to construct the recombinant vector P15A-C12-279 (SEQ ID NO: 719). The reaction solution was transformed into Stbl3 competent cells, coated on the LB plate containing chloramphenicol, and cultured overnight at 37°C, and a single clone was picked for sequencing verification.

### b. Preparation of competent cells containing bacterial expression plasmid P15A-C12-279

The P15A-C12-279 plasmid verified to be correct by sequencing was transformed into DH5a competent cells (Vidyabiotech, CAT#: DL1001), and the single clone was picked and inoculated into LB medium containing chloramphenicol and cultured at 37°C overnight.

Electrocompetent cells were prepared as follows.

The culture bacterial solution was inoculated into 100 mL of fresh LB medium containing chloramphenicol at a ratio of 1:100 for amplification culture at 37°C and 220 rpm.

Then the culture bacterial solution was cultured until OD600 reached 0.5, the bacterial solution was transferred to a 50 mL centrifuge tube and pre-cooled on ice for 30 min.

Then the culture bacterial solution was centrifuged at 4000 rpm and 4°C for 10 min, the cells were collected and resuspend in an equal volume of pre-cooled sterile water.

The above operations were repeated.

The cells were resuspended in 1/10 volume of pre-cooled sterile water containing 10% glycerol, divided into 50 uL/tube, and stored at -80°C to obtain the P15A-C12-279 competent cells.

### c. Performing plasmid elimination to identify the PAM sequence of the C12-279 protein

100 ng of pLVX-7NN-Puro library plasmid was electroporated into the prepared P15A-C12-279 competent cells and DH5a electroporated cells, respectively, and marked as Lib1 (electroporated P15A-C12-279 competent cells) and Lib2 (electroporated DH5a competent cells), respectively.

After electroporation, 10 mL of LB medium was added, and the cells were revived and cultured at 37°C and 220 rpm for 2 h.

The revived bacterial solution was centrifuged at 4000 rpm for 2 min to collect the bacteria, and then resuspended in 400 uL LB and coated on the LB plate. The electroporated DH5a bacterial solution was coated on the LB plate containing ampicillin, and the electroporated P15A-C12-279 competent cells were coated on the LB plate containing chloramphenicol and ampicillin and cultured at 37°C overnight.

The bacterial cells were scraped from the culture plate and the plasmid DNA was extracted by alkaline lysis manner.

100 ng of each of the two extracted plasmid DNA samples was used as the PCR template, and a PCR amplification was performed using primers SiteSeq -PF1 (SEQ ID NO: 720) and SiteSeqPuro-PR (SEQ ID NO: 721). The obtained fragments were subjected to amplicon library construction using an NGS library construction kit (Xunshi Biotechnology, SynplSeq DNA Library Prep Kit for Illumina), followed by NGS sequencing.

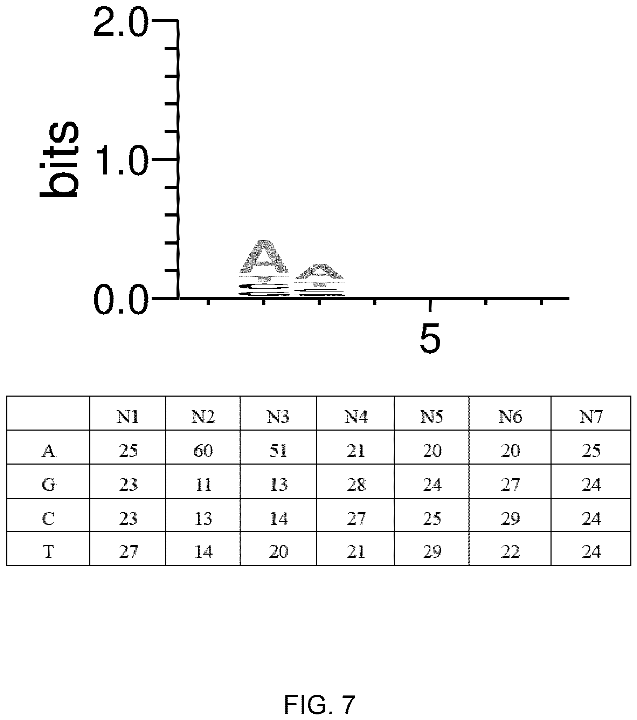

The NGS sequencing data from Lib1 and Lib2 cells is compared and analyzed, as shown in FIG. 7. It is proved that the PAM sequence recognized by the C12-279 is 5'-TTN-3'.

### Example 5. Cleavage activity of C12-279 protein on target nucleic acid in 293T cells

In this example, sgRNA targeting TTR genes in HEK293T cells was first designed and constructed into a pXC12-279-GFPPAM-DR5 plasmid (SEQ ID NO: 697) to obtain a plasmid pXC12-279-TTR01 targeting the TTR genes. After transfecting the HEK293T cells, an indel ratio was verified by NGS to verify the cleavage activity of the C12-279 protein in 293T cells. The specific operations were as follows.

### 1. Construction of sgRNA plasmid

An sgRNA target site catgagcatgcagaggtgagtat (SEQ ID NO: 722) was designed based on TTR gene sequence information in a HEK293T cell line.

**Table 11. sgRNA annealing primer**

| Plasmid name | Primer name | Primer sequence |
|---|---|---|
| pXC12-279-TTR01 | TTR01-PF1 | cattgacgccatgagcatgcagaggtgagtatTTTTTG (SEQ ID NO: 723) |
| | TTR01-PR1 | gtaccAAAAAatactcacctctqcatgctcatggcgtc (SEQ ID NO: 724) |

The annealing primers were designed according to the sgRNA target site information, and the plasmid pXC12-279-GFPPAM-DR5 (SEQ ID NO: 697) was digested with BsmBI (Thermo Scientific^{™}, ER0451) and Acc65I (Thermo Scientific^{™}, ER0901). The primers (Table 11) were annealed and then connected with the vector to obtain a pXC12-279-TTR01 expression clone.

### 2. Detection of TTR gene editing efficiency

Plating: 293T cell lines were plated when a confluency reached 70-80%, and a count of cells seeded in a 24-well plate was 5*10^5 cells/well.

Transfection: Transfection was performed 12-14 h after plating. 100 µL Opti-MEM, 1.5 µL PEI (Yeasen Biotechnology, Polyethylenimine Linear (PEI) MW25000), and 500 ng pXC12-279-TTR01 plasmid were added to each well of a 24-well plate, mixed, and added to 293T cells for cell transfection after placing at room temperature for 20 min. After overnight transfection, a fresh culture medium was replaced, and culture was continued.

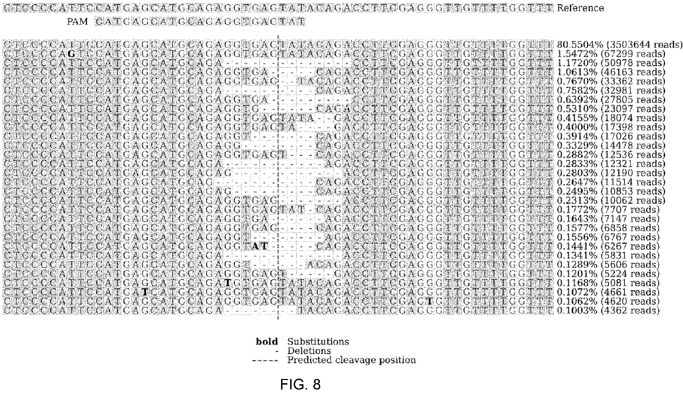

DNA extraction, PCR amplification, and NGS library construction: After 72 h of culture, the cells were washed with PBS, and then 100 µL of cell lysis solution (Viagen, DirectPCR^{®} Lysis Reagent (Cell)) was added for lysis to obtain lysate containing genomic DNA. The region near the target sequence was amplified for the genomic DNA. The PCR product was subjected to the NGS library construction and sequencing, and the sequencing result was analyzed. The indel rate is higher than 14%, as shown in FIG. 8.

### Example 6. PAM recognition of CI1062732

Compared with the C12-279 protein (SEQ ID NO: 696), the protein CI1062732 (SEQ ID NO: 46) lacks dozens of amino acid residues at the N-terminal.

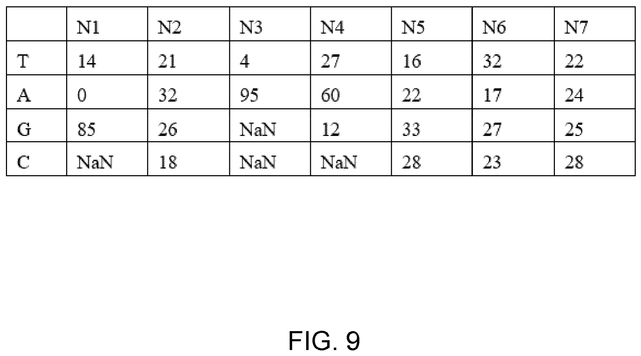

A PAM sequence recognized by CI1062732 was identified using a manner substantially the same as that in Example 4. A gRNA containing a "false" DR sequence GTAATGCGTCTCCCATTGACGCC (SEQ ID NO: 529) was combined with CI1062732 to target a 7nt random sequence plasmid library in bacteria. The sequencing analysis (as shown in FIG. 9) indicates that a "false" PAM motif identified by CI1062732 is 5'-TTNC-3'.

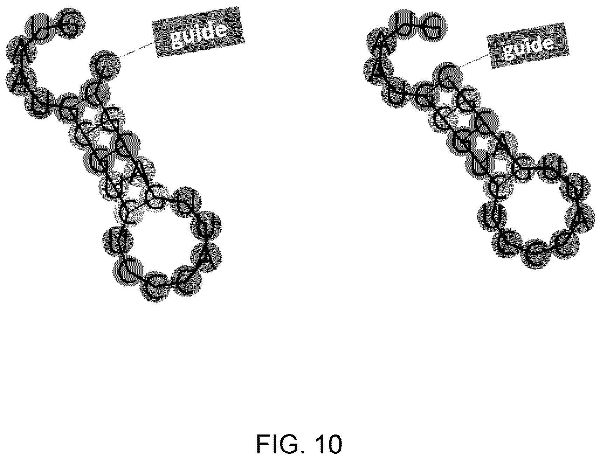

Subsequently, by analyzing a secondary structure of the "false" DR sequence, it is hypothesized that the 3'-end C base of the identified "false" PAM motif TTNC might be caused by an extra C at the 3' end of DR (FIG. 10). Therefore, in subsequent CI1062732 test and C12-279 test, the DR sequence is GTAATGCGTCTCCCATTGACGC (SEQ ID NO: 704).

### Example 7. Screening, design, preparation, and testing of C12-101-07 protein

(1) The C12-101-07 protein was ultimately obtained through screening and design using complex bioinformatics manners.

An amino acid sequence of the C12-101-07 protein is:

IFPTQRRNTMSGNVVRSYKSVLRPNIRKRELLDATFNWFDRAYKKFFDVFVCLYGGVEHDTVHSALLKETT
DPDLVCATMWFRVVPKSSCDGISAQEMIRRFGVYAGHEPSAVAMSYLTGNFDDKKNHWIDCREKFVALAR
DMVVKPESLSIDIKSMIEHKILPICSQDNWQAWNAISQLFGEGKKENKAEKAQIFVDVVSMLSNNSVQTWE
DYKEVISKATGCRTAGEINSKFGGRPGILSVDFSKDDTGSLPKDFIERRIKDLSQKAKEKSAAYELPNRMRLR
ELIASVIGPYRLETWSSVAQRACGDIRSKNSNNLLYASERFNRTKEIEEILSAKGDVARAQNILGQFRSGEFN
EFAVEKRHLGNLESLYNIWSKSDMDTGIEEYSSIHKDEYDRDPIVDLYRHIYPHREVISAKNFIDAAVLNKLL
RLNASRHVHPTVFGKTVISFSPKSSAYGRITPPSEIVRGRPAGSHGMIWLTMELFDDGKWIEHHLPFHNSR
YYEEVYCYQEGLPVKDEPRSPMFGYRVGNAIADTSKIDNRRRKASKQFLRAQQNITHNVSFDENTAFSVVR
NGEDFSITISSRIKASNAKSLMTIGDRIMGIDQNQTAANTYSIWEVVGDGDAGSIQHGNLSLKRIEDGHITS
IIRGRGGNFDQLNYGGLSYAKFSEWRSARLGFISSLNAELASKMNWDWCCLYEWNARYASALRKIIYSHK
GIDIERVFRREIEDFVEGVLRIGSLSSDALQCLTNAKSLISSYFFLNGKKEPEEQRKFDQGLFDLSAKIDKKRV
NKRQQKTKRIMSSIMQIARDRGVSFIVVEGKLSTATKDNKSKANQKAIDWCARAVVENLEHSCSLVGIKLV
AIDPMNTSHLDPFVYVLKTSLGKEARFASVVPAKINARHMKNFKSWSSLLAGNGKIKKTTDAIYVGAFQDFC
REYGFVAEDISKMSQSEIQDKLSSHQFVLVPQRGGRVYMSTHPVSSEAKKIVYAGRERWYNNADVVAAVN
ITLRGCERLSASRKTASTR (SEQ ID NO: 52).

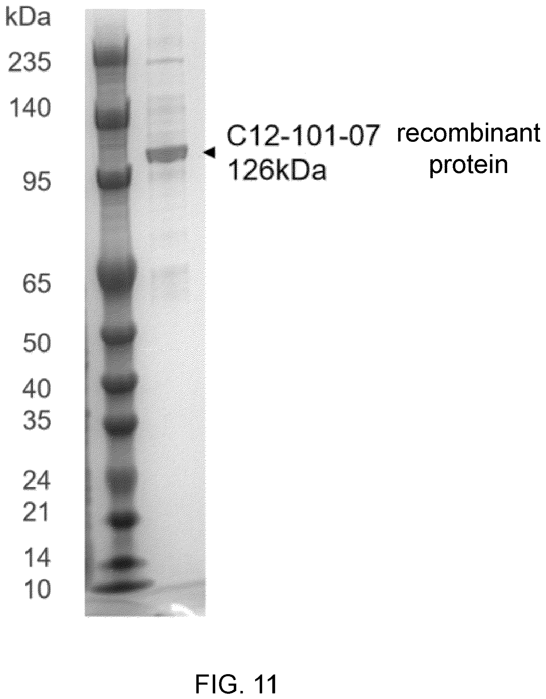

The recombinant vector C12-101-07-pET28a-01 (SEQ ID NO: 725) was constructed by a manner substantially the same as that in Example 2, which was expressed and purified to obtain the C12-101-07 recombinant protein, with an amino acid count of 1122 aa and a structure of His tag-NLS-C12-101-07-SV40 NLS-nucleoplasmin NLS. The purified recombinant protein was detected by SDS-PAGE electrophoresis, and the result is shown in FIG. 11.

(2) The in vitro cleavage of PAM library by the C12-101-07 protein was prepared using a manner substantially the same as that in Example 3 to grab the PAM.

In vitro transcription was used to synthesize sgRNA containing a specific guide sequence, the sequence of which is as follows:
>C12-101-07-sgRNA
   5'-atcgcaacatctcagaaacccgtcctaagttgacggGTGAGCAAGGGCGAGGAGCTGTTC-3' (SEQ ID NO: 726).
>C12-101-07-sgRNA-Rev
   5'-atcgcaacatctcagaaacccgtcctaagttgacggCGCAATGATGATCTCCGAGCCGTTCC-3' (SEQ ID NO: 727).

The sgRNA scaffold sequence is: 5'-atcgcaacatctcagaaacccgtcctaagttgacgg-3' (SEQ ID NO: 534).

The C12-101-07 protein was combined with forward and reverse gRNAs respectively for editing.

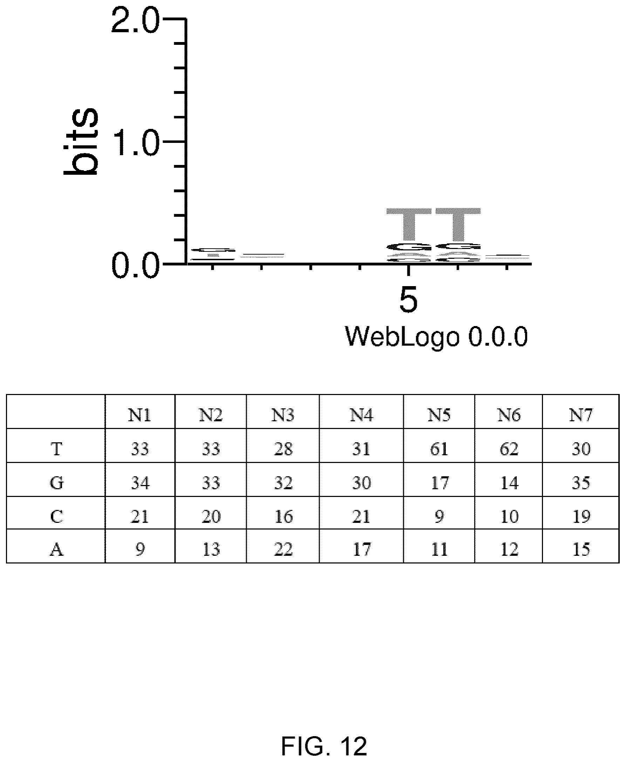

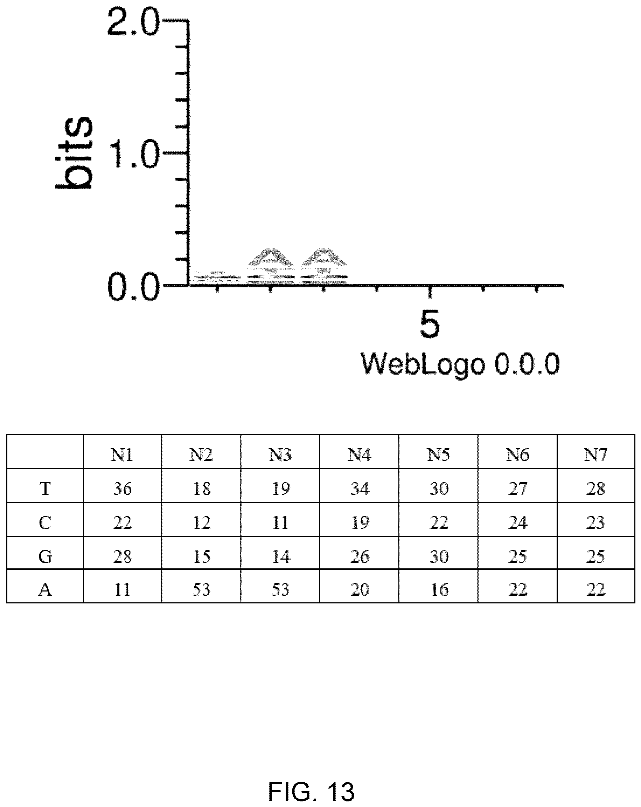

The PAM motifs shown in FIGs. 12 and 13 were identified during the experiment.

It is demonstrated that the PAM sequence recognized by C12-101-07 is 5'-TTN-3'.

### Example 8. In vivo editing activity of C12-101-09 protein in bacteria and verification of PAM

A similar protein C12-101-09 was designed based on C12-101-07, with the sequence as follows:
>C12-101-09

IFPTQRRNTMSGNVVRSYKSVLRPNIRKRELLDATFNWFDRAYKKFFDVFVCLYGGVEHDTVHSALLKETTD
PDLVCATMWFRVVPKSSCDGISAQEMIRRFGVYAGHEPSAVAMSYLTGNFDDKKNHWIDCREKFVALARD
MVVKPESLSIDIKSMIEHKILPICSQDNWQAWNAISQLFGEGKKENKAEKAQIFVDVVSMLSNNSVQTWED
YKEVISKATGCRTAGEINSKFGGRPGILSVDFSKDDTGSLPKDFIERRIKDLSQKAKEKSAAYELPNRMRLRE
LIASVIGPYRLETWSSVAQRACGDIRSKNSNNLLYASERFNRTKEIEEILSAKGDVARAQSILGQFRSGEFNE
FAVEKRHLGNLESLYNIWSKSDMDTGIEEYSSIHKDEYDRDPIVDLYRHIYPHREVISAKNFIDAAVLNKLLR
LNASRHVHPTVFGKTVISFSPKSSAYGRITPPSEIVRGRPAGSHGMIWLTMELFDDGKWIEHHLPFHNSRYY
EEVYCYQEGLPVKDEPRSPMFGYRVGNAIADTSKIDNRRRKASKQFLRAQQNITHNVSFDENTAFSVVRNG
EDFSITISSRIKASNAKSLMTIGDRIMGIDQNQTAANTYSIWEVVGDGDAGSIQHGNLSLKRIEDGHITSIIR
GRGGNFDQLNYGGLSYAKFSEWRSARLGFISSLNAELASKMNWDWCCLYEWNARYASALRKIIYSHKGIDI
ERVFRREIEDFVEGVLRIGSLSSDALQCLTNAKSLISSYFFLNGKKEPEEQRKFDQGLFDLSAKIDKKRVNKR
QQKTKRIMSSIMQIARDRGVSFIVVEGKLSTATKDNKSKANQKAIDWCARAVVENLEHSCSLVGIKLVAIDP
MNTSHLDPFVYVLKTSLGKEARFASVVPAKINARHMKNFKSWSSLLAGNGKIKKTTDAIYVGAFQDFCREY
GFVAEDISKMSQSEIQDKLSSHQFVLVPQRGGRVYMSTHPVSSEAKKIVYAGRERWYNNADVVAAVNITLR
GCERLSASRKTASTR (SEQ ID NO: 728).

The recombinant vector plasmid P15A-C12-101-09 (SEQ ID NO: 729) was constructed by conventional manners, and then tests were performed using manners substantially the same as those in Example 4.

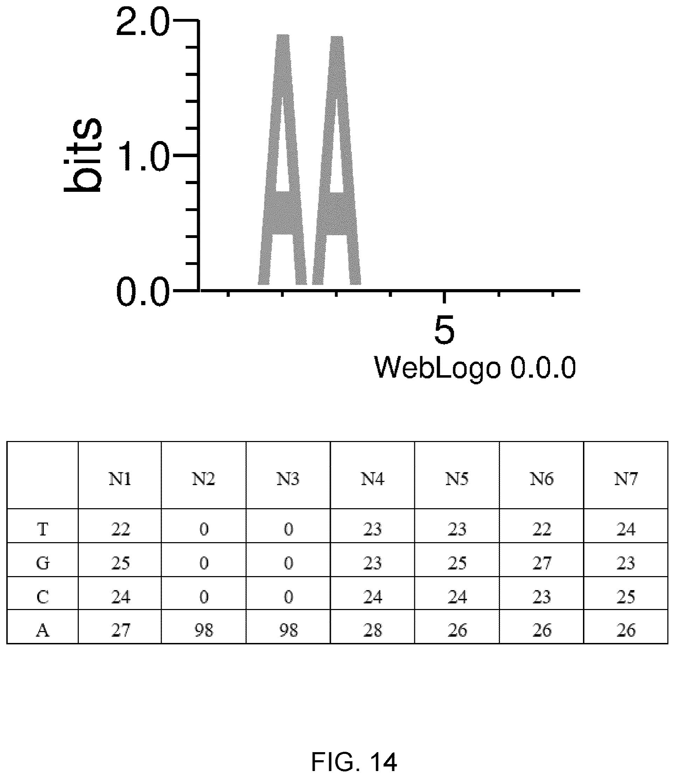

The motif recognized by C12-101-09 was identified, as shown in FIG. 14.

It is demonstrated that the PAM sequence recognized by C12-101-09 is 5'-TTN-3'.

### Example 9. Construction of cell lines containing different PAM reporter systems

Stable cell lines were prepared by lentiviral infection. By constructing lentiviral expression plasmids containing different PAM sequences, 293T cells were infected after virus packaging to construct cell lines containing GFP reporter systems with different PAM sequences for subsequent mutation screening.

### (1) Construction of lentiviral expression plasmids for GFP reporter systems with different PAM sequences

In addition to the prepared plasmid pCDH-CMV-EGFP-reporter3-EF1-Puro (SEQ ID NO: 712), a plasmid library with a PAM composition of NAAN was simultaneously constructed. A specific construction scheme was as follows.

A mixed library of EGFP fragments containing a detection system was synthesized by gene synthesis, and digested with XbaI+NotI, then the digested fragments were ligated to the XbaI+NotI digested vector of the plasmid pCDH-CMV-EGFP-reporter3-EF1-Puro using T4 DNA ligase. The ligation products were transformed into Stbl3 cells, and cultured on ampicillin-containing plates at 37°C overnight.

For the mixed fragment library, multiple clones were picked for sequencing and identification to obtain 16 plasmids with different sequence compositions (i.e., PAM sequences being AAAA, AAAT, AAAG, AAAC, TAAA, TAAT, TAAG, TAAC, GAAA, GAAT, GAAG, GAAC, CAAA, CAAT, CAAG, CAAC), and then the plasmids were mixed at equal mass to obtain a Plasmid Lib (Puro-NAAN-eGFP-Lib, SEQ ID NO: 730) with a PAM sequence composition of NAAN for subsequent lentiviral packaging and construction of stable cell line.

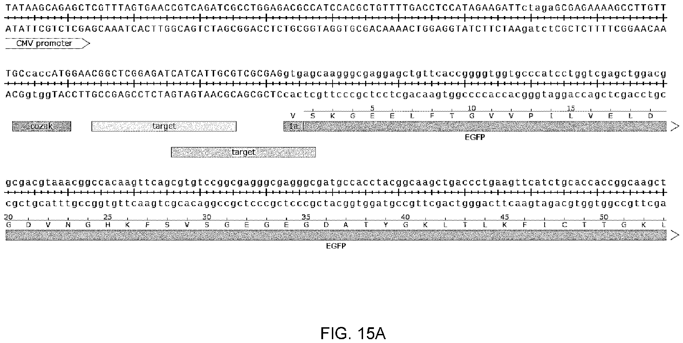

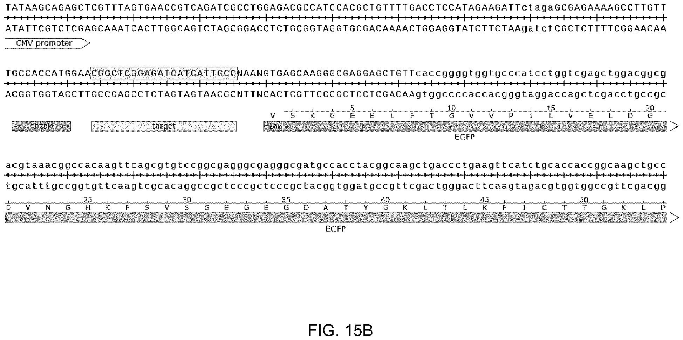

In the unedited reporter system, there is a base insertion that is not a multiple of 3 from a start codon to a normal GFP reading frame (pCDH-CMV-EGFP-reporter3-EF1-Puro has a 32bp base insertion, and the Plasmid Lib has a 29bp base insertion, as shown in FIG. 15), which causes the GFP normal reading frame to be interrupted, and GFP is not expressed. Then an sgRNA target site was set inside a GFP expression frame, and indel was generated by the editing of Cas12, having a probability to restore the normal GFP reading frame, so that GFP was expressed normally, and the higher an editing efficiency, the higher the probability of the generated indel to restore the normal GFP reading frame. The editing efficiency of the Cas12 protein was characterized by counting GFP-expressing cells using flow cytometry.

### (2) Lentiviral packaging of GFP reporter system plasmids with different PAM sequences

The constructed pCDH-CMV-EGFP-reporter3-EF1-Puro plasmid and Plasmid Lib were mixed with virus packaging auxiliary plasmids pMD2.G (Miaoling Biosciences) and psPAX2 (Miaoling Biosciences) at a molar ratio of 1:1:1, respectively, and then transfected into 293T cells using PEI. After 48 h of transfection, a culture supernatant was taken and filtered with 0.45 µm of a filter film to obtain two crude viruses, namely pCDH-CMV-EGFP-reporter3-EF1-Puro and Plasmid Lib.

### (3) Construction of the detection cell line by infecting 293T cells using the crude viruses pCDH-CMV-EGFP-reporter3-EF1-Puro and Plasmid Lib

293T cells were infected with 1/4 volume of crude viruses pCDH-CMV-EGFP-reporter3-EF1-Puro and Plasmid Lib in culture medium. After 48 h of infection, the medium was changed and 2 ug/mL of Puromycin was added for screening.

For the 293T cells infected with pCDH-CMV-EGFP-reporter3-EF1-Puro, the screened cells were subjected to monoclonal screening by limited dilution, and the screened monoclonal cell lines were the cell line used for detection (namely, Reporter3 cell line).

For the 293T cells infected with Plasmid Lib, the cell pool obtained after drug screening was the cell line used for detection (namely, NAAN cell line).

### Example 10. Design of C12-279 protein mutation and detection of editing efficiency

### a. Determination of mutation position

A 3D structure of C12-279 protein was predicted and simulated through bioinformatics analysis and AI manners. Possible binding, recognition and cleavage sites of DNA of C12-279 were identified in combination with the 3D structure. The molecular cloning point mutation manner was used to construct mutation clones for these sites.

First, the mutations shown in Table 12 were designed.

**Table 12. Editing efficiency of different mutation positions of C12-279 in NAAN cell line**

| Mutation position | Mutant vector | Editing efficiency in NAAN cell line, expressed as a multiple of the editing efficiency of C12-279 |
|---|---|---|
| D352R | C12-279-GFPPAM-01 | 1.45±0.36 |
| K631R | C12-279-GFPPAM-02 | 0.99±0.18 |
| A988R | C12-279-GFPPAM-03 | 1.24±0.14 |
| G989R | C12-279-GFPPAM-04 | 1.23±0.44 |
| Q186R | C12-279-GFPPAM-05 | 1.74±0.38 |
| G194R | C12-279-GFPPAM-06 | 0.01 |
| N195R | C12-279-GFPPAM-07 | 1.64 |
| G196R | C12-279-GFPPAM-08 | 0.31±0.12 |
| G197R | C12-279-GFPPAM-09 | 1.44±0.45 |
| N245R | C12-279-GFPPAM-10 | 0.46±0.06 |
| L260R | C12-279-GFPPAM-11 | 1.56±0.07 |
| A355R | C12-279-GFPPAM-12 | 1.31±0.36 |
| C385R | C12-279-GFPPAM-13 | 1.20±0.37 |
| P386R | C12-279-GFPPAM-14 | 1.40±0.48 |
| H387R | C12-279-GFPPAM-15 | 0.92±0.33 |
| G390R | C12-279-GFPPAM-16 | 0.00 |
| K391R | C12-279-GFPPAM-17 | 1.14±0.34 |
| N392R | C12-279-GFPPAM-18 | 1.39±0.18 |
| D429R | C12-279-GFPPAM-19 | 0.34±0.06 |
| Q461R | C12-279-GFPPAM-20 | 0.99±0.3 |
| Q462R | C12-280-GFPPAM-21 | 1.36±0.39 |
| E485R | C12-280-GFPPAM-22 | 1.57±0.37 |
| L611R | C12-280-GFPPAM-23 | 0.43±0.09 |
| Q990R | C12-280-GFPPAM-24 | 0.94±0.09 |
| A1136R | C12-280-GFPPAM-25 | Not detected |
| K1138R | C12-280-GFPPAM-26 | Not detected |
| T1139R | C12-280-GFPPAM-27 | Not detected |
| N/A | NAAN cell line | 0.00 |
| N/A | PEI-control | 0.00 |
| N/A | C12-279-GFPPAM-DR5 | 1.00±0.28 |

### b. Construction of mutation clones

After a specific mutation position was determined, the mutation base was introduced through primers to construct expression clones containing different mutation positions. The following was an example of the construction of a D352R mutation clone.

**Table 13. Primer sequences for the construction of mutation clone C12-279-GFPPAM-01 of C12-279**

| Primer name | Primer sequences |
|---|---|
| ChkCas12-PF1 | CCAAGCTGGCTAGCGTTTAAACTTAAG (SEQ ID NO: 731) |
| ChkCas12-PR1 | ATGATCTCCGAGCCGTTTTTGGTACC (SEQ ID NO: 732) |
| C279-D352R-PF1 | GCTGAAGAGACACAGCagaATCGCCGCCGCT (SEQ ID NO: 733) (underlined being introduced amino acid mutations) |
| C279-D352R-PR1 | GGGAAGCGGCGGCGATtctGCTGTGTCTCT (SEQ ID NO: 734) (underlined being introduced amino acid mutations) |

Primers were designed for the mutation position D352R (as shown in Table 13), and the mutation position was introduced by primers C279-D352R-PF1 and C279-D352R-PR1. Using pXC12-279-GFPPAM-DR5 (SEQ NO: 1) as a template, ChkCas12-PF1+C279-D352R-PR1 was subjected to PCR amplification (Yijin Bio, PC019, UltraHiPF^{™} DNA Polymerase Kit) to obtain fragment C12-279-D352R-F1, ChkCas12-PR1+C279-D352R-PF1 was subjected to PCR amplification to obtain fragment C12-279-D352R-F2, plasmid pXC12-279-GFPPAM-DR5 (SEQ ID NO: 697) was digested using HindIII+KpnI, and 5646 bp vector fragment was gel recovered (Guangzhou Meiji Biotechnology Co., Ltd., D2110, HiPure Gel Pure Micro Kit), and was recombined in vitro with fragments C12-279-D352R-F1 and C12-279-D352R-F2 (NEB, E2611L, Gibson Assembly^{®} Master Mix), and the mutation clone plasmid C12-279-GFPPAM-01 was obtained by heat-shock transformation of *Escherichia coli.*

### c. Detection of the editing efficiency of mutants in NAAN reporter system

Plating: cells were plated when a confluence of the NAAN cell line reached 70-80%, and a number of cells seeded in a 24-well plate was 5*10^5 cells/well.

Transfection: transfection was performed 12-14 h after plating. 1.5 uL PEI (Yeasen Biotechnology, 40815ES03, Polyethylenimine Linear (PEI) MW25000) and 500 ng mutation clone plasmid were added to 100 µL Opti-MEM per well of the 24-well plate, mixed, and added to the NAAN cell line for cell transfection after placing at room temperature for 20 min. After overnight transfection, a fresh culture medium was replaced, and the culture was continued for 72 h, followed by flow cytometry. Editing efficiencies of different mutant clones were characterized by the proportion of GFP-positive cells.

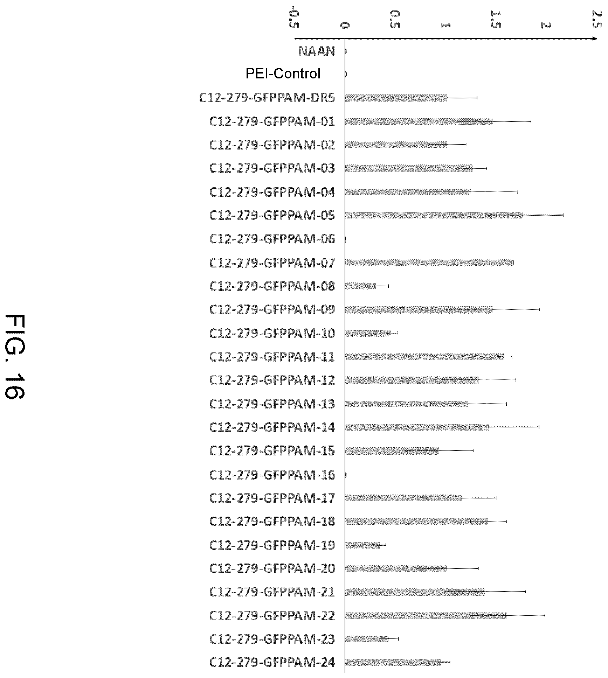

The results are shown in Table 16 and FIG. 16. The editing efficiency of different C12-279 mutants in NAAN cells is expressed as a multiple of the editing efficiency of C12-279. Multiple mutants improve the editing efficiency.

d. According to the results of a first round of mutation, a portion of mutation positions that significantly improved the editing efficiency were combined, and multiple mutation position combinations were tried to further improve the editing efficiency. The designed mutants were shown in Table 14.

**Table 14. Editing efficiency of different mutation position combinations of C12-279 in NAAN cell line**

| Mutation position 1 | Mutation position 2 | Vector name of the mutant |
|---|---|---|
| D352R | Q186R | C12-279-GFPPAM-28 |
| D352R | L260R | C12-279-GFPPAM-29 |
| D352R | A355R | C12-279-GFPPAM-30 |
| A355R | L260R | C12-279-GFPPAM-31 |
| P386R | C385R | C12-279-GFPPAM-32 |
| E485R | Q462R | C12-279-GFPPAM-33 |

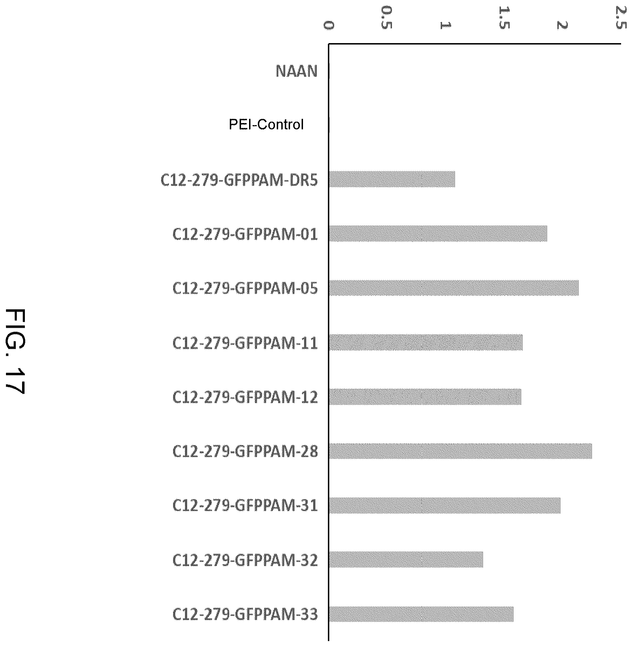

The vector was constructed using essentially the same manner as described above, and the editing efficiency was tested in the NAAN cell line. The results are shown in FIG. 17.

### e. Construction of mutant plasmid targeting fixed PAM combination

As the PAM of the NAAN cell line was a mixed library, it may theoretically have a certain impact on an actual editing efficiency. To more intuitively and efficiently demonstrate the effect of mutation on the editing efficiency, based on a sequence between a start codon of the Reporter3 cell line and a GFP reading frame, a target site with a PAM sequence of TTG and a target sequence of CTCACCTCGCGACGCAATGATG (SEQ ID NO: 735) was selected for subsequent editing efficiency test.

### Construction of mutation clones targeting Reporter3 cell line

Based on C12-279-GFPPAM-DR5, the sgRNA was changed to target CTCACCTCGCGACGCAATGATG (SEQ ID NO: 735), the synthesized primers pCDH-PF1: GTACCGAAAAACATCATTGCGTCGCGAGGTGAGGCGTC (SEQ ID NO: 754) and pCDH-PR1: CATGACGCCTCACCTCGCGACGCAATGATGTTTTTCG (SEQ ID NO: 755) were annealed. The plasmid C12-279-GFPPAM-DR5 was digested with Acc65I (Thermo Scientific) and BsmBI (Thermo Scientific) to recover the vector and then connected with an annealing product to obtain the mutation clone C12-279-pCDH targeting the Reporter3 cell line. The construction manner of the mutation clones targeting Reporter3 cell lines was the same as the manner described in this example, except that the vector plasmid needed to be changed from C12-279-GFPPAM-DR5 to C12-279-pCDH, and the primer ChkCas12-PR1 needed to be replaced by ChkCas12-PR2: GCGACGCAATGATGTTTTTCGGTACC (SEQ ID NO: 736).

Editing efficiency of some selected mutants was tested in Reporter3 cell line using the same manner as described for the NAAN reporter system, except that the cell line was changed from NAAN cell line to Reporter3 cell line.

According to the results of the first round of mutation, the entire 3D structure was corrected and marked, and data of the first round was placed in a new model for predictive analysis. Finally, possible second round mutation positions were analyzed and predicted, and mutations and detections were performed. By analogy, the best mutation combination of C12-279 was determined through a plurality of rounds of mutations, selections, and iterations. Table 15 shows the editing efficiency of C12-279 mutants in the Reporter3 cell line. An absolute value (average) of the editing efficiency of the C12-279-pCDH group, i.e., the C12-279 protein, is 8.55%.

**Table 15. Editing efficiency of C12-279 mutants in Reporter3 cell line**

| (Expressed as multiple of editing efficiency of C12-279) | | | | |
|---|---|---|---|---|
| Mutation position 1 | Mutation position 2 | Mutation position 3 | Group | Multiple of editing efficiency of C12-279 |
| N/A | N/A | N/A | PEI-control | 0.02±0.01 |
| N/A | N/A | N/A | Reporter3 cell line | 0.03±0.02 |
| N/A | N/A | N/A | C12-279-pCDH | 1.00±0.18 |
| D352R | N/A | N/A | C12-279-pCDH-01 | 1.18±0.22 |
| G989R | N/A | N/A | C12-279-pCDH-04 | 1.27±0.2 |
| Q186R | N/A | N/A | C12-279-pCDH-05 | 1.60±0.32 |
| L260R | N/A | N/A | C12-279-pCDH-11 | 0.66±0.14 |
| A355R | N/A | N/A | C12-279-pCDH-12 | 0.96±0.14 |
| E485R | N/A | N/A | C12-279-pCDH-22 | 1.15±0.32 |
| D352R | Q186R | N/A | C12-279-pCDH-28 | 1.39±0.25 |
| A355R | L260R | N/A | C12-279-pCDH-31 | 0.95±0.22 |
| D352R | Q186R | I33R | C12-279-pCDH-34 | 1.51±0.31 |
| D352R | Q186R | G184R | C12-279-pCDH-35 | 2.28 |
| D352R | Q186R | S185R | C12-279-pCDH-36 | 1.91 |
| D352R | Q186R | G256R | C12-279-pCDH-37 | 0.06±0.01 |
| D352R | Q186R | Y278R | C12-279-pCDH-38 | 1.86±0.06 |
| D352R | Q186R | S285R | C12-279-pCDH-39 | 1.87±0.06 |
| D352R | Q186R | Y316R | C12-279-pCDH-40 | 1.12±0.2 |
| D352R | Q186R | H350R | C12-279-pCDH-41 | 2.11 |
| D352R | Q186R | A356R | C12-279-pCDH-42 | 2.01±0.18 |
| D352R | Q186R | Q469R | C12-279-pCDH-43 | 1.61±0.22 |
| D352R | Q186R | S491R | C12-279-pCDH-44 | 1.41±0.48 |
| D352R | Q186R | K521R | C12-279-pCDH-45 | 1.73±0.07 |
| D352R | Q186R | P525R | C12-279-pCDH-46 | 1.40±0.09 |
| D352R | Q186R | K629R | C12-279-pCDH-47 | 1.74±0.09 |
| D352R | Q186R | N633R | C12-279-pCDH-48 | 1.84±0.06 |
| D352R | Q186R | D841R | C12-279-pCDH-49 | 1.83±0.00 |
| D352R | Q186R | N898R | C12-279-pCDH-50 | 1.69±0.05 |
| D352R | Q186R | K987R | C12-279-pCDH-51 | 1.76±0.07 |
| D352R | Q186R | T991R | C12-279-pCDH-52 | 1.32 |
| D352R | Q186R | D1010R | C12-279-pCDH-53 | 1.58±0.29 |
| D352R | Q186R | E1013R | C12-279-pCDH-54 | 2.00±0.04 |

### Example 11. Design of C12-101-07 protein mutants and detection of editing efficiency

Mutants shown in Table 16 were designed for the C12-101-07 protein.

The vector plasmid of mutant was constructed using a manner basically the same as that in Example 10, and the editing efficiency was detected.

a. Detection of the editing efficiency of single point mutation based on NAAN reporter system.

For selected mutation positions, C12-101-07-GFPPAM (SEQ ID NO: 737) was used as a cloning template plasmid and a control plasmid for transfection test, and the mutation clone was constructed by molecular cloning point mutation, and the editing efficiency was detected. The results are shown in Table 16. The editing efficiency of different C12-101-07 mutants in NAAN cell line is expressed as a multiple of the editing efficiency of C12-101-07. An absolute value (average) of the editing efficiencies of the C12-101-07-GFPPAM group, i.e., the C12-101-07 protein, is 0.23%.

**Table 16. Different mutants of C12-101-07**

| Mutation position | Mutation vector | Editing efficiency in NAAN cell line, expressed as multiple of the editing efficiency of C12-101-07 |
|---|---|---|
| Q172R | C12-101-07-GFPPAM-01 | 2.08±1.05 |
| G182R | C12-101-07-GFPPAM-02 | 0.01±0.02 |
| E183R | C12-101-07-GFPPAM-03 | 0.95±0.56 |
| G184R | C12-101-07-GFPPAM-04 | 0.02±0.04 |
| K185R | C12-101-07-GFPPAM-05 | 1.01±0.47 |
| K186R | C12-101-07-GFPPAM-06 | 0.02±0.02 |
| V243R | C12-101-07-GFPPAM-07 | 0.71±0.27 |
| L297R | C12-101-07-GFPPAM-08 | 0.04±0.00 |
| N317R | C12-101-07-GFPPAM-09 | 0.52±0.12 |
| E363R | C12-101-07-GFPPAM-10 | 0.19±0.12 |
| H366R | C12-101-07-GFPPAM-11 | 0.02±0.02 |
| V426R | C12-101-07-GFPPAM-12 | 1.15±0.59 |
| K429R | C12-101-07-GFPPAM-13 | 0.80±0.36 |
| L433R | C12-101-07-GFPPAM-14 | 0.57±0.28 |
| S452R | C12-101-07-GFPPAM-15 | 1.09±0.26 |
| S455R | C12-101-07-GFPPAM-16 | 0.07±0.02 |
| K918R | C12-101-07-GFPPAM-17 | 0.50±0.11 |
| T919R | C12-101-07-GFPPAM-18 | 0.29±0.15 |
| T920R | C12-101-07-GFPPAM-19 | 1.21±0.70 |
| A922R | C12-101-07-GFPPAM-20 | 1.18±0.41 |
| N/A | C12-101-07-GFPPAM | 1.00±0.67 |

b. The mutation positions that significantly improved the editing efficiency in the first round of mutations were combined to try two-point mutations. The mutants shown in Table 17 were designed.

Mutation clones were constructed using a point mutation molecular cloning method and the editing efficiency was determined based on the NAAN cell line.

The results are shown in Table 17. The editing efficiency of different C12-101-07 mutants in the NAAN cell line is expressed as the multiple of the editing efficiency of C12-101-07.

**Table 17. C12-101-07 mutants**

| Mutation position 1 | Mutation position 2 | Mutation vector | Editing efficiency in NAAN cell line, expressed as multiple of the editing efficiency of C12-101-07 |
|---|---|---|---|
| Q172R | V426R | pXC12-101-07-TwoMut-01 | 2.15±1.05 |
| Q172R | S452R | pXC12-101-07-TwoMut-02 | 1.89±0.68 |
| Q172R | T920R | pXC12-101-07-TwoMut-03 | 4.04±1.00 |
| V426R | S452R | pXC12-101-07-TwoMut-04 | 1.56±0.52 |
| V426R | T920R | pXC12-101-07-TwoMut-05 | 2.11±0.79 |
| S452R | T920R | pXC12-101-07-TwoMut-06 | 1.19±0.21 |
| N/A | N/A | pXC12-101-07-GFPPAM | 1.00±0.37 |

### c. Construction of mutation clones targeting Reporter3 cell lines and detection of the editing efficiency

Using a manner basically the same as that of Example 10, the target sequence on the original C12-101-07-GFPPAM vector was replaced. The sgRNA target site for the NAAN cell line was replaced with a site targeting the Reporter3 cell line, resulting in the control plasmid 101-07-sgRNA02. Then, the mutant vector was constructed, and the editing efficiency was tested in the Reporter3 cell line.

The results are shown in Table 18. The editing efficiency of different C12-101-07 mutations in the Reporter3 cell line is expressed as a multiple of the editing efficiency of C12-101-07. The absolute value (average) of the editing efficiency of the 101-07-sgRNA02 control group, i.e., the C12-101-07 protein, is 10.32%.

**Table 18. Editing efficiency of different mutants of C12-101-07 in Reporter3 cell lines**

| (Expressed as multiple of editing efficiency of C12-101-07) | | | |
|---|---|---|---|
| Mutation position 1 | Mutation position 2 | Vector | Editing efficiency, expressed as multiple of the editing efficiency of C12-101-07 |
| V15R | N/A | pXC12-101-07-21 | 1.67±0.04 |
| A173W | N/A | pXC12-101-07-22 | 0.1±0.01 |
| G239R | N/A | pXC12-101-07-23 | 0.04±0.00 |
| D264R | N/A | pXC12-101-07-24 | 1.28±0.16 |
| E271R | N/A | pXC12-101-07-25 | 0.96±0.1 |
| Y295R | N/A | pXC12-101-07-26 | 0.53±0.03 |
| T329R | N/A | pXC12-101-07-27 | 1.3±0.17 |
| E331R | N/A | pXC12-101-07-28 | 1.06±0.01 |
| I335R | N/A | pXC12-101-07-29 | 1.52±0.16 |
| S430R | N/A | pXC12-101-07-30 | 0.79±0.15 |
| S465R | N/A | pXC12-101-07-31 | 1.18±0.37 |
| E493R | N/A | pXC12-101-07-32 | 1.61±0.02 |
| P497R | N/A | pXC12-101-07-33 | 0.15±0.04 |
| K587R | N/A | pXC12-101-07-34 | 0.3±0.01 |
| E768R | N/A | pXC12-101-07-35 | 1.4±0.07 |
| T825R | N/A | pXC12-101-07-36 | 0.95±0.04 |
| A911R | N/A | pXC12-101-07-37 | 1.33±0.21 |
| G914R | N/A | pXC12-101-07-38 | 0.26±0.05 |
| K915R | N/A | pXC12-101-07-39 | 1.03±0.26 |
| I916R | N/A | pXC12-101-07-40 | 0.51±0.12 |
| E940R | N/A | pXC12-101-07-42 | 1.13±0.11 |
| N347E | N/A | pXC12-101-07-43 | 0.99±0.13 |
| K339R | N/A | pXC12-101-07-44 | 1.13±0.01 |
| N347E | K339R | pXC12-101-07-45 | 1.16±0.08 |
| Q172R | V426R | pXC12-101-07-TwoMut-01 | 2.02±0.06 |
| Q172R | S452R | pXC12-101-07-TwoMut-02 | 1.72±0.09 |
| Q172R | T920R | pXC12-101-07-TwoMut-03 | 2.15±0.12 |
| V426R | S452R | pXC12-101-07-TwoMut-04 | 1.56±0.12 |
| V426R | T920R | pXC12-101-07-TwoMut-05 | Not detected |
| S452R | T920R | pXC12-101-07-TwoMut-06 | Not detected |
| N/A | N/A | 101-07-sgRNA02 control | 1.00±0.11 |

### Example 12. Construction of different mutants by site-directed mutagenesis of C12-279 protein

The amino acid sequence of C12-279 protein was SEQ ID NO: 696. Two mutation primers F/R were designed at the mutation position, and the required mutation sequence was introduced through the primers. Combined with the universal primers at both ends of the vector, PCR amplification was performed to obtain two mutation fragments F1 and F2. The mutation fragments F1 and F2 were homologously recombined with the linearized vector to obtain a mutation plasmid. In this example, single-point mutation (D426R, L860R) mutants and multi-point mutation (D426R&L860R) mutants were constructed using the two amino acid positions 426 and 860. The specific steps were as follows.

The primers for site-directed mutagenesis at positions 426 and 860 are shown in Table 19.

**Table 19. Primers**

| Primer name | Primer sequences |
|---|---|
| ChkCas12-PF1 | CCAAGCTGGCTAGCGTTTAAACTTAAG (SEQ ID NO: 731) |
| ChkCas12-PR2 | GCGACGCAATGATGTTTTTCGGTACC (SEQ ID NO: 736) |
| 279_426_F | gagttcaagAGAggcttcgacagagagc (underlined being introduced amino acid mutation) (SEQ ID NO: 756) |
| 279_426_R | tcgaagccTCTcttgaactcggcgcagtag (underlined being introduced amino acid mutation) (SEQ ID NO: 757) |
| 279_860_F | aaccAGGagacagaacaagggcgagg (underlined being introduced amino acid mutation) (SEQ ID NO: 758) |
| 279_860_R | ccttgttctgtctCCTggtttccagcttgttcag (underlined being introduced amino acid mutation) (SEQ ID NO: 759) |

### Construction of single point mutation clones at positions 426 and 860

Using C12-279-pCDH plasmid as a template, ChkCas12-PF1+279_426_R was subjected to PCR amplification (Yijin Bio, PC019, UltraHiPF^{™} DNA Polymerase Kit) to obtain mutation fragment D426R-F1, and ChkCas12-PR2+279_426_F was subjected to PCR amplification to obtain mutation fragment D426R-F2. Plasmid C12-279-pCDH was digested with HindIII+KpnI, and 5647bp vector fragment was gel recovered (Guangzhou Meiji Biotechnology Co., Ltd., D2110, HiPure Gel Pure Micro Kit). The 5647bp vector fragment was recombined in vitro with fragments D426R-F1 and D426R-F2 (NEB, E2611L, Gibson Assembly^{®} Master Mix), and the plasmid C12-279-pCDH-426 with position 426 mutation was obtained by heat shock transformation of *Escherichia coli.*

The same steps and methods were used, except that primer 279_426_F was replaced with 279_860_F and primer 279_426_R was replaced with 279_860_R, and the same amplification and recombination were performed. The plasmid C12-279-pCDH -860 of the position 860 mutation was obtained by heat shock transformation of *Escherichia coli.* The construction method of the Cas protein mutation plasmids of other different positions was consistent with the method for the positions 426 and 860, except that the mutation primers for each different position needed to be designed.

### Verification of editing activity of Cas protein mutants

The test was conducted based on the Reporter3 cell line constructed in the above examples. The sequence between the start codon and the GFP reading frame was 32 bases, which was not an integer multiple of 3, resulting in abnormal reading frame of GFP and no expression of GFP. The Reporter3 cell line was edited using a target site whose PAM recognized by the Cas protein mutant was TTG and target sequence was CTCACCTCGCGACGCAATGATG (SEQ ID NO: 735), indels were generated, and a normal reading frame of GFP was restored. A flow cytometry was used to detect the proportion of cells that restored GFP expression to characterize the editing efficiency of different mutants. The specific operations were as follows.

Cell culture and plating: when the cell line was cultured to 70-80% confluence, the plating was performed, and the count of cells seeded in a 24-well plate was 5*10^5 cells/well.

Transfection: the transfection was performed 12-14 h after plating. 1.5 uL PEI (Yeasen Biotechnology) and 500 ng mutation plasmid were added to 100 µL Opti-MEM per well of the 24-well plate, mixed, and added to the Reporter3 cell line for cell transfection after placing at room temperature for 20 min. Fresh culture medium was replaced after overnight transfection and continued to be cultured for 72 h, the flow cytometry was used for detection. The editing efficiency of different mutation clones were characterized according to a GFP-positive cell proportion, and the average of a plurality of batches of data was taken. The specific results are shown in Table 20 and FIG. 18. An average absolute value of the editing efficiency of a wild-type C12-279 group is 9.0%.

**Table 20. Editing efficiency of each mutant of C12-279 in Reporter3 cell line (Expressed as multiple of editing efficiency of C12-279)**

| Group | Editing efficiency (expressed as multiple of the editing efficiency of C12-279) | Group | Editing efficiency (expressed as multiple of the editing efficiency of C12-279) | Group | Editing efficiency (expressed as multiple of the editing efficiency of C12-279) |
|---|---|---|---|---|---|
| Wild-type C12-279 | 1.00 | 376R | 1.60 | 749R | 1.01 |
| Mut-01 (Q186R) | 1.58 | 377R | 1.20 | 751R | 1.39 |
| Mut-02 (Q186 & D352R) | 1.62 | 378R | 1.41 | 752R | 0.95 |
| Mut-03 (G184R & Q186R & D352R) | 1.87 | 379R | 1.57 | 753R | 1.01 |
| Cell Line NC negative control | 0.05 | 380R | 1.07 | 754R | 1.24 |
| PEI NC negative control | 0.08 | 381R | 1.05 | 755R | 1.42 |
| 1R | 2.07 | 382R | 1.01 | 756R | 1.18 |
| 2R | 1.79 | 383R | 1.51 | 758R | 1.41 |
| 3R | 2.05 | 384R | 0.04 | 759R | 1.13 |
| 4R | 1.53 | 385R | 1.20 | 760R | 1.39 |
| 5R | 2.00 | 386R | 1.09 | 761R | 0.19 |
| 6R | 0.98 | 387R | 1.14 | 762R | 0.25 |
| 7R | 1.91 | 388R | 0.99 | 764R | 0.64 |
| 8R | 0.81 | 389R | 0.06 | 765R | 0.91 |
| 9R | 0.82 | 390R | 0.26 | 766R | 1.18 |
| 10R | 1.60 | 391R | 1.05 | 767R | 1.34 |
| 12R | 0.10 | 392R | 1.15 | 768R | 1.36 |
| 13R | 0.91 | 393R | 0.04 | 769R | 0.24 |
| 14R | 1.13 | 394R | 0.87 | 771R | 1.63 |
| 15R | 1.32 | 395R | 0.10 | 772R | 0.11 |
| 16R | 1.37 | 396R | 1.28 | 773R | 1.56 |
| 19R | 1.26 | 397R | 1.40 | 774R | 1.19 |
| 21R | 1.29 | 398R | 0.99 | 775R | 1.09 |
| 22R | 1.19 | 399R | 1.01 | 776R | 1.19 |
| 23R | 1.07 | 400R | 1.62 | 779R | 1.55 |
| 24R | 1.56 | 401R | 1.03 | 780R | 1.18 |
| 25R | 1.05 | 402R | 1.21 | 781R | 1.66 |
| 26R | 1.18 | 403R | 1.20 | 782R | 1.24 |
| 27R | 1.33 | 404R | 1.02 | 783R | 0.06 |
| 28R | 0.51 | 405R | 1.33 | 784R | 1.51 |
| 29R | 0.08 | 406R | 1.13 | 785R | 1.77 |
| 30R | 1.59 | 407R | 1.07 | 786R | 1.54 |
| 32R | 1.30 | 408R | 1.38 | 787R | 0.06 |
| 33R | 0.80 | 409R | 1.09 | 789R | 1.41 |
| 34R | 1.08 | 410R | 0.81 | 790R | 1.06 |
| 35R | 0.03 | 411R | 1.20 | 791R | 0.11 |
| 36R | 0.07 | 412R | 1.21 | 792R | 1.84 |
| 37R | 0.94 | 413R | 1.01 | 794R | 1.49 |
| 38R | 0.17 | 414R | 0.91 | 795R | 0.30 |
| 39R | 1.23 | 415R | 0.03 | 797R | 1.08 |
| 40R | 0.08 | 416R | 1.48 | 798R | 1.40 |
| 41R | 0.52 | 417R | 0.92 | 800R | 0.09 |
| 42R | 0.45 | 418R | 1.30 | 801R | 0.23 |
| 43R | 1.14 | 419R | 0.87 | 802R | 1.33 |
| 44R | 0.93 | 420R | 1.10 | 804R | 0.61 |
| 46R | 1.10 | 421R | 1.17 | 805R | 0.22 |
| 47R | 1.34 | 422R | 1.19 | 806R | 0.13 |
| 48R | 1.43 | 423R | 0.79 | 807R | 0.11 |
| 49R | 1.17 | 424R | 1.12 | 808R | 0.90 |
| 50R | 0.20 | 425R | 1.24 | 809R | 0.62 |
| 51R | 1.46 | 426R | 2.22 | 810R | 1.11 |
| 53R | 0.05 | 427R | 0.68 | 811R | 0.22 |
| 54R | 0.42 | 428R | 0.44 | 812R | 0.94 |
| 55R | 1.40 | 429R | 0.74 | 813R | 0.99 |
| 56R | 0.28 | 431R | 1.15 | 814R | 0.42 |
| 57R | 0.07 | 432R | 0.05 | 815R | 0.47 |
| 58R | 1.42 | 433R | 0.91 | 817R | 1.16 |
| 59R | 1.51 | 435R | 1.29 | 818R | 0.23 |
| 60R | 0.05 | 436R | 0.04 | 819R | 1.14 |
| 61R | 0.84 | 437R | 0.29 | 821R | 0.91 |
| 62R | 0.71 | 439R | 0.50 | 822R | 1.48 |
| 64R | 0.06 | 440R | 0.04 | 823R | 1.76 |
| 65R | 0.19 | 441R | 1.18 | 824R | 0.32 |
| 66R | 1.42 | 442R | 1.06 | 825R | 1.46 |
| 67R | 0.47 | 443R | 1.44 | 826R | 1.60 |
| 68R | 0.12 | 444R | 1.10 | 827R | 0.34 |
| 69R | 0.65 | 446R | 1.24 | 828R | 1.19 |
| 70R | 0.98 | 447R | 1.23 | 829R | 1.73 |
| 71R | 0.06 | 448R | 1.12 | 830R | 1.79 |
| 72R | 0.08 | 449R | 1.46 | 831R | 0.76 |
| 73R | 0.03 | 450R | 1.27 | 832R | 1.25 |
| 74R | 0.44 | 451R | 0.97 | 833R | 1.51 |
| 75R | 0.05 | 452R | 1.04 | 834R | 1.40 |
| 76R | 1.12 | 453R | 0.04 | 835R | 1.32 |
| 77R | 0.72 | 454R | 1.29 | 836R | 1.53 |
| 78R | 1.28 | 455R | 1.04 | 837R | 1.32 |
| 79R | 0.64 | 456R | 1.45 | 838R | 1.21 |
| 80R | 0.20 | 457R | 0.11 | 839R | 1.30 |
| 81R | 1.19 | 458R | 0.03 | 840R | 1.36 |
| 82R | 0.99 | 459R | 1.53 | 841R | 0.11 |
| 83R | 0.89 | 460R | 0.03 | 842R | 1.58 |
| 84R | 0.97 | 461R | 1.11 | 844R | 1.14 |
| 85R | 1.35 | 462R | 1.58 | 845R | 1.72 |
| 86R | 1.01 | 463R | 1.05 | 846R | 1.91 |
| 88R | 0.13 | 464R | 0.81 | 847R | 1.61 |
| 89R | 1.34 | 467R | 1.22 | 848R | 1.38 |
| 90R | 1.30 | 469R | 1.46 | 849R | 0.07 |
| 91R | 0.84 | 470R | 1.03 | 850R | 1.74 |
| 92R | 0.81 | 471R | 0.10 | 851R | 1.59 |
| 93R | 0.27 | 472R | 0.10 | 852R | 1.22 |
| 94R | 0.07 | 473R | 1.19 | 853R | 1.67 |
| 95R | 0.10 | 474R | 0.03 | 854R | 0.08 |
| 96R | 0.05 | 475R | 1.28 | 855R | 1.48 |
| 97R | 0.09 | 476R | 0.99 | 856R | 1.45 |
| 98R | 0.12 | 477R | 0.20 | 857R | 1.19 |
| 99R | 0.07 | 478R | 1.13 | 858R | 1.87 |
| 101R | 0.39 | 479R | 0.48 | 859R | 1.40 |
| 102R | 0.98 | 480R | 1.03 | 860R | 1.84 |
| 103R | 1.25 | 481R | 0.46 | 862R | 1.14 |
| 104R | 1.00 | 482R | 1.30 | 863R | 1.27 |
| 105R | 1.13 | 483R | 0.41 | 864R | 0.80 |
| 106R | 0.33 | 484R | 1.68 | 865R | 1.07 |
| 108R | 1.41 | 485R | 1.52 | 866R | 1.58 |
| 109R | 0.81 | 486R | 0.47 | 867R | 0.19 |
| 110R | 1.03 | 487R | 0.22 | 868R | 0.54 |
| 111R | 0.61 | 488R | 0.91 | 870R | 0.90 |
| 112R | 0.78 | 489R | 1.16 | 872R | 0.14 |
| 114R | 1.25 | 490R | 0.77 | 873R | 1.11 |
| 115R | 1.06 | 491R | 1.12 | 874R | 0.49 |
| 116R | 0.95 | 492R | 1.04 | 875R | 1.03 |
| 117R | 0.98 | 493R | 0.82 | 876R | 0.05 |
| 118R | 1.41 | 494R | 1.31 | 877R | 0.96 |
| 119R | 0.80 | 496R | 1.18 | 879R | 1.23 |
| 120R | 0.29 | 497R | 0.75 | 880R | 0.85 |
| 121R | 1.35 | 499R | 1.07 | 881R | 1.39 |
| 123R | 1.34 | 500R | 0.94 | 882R | 0.93 |
| 124R | 1.29 | 501R | 1.33 | 883R | 1.13 |
| 125R | 1.33 | 502R | 1.02 | 884R | 1.43 |
| 126R | 0.93 | 503R | 0.61 | 885R | 0.13 |
| 127R | 1.20 | 504R | 0.56 | 886R | 1.25 |
| 128R | 1.31 | 506R | 0.10 | 887R | 1.12 |
| 129R | 1.30 | 507R | 0.93 | 888R | 0.05 |
| 130R | 0.97 | 508R | 0.05 | 890R | 0.05 |
| 131R | 1.00 | 509R | 1.47 | 891R | 0.06 |
| 132R | 0.43 | 510R | 0.07 | 892R | 1.42 |
| 133R | 0.92 | 511R | 0.85 | 893R | 1.64 |
| 134R | 0.98 | 512R | 0.60 | 894R | 0.04 |
| 135R | 0.12 | 513R | 0.22 | 895R | 0.31 |
| 136R | 0.39 | 514R | 1.10 | 896R | 0.74 |
| 137R | 0.09 | 515R | 1.36 | 897R | 0.53 |
| 138R | 1.43 | 516R | 0.85 | 898R | 1.07 |
| 139R | 1.37 | 517R | 0.62 | 899R | 1.33 |
| 140R | 1.26 | 518R | 1.11 | 900R | 1.44 |
| 141R | 1.60 | 519R | 0.09 | 901R | 1.30 |
| 142R | 1.02 | 520R | 0.86 | 902R | 0.30 |
| 143R | 1.20 | 521R | 0.90 | 903R | 1.27 |
| 144R | 1.11 | 522R | 1.17 | 904R | 1.62 |
| 145R | 0.95 | 523R | 0.10 | 905R | 1.36 |
| 146R | 1.16 | 524R | 0.33 | 906R | 0.15 |
| 147R | 0.92 | 525R | 0.89 | 909R | 1.05 |
| 148R | 0.10 | 526R | 0.08 | 910R | 1.18 |
| 149R | 0.10 | 527R | 1.00 | 911R | 0.17 |
| 151R | 1.06 | 528R | 0.04 | 912R | 0.06 |
| 152R | 1.20 | 529R | 0.38 | 913R | 1.32 |
| 153R | 0.08 | 531R | 0.07 | 914R | 0.26 |
| 154R | 0.97 | 532R | 0.42 | 916R | 1.29 |
| 155R | 1.20 | 533R | 1.07 | 917R | 0.31 |
| 156R | 0.16 | 534R | 0.09 | 918R | 0.40 |
| 157R | 0.99 | 535R | 0.31 | 919R | 1.04 |
| 158R | 0.85 | 536R | 0.39 | 920R | 0.47 |
| 159R | 1.09 | 537R | 0.62 | 921R | 0.38 |
| 160R | 0.12 | 538R | 1.02 | 922R | 1.36 |
| 161R | 1.23 | 539R | 0.83 | 923R | 0.43 |
| 162R | 0.57 | 540R | 1.04 | 924R | 1.25 |
| 163R | 0.31 | 541R | 0.91 | 925R | 0.11 |
| 164R | 0.09 | 542R | 1.21 | 926R | 1.49 |
| 165R | 0.92 | 543R | 0.12 | 927R | 0.19 |
| 166R | 0.95 | 544R | 0.05 | 928R | 0.59 |
| 167R | 0.08 | 545R | 0.81 | 930R | 0.83 |
| 169R | 1.15 | 546R | 1.11 | 931R | 1.10 |
| 170R | 0.47 | 547R | 0.72 | 932R | 1.21 |
| 171R | 0.07 | 548R | 1.02 | 933R | 0.87 |
| 172R | 0.10 | 549R | 1.21 | 934R | 0.94 |
| 174R | 0.09 | 550R | 0.08 | 935R | 0.13 |
| 175R | 1.40 | 552R | 0.68 | 936R | 0.45 |
| 176R | 0.16 | 553R | 1.23 | 937R | 0.19 |
| 177R | 0.10 | 555R | 0.35 | 938R | 0.90 |
| 178R | 1.60 | 556R | 1.17 | 939R | 0.49 |
| 179R | 0.19 | 557R | 0.79 | 940R | 0.04 |
| 180R | 0.34 | 558R | 1.37 | 941R | 0.05 |
| 182R | 1.09 | 559R | 1.17 | 942R | 0.10 |
| 183R | 1.08 | 560R | 0.92 | 943R | 0.11 |
| 184R | 1.22 | 561R | 1.41 | 944R | 0.11 |
| 185R | 1.41 | 562R | 1.08 | 945R | 0.08 |
| 186R | 1.23 | 563R | 1.12 | 946R | 0.05 |
| 187R | 0.30 | 564R | 1.08 | 947R | 0.94 |
| 188R | 0.39 | 565R | 1.23 | 948R | 0.10 |
| 189R | 0.11 | 566R | 1.07 | 949R | 1.05 |
| 190R | 0.08 | 567R | 1.25 | 950R | 0.95 |
| 191R | 1.01 | 568R | 1.34 | 951R | 0.88 |
| 192R | 0.04 | 569R | 0.67 | 952R | 0.85 |
| 194R | 0.10 | 570R | 0.07 | 953R | 1.02 |
| 195R | 1.22 | 571R | 0.07 | 954R | 1.11 |
| 196R | 0.40 | 572R | 1.29 | 955R | 0.42 |
| 197R | 1.34 | 573R | 0.10 | 956R | 1.44 |
| 198R | 0.19 | 574R | 1.16 | 957R | 0.85 |
| 199R | 0.07 | 575R | 0.85 | 958R | 1.20 |
| 200R | 1.06 | 576R | 0.67 | 959R | 0.46 |
| 202R | 0.19 | 577R | 1.28 | 960R | 1.20 |
| 203R | 1.31 | 578R | 1.15 | 961R | 0.07 |
| 204R | 0.03 | 579R | 0.96 | 963R | 0.11 |
| 205R | 0.60 | 580R | 1.38 | 964R | 1.10 |
| 206R | 1.07 | 581R | 1.19 | 965R | 1.06 |
| 207R | 0.06 | 582R | 1.16 | 966R | 0.95 |
| 208R | 0.04 | 583R | 0.72 | 967R | 1.17 |
| 209R | 0.89 | 584R | 1.18 | 968R | 0.19 |
| 210R | 0.91 | 585R | 1.31 | 969R | 0.84 |
| 211R | 0.05 | 586R | 1.12 | 970R | 0.57 |
| 212R | 0.49 | 587R | 0.73 | 971R | 0.09 |
| 213R | 1.24 | 589R | 1.01 | 972R | 0.84 |
| 214R | 1.02 | 590R | 1.13 | 973R | 0.46 |
| 215R | 0.06 | 592R | 0.10 | 974R | 0.96 |
| 216R | 1.08 | 593R | 1.20 | 975R | 0.36 |
| 217R | 1.10 | 594R | 0.09 | 976R | 0.53 |
| 218R | 0.94 | 595R | 1.16 | 977R | 0.76 |
| 219R | 0.97 | 596R | 0.07 | 979R | 0.07 |
| 220R | 1.07 | 597R | 1.45 | 980R | 0.55 |
| 221R | 0.95 | 598R | 1.22 | 981R | 1.20 |
| 222R | 0.67 | 599R | 1.23 | 982R | 0.67 |
| 223R | 1.27 | 601R | 0.25 | 983R | 0.15 |
| 224R | 1.06 | 602R | 0.74 | 985R | 1.81 |
| 225R | 0.72 | 603R | 1.01 | 986R | 0.89 |
| 227R | 0.45 | 604R | 0.79 | 987R | 0.08 |
| 228R | 0.06 | 605R | 1.26 | 988R | 1.60 |
| 229R | 0.70 | 606R | 1.13 | 989R | 1.55 |
| 230R | 0.47 | 607R | 1.52 | 990R | 1.06 |
| 231R | 0.39 | 608R | 0.82 | 991R | 0.84 |
| 232R | 0.05 | 609R | 1.45 | 992R | 1.56 |
| 233R | 0.59 | 610R | 1.15 | 993R | 1.43 |
| 234R | 1.38 | 611R | 0.68 | 994R | 0.80 |
| 235R | 0.51 | 612R | 1.20 | 995R | 0.26 |
| 236R | 0.06 | 613R | 0.07 | 996R | 1.51 |
| 237R | 0.99 | 614R | 0.79 | 997R | 1.32 |
| 238R | 0.05 | 615R | 0.04 | 998R | 0.10 |
| 239R | 1.10 | 616R | 0.06 | 999R | 0.12 |
| 240R | 0.36 | 618R | 1.35 | 1000R | 0.08 |
| 242R | 0.59 | 619R | 0.09 | 1001R | 0.97 |
| 243R | 0.45 | 620R | 0.77 | 1002R | 0.07 |
| 244R | 0.05 | 621R | 1.28 | 1003R | 0.17 |
| 245R | 0.41 | 622R | 0.05 | 1004R | 0.13 |
| 247R | 0.70 | 623R | 1.44 | 1005R | 0.45 |
| 248R | 0.04 | 624R | 0.05 | 1006R | 0.09 |
| 249R | 0.12 | 625R | 1.00 | 1007R | 0.91 |
| 250R | 1.29 | 626R | 0.07 | 1008R | 0.13 |
| 251R | 0.86 | 627R | 0.24 | 1009R | 1.39 |
| 252R | 1.18 | 628R | 0.47 | 1010R | 0.67 |
| 253R | 0.06 | 630R | 0.55 | 1011R | 0.99 |
| 255R | 0.30 | 631R | 1.17 | 1012R | 1.06 |
| 256R | 0.05 | 632R | 1.05 | 1013R | 0.10 |
| 257R | 1.41 | 633R | 1.19 | 1014R | 1.04 |
| 258R | 0.06 | 634R | 1.26 | 1015R | 0.09 |
| 259R | 0.36 | 635R | 1.18 | 1016R | 1.42 |
| 260R | 0.99 | 636R | 1.38 | 1017R | 1.06 |
| 261R | 0.07 | 637R | 1.17 | 1018R | 0.77 |
| 262R | 0.09 | 638R | 1.69 | 1021R | 0.89 |
| 263R | 0.69 | 639R | 1.55 | 1023R | 1.00 |
| 265R | 0.87 | 640R | 1.55 | 1024R | 1.12 |
| 266R | 0.96 | 641R | 0.95 | 1025R | 0.86 |
| 267R | 0.80 | 642R | 1.30 | 1026R | 0.56 |
| 268R | 1.18 | 643R | 1.14 | 1027R | 0.98 |
| 269R | 1.32 | 644R | 0.69 | 1028R | 0.78 |
| 270R | 0.11 | 645R | 0.44 | 1029R | 1.00 |
| 271R | 0.93 | 646R | 1.24 | 1030R | 0.90 |
| 272R | 0.76 | 647R | 0.53 | 1031R | 0.11 |
| 273R | 0.77 | 648R | 0.04 | 1032R | 1.17 |
| 274R | 1.14 | 649R | 0.12 | 1033R | 1.40 |
| 275R | 0.10 | 650R | 0.04 | 1035R | 0.09 |
| 276R | 1.20 | 651R | 0.04 | 1036R | 1.33 |
| 278R | 0.60 | 652R | 0.04 | 1037R | 1.17 |
| 279R | 0.12 | 653R | 0.37 | 1038R | 1.31 |
| 281R | 1.03 | 654R | 0.56 | 1039R | 1.17 |
| 282R | 0.09 | 655R | 0.69 | 1040R | 1.10 |
| 283R | 0.13 | 656R | 0.83 | 1041R | 1.18 |
| 284R | 0.93 | 657R | 1.10 | 1042R | 0.06 |
| 285R | 0.93 | 658R | 0.07 | 1043R | 1.09 |
| 286R | 0.05 | 659R | 0.14 | 1044R | 1.29 |
| 287R | 1.08 | 660R | 0.54 | 1045R | 1.42 |
| 288R | 0.87 | 661R | 0.10 | 1046R | 0.11 |
| 289R | 0.68 | 662R | 0.75 | 1047R | 0.83 |
| 290R | 0.76 | 663R | 0.09 | 1048R | 0.05 |
| 291R | 0.83 | 664R | 1.06 | 1049R | 0.05 |
| 292R | 0.09 | 665R | 1.17 | 1050R | 1.50 |
| 293R | 0.95 | 666R | 1.04 | 1052R | 0.07 |
| 294R | 0.11 | 667R | 1.06 | 1053R | 0.05 |
| 295R | 1.18 | 668R | 1.10 | 1055R | 1.19 |
| 296R | 1.12 | 669R | 1.09 | 1056R | 0.12 |
| 297R | 1.07 | 670R | 1.25 | 1057R | 0.08 |
| 298R | 0.04 | 671R | 1.06 | 1058R | 0.06 |
| 299R | 1.26 | 672R | 1.16 | 1059R | 1.05 |
| 300R | 0.04 | 673R | 1.20 | 1060R | 0.84 |
| 301R | 0.43 | 674R | 1.22 | 1061R | 1.29 |
| 302R | 0.05 | 675R | 1.21 | 1062R | 0.11 |
| 303R | 1.21 | 676R | 0.21 | 1063R | 0.07 |
| 305R | 1.07 | 678R | 1.19 | 1064R | 0.69 |
| 306R | 0.21 | 679R | 1.20 | 1065R | 1.05 |
| 308R | 1.22 | 680R | 1.01 | 1066R | 0.43 |
| 309R | 1.26 | 681R | 0.12 | 1067R | 1.33 |
| 310R | 0.80 | 683R | 0.46 | 1068R | 1.11 |
| 313R | 0.94 | 684R | 0.10 | 1069R | 0.84 |
| 315R | 1.15 | 685R | 1.02 | 1070R | 1.07 |
| 316R | 0.49 | 686R | 0.05 | 1071R | 1.10 |
| 317R | 0.38 | 688R | 0.39 | 1072R | 1.10 |
| 318R | 1.06 | 689R | 0.04 | 1073R | 1.44 |
| 319R | 0.54 | 691R | 0.13 | 1074R | 1.50 |
| 320R | 0.05 | 692R | 0.86 | 1075R | 1.16 |
| 321R | 0.23 | 693R | 0.10 | 1076R | 0.62 |
| 322R | 1.29 | 694R | 1.20 | 1077R | 0.34 |
| 323R | 0.55 | 695R | 0.54 | 1078R | 1.05 |
| 324R | 0.07 | 696R | 1.32 | 1079R | 0.07 |
| 325R | 0.44 | 697R | 1.70 | 1080R | 0.23 |
| 327R | 1.19 | 698R | 0.90 | 1081R | 0.09 |
| 328R | 0.08 | 699R | 1.37 | 1082R | 0.08 |
| 329R | 1.04 | 700R | 0.84 | 1083R | 0.06 |
| 330R | 0.29 | 701R | 0.70 | 1084R | 0.06 |
| 331R | 0.21 | 702R | 0.11 | 1085R | 0.10 |
| 332R | 0.04 | 703R | 0.12 | 1086R | 0.06 |
| 333R | 1.67 | 704R | 0.08 | 1087R | 0.06 |
| 334R | 0.68 | 705R | 0.53 | 1088R | 0.05 |
| 335R | 0.77 | 706R | 0.35 | 1089R | 0.06 |
| 336R | 0.04 | 707R | 0.49 | 1090R | 0.03 |
| 337R | 0.88 | 708R | 0.12 | 1091R | 0.10 |
| 339R | 0.06 | 709R | 0.51 | 1092R | 0.20 |
| 340R | 0.91 | 710R | 1.14 | 1093R | 0.27 |
| 341R | 0.99 | 711R | 0.77 | 1094R | 0.23 |
| 342R | 0.60 | 712R | 1.35 | 1095R | 1.41 |
| 343R | 0.12 | 713R | 0.90 | 1096R | 1.16 |
| 344R | 0.78 | 715R | 1.17 | 1097R | 1.15 |
| 345R | 0.70 | 716R | 1.19 | 1098R | 1.15 |
| 346R | 0.92 | 717R | 0.69 | 1100R | 1.59 |
| 347R | 1.37 | 719R | 1.23 | 1101R | 1.38 |
| 348R | 1.28 | 720R | 0.86 | 1102R | 1.29 |
| 350R | 1.19 | 721R | 1.24 | 1103R | 1.32 |
| 351R | 0.97 | 722R | 1.42 | 1104R | 1.35 |
| 352R | 1.15 | 723R | 1.08 | 1105R | 1.34 |
| 353R | 1.15 | 724R | 1.14 | 1107R | 1.39 |
| 354R | 1.15 | 725R | 1.24 | 1108R | 1.22 |
| 355R | 1.13 | 727R | 1.32 | 1109R | 0.20 |
| 356R | 1.56 | 728R | 1.26 | 1110R | 0.44 |
| 357R | 1.03 | 729R | 1.21 | 1111R | 1.20 |
| 358R | 1.00 | 730R | 1.20 | 1112R | 1.20 |
| 359R | 1.03 | 731R | 1.44 | 1113R | 1.02 |
| 360R | 1.12 | 732R | 1.39 | 1115R | 1.21 |
| 361R | 1.25 | 733R | 1.49 | 1116R | 1.28 |
| 362R | 0.96 | 734R | 1.34 | 1117R | 1.06 |
| 363R | 1.13 | 736R | 0.92 | 1120R | 1.26 |
| 364R | 0.81 | 737R | 1.22 | 1122R | 1.21 |
| 365R | 0.84 | 738R | 1.05 | 1123R | 1.22 |
| 366R | 0.03 | 739R | 1.08 | 1124R | 1.40 |
| 367R | 0.18 | 740R | 1.17 | 1125R | 1.30 |
| 368R | 1.00 | 741R | 1.16 | 1128R | 0.78 |
| 369R | 1.29 | 742R | 1.27 | 1129R | 1.40 |
| 370R | 0.42 | 743R | 0.99 | 1130R | 1.05 |
| 371R | 1.18 | 744R | 0.08 | 1131R | 1.34 |
| 372R | 1.06 | 745R | 1.33 | 1132R | 1.92 |
| 373R | 0.04 | 746R | 1.16 | 1133R | 1.32 |
| 374R | 0.02 | 747R | 1.03 | 1134R | 1.29 |
| 375R | 1.40 | 748R | 1.19 | 1135R | 1.37 |

In the table, nR (n is an integer) denotes that the amino acid residue at position n is mutated to R.

Construction of clones with different mutation position combinations of Cas mutant proteins and verification of editing activity

According to the editing efficiency results of different mutation positions mentioned above, different mutation positions were selected for multiple mutation combinations, and the construction of plasmid of the Cas mutant with double-position 426 and 860 was introduced as an example.

Using C12-279-pCDH plasmid as the template, ChkCas12-PF1+279_426_R was subjected to PCR amplification to obtain the mutation fragment D426R-F1, 279_426_F+279_860_R was subjected to PCR amplification to obtain the mutation fragment D426R-L860R-F1, and ChkCas12-PR2+279_860_F was subjected to PCR amplification to obtain the mutation fragment L860R-F2. The plasmid C12-279-pCDH was digested with HindIII+KpnI, and the 5647 bp vector fragment was recovered by gel extraction, and was recombined in vitro with fragments D426R-F1, D426R-L860R-F1, and L860R-F2, and the mutant plasmid C12-279-pCDH-426-860 was obtained by heat shock transformation of *Escherichia coli.* The construction manner of other plasmids with multiple mutations was consistent with that of C12-279-pCDH-426-860.

The amino acid sequence of the Mut-02-1-426-846-858-860 mutation is:

RFVVLSVNQFRCNPNAFRQASMKTDDEKKDTAIKSYTSWLLPNDRKEKDMVRSFLALDQGSRFFFDLMQA
WYGGLTPDIIKSVTKERDLVDLWCAIYWFRPMKKELASHPIDRVDLAKTFQRYYGGPASDVVQEYLSASIGE
DCCWNDCRQKYQEFCKNLGVDFTPDLKTLVREKLIAVALNDGSRAAISNLFGNGGKEDRNVKVNICKKILIA
LEKAGKSVKYVRDVQSIILKCANAKDREDFNRIYADKNGRPGTLLLLLDRKGENTYDAEVLKRYLRKTIKSKN
APLVWNYNQKLFEYIERGIRNLLRRTINYDAWAWGEMWRVGHTPLQTKATRNYHFAQEQLKRHSRIAAAS
QSSHAIAVALNEFFESPFFDGENPFTICPHHLGKNLEKLFKAWADEKNQTDEEDAGETAITDYCAEFKRGFD
REPIRNLLRYVYASLRSKYSAKELIQAAKYNQQFERYQRQKAHPVTPDNQGYTWPEAVIKPSKAERKNREN
SLDTRIWVSVKLLQKDGTWEKHHIPFYNLRFFEEVYAAASIAEDMPSPTFRNSRFGFKLPKLLNTHAKINKK
KKDGKKDKKAVNAAKREARIYLAAKEGRLPAPSVPLDKLSAVIARVNGKFQVTVPVKFKVNKQPKYPLPQLG
QIILGYDQNLTANHAWALFEVVTEDTPGAHFCRNQYLRVLKTGQVRSITKTKDGQEIDQLSYDGLEYKEYG
EWRKQAKKFASQWYITTKVRKGKETTLLSQPALERFESIEKYKPALYRFNKEYARLLLAVIRRKTLNELEEIRP
EIFRLVEQGFGICRFGSLSLSSFEAIRAAKAVIYGYFSTALKHDHNTPITDDERQARDPELFGLLNKLRTRRQ
NKGEEKINRAANALIRIALENKADFIRGEGDLPTTNKSVKKGQNSRSMDWLARGLFNKICQLAVMHNIIPTA

ANPHSTSHQDPFEHNKDFDDPQPAMKCRYAEFKIEDIGDSVLERLGTSLRNAKAGQTGAYYYKGAQDFLA
HYGLQDIEEELKKCRRGRKANMPCWELQKRLIQKLGSKDAVVYIPMRGGRIYLATHSVTTGAKPIIFNGEEV
WVSNADEIAAVNIGLTIIPTSKKDEEHPDRENDDKAIRKSVRRPRKSGERRSGDKMPATARKT (SEQ ID NO:
760).

The editing activity of multi-point mutations was verified using the same manner as described above in this example.

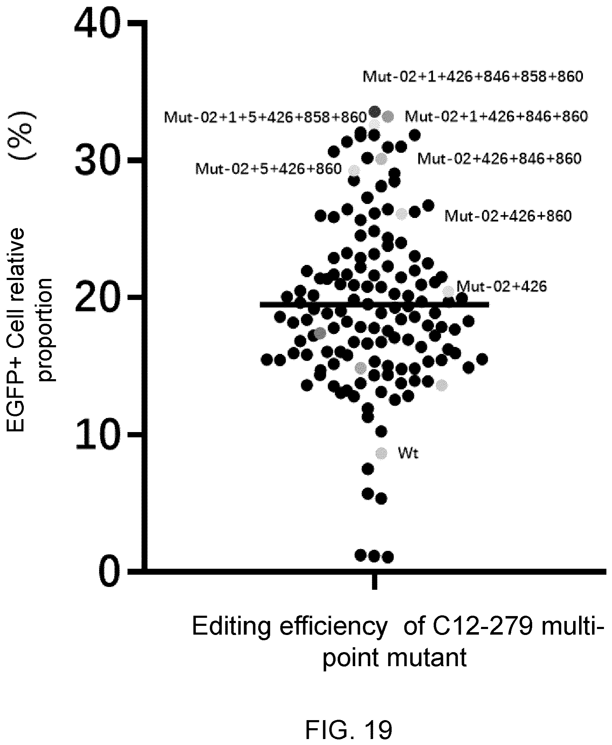

The results indicate that the editing efficiency of most multi-point mutants are improved compared with the wild-type, and specific results are shown in Table 21 and FIG. 19. The absolute value of the editing efficiency of the wild type C12-279 group is 8.7%.

**Table 21. Editing efficiency of each mutant of C12-279 in Reporter3 cell line (expressed as multiple of editing efficiency of C12-279)**

| Group | Editing efficiency (expressed as multiple of the editing efficiency of C12-279) | Group | Editing efficiency (expressed as multiple of the editing efficiency of C12-279) |
|---|---|---|---|
| Wt wild type | 1.00 | 858+988+1132 | 1.97 |
| 3 | 1.71 | 988+1132 | 1.53 |
| 5 | 1.66 | Mut-01 | 1.58 |
| 7 | 1.48 | Mut-01+376 | 1.99 |
| 333 | 1.57 | Mut-01+485 | 1.59 |
| 376 | 1.59 | Mut-02 | 1.72 |
| 426 | 1.85 | Mut-02+1+3+426+858+860 | 3.70 |
| 485 | 1.48 | Mut-02+1+3+426+860 | 3.35 |
| 846 | 1.61 | Mut-02+1+333+426+858+860 | 3.68 |
| 858 | 1.57 | Mut-02+1+333+426+860 | 2.84 |
| 860 | 1.78 | Mut-02+1+426+485+858+860 | 3.67 |
| 988 | 1.66 | Mut-02+1+426+485+860 | 3.25 |
| 1132 | 1.70 | Mut-02+1+426+846+858+860 | 3.88 |
| 2+5+846+85 8 | 2.68 | Mut-02+1+426+846+860 | 3.83 |
| 3+1132 | 0.66 | Mut-02+1+5+426+858+860 | 3.77 |
| 3+333+426 | 2.29 | Mut-02+1+5+426+860 | 3.62 |
| 3+426+858 | 2.51 | Mut-02+1132 | 2.10 |
| 3+426+860 | 2.42 | Mut-02+3 | 0.13 |
| 3+5 | 2.06 | Mut-02+3+376+426 | 2.57 |
| 3+7 | 1.38 | Mut-02+3+426 | 2.66 |
| 3+846 | 1.71 | Mut-02+3+426+858+860 | 3.58 |
| 3+846+1132 | 1.92 | Mut-02+3+426+860 | 3.68 |
| 3+846+858 | 2.50 | Mut-02+333 | 2.03 |
| 3+846+860 | 2.53 | Mut-02+333+336+352+376+4 26 | 0.13 |
| 3+846+988 | 2.32 | Mut-02+333+352+376+426 | 2.24 |
| 3+858 | 2.15 | Mut-02+333+426 | 2.40 |
| 3+858+860 | 2.48 | Mut-02+333+426+858+860 | 3.54 |
| 3+858+988 | 2.18 | Mut-02+333+426+860 | 3.49 |
| 3+860 | 2.41 | Mut-02+376 | 0.14 |
| 333+376 | 1.94 | Mut-02+376+426 | 2.54 |
| 333+376+42 6 | 2.75 | Mut-02+376+426+485+860 | 2.21 |
| 333+376+48 5 | 1.83 | Mut-02+376+426+860+865 | 2.57 |
| 333+426 | 2.47 | Mut-02+426 | 2.36 |
| 333+426+11 32 | 2.27 | Mut-02+426+1132 | 1.87 |
| 333+426+48 5 | 2.33 | Mut-02+426+485 | 2.28 |
| 333+426+84 6 | 2.34 | Mut-02+426+485+858+860 | 3.06 |
| 333+426+85 8 | 2.28 | Mut-02+426+485+860 | 2.04 |
| 333+426+86 0 | 1.77 | Mut-02+426+485+860 | 3.04 |
| 333+485 | 1.79 | Mut-02+426+846 | 2.65 |
| 352+426 | 1.79 | Mut-02+426+846+858+860 | 3.29 |
| 376+426 | 1.94 | Mut-02+426+846+860 | 3.48 |
| 376+426+48 5+660 | 1.86 | Mut-02+426+860 | 3.06 |
| 376+485 | 1.51 | Mut-02+485 | 1.31 |
| 426+649 | 2.05 | Mut-02+5 | 2.69 |
| 426+846+85 8+860 | 3.02 | Mut-02+5+426 | 2.77 |
| 426+858+86 0 | 3.15 | Mut-02+5+426+858+860 | 3.58 |
| 426+858+98 8 | 0.62 | Mut-02+5+426+860 | 3.38 |
| 428+485 | 2.26 | Mut-02+639 | 2.18 |
| 5+1132 | 1.45 | Mut-02+7 | 2.60 |
| 5+333+426 | 2.97 | Mut-02+858 | 2.48 |
| 5+426+858 | 2.87 | Mut-02+860 | 3.00 |
| 5+426+860 | 3.30 | Mut-02+988 | 1.75 |
| 5+846 | 1.84 | Mut-03 | 2.01 |
| 5+846+1132 | 1.90 | Mut-03+1132 | 1.61 |
| 5+846+858 | 2.65 | Mut-03+3 | 1.94 |
| 5+846+860 | 2.32 | Mut-03+3+107+426 | 2.81 |
| 5+858 | 2.15 | Mut-03+3+376 | 2.44 |
| 5+858+988 | 2.18 | Mut-03+3+639 | 2.40 |
| 5+860 | 1.73 | Mut-03+333 | 1.78 |
| 7+426+846 | 3.02 | Mut-03+333+336 | 0.87 |
| 7+426+858 | 3.09 | Mut-03+376 | 2.30 |
| 846+1132 | 1.66 | Mut-03+376+1132 | 2.99 |
| 846+585+86 0 | 2.36 | Mut-03+426 | 2.20 |
| 846+858 | 1.96 | Mut- 03+426+1132 | 2.11 |
| 846+858+11 32 | 1.77 | Mut-03+485 | 1.51 |
| 846+858+98 8 | 2.51 | Mut-03+5 | 2.08 |
| 846+860 | 1.99 | Mut-03+639 | 2.13 |
| 846+860+11 32 | 1.84 | Mut-03+639+1132 | 1.82 |
| 846+860+98 8 | 2.42 | Mut-03+7 | 1.19 |
| 846+988 | 1.72 | Mut-03+846 | 2.13 |
| 858+860 | 2.23 | Mut-03+858 | 2.06 |
| 858+860+11 32 | 2.06 | Mut-03+860 | 2.47 |
| 858+988 | 2.11 | | |

In the table, the integer n indicates that the amino acid residue at position n is mutated to R. Mut-01 is a Q186R mutant, Mut-02 is a Q186R & D352R double-point mutant, and Mut-03 is a G184R+Q186R+D352R triple-point mutant; 5+426+860 indicates that the amino acid residues at positions 5, 426, and 860 are mutated to R, simultaneously; Mut-02+860 indicates a multi-point mutant obtained by introducing an additional mutation at position 860 (mutated to R) based on the Mut-02 mutant; and other multi-point mutants are similar (mutated to R).

### Example 13. Detection of editing efficiency of different mutants for different target sites of TTR and HBG

In this example, for the TTR gene (GeneBank: NG_009490.1) and the HBG gene (GeneBank: NC_000011.10), the PAM sequence was selected as the target site of TTN, and sgRNAs targeting different positions were designed and constructed (as shown in Table 22) for combination with different mutants to detect the editing efficiency.

**Table 22. sgRNA targeting TTR and HBG**

| sgRNA name | PAM | Target site | sgRNA sequence |
|---|---|---|---|
| C12-279-TTR01 | TTC | catgagcatgcagaggtgag tat (SEQ ID NO: 722) | gtaatgcgtctcccattgacgccatgagcatgcagaggt gagtat (SEQ ID NO: 783) |
| C12-279-TTR02 | TTT | tcctataaggtgtgaaagtct gg (SEQ ID NO: 761) | gtaatgcgtctcccattgacgctcctataaggtgtgaaag tctgg (SEQ ID NO: 784) |
| C12-279-TTR03 | TTC | cagtaagatttggtgtctat (SEQ ID NO: 762) | [?]ggg ([?]) gtaatgcgtctcccattgacgccagtaagatttggtgtct at (SEQ ID NO: 785) |
| C12-279-TTR04 | TTG | tatatcccttctacaaattcctc (SEQ ID NO: 763) | [?] gtaatgcgtctcccattgacgctatatcccttctacaaatt cctc (SEO ID NO: 786) |
| C12-279-TTR05 | TTT | GTGTCTGAGGCTGGCC CTACGGTG (SEQ ID NO: 764) | [?] gtaatgcgtctcccattgacgcGTGTCTGAGGCTG GCCCTACGGTG (SEQ ID NO: 787) |
| C12-279-TTR06 | TTC | AGAAAGGCTGCTGATG ACACCTG (SEQ ID NO: 765) | gtaatgcgtctcccattgacgcAGAAAGGCTGCTG ATGACACCTG (SEQ ID NO: 788) |
| C12-279-TTR07 | TTG | GCATCTCCCCATTCCA TGAGCATG (SEQ ID NO: 766) | gtaatgcgtctcccattgacgcGCATCTCCCCATTC CATGAGCATG (SEQ ID NO: 789) |
| C12-279-TTR08 | TTC | ACAGCCAACGACTCCG GCCCCG (SEQ ID NO: 767) | gtaatgcgtctcccattgacgccatgagcatgcagaggt gagtat (SEQ ID NO: 783) gtaatgcgtctcccattgacgctcctataaggtgtgaaag tctgg (SEQ ID NO: 784) gtaatgcgtctcccattgacgccagtaagatttggtgtct at (SEQ ID NO: 785) gtaatgcgtctcccattgacgctatatcccttctacaaatt cctc (SEQ ID NO: 786) gtaatgcgtctcccattgacgcGTGTCTGAGGCTG GCCCTACGGTG (SEQ ID NO: 787) gtaatgcgtctcccattgacgcAGAAAGGCTGCTG ATGACACCTG (SEQ ID NO: 788) gtaatgcgtctcccattgacgcGCATCTCCCCATTC CATGAGCATG (SEQ ID NO: 789) gtaatgcgtctcccattgacgcACAGCCAACGACT CCGGCCCCCG (SEQ ID NO: 790) gtaatgcgtctcccattgacgcTAGAAGGGATATA CAAAGTGGAA (SEQ ID NO: 791) gtaatgcgtctcccattgacgcTAGATGCTGTCCG AGGCAGTCCTG (SEQ ID NO: 792) gtaatgcgtctcccattgacgcGGTGTCTATTTCCA CTTTGTATA (SEQ ID NO: 793) gtaatgcgtctcccattgacgcCTGAACACATGCAC GGCCACATTG (SEQ ID NO: 794) gtaatgcgtctcccattgacgcCCCAGAGGCAAAT GGCTCCCAGG (SEQ ID NO: 795) gtaatgcgtctcccattgacgcCTCCTCAGTTGTG AGCCCATGCA (SEQ ID NO: 796) |
| C12-279-TTR09 | TTG | TAGAAGGATATACAA AGTGGAA (SEQ ID NO: 768) | gtaatgcgtctcccattgacgcTAGAAGGGATATA CAAAGTGGAA (SEQ ID NO: 791) |
| C12-279-TTR10 | TTC | TAGATGCTGTCCGAG GCAGTCCTG (SEQ ID NO: 769) | gtaatgcgtctcccattgacgcTAGATGCTGTCCG AGGCAGTCCTG (SEQ ID NO: 792) |
| C12-279-TTR11 | TTT | GGTGTCTATTTCCACT TTGTATA (SEQ ID NO: 770) | gtaatgcgtctcccattgacgcGGTGTCTATTTCCA CTTTGTATA (SEQ ID NO: 793) |
| C12-279-TTR12 | TTT | CTGAACACATGCACGG CCACATTG (SEQ ID NO: 771) | gtaatgcgtctcccattgacgcCTGAACACATGCAC GGCCACATTG (SEQ ID NO: 794) |
| C12-279-TTR13 | TTA | CCCAGAGGCAAATGGC TCCCAGG (SEQ ID NO: 772) | gtaatgcgtctcccattgacgcCCCAGAGGCAAAT GGCTCCCAGG (SEQ ID NO: 795) |
| C12-279-TTR14 | TTC | CTCCTCAGTTGTGAGC CCATGCA (SEQ ID NO: 773) | gtaatgcgtctcccattgacgcCTCCTCAGTTGTG AGCCCATGCA (SEQ ID NO: 796) |
| C12-279-HBG01 | TTG | GCCAGCCTTGCCTTGA CCAATAG (SEQ ID NO: 774) | gtaatgcgtctcccattgacgcGCCAGCCTTGCCT TGACCAATAG (SEQ ID NO: 797) |
| C12-279-HBG02 | TTG | ACCAATAGTCTTAGAG TATCCAG (SEQ ID NO: 775) | gtaatgcgtctcccattgacgcACCAATAGTCTTAG AGTATCCAG (SEQ ID NO: 798) |
| C12-279-HBG03 | TTG | CCTTGTCAAGGCTATT GGTCAAG (SEQ ID NO: 776) | gtaatgcgtctcccattgacgcCCTTGTCAAGGCT ATTGGTCAAG (SEQ ID NO: 799) |
| C12-279-HBG04 | TTG | CCTTGACCAATAGCCT TGACAAG (SEQ ID NO: 777) | gtaatgcgtctcccattgacgcCCTTGACCAATAGC CTTGACAAG (SEQ ID NO: 800) |
| C12-279-HBG05 | TTG | ACCAATAGCCTTGACA AGGCAAA (SEQ ID NO: 778) | gtaatgcgtctcccattgacgcACCAATAGCCTTGA CAAGGCAAA (SEQ ID NO: 801) |
| C12-279-HBG06 | TTG | GTCAAGGCAAGGCTGG CCAACCC (SEQ ID NO: 779) | gtaatgcgtctcccattgacgcGTCAAGGCAAGGC TGGCCAACCC (SEQ ID NO: 802) |
| C12-279-HBG07 | TTG | GTCAAGTTTGCCTGTC AAGGC (SEQ ID NO: 780) | gtaatgcgtctcccattgacgcGTCAAGTTTGCCT TGTCAAGGC (SEQ ID NO: 803) |
| C12-279-HBG08 | TTG | TCAAGGCTATTGGTCA AGGCAAG (SEQ ID NO: 781) | gtaatgcgtctcccattgacgcTCAAGGCTATTGG TCAAGGCAAG (SEQ ID NO: 804) |
| C12-279-HBG09 | TTG | ACAAGGCAAACTTGAC CAATAGTC (SEQ ID NO: 782) | gtaatgcgtctcccattgacgcACAAGGCAAACTT GACCAATAGTC (SEQ ID NO: 805) |

The sgRNA plasmids were constructed according to the sgRNA sequences in the above table: the vector plasmid SpCas9-gRNA-pUC57Kan was linearized using BbsI (Thermofisher) and XhoI (Thermofisher), the primers were synthesized for different sgRNA sequences, annealed and connected to the linearized vector, and *Escherichia coli* was transformed to obtain the final sgRNA expression vector plasmid.

Different mutant plasmids were combined with different sgRNA plasmids, and HEK293T cells were transfected with PEI. After 48 h, the cells were collected and lysed using DirectPCR Lysis Reagent (Cell) (VIAGEN: 302-C). Different primers were selected according to different target sites for PCR amplification and Sanger sequencing. The editing efficiency was analyzed using TIDE.

Specific amplification and sequencing primers were shown in Table 23. The experiment was repeated for three times, and the editing efficiency results are shown in FIGs. 20A and 20B. Each sgRNA group in each batch of experiments generates effective editing, and the editing efficiency at many target sites is better than that of wild-type C12-297.

**Table 23. Primers for amplification and sequencing of different target sites**

| Amplification primer name | Primer sequence | Corresponding sgRNA | Sequencing primer |
|---|---|---|---|
| ChkTTR-PF1 | GAACGAATGTTCCGATGCTCTAAT C (SEQ ID NO: 806) | C12-279-TTR05 | ChkTTR-PF1 |
| ChkTTR-PR1 | AATGAGGTAGAATGCTCAGAGTTCAA GT (SEQ ID NO: 807) | | |
| ChkTTR-PF2 | CAACTGTTCTCAGGGGGACCTATTTT C (SEQ ID NO: 808) | C12-279-TTR06, C12-279-TTR10, C12-279-TTR12, C12-279-TTR13 | ChkTTR-PF2 |
| ChkTTR-PR2 | CTCTACTGTCTGCCCCTAAATGATGC (SEQ ID NO: 809) | | |
| ChkTTR-PF3 | TGCCATTTAATTTCAGTTAGGAGTTT TC (SEQ ID NO: 810) | C12-279-TTR01, C12-279-TTR02, C12-279-TTR03, C12-279-TTR04, C12-279-TTR07, C12-279-TTR09, C12-279-TTR11 C12-279-TTR14 | ChkTTR-PF3 |
| ChkTTR-PR3 | CAGACCTCCGAGAAGCCAGATGT (SEQ ID NO: 811) | | |
| ChkTTR-PF4 | CAAGACCTGACTCTGTACTCCTGCTC T (SEQ ID NO: 812) | C12-279-TTR08 | ChkTTR-PR4 |
| ChkTTR-PR4 | ATTTGGATCTCTCCTAGCGTTCTGC (SEQ ID NO: 813) | | |
| ChkHBG01-PF1 | ACGGCTGACAAAAGAAGTCCTGG (SEQ ID NO: 814) | C12-279-HBG01 to C12-279-HBG09 | ChkHBG01-PR1 |
| ChkHBG01-PR1 | CATGGGTAGACAACCAGGAGCC (SEQ ID NO: 815) | | |

### Example 14. Preparation and activity detection of inactivated C12-279 mutant

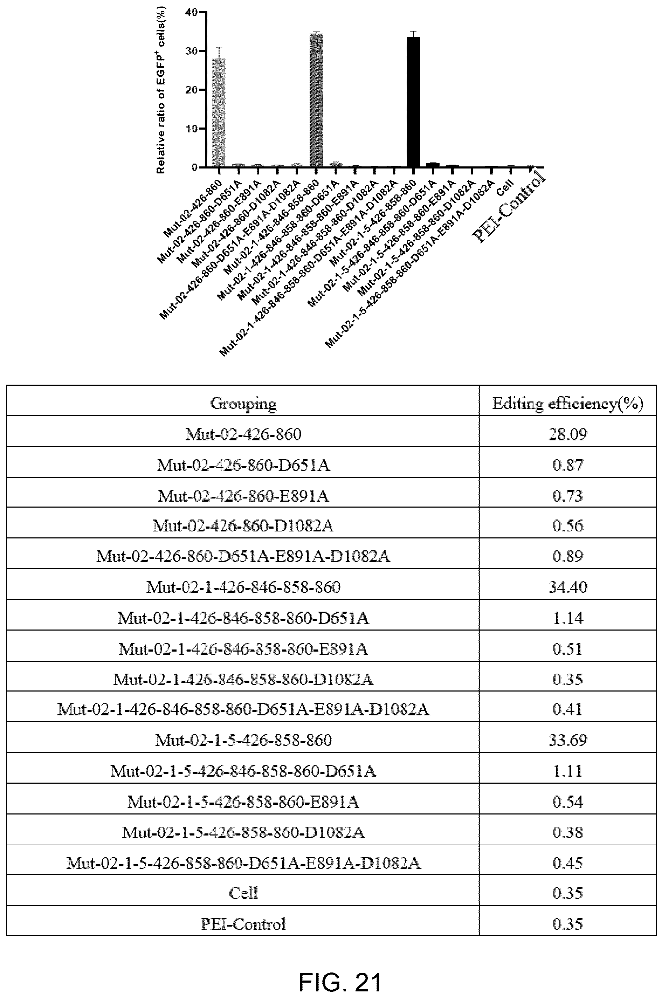

On the basis of the multi-point mutants designed in the previous examples, any one or more of the D651A, E891A, and D1082A mutations were further introduced, and the mutation clone plasmid was constructed using the same manner as in the previous example 10. Editing was performed in the Reporter3 cell line, and an analysis was performed on the proportion of cells that restored GFP expression after editing by flow cytometry to determine the editing efficiency of the mutant. Specific results are shown in FIG. 21. As long as each mutation in the figure contains at least one of D651A, E891A, and D1082A mutations, the editing efficiency is reduced to less than 1.3%. DeadCas12 (dCas12) may be obtained by introducing any of D651A, E891A, and D1082A.

The amino acid sequence of the dCas mutant Mut-02-1-426-846-858-860-D651A-E891A-D1082A is:

RFVVLSVNQFRCNPNAFRQASMKTDDEKKDTAIKSYTSWLLPNDRKEKDMVRSFLALDQGSRFFFDLMQA
WYGGLTPDIIKSVTKERDLVDLWCAIYWFRPMKKELASHPIDRVDLAKTFQRYYGGPASDVVQEYLSASIGE

DCCWNDCRQKYQEFCKNLGVDFTPDLKTLVREKLIAVALNDGSRAAISNLFGNGGKEDRNVKVNICKKILIA
LEKAGKSVKYVRDVQSIILKCANAKDREDFNRIYADKNGRPGTLLLLLDRKGENTYDAEVLKRYLRKTIKSKN
APLVWNYNQKLFEYIERGIRNLLRRTINYDAWAWGEMWRVGHTPLQTKATRNYHFAQEQLKRHSRIAAAS
QSSHAIAVALNEFFESPFFDGENPFTICPHHLGKNLEKLFKAWADEKNQTDEEDAGETAITDYCAEFKRGFD
REPIRNLLRYVYASLRSKYSAKELIQAAKYNQQFERYQRQKAHPVTPDNQGYTWPEAVIKPSKAERKNREN
SLDTRIWVSVKLLQKDGTWEKHHIPFYNLRFFEEVYAAASIAEDMPSPTFRNSRFGFKLPKLLNTHAKINKK
KKDGKKDKKAVNAAKREARIYLAAKEGRLPAPSVPLDKLSAVIARVNGKFQVTVPVKFKVNKQPKYPLPQLG
QIILGYAQNLTANHAWALFEVVTEDTPGAHFCRNQYLRVLKTGQVRSITKTKDGQEIDQLSYDGLEYKEYG
EWRKQAKKFASQWYITTKVRKGKETTLLSQPALERFESIEKYKPALYRFNKEYARLLLAVIRRKTLNELEEIRP
EIFRLVEQGFGICRFGSLSLSSFEAIRAAKAVIYGYFSTALKHDHNTPITDDERQARDPELFGLLNKLRTRRQ
NKGEEKINRAANALIRIALENKADFIRGAGDLPTTNKSVKKGQNSRSMDWLARGLFNKICQLAVMHNIIPTA
ANPHSTSHQDPFEHNKDFDDPQPAMKCRYAEFKIEDIGDSVLERLGTSLRNAKAGQTGAYYYKGAQDFLA
HYGLQDIEEELKKCRRGRKANMPCWELQKRLIQKLGSKDAVVYIPMRGGRIYLATHSVTTGAKPIIFNGEEV
WVSNAAEIAAVNIGLTIIPTSKKDEEHPDRENDDKAIRKSVRRPRKSGERRSGDKMPATARKT (SEQ ID NO:
816).

### Example 15. mRNA+gRNA delivery

The TTR gene was edited using the encoding mRNA of the Mut-02-3-426-860 mutant (obtained by in vitro transcription) together with the modified gRNA (as shown in Table 24).

**Table 24. Modified gRNA**

| gRNA name | gRNA sequence (including modifications) | Modification | Editing efficiency determined by Sanger sequencing |
|---|---|---|---|
| C279-dmTTR01-01 | dAdTdGdTdGdTdTdTdTdTdGd TdCdAdAdAdAdGdAdCdCdTdTd TdTrGrUrArArUrGrCrGrUrCrUrC rCrArUrUrGrArCrGrCrCrArUrGrA rGrCrArUrGrCrArGrArGrGrUrGr ArGrUrA*mU (SEQ ID NO: 817) | adding a 25-nt DNA sequence to the 5' end and modifying the last nucleotide at the 3' end with phosphorothioate and 2'-oxymethylation. | 52.0% |
| C279-mTTR01-01 | mG*mU*mA*rArUrGrCrGrUrCr UrCrCrArUrUrGrArCrGrCrArUrGr ArGrCrArUrGrCrArGrArGrGrUrG rArG*mU*mA*mU (SEQ ID NO: 818) | modifying three nucleotides at the 5' and 3' ends with phosphorothioate and 2'-oxymethyl | 29.5% |
| C279-dmTTR01-02 | dGdTdTdGdCdAdAdTdCdCdCd AdAdGrGrUrArArUrGrCrGrUrCr UrCrCrArUrGrArCrGrCrCrArUrGr ArGrCrArUrGrCrArGrArGrGrUrG rArGrUrA*mU (SEQ ID NO: 819) | adding a 14-nt DNA sequence to the 5' end and modifying the last nucleotide at the 3' end with phosphorothioate and 2'-oxymethyl. | 47.5% |
| C279-mTTR01-02 | mG*rUrArArUrGrCrGrUrCrUrCr CrArUrUrGrArCrGrCrArUrGrArGr CrArUrGrCrArGrArGrGrUrGrArG rUrA*mU (SEQ ID NO: 820) | modifying a base at the 5' and 3' ends with phosphorothioate and 2'-oxymethyl | 26.8% |

In the table, "r" indicates a natural base, "d" indicates a deoxy modification, "m" indicates a methylation modification, and "*" indicates a phosphorothioate modification.

HEK293 cells were transformed with mRNA and gRNA by electroporation. After 48 h, cells were collected and lysed using DirectPCR Lysis Reagent (Cell) (VIAGEN: 302-C). After that, a Sanger sequencing was performed on the lysed cells, and the editing efficiency was analyzed using TIDE. The results are shown in Table 24.

After adding DNA nucleotide sequence at the 3' end, the editing activity is significantly improved. For example, the added sequence is as follows. dAdTdGdTdGdTdTdTdTdTdGdTdCdAdAdAdAdGdAdCdCdTdTdTdT (SEQ ID NO: 821) or dGdTdTdGdCdAdAdTdCdCdCdAdAdG (SEQ ID NO: 822).

In addition, primers TTR-NGS-PF2 (GCGTAACTTAATCCAGACTTTCACACCTT, SEQ ID NO: 823) and TTR-NGS-PR2 (GGTCATTCATCACCTTCCTTAGGACA, SEQ ID NO: 824) were used for PCR amplification and library construction, and an NGS sequencing was performed to detect the editing efficiency. The electroporation editing efficiency of a combination of the mutants Mut-02-3-426-860 and C279-dmTTR01-01 reaches 79.64%.

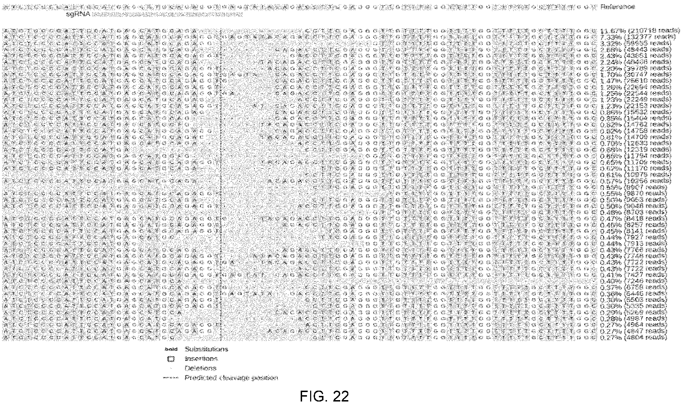

Subsequently, C279-dmTTR01-02 was tested in combination with different mutants. The specific NGS sequencing results are shown in Table 25 and FIG. 22.

**Table 25. Editing efficiency of modified gRNA (C279-dmTTR01-02) combined with different mutants (NGS assay)**

| Mutant | sgRNA | Editing efficiency determined by NGS |
|---|---|---|
| Mut-02-426-860 | C279-dmTTR01-02 | 24.63% |
| Mut-02-5-426-860 | C279-dmTTR01-02 | 63.48% |
| Mut-02-1-5-426-858-860 | C279-dmTTR01-02 | 92.18% |
| Mut-02-1-426-846-858-860 | C279-dmTTR01-02 | 88.05% |
| Mut-02-1-3-426-858-860 | C279-dmTTR01-02 | 89.67% |
| Mut-02-3-426-860 | C279-dmTTR01-02 | 79.99% |

### Example 16. Verification of editing efficiency of different endogenous target genes

Different gRNA target sites were designed according to different disease-related target sites (Table 26), and the mutant Mut-02-1-426-846-858-860 protein was edited in combination with the gRNA in Table 26. The same manner as in example 13 was used, including the plasmid construction, the transfection, and the editing efficiency detection. The results are shown in Table 26.

**Table 26. Editing efficiency of targeting different endogenous genes**

| Name | Target gene | Sequence of PAM position | gRNA guide sequence | Editing efficiency |
|---|---|---|---|---|
| sgRNA-BCL11A-01-CTX-001-PAM-TTC | BCL11A | TTC | ctggagcctgtgataaaagcaac (SEQ ID NO: 825) | 27.40% |
| sgRNA-BCL11A-02-CTX-001-PAM-TGC | BCL11A | TGC | aagctaacagttgcttttatcac (SEQ ID NO: 826) | 1% |
| sgRNA-HBG-01-ZBTB7A-binding-site-PAM-TTG | HBG | TTG | cattgagatagtgtggggaaggg (SEQ ID NO: 827) | 10.40% |
| sgRNA-HBG-02-ZBTB7A-binding-site-PAM-TTT | HBG | TTT | gcattgagatagtgtggggaagg (SEQ ID NO: 828) | 10.90% |
| sgRNA-HBG-03-ZBTB7A-binding-site-PAM-TTG | HBG | TTG | agatagtgtggggaaggggcccc (SEQ ID NO: 829) | 23.20% |
| sgRNA-HBG-04-ZBTB7A-binding-site-PAM-TTG | HBG | TTG | ggggccccttccccacactatct (SEQ ID NO: 830) | 18.80% |
| sgRNA-HBG-05-ZBTB7A-binding-site-PAM-TAT | HBG | TAT | cctcttgggggccccttccccac (SEQ ID NO: 831) | 1% |
| sgRNA-HBG-06-ZBTB7A-binding-site-PAM-GTA | HBG | GTA | tcctcttgggggccccttcccca (SEQ ID NO: 832) | 1.20% |
| sgRNA-HBG-07-ZBTB7A-binding-site-PAM-TTA | HBG | TTA | gcagtatcctcttgggggcccct (SEQ ID NO: 833) | 1.60% |
| sgRNA-HBG-08-(-175)-PAM-TTC | HBG | TTC | cccacactatctcaatgcaaata (SEQ ID NO: 834) | 43.10% |
| sgRNA-HBG-09-(-175)-PAM-TTT | HBG | TTT | cagacagatatttgcattgagat (SEQ ID NO: 835) | 39.40% |
| sgRNA-HBG-10-(-175)-PAM-TTT | HBG | TTT | agccagggaccgtttcagacaga (SEQ ID NO: 836) | 34.20% |
| sgRNA-HBG-11-(-88)-PAM-TTG | HBG | TTG | acaaggcaaacttgaccaatagt (SEQ ID NO: 837) | 33.20% |
| sgRNA-HBG-12-(-73)-PAM-TTG | HBG | TTG | accaatagtcttagagtatccag (SEQ ID NO: 775) | 14.80% |
| sgRNA-HBG-13-(-56)-PAM-TTC | HBG | TTC | atccctagccagccgccggcccc (SEQ ID NO: 838) | 14.70% |
| sgRNA-BACH2-01-PAM-TTG | BACH2 | TTG | tagggtaccctcgtgcacccggg (SEQ ID NO: 839) | 9.60% |
| sgRNA-BACH2-02-PAM-TTC | BACH2 | TTC | cgcggcagccactcgctggtatc (SEQ ID NO: 840) | 29.70 % |
| sgRNA-BACH2-03-PAM-TTG | BACH2 | TTG | gtccttgaggacagacaccactg (SEQ ID NO: 841) | 7.10% |
| sgRNA-BACH2-04-PAM-TTC | BACH2 | TTC | atccttgttttccagtggtgtct (SEQ ID NO: 842) | 17% |
| sgRNA-BACH2-05-PAM-TTG | BACH2 | TTG | gaagagttgaccatcagttatgg (SEQ ID NO: 843) | 28.70% |
| KLKB1-sgRNA01 | KLKB1 | TTC | CTCTACTCCTCAAGAAACAC CA (SEQ ID NO: 844) | 19% |
| KLKB1-sgRNA02 | KLKB1 | TTG | ACACAGAACCCTGCCATTC TAAA (SEQ ID NO: 845) | 27.20% |
| KLKB1-sgRNA03 | KLKB1 | TTC | AGTCTGCACAACAAAAACA AGCA (SEQ ID NO: 846) | 24.30% |
| KLKB1-sgRNA04 | KLKB1 | TTG | GGGTGATAGGTGCAGATGG TCCG (SEQ ID NO: 847) | 21.20% |
| KLKB1-sgRNA05 | KLKB1 | TTA | CCCAACAGTTGGTATAAATT GTG (SEQ ID NO: 848) | Not detected |
| KLKB1-sgRNA06 | KLKB1 | TTT | CAGTTCTGCACAACAAAAA CAAGC (SEQ ID NO: 849) | 25.50% |
| KLKB1-sgRNA07 | KLKB1 | TTC | AGGTGGTCCCTTAGTTTGC AAAC (SEQ ID NO: 850) | 14.60% |
| KLKB1-sgRNA08 | KLKB1 | TTT | GGGGTGTACATGGAAGCTA CATC (SEQ ID NO: 851) | 23.40% |
| KLKB1-sgRNA09 | KLKB1 | TTG | CCTATTAAAGTACAGTCC CGGAG (SEQ ID NO: 852) | 1% |
| KLKB1-sgRNA10 | KLKB1 | TTA | TAGCGGTAGGGTTCCTCCG GGA (SEQ ID NO: 853) | 9.40% |
| PCSK9-sgRNA01 | PCSK9 | TTC | CGTGCTCGGGTGCTTCGGC CAGG (SEQ ID NO: 854) | 10% |
| PCSK9-sgRNA02 | PCSK9 | TTA | CCCCTCCACGGTACCGGGC GGAT (SEQ ID NO: 855) | 20.40% |
| PCSK9-sgRNA03 | PCSK9 | TTC | ATCCGCCCGGTACCGTGGA GGGG (SEQ ID NO: 856) | 17.60% |
| PCSK9-sgRNA04 | PCSK9 | TTG | GCAGTTGAGCACGCGCAGG CTGC (SEQ ID NO: 857) | 21% |
| PCSK9-sgRNA05 | PCSK9 | TTG | GCCACGCCGGCATCCCGCC CGCT (SEQ ID NO: 858) | 4.90% |
| PCSK9-sgRNA06 | PCSK9 | TTG | AGGACGCGGCTGTACCCAC CCGC (SEQ ID NO: 859) | 8.20% |
| PCSK9-sgRNA07 | PCSK9 | TTT | GGCCGCTGTGTGGACCTCT TTGC (SEQ ID NO: 860) | 7.80% |
| PCSK9-sgRNA08 | PCSK9 | TTG | TCTACGGCGTAGGCCCCCA GGAC (SEQ ID NO: 861) | 29.00% |
| SOD1-sgRNA01 | SOD1 | TTG | CCCAAGTCTCCAACATGCC TAAT (SEQ ID NO: 862) | 35.60% |
| SOD1-sgRNA02 | SOD1 | TTC | CCCACAACCTTCACTGGTC CATTA (SEQ ID NO: 863) | 32.10% |
| SOD1-sgRNA03 | SOD1 | TTA | ATGCTTCCCCACACCTTCAC TGG (SEQ ID NO: 864) | 34.30% |
| SOD1-sgRNA04 | SOD1 | TTA | AGCATCTTGTTACCTCTCTT CAT (SEQ ID NO: 865) | Not detected |
| SOD1-sgRNA05 | SOD1 | TTG | GGCAATGTGACTGCTGACA AAGA (SEQ ID NO: 866) | 38.20% |
| SOD1-sgRNA06 | SOD1 | TTC | AGCACGCACACGGCCTTCG TCGC (SEQ ID NO: 867) | 21.50% |
| SOD1-sgRNA07 | SOD1 | TTA | AAGGACTGACTGAAGGCCT GCAT (SEQ ID NO: 868) | 33.20% |
| BACE1-sgRNA01 | BACE1 | TTG | AGGGGGAAGCCAGCACCAC AAAG (SEQ ID NO: 869) | 28.90% |
| BACE1-sgRNA02 | BACE1 | TTC | CCTCTTAGTACAACTATGAC AAG (SEQ ID NO: 870) | Not detected |
| BACE1-sgRNA03 | BACE1 | TTC | CCAGTCATCTCACTCTACCT AAT (SEQ ID NO: 871) | 24.50% |
| BACE1-sgRNA04 | BACE1 | TTG | CCCTCTCCCCAGTGCACGA TGAG (SEQ ID NO: 872) | 18.90% |
| BACE1-sgRNA05 | BACE1 | TTC | AGCAGGGAGATGTCATCAG CAAA (SEQ ID NO: 873) | 27.90% |
| BACE1-sgRNA06 | BACE1 | TTA | GGTAGAGTGAGATGACTGG GAAA (SEQ ID NO: 874) | 10.80% |
| BACE1-sgRNA07 | BACE1 | TTC | CCCCTCAACCAGTCTGAAG TGCT (SEQ ID NO: 875) | 35.30% |
| BACE1-sgRNA08 | BACE1 | TTC | AGATCCTGTCCATTGATCTC CAC (SEQ ID NO: 876) | 5.70% |
| BACE1-sgRNA09 | BACE1 | TTG | GCACGCACAGTGACGTTGG GGCC (SEQ ID NO: 877) | 4.30% |

### Example 17. PAM recognition of C12-279 mutant Mut-02-1-426-846-858-860

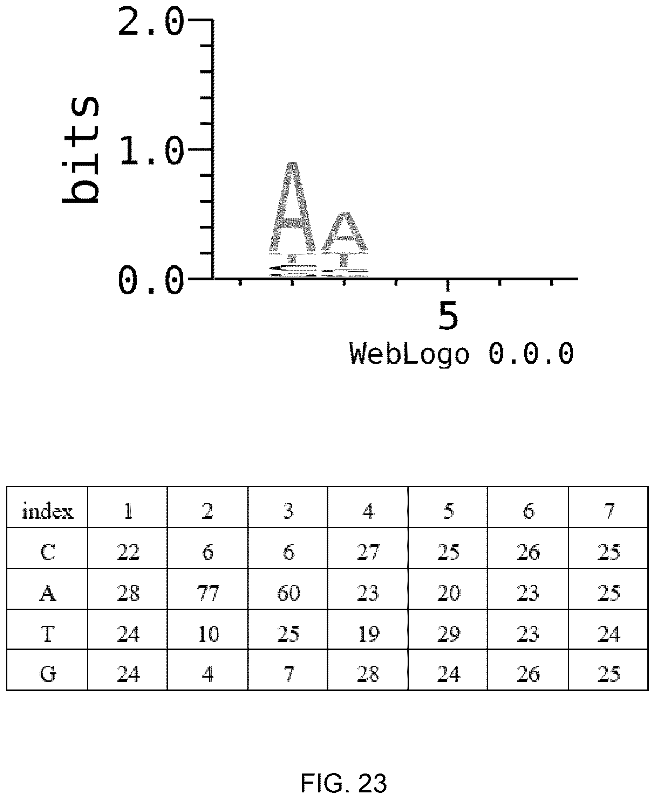

Using the same manner as in the previous examples, the PAM recognized by the mutant Mut-02-1-426-846-858-860 was 5'-WTN-3' (W is A or T) by performing bacterial editing experiments in vivo and NGS sequencing, as shown in FIG. 23.

Although the specific examples of the present disclosure are described above, those skilled in the art should understand that these are only examples, and that various changes or modifications may be made to these examples without departing from the principles and essence of the present disclosure. Therefore, the scope of protection of the present disclosure is limited by the attached claims.

## Claims

1. A Cas12 protein, wherein
(a) the Cas12 protein is a CLUSTER1 protein, a CLUSTER2 protein, a CLUSTER3 protein, a CLUSTER4 protein, a CLUSTERS protein, a CLUSTER6 protein, a CLUSTER7 protein, a CLUSTER8 protein, a CLUSTER9 protein, a CLUSTER10 protein, a CLUSTER11 protein, a CLUSTER12 protein, or a CLUSTER13 protein;
or,
(b) the Cas12 protein comprises an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity with any one of the amino acid sequences shown in SEQ ID NO: 1-53, SEQ ID NO: 696, and SEQ ID NO: 728;
preferably, wherein the Cas12 protein retains a function of a protein with an amino acid sequence as shown in any one of SEQ ID NO: 1-53, SEQ ID NO: 696, and SEQ ID NO: 728;
preferably, wherein a protospacer adjacent motif (PAM) sequence (5'→3') recognized by the Cas12 protein is selected from any one or more of the following:
A, C, T, G,
TA, TC, GN, AA, AG, TG, AN, GG, CG, TN, NT, NG, GT, NA, CC, AC, GC, AT, CT, GA, TT, CN, NC, CA,
NTN, ANN, TTN, ATC, NAC, AGA, TGC, TCT, NGN, CGC, NTC, GCA, TCG, TTT, CCG, GGG, NAG, ACA, CGG, CNG, ACN, GTG, CNT, TTG, TCN, GGT, TNC, CCN, CGT, TGG, CGA, NGG, TCC, AGT, NCA, CAN, TCA, NNG, TAC, CCT, NTG, CGN, TGN, CAT, NGC, GNG, GNC, NNA, GAA, TTC, CTT, ATA, TAT, GCT, NCC, TTA, AGN, GNN, CAA, CAC, AGG, NTT, ANG, GNA, GTT, NGA, TAA, GTA, GGN, GNT, NCG, ATT, CCA, CNN, AAA, AAC, ATN, GAG, CTG, ACG, NAA, TAN, NAT, CNA, GCN, GTC, NCN, CTN, CNC, ANT, NNC, CAG, NAN, ATG, NCT, CCC, AAN, TGT, TNA, ACC, GAT, ACT, AAT, GGA, GAN, ANC, GAC, NNT, CTA, TNN, GCG, GTN, TNT, AAG, TAG, NGT, NTA, ANA, CTC, GCC, TGA, GGC, AGC, TNG,
NGAA, GANC, GCNC, NTNT, TGGG, AAGG, AAGN, NTNN, TCGT, CNTG, NTGG, CCGN, ATAT, TGCA, NGGT, TGNT, NNTG, NCCG, ACAT, GNTG, CGCG, GACN, NTCG, TCNG, CTGC, TNNC, GGTN, CGNN, TCCA, AGCN, TNAG, GGAC, GATC, AANA, NATG, CCAG, NAAT, TCNT, CACT, CGGC, CGAN, CNCA, ATNT, NNNG, NGCT, CTGG, GGAN, NTNC, ATTC, AATG, CNTC, TGGN, NATC, GTCG, ACNC, GCNN, GACT, CTNT, NCTT, NAGG, NANC, CTTA, GTCT, ANAG, NGCN, CNNA, TCAG, ACAC, NCGG, TNNT, CAAG, ACCT, CCCA, GTNC, ANTC, GACC, AACG, TTAA, TCCG, CGCC, NCCN, TTNA, NCNT, NGCA, AGNN, AATC, GGGA, GNAN, NAGA, CGNA, GTAT, GTNA, ATNC, ACNA, GGAA, NTCC, GGCG, AATN, CNNT, AGGC, GCGN, GTGC, TTGA, AAGC, GAAG, ATNG, TGCT, TACT, CTAN, GGCT, GNGC, GTCN, CGAA, CNAC, GCCT, TAGG, ANGC, TNAA, GANT, NCNA, NCCT, AGAN, GTAA, TTTN, ATGA, TGNA, CANC, ACGA, CCAC, CCGG, CTNG, CNGN, GGTA, NGNC, GTTT, CTAA, TNCT, CTGN, NGAC, TGTA, TANN, GCNT, GCTC, CNCG, AAAN, CCNT, GANA, CACA, CTNA, ANTN, TTNT, CCTG, TNTT, CANA, NTAN, CACG, GGAT, TTTC, GNCG, TACA, GTAC, GAGC, ACNN, ATGG, AANT, ATCC, ACCG, AGNC, TGTT, NCAT, ATTA, GNTT, GAGN, TNAC, GCCG, NTNG, GTGG, GNGN, ACCA, NTAA, ACTN, NCTG, NCTA, TTTT, GCNG, NTAG, CAAA, GGNA, CNTN, TTAG, TCTG, NCTN, TATG, GCGT, TANT, GGGT, NACN, ACTG, CCNG, GNNT, CCAT, GNTA, NANT, TACN, TGTN, ATCT, NCAN, TNGG, CNNN, AAGT, ATTN, GGNN, CAGC, CGTN, GCCC, GCTT, CNAT, NANA, CCNN, GNGA, TNGN, GCAG, CGNG, CCTT, NGAG, NCNG, AANG, GGTC, ACTC, TGAA, NAGN, NNCA, ACGG, TGAC, TCCN, ANNN, TCGN, TAAN, CAGG, TTAN, NGAN, NTGC, CCNC, TNTN, ATGN, GTGN, GCAT, NNGN, NNCC, CCNA, CNAG, GNAC, CGNT, TTCN, TAGN, ANCT, NATN, GTGA, TNGT, CTAT, CCCG, TNCA, NGTA, NNGA, CGTG, TAAT, CGCA, NNCG, NGTC, NAGT, GNAT, TNTC, NCGC, NGGN, CATN, GTTN, AGTA, GNNG, TTNN, TGNC, NAAA, TNCC, CACC, CTCT, TTGN, GCTA, NTTT, TGAN, TNAN, NGAT, CCTN, GAAT, GTCA, NTCN, GCCA, ANTG, TGGC, CAAC, TTTA, TGTC, CGGA, NCGN, AGNT, NCGA, ANCG, ACAA, TAGT, CGAG, NCAA, AATA, AGGG, GNGT, CAGA, AGGT, GGGG, ANAC, TGGT, GTGT, GNCA, GTTA, NGTT, TNNG, NCAG, CACN, GCAN, GAAC, NCCA, TTCC, NCNN, GNNN, ANGT, NTNA, CCCT, GNAA, TTNG, GTNN, GGNG, TCTA, NCAC, GANG, TTCG, CCTC, CNGG, ANNA, TCAN, ATCG, NTGA, CGTA, TTAC, GCTN, GCTG, NGTG, TCCC, CANN, NNNA, TAGA, ACGT, AGAT, GATG, GCCN, TGNG, GCGC, CCGA, GNCN, NTTG, NNAT, TNCG, NANG, GGTG, NCCC, GNCC, CAAT, CGCN, CNGA, NTTC, TTCT, NGGA, AGTC, CNNC, NACG, AGTN, NANN, ACAG, GNCT, TACC, CNTA, TGTG, CATC, GACA, TCTT, NTCT, CTGA, AGGA, GATA, TNAT, CCTA, GGAG, ANCC, AANC, GTAN, GCNA, TGNN, TANC, GNTN, AGCG, CTAG, NNAA, AGTT, CTAC, TACG, TTNC, TNTA, ANTT, ATAC, TCCT, TCAC, NGGC, NTTN, NNTC, CANT, ATAA, TGCC, CTCC, TNNA, GTNG, ACGN, GGCA, AAAG, TTGT, NGNA, NAAN, TATN, CGGG, CATA, ATGC, ACGC, ACCN, ATTT, TCNA, TNGC, NACA, NACC, CTCN, GGCC, TANG, AGAA, TNGA, TAGC, CAGN, GGCN, ANNT, NNNC, TCAT, CATT, TAAA, ATGT, TGAG, CGCT, TCGG, GCAC, GTAG, NTCA, NATT, ANTA, CCCN, ACTA, AAAA, GAAN, TATT, NNAC, TGAT, GGGN, CCAA, GNGG, CCAN, GTCC, NNCT, AGNG, CNTT, CNCT, GANN, GGTT, AGCT, CATG, NTAC, TNCN, NNTN, TGGA, GATT, AGCA, TAAG, GCGA, ACTT, ANGN, NTGN, AACN, AACT, TCAA, NTAT, TCGA, NCTC, NNGG, ANGG, NNTT, GTNT, CTNN, CGGN, TAAC, GGNC, GAAA, ACNG, GNAG, TTGG, CTTC, CNGT, TNNN, TNTG, GTTG, TCNN, CGGT, GAGA, CNNG, NCNC, GAGG, AGCC, ATNN, NNNT, AGAC, AACC, ANNC, ANNG, ACAN, GTTC, TATA, GNTC, NCGT, NGNT, CGTC, CCGC, CGAC, GACG, ATTG, GNNC, CNAA, TATC, AGNA, CTNC, TTCA, ANCA, ACCC, AGTG, CCGT, ANAT, CTGT, GGGC, NTTA, NAAG, AANN, CNAN, NNCN, ANAA, ANAN, CTTG, NGNN, AGAG, TANA, TCNC, GCAA, NGNG, NAGC, NATA, ATCN, CGTT, CNGC, GATN, NNTA, AAGA, CTTT, AAAC, AGGN, ACNT, NTGT, CTTN, ATCA, NACT, NNAG, NGTN, NAAC, TGCG, GGNT, ATAN, TTGC, ANCN, CCCC, ANGA, NGCG, TCTC, CTCG, ATNA, AATT, NNAN, NNGT, TCGC, ATAG, CAAN, AACA, TTAT, CAGT, GNNA, TGCN, GCGG, NGGG, CANG, TTTG, GAGT, AAAT, CTCA, CNCN, CNCC, TCTN, CGNC, NGCC, CGAT, and NNGC;
preferably, the Cas12 protein has at least one mutation in at least 1, at least 2, at least 3, at least 4, at least 5, at least 6, at least 7, at least 8, at least 9, at least 10, at least 11, or at least 12 of the amino acid residues corresponding to positions 1-10, 12-16, 19, 21-30, 32-44, 46-51, 53-62, 64-86, 88-99, 101-106, 108-112, 114-149, 151-167, 169-180, 182-192, 194-200, 202-225, 227-240, 242-245, 247-253, 255-263, 265-276, 278-279, 281-303, 305- 306, 308-310, 313, 315-325, 327-337, 339-348, 350-429, 431-433, 435-437, 439-444, 446-464, 467, 469-494, 496-497, 499-504, 506-529, 531-550, 552- 553, 555-587, 589-590, 592-599, 601-616, 618-628, 630-676, 678-681, 683-686, 688-689, 691-713, 715-717, 719-725, 727-734, 736-749, 751-762, 764- 769, 771-776 769, 771-776, 779-787, 789-792, 794-795, 797-798, 800-802, 804-815, 817-819, 821-842, 844-860, 862-868, 870, 872-877, 879-906, 909-914, 916-928, 930-961, 963-977, 979-983, 985-1018, 1021, 1023-1033, 1035-1050, 1052-1053, 1055-1105, 1107-1113, 1115-1117, 1120, 1122-1125, and 1128-1135 of the amino acid sequence shown in SEQ ID NO: 696; preferably, wherein the mutation is a mutation to any other natural amino acid residue; preferably, the mutation is to mutate into residues R, H, K, or A;
preferably, the Cas12 protein has any 1, any 2, any 3, any 4, any 5, any 6, any 7, any 8, any 9, any 10, any 11, any 12, any 13, any 14, any 15, any 16, or more amino acid mutations of the amino acid sequence shown in SEQ ID NO: 696 at positions corresponding to the amino acid sequence shown in SEQ ID NO: 696, and the amino acid mutations are selected from: I33R, G184R, S185R, Q186R, G194R, N195R, G196R, G197R, N245R, G256R, L260R, G197R, N245R, G256R, L260R, Y278R, S285R, Y316R, H350R, D352R, A355R, A356R, C385R, P386R, H387R, G390R, K391R, N392R, D429R, Q461R, Q462R, Q469R, E485R, S491R, K521R, P525R, L611R, K629R, K631R, N633R, D841R, N898R, K987R, A988R, G989R, Q990R, T991R, D1010R, E1013R, A1136R, K1138R, and T1139R;
preferably, the Cas12 protein has any 1, any 2, any 3, any 4, any 5, or more amino acid mutations of the amino acid sequence shown in SEQ ID NO: 696 at positions corresponding to the amino acid sequence shown in SEQ ID NO: 696, and the amino acid mutations are selected from: D352R+Q186R, D352R+L260R, D352R+A355R, A355R+L260R, P386R+C385R, E485R+Q462R, D352R+Q186R, A355R+L260R, G184R+R186Q, D352R+ Q186R+I33R, D352R+Q186R+G184R, D352R+Q186R+S185R, D352R+Q186R+G256R, D352R+Q186R+Y278R, D352R+Q186R+S285R, D352R+Q186R+Y316R, D352R+Q186R +H350R, D352R+Q186R+A356R, D352R+Q186R+Q469R, D352R+Q186R+S491R, D352R+Q186R+K521R, D352R+Q186R+P525R, D352R+Q186R+K629R, D352R+Q186R+ N633R, D352R+Q186R+D841R, D352R+Q186R+N898R, D352R+Q186R+K987R, D352R+Q186R+T991R, D352R+Q186R+D1010R, and D352R+Q186R+E1013R;
preferably, wherein the Cas12 protein forms a complex with a guide polynucleotide; the complex specifically binds to a target nucleic acid; the complex cleaves the target nucleic acid, modifies the target nucleic acid, or modulates a protein expressed by the target nucleic acid;
preferably, wherein the Cas12 protein forms a complex with a guide polynucleotide, the guide polynucleotide includes a guide sequence that is reverse complementary to the target nucleic acid; the guide polynucleotide includes a scaffold sequence interacting with the Cas12 protein; the scaffold sequence includes a direct repeat (DR) sequence;
preferably, the scaffold sequence does not include a tracrRNA sequence;
preferably, wherein the recognizable PAM sequence of the Cas12 protein is at least one of 5'-TTN-3' or 5'-TTNC-3'.
preferably, wherein the Cas12 protein is a nuclease-inactive variant; preferably, the Cas12 protein is a dead Cas12 inactive variant or a nickase Cas12 inactive variant; preferably, the Cas12 protein has an inactive Ruvc domain.

2. A guide polynucleotide, comprising:
(i) a direct repeat (DR) sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity with a sequence shown in any one of SEQ ID NO: 54-583, and
(ii) a guide sequence engineered to hybridize to a target nucleic acid; wherein the DR sequence is linked to the guide sequence, and the guide polynucleotide forms a complex with the Cas12 protein, and guides a sequence-specific binding of the complex to the target nucleic acid through the guide sequence;
preferably, the Cas12 protein is the Cas12 protein of claim 1;
preferably, the guide sequence includes 15-60 nucleotides;
preferably, the guide sequence hybridizes to the target nucleic acid, and the guide sequence is mismatched to the target nucleic acid by no more than one nucleotide;
preferably, a nucleotide sequence of the guide sequence is shown in any one of SEQ ID NO: 54-583 and SEQ ID NO: 704;
preferably, the guide polynucleotide further includes a tracrRNA; preferably, a sequence of the tracrRNA is linked to the DR sequence; preferably, the tracrRNA includes 10-200 nucleotides
preferably, the guide sequence is located at a 3' end of the DR sequence;
preferably, the guide sequence is located at a 5' end of the DR sequence;
preferably, the sequence of the tracrRNA is located at the 5' or 3' end of the DR sequence; preferably, the sequence of the tracrRNA has at least 50% sequence identity with any one of the sequences shown in SEQ ID NO: 584-695.

3. A Cas12 inactive variant, wherein the Cas12 inactive variant is a nuclease-inactive variant of the Cas12 protein of claim 1; wherein the nuclease-inactivation refers to an inability to generate a double-stranded break in a target nucleic acid;
preferably, the Cas12 inactive variant is a dead Cas12 inactive variant or a nickase Cas12 inactive variant;
preferably, the Cas12 inactive variant is a variant obtained by inactivating a Ruvc domain of the Cas12 protein.

4. A fusion protein or conjugate, comprising:
(1) the Cas12 protein of claim 1, or the Cas12 inactive variant of claim 3; and
(2) a homologous or heterologous functional domain;
preferably, the functional domain has an enzyme activity that modifies a target nucleic acid sequence; and the functional domain includes at least one of a nuclease activity, a methyltransferase activity, a demethylase activity, a DNA repair activity, a DNA damage activity, a deamination activity, a dismutase activity, an alkylation activity, a depurination activity, an oxidation activity, a pyrimidine dimer formation activity, an integrase activity, a transposase activity, a recombinase activity, a polymerase activity, a ligase activity, a helicase activity, a photolyase activity, a glycosylase activity, a deglycosylation activity, an acetyltransferase activity, a deacetylase activity, a kinase activity, a phosphatase activity, a ubiquitin ligase activity, a deubiquitination activity, an adenylylation activity, a deadenylation activity, a SUMOylating activity, a deSUMOylating activity, a myristoylation activity, or a demyristoylation activity;
preferably, the functional domain is selected from one or more of: a subcellular positioning signal, a DNA binding domain, a protease domain, a transcriptional activation domain, a transcriptional repression domain, a nuclease domain, a deaminase domain, a uracil DNA glycosylase domain (UDG), a uracil DNA glycosylase inhibitor domain (UGIs), a methylase, a demethylase, a transcriptional release factor, a histone acetylase domain, a histone deacetylase domain, a DNA ligase, an affinity tag, a reporter tag, an affinity domain, and a reporter domain;
preferably, the nuclease domain includes at least one of a polypeptide having ssDNA cleavage activity or a polypeptide having dsDNA cleavage activity;
preferably, the Cas12 protein or the Cas12 inactive variant is directly or indirectly linked to the homologous or heterologous functional domain; preferably, the direct linkage is a covalent linkage, and the indirect linkage is a linkage through an amino acid linker or a non-amino acid linker; and
preferably, the homologous or heterologous functional domain is fused or conjugated at an N-terminal, a C-terminal, or internally with respect to the Cas12 protein or the Cas12 inactivation variant.

5. An isolated nucleic acid, wherein the isolated nucleic acid encodes the Cas12 protein of claim 1, the Cas12 inactive variant of claim 3, or the fusion protein or conjugate of claim 4;
preferably, the isolated nucleic acid is codon optimized for expression in a cell;
preferably, the isolated nucleic acid is codon optimized for expression in a prokaryotic cell;
preferably, the isolated nucleic acid is codon optimized for expression in a eukaryotic cell; or
preferably, the isolated nucleic acid is codon-optimized for expression in eukaryote, a mammal, a plant, an insect, a bird, a reptile, a rodent, a fish, a worm/nematode, or a yeast.

6. A CRISPR-Cas12 system, comprising:
a. the Cas12 protein of claim 1, the Cas12 inactive variant of claim 3, the fusion protein or conjugate of claim 4, or the nucleic acid of claim 5; and
b. the guide polynucleotide of claim 2, or a polynucleotide sequence encoding the guide polynucleotide;
wherein a Cas12 functional domain or the fusion protein or conjugate forms a complex with the guide polynucleotide; the guide polynucleotide including a guide sequence engineered to guide a sequence-specific binding of the complex to the target nucleic acid;
preferably, the guide polynucleotide further includes a direct repeat (DR) sequence linked to the guide sequence, and the DR sequence has at least 50% sequence identity with a sequence shown in any one of SEQ ID NO: 54-583 and SEQ ID NO: 704;
preferably, the guide sequence includes 15-35 nucleotides, the guide sequence hybridizes to the target nucleic acid, and the guide sequence is 90-100% complementary to the target nucleic acid, and the guide sequence is mismatched to the target nucleic acid by no more than one nucleotide;
preferably, the guide sequence includes 15-60 nucleotides;
preferably, the guide sequence hybridizes to the target nucleic acid;
preferably, the guide sequence is mismatched to the target nucleic acid by no more than one nucleotide;
preferably, the guide polynucleotide further includes the tracrRNA; preferably, the tracrRNA sequence is linked to the DR sequence; and preferably, the tracrRNA includes 10-200 nucleotides;
preferably, the guide sequence is located at the 3' end of the DR sequence;
preferably, the guide sequence is located at the 5' end of the DR sequence;
preferably, the tracrRNA sequence is located at the 5' or 3' end of the DR sequence;
preferably, the target nucleic acid is DNA, RNA, dsDNA, or ssDNA;
preferably, the DNA is eukaryotic DNA; wherein the eukaryotic DNA is non-human mammalian DNA, non-human primate DNA, human DNA, plant DNA, insect DNA, bird DNA, reptile DNA, rodent DNA, fish DNA, worm/nematode DNA, or yeast DNA; and
preferably, the target nucleic acid is a gene associated with disease or disorder or a gene associated with a signal transduction biochemical pathway, or the target nucleic acid is a reporter gene; preferably, the target nucleic acids are the genes listed in Table 27.

7. A vector system, comprising one or more recombinant vectors, wherein the one or more recombinant vectors include the isolated nucleic acid of claim 5, or the CRISPR-Cas12 system of claim 11;
preferably, the one or more recombinant vectors further include a regulatory sequence;
preferably, a polynucleotide sequence encoding the Cas12 protein, the Cas12 inactive variant, or the fusion protein or conjugate is operably linked to the regulatory sequence, or the polynucleotide sequence encoding the guide polynucleotide is operably linked to the regulatory sequence; the regulatory sequence is preferably selected from one or more of: a promoter, an enhancer, an internal ribosome entry site, and a transcriptional termination signal, wherein the promoter includes a constitutive promoter, an inducible promoter, a broad-spectrum promoter, or a tissue-specific promoter, or the transcriptional termination signal includes a polyadenylation signal or a poly-U sequence;
preferably, a scaffold of the one or more recombinant vectors is an adeno-associated virus vector, a lentiviral vector, or a virus-like particle; wherein when the scaffold is the adeno-associated virus vector, the adeno-associated virus vector is a recombinant adeno-associated virus vector of serotype AAV1, AAV2, AAV4, AAVS, AAV6, AAV7, AAVrh74, AAV8, AAV9, AAV10, AAV11, AAV12, or AAV13, and when the scaffold is the lentiviral vector, the lentiviral vector is pseudotyped with an envelope protein;
preferably, the isolated nucleic acid is linked to an aptamer sequence; and
when the scaffold is the virus-like particle, the isolated nucleic acid is linked to a gene encoding a gag protein.

8. A delivery system, comprising:
(1) a delivery tool, and
(2) the Cas12 protein of claim 1, the guide polynucleotide of claim 2, the Cas12 inactive variant of claim 3, the fusion protein or conjugate of claim 4, the isolated nucleic acid of claim 5, the CRISPR-Cas12 system of claim 6, or the vector system of claim 7; wherein
preferably, the delivery tool is a virus, a lipid nanoparticle, a nanoparticle, a liposome, an exosome, a microbubble, or a gene gun; or
preferably, the delivery tool is the lipid nanoparticle including the guide polynucleotide and mRNA encoding the Cas12 protein, the Cas12 inactive variant, or the fusion protein or conjugate.

9. A cell, comprising the Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7 or the delivery system of claim 8; wherein
preferably, the cell is a prokaryotic cell or a eukaryotic cell; wherein the eukaryotic cell is a mammalian cell.

10. A pharmaceutical composition, comprising the Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, or the cell of claim 9; wherein
preferably, the pharmaceutical composition includes pharmaceutically acceptable excipients.

11. A kit, comprising the Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, the cell of claim 9, or the pharmaceutical composition of claim 10.

12. The Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, the cell of claim 9, the pharmaceutical composition of claim 10, or the kit of claim 11 for use as a reagent or medicament for diagnosing, treating, or preventing a disease or disorder associated with a target nucleic acid;
preferably, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease; the reagent or medicament is used to: cleave one or more target nucleic acid molecules or introduce nicks into the one or more target nucleic acid molecules, activate or upregulate an expression of the one or more target nucleic acid molecules, activate or inhibit transcription of the one or more target nucleic acid molecules, inactivate the one or more target nucleic acid molecules, visualize, label, or detect the one or more target nucleic acid molecules, bind the one or more target nucleic acid molecules, transport the one or more target nucleic acid molecules, and mask the one or more target nucleic acid molecules;
preferably, the disease or disorder is the disease or disorder as listed in Table 27;
preferably, the disease or disorder is selected from: hemophilia A, Best yolk-like macular dystrophy, B-cell acute lymphoblastic leukemia, hemophilia B, CDKL5deficiency, CLN2 disease, Niemann-Pick disease type C, Dravet syndrome, FOXG1syndrome, GM1 ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5deficiency, X-linked myotubular myopathy, X-linked retinoschisis, X-linked retinitis pigmentosa, α1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Bietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1,spinal muscular atrophy, Tay-Sachs disease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase deficiency, Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune deficiency, heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin (ATTR) amyloidosis, alpha-1-antitrypsin deficiency (AATD) liver disease, and AATD lung disease.

13. A method for detecting, binding, or cleaving a target nucleic acid, comprising using the Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, the cell of claim 9, the pharmaceutical composition of claim 10, or the kit of claim 11 to contact the target nucleic acid;
preferably, the method is for non-diagnostic and/or non-therapeutic purposes; and/or the fusion protein or conjugate includes a detectable marker that is detected by fluorescence, DNA blotting, or FISH.

14. A method for altering a cell state, comprising using the Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, the cell of claim 9, the pharmaceutical composition of claim 10, or the kit of claim 11 to contact a cell to alter the cell state; wherein
the method results in one or more of: an increase or decrease in an expression of a specific gene, an induction of cellular senescence in vitro or in vivo, an induction of cellular cycle arrest in vitro or in vivo, a cellular growth promotion or a cellular growth inhibition in vitro or in vivo, an induction of anergy in vitro or in vivo, an induction of apoptosis in vitro or in vivo, and an induction of necrosis in vitro or in vivo;
preferably, the method is for non-diagnostic and/or non-therapeutic purposes.

15. A method for diagnosing, treating, or preventing a disease or disorder associated with a target nucleic acid, comprising applying the Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, the cell of claim 9, the pharmaceutical composition of claim 10, or the kit of claim 11 to a sample from a subject in need or the subject in need;
preferably, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.
preferably, the disease or disorder is the disease or disorder as listed in Table 27.
preferably, the disease or disorder is selected from: hemophilia A, Best yolk-like macular dystrophy, B-cell acute lymphoblastic leukemia, hemophilia B, CDKL5 deficiency, CLN2 disease, Niemann-Pick disease type C, Dravet syndrome, FOXG1 syndrome, GM1 ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5 deficiency, X-linked myotubular myopathy, X-linked retinoschisis, X-linked retinitis pigmentosa, a1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Bietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1, spinal muscular atrophy, Tay-Sachs disease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase deficiency, Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune deficiency, heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin amyloidosis (ATTR), alpha-1-antitrypsin deficiency (AATD) liver disease, and AATD lung disease.

16. The Cas12 protein of claim 1, the guide polynucleotide of claim 2, the Cas12 inactive variant of claim 3, the fusion protein or conjugate of claim 4, the nucleic acid of claim 5, the CRISPR-Cas12 system of claim 6, or the vector system of claim 7, the delivery system of claim 8, the cell of claim 9, the pharmaceutical composition of claim 10, or the kit of claim 11 for use in diagnosing, treating, or preventing a disease or disorder associated with the target nucleic acid;
preferably, the disease or disorder is a hematologic disease or disorder, an ophthalmic disease or disorder, a neurological disease or disorder, a respiratory disease or disorder, a hepatic disease or disorder, a metabolic disease or disorder, a cancer, or an infectious disease.
preferably, the disease or disorder is the disease or disorder as listed in Table 27.
preferably, the disease or disorder is selected from: hemophilia A, Best yolk-like macular dystrophy, B-cell acute lymphoblastic leukemia, hemophilia B, CDKL5 deficiency, CLN2 disease, Niemann-Pick disease type C, Dravet syndrome, FOXG1 syndrome, GM1 ganglioside storage disease, GM2 ganglioside deposition disease, HIV infection, HSV infection, Usher syndrome type IB, Usher syndrome type IIA, Mucopolysaccharidosis type IIIA, Mucopolysaccharidosis type IIIB, Gaucher disease type III, Mucopolysaccharidosis type II, type II diabetes, Mucopolysaccharidosis type IV, Gaucher disease type I, Mucopolysaccharidosis type I, type I diabetes, Usher syndrome type I, KCNQ2 epileptic encephalopathy, Leber hereditary optic neuropathy, Leigh syndrome, Prader-Willi syndrome, SLC13A5 deficiency, X-linked myotubular myopathy, X-linked retinoschisis, X-linked retinitis pigmentosa, a1-antitrypsin deficiency, α-mannoside storage disease, α-thalassemia, β-thalassemia, Alzheimer's disease, Bardet-Biedl syndrome, white dot retinal degeneration, leukocyte adhesion deficiency type I, galactosemia, bladder cancer, overactive bladder, phenylketonuria, nasopharyngeal carcinoma, Bietti's crystalline dystrophy, pyruvate kinase deficiency, erectile dysfunction, autosomal recessive congenital ichthyosis, adult glucan body disease, traumatic arthritis, homozygous familial hypercholesterolemia, Fragile X syndrome, thalassemia, hypophosphatasia, epilepsy, multiple myeloma, multiple system atrophy, frontotemporal dementia, catecholamine-sensitive polymorphic ventricular tachycardia, Fabry's disease, Fanconi's anemia, aromatic L-amino acid decarboxylase deficiency, radiation-induced xerostomia, non-Hodgkin's lymphoma, non-muscle invasive bladder carcinoma, non-alcoholic fatty liver disease, non-small cell lung cancer, hypertrophic cardiomyopathy, hypertrophic scar, obesity, peroneal muscular dystrophy type 1A, peroneal muscular dystrophy type 2A, pulmonary hypertension, Friedrich's ataxia, peritoneal carcinoma, liver cancer, hepatocellular carcinoma, dry age-related macular degeneration, sicca syndrome, hyperuricemia, hyperlipidemia, Gaucher disease, autism spectrum disorders, osteoarthritis, bone marrow failure syndromes, citrullinemia type I, coronary heart disease, cystinosis, melanoma, Huntington's disease, amyotrophic lateral sclerosis, urge incontinence, acute intermittent porphyria, acute lymphoblastic leukemia, spinal cerebellar ataxia, spinal muscular atrophy with respiratory distress type 1, spinal muscular atrophy, Tay-Sachs disease, methylmalonic acidemia, thyroid carcinoma, pseudohypertrophic muscular dystrophy, anaplastic astrocytoma, intermittent claudication, junctional epidermolysis bullosa, glioma, glioblastoma, corneal graft rejection, colorectal cancer, progressive multifocal leukoencephalopathy, progressive familial intrahepatic cholestasis, giant-axonal neuropathy, Canavan's disease, cocaine addiction, Klaber's disease, Kriegler-Najjar syndrome, oral cancer, Angelman syndrome, diffuse intrinsic pontine glioma, Lafora's disease, rheumatoid arthritis, sickle cell disease, lymphedema, ovarian cancer, chronic lymphocytic leukemia, chronic granulomatous disease, chronic nephrogenic anemia, chronic pain, chronic hepatitis B, Menkes' disease, cystic fibrosis, Netherseton's syndrome, ornithine transcarbamylase Parkinson's disease, Pompe's disease, uveitis, prostate cancer, vestibular schwannoma, ankylosing muscular dystrophy, ankylosing spondylitis, castration-resistant prostate cancer, glaucoma, achromatopsia, ischemic heart failure, lysosomal storage disease, sarcoma, breast cancer, Rett's syndrome, triple-negative breast cancer, Sandhoff's disease, color blindness, heart failure with reduced ejection fraction, neuronal ceroid lipofuscinosis, adrenoleukodystrophy, renal cell carcinoma, wet age-related macular degeneration, eczema, thrombocytopenia with immunodeficiency syndrome, esophageal cancer, optic neuropathy, optic nerve atrophy, retinal vein occlusion, retinitis pigmentosa, rhodopsin-mediated autosomal dominant retinitis pigmentosa, ependymoma, fallopian tube carcinoma, bilateral vestibulopathies, Stargardt's disease, diabetic macular edema, diabetic neuropathy, diabetic retinopathy, diabetic peripheral neuralgia, diabetic foot, glycogenosis, glycogenosis type Ia, glycogenosis type IIb, atopic dermatitis, hearing loss, hearing impairment, head and neck cancer, squamous cell carcinoma of the head and neck, Wilson's disease, stable angina pectoris, Usher's syndrome, choroideremia, Leber's congenital amaurosis, congenital adrenal hyperplasia, cardiomyopathy, angina pectoris, heart failure, COVID-19 infection, pleural mesothelioma, acne vulgaris, severe combined immunodeficiency diseases, severe limb ischemia, oculopharyngeal muscular dystrophy, pancreatic cancer, graft-versus-host disease, hereditary retinal dystrophy, hereditary angioedema, hepatitis B, heterotrophic cerebral leukoencephalic dystrophy, psoriatic arthritis, recessive genetic dystrophic epidermolysis bullosa, infantile malignant osteosclerosis, dystrophic epidermolysis bullosa, morphea, primary immune heterozygous familial hypercholesterolemia, limb-girdle muscular dystrophy type 2B, limb-girdle muscular dystrophy type 2C, limb-girdle muscular dystrophy type 2D, limb-girdle muscular dystrophy type 2E, limb-girdle muscular dystrophy type 2I, limb-girdle muscular dystrophy type 2L, limb ischemic disease, lipoprotein lipase deficiency, severe congenital neutrophilic dysphoria, wrinkles, stroke, sciatica, schizophrenia, depression, drug addiction, autism, idiopathic pulmonary fibrosis, hyperlipidemia, transthyretin amyloidosis (ATTR), alpha-1-antitrypsin deficiency (AATD) liver disease, and AATD lung disease.
